# EUROPEAN PATENT SPECIFICATION

(11) **EP 3 045 043 B1**
(45) Date of publication and mention of the grant of the patent: **29.04.2020**
(21) Application number: 16158311.7
(22) Date of filing: 26.02.2010
(51) Int. Cl.: A01N 43/42, A61K 31/44, A61P 23/00

(54) **EXTENDED RELEASE ORAL PHARMACEUTICAL COMPOSITIONS OF 3-HYDROXY-N-METHYLMORPHINAN AND METHOD OF USE**
ORAL EINNEHMBARE PHARMAZEUTISCHE ZUSAMMENSETZUNGEN AUS 3-HYDROXY-N-METHYLMORPHINAN MIT VERZÖGERTER FREISETZUNG UND VERWENDUNGSVERFAHREN DAFÜR
COMPOSITIONS PHARMACEUTIQUES ORALES À LIBÉRATION PROLONGÉE DE 3-HYDROXY-N-MÉTHYLMORPHINANE ET PROCÉDÉ D'UTILISATION

(30) Priority: 26.02.2009 US 202447 P; 14.10.2009 US 272650 P; 02.12.2009 US 265884 P
(43) Date of publication of application: 20.07.2016
(62) Divisional of application: 10746955.3
(73) Proprietor: Relmada Therapeutics, Inc., Blue Bell, PA 19422-3212 (US)
(72) Inventor: BABUL, Najib, Blue Bell, PA 19422-3212 (US)
(74) Representative: Bond, Christopher William

(56) References cited:
- EP-A2- 1 020 185
- WO-A1-01/58447
- WO-A1-82/03768
- WO-A2-03/013535
- WO-A2-2007/005716

## Description

### FIELD OF THE INVENTION

The present invention is directed to extended release oral pharmaceutical compositions and to delayed onset, extended release oral pharmaceutical compositions of *d,l* 3-hydroxy-N-methylmorphinan and *l* 3-hydroxy-N-methylmorphinan and their pharmaceutically acceptable salts, and the use thereof.

### BACKGROUND OF THE INVENTION

This application concerns therapeutically effective dosage forms of extended release levorphanol and delayed onset, extended release levorphanol, their manufacture and their use in subjects in need of levorphanol.

Extended release opioid formulations have now become the standard of care for the management of chronic pain and a number of extended release opioids have been commercialized. Despite this, chronic pain continues to be a major therapeutic challenge for both patients and their caregivers. There is therefore a need for new therapeutic alternatives for the management of pain, including alternative extended release opioids that are bioavailable, therapeutically effective and pharmacologically differentiated from existing extended release opioids

Levorphanol (or *l* 3-hydroxy-N-methylmorphinan) is a potent opioid analgesic. It is the mirror image of dextrorphan, which is the primary metabolite of the cough suppressant dextromethorphan. Dextrorphan (*l* 3-hydroxy-N-methylmorphinan) is devoid of significant opioid agonist activity. Instead, substantially all the opioid agonist activity resides in the levo isomer, levorphanol.

Levorphanol is differentiated from morphine, hydromorphone, oxymorphone, oxycodone, hydrocodone and other commonly used opioids, by virtue of its different structural class and pharmacology, which confer significant advantages over currently available extended release opioids. For example, levorphanol has significant binding at the kappa and delta opioid receptors as an agonist, and to the NMDA receptor as an antagonist, in addition to its mu [morphine like] opioid agonism. When adjusted for their potencies at the mu opioid receptor, levorphanol has significant greater activity than morphine at the kappa and delta opioid receptor, and robust activity as an NMDA antagonist, the latter being important in modulating pain and opioid tolerance. Levorphanol has also been shown to substantially reverse analgesic tolerance to morphine.

Additionally, levorphanol is purported to have a long half life and a long duration of analgesic action. It would therefore be reasonable to expect widespread use of levorphanol, particularly given expert and consensus opinion about the need for additional (and preferably pharmacologically differentiated) alternatives to "morphine-like" opioids for "opioid rotation". Despite this, compared to other opioids, levorphanol has been a commercial failure. It is hardly ever prescribed and its U.S. unit sales are negligible. Furthermore, levorphanol is not actively promoted by any pharmaceutical company.

Levorphanol is commercially available only in the United States, and only in one (2 mg) strength, providing little dosing flexibility for the treating physician and for the patient with pain. For example, using the purported 5:1 potency ratio of levorphanol to morphine, 2 mg or levorphanol would be approximately equal to 10 mg of morphine and approximately equal to 2 mg of hydromorphone. However, oral immediate release morphine is available in tablet form at an equianalgesic dose to levorphanol which is 3 times higher and in an almost unlimited dose as a 20 mg/mL concentrate. Hydromorphone is available in tablet form at an equianalgesic dose to levorphanol which is 4 times higher and in an almost unlimited dose as a 1 mg/mL concentrate.

To the applicant's knowledge, no extended release dosage forms of levorphanol have been developed or commercialized in any country. There is therefore a need for a therapeutically effective extended release dosage form of levorphanol: (i) as first line opioid therapy in chronic pain; (ii) as an alternative to extended release dosage forms of morphine and morphine-like opioids (e.g., hydromorphone, oxycodone and oxymorphone); (iii) for use in patients who have a suboptimal efficacy or safety response to currently available extended release dosage forms of opioids; and (iv) in the setting of pharmacologic tolerance to morphine-like drugs.

Commercially available levorphanol tartrate tablets (from Roxane Laboratories, Columbus, Ohio) must be stored in the narrow range of 20 °C to 25 °C (USP Controlled Room Temperature) and dispensed in a Tight Container as defined by United States Pharmacopeia, USP/NF. Storage specifications which are restrictive can add to the economic cost of the product, reduce its shelf life, and inconvenience the patients. Another aspect of the invention provides for improved storage conditions for once-daily extended release levorphanol.

In view of the foregoing, it is immediately apparent that a serious need exists for an improvement in the dosage form of oral levorphanol for optimal therapeutic effect. There are other product performance issues associated with commercially available oral levorphanol tartrate IR tablets.

According to the FDA approved U.S. prescribing information for commercially available levorphanol tartrate tablets, (i) the onset of analgesia following administration of levorphanol is similar to morphine; (ii) the peak analgesic effect following administration of levorphanol is similar to morphine; and (iii) levorphanol is well absorbed after oral administration with peak plasma concentrations occurring approximately 1 hour after dosing. The applicant has determined that the foregoing statements are substantially incorrect when applied to the commercially available oral IR levorphanol tartrate tablets.

Commercially available IR levorphanol tablets have an in-vitro release rate of more than about 85% at about 10 minutes and more than about 95% at about 20 minutes when tested by the USP Paddle Method at 50 rpm. Despite this, the onset and peak analgesia of commercially available levorphanol tartrate tablets can be demonstrated to be significantly slower than commercially other commercially available opioids.

In view of the foregoing, it is immediately apparent that a serious that a serious need exists for an improvement in the dosage forms of oral levorphanol for the treatment of pain. In view of the shift in standard of care for chronic pain, there is a need for robust extended release dosage forms of oral levorphanol.

Commercially available immediate-release dosage of levorphanol are available only as the tartrate salt in about a 1:1 molar ratio and comprising, in addition to levorphanol tartrate dihydrate, lactose, corn starch, stearic acid, magnesium stearate and talc. These excipients account for about 98% of the tablet weight. These pharmaceutical excipients are ubiquitous in modern pharmaceutical dosage forms. However, in the case of the commercially available dosage form of levorphanol tartrate, these excipients may provide an inefficient dosage form.

There is therefore a need for therapeutically effective dosage forms of levorphanol which are devoid of the excipients found in commercially available oral levorphanol tablets and which provide more rapid and consistent in vivo performance.

There is therefore a need for therapeutically effective dosage forms of levorphanol which provide a robust therapeutic response, suitable for up to once-a-day administration and up to 24 hours of therapeutic effect.

Continuous suppression of pain through the use of around the clock opioid analgesics is now recommended in numerous guidelines for pain management. Extended release opioids can result in fewer interruptions in sleep, reduced dependence on caregivers, improved compliance, enhanced quality of life outcomes, and increased control over the management of their pain. In addition, such formulations can provide more constant plasma concentrations and clinical effects, less frequent peak to trough fluctuations and fewer side effects, compared with short acting opioids.

A number of oral extended release formulations of opioid analgesics have been developed or commercialized, including morphine, hydromorphone, oxycodone, hydrocodone and oxymorphone. However, these opioid analgesics have very similar pharmacologic profiles and do not provide adequate clinical differentiation.

To the applicant's knowledge, there are no marketed extended release formulations of levorphanol.

In addition to the pharmacologic differences with existing extended release opioids, the effect of levorphanol on monoaminergic reuptake inhibition has previously been underestimated. Monoamines play an important role in modulating analgesia. This finding provides an important additional impetus for the development of an extended release dosage form, since it has been shown that extended release dosage forms of monoaminergic reuptake inhibitors produce fewer adverse effects.

There are important additional reasons for the development of therapeutically effective and efficient dosage forms of extended release levorphanol. For example, pain clinicians frequently report that patients with poor analgesic efficacy or safety outcomes on one opioid do well on alternate opioids. Many opioid side effects are sufficiently bothersome as to require: (i) use of additional medications to treat the iatrogenic symptoms; (ii) more intensive patient management; (iii) use of lower doses that leave patients in continued pain; or (iv) in other cases, complete discontinuation of analgesic therapy. Patients receiving chronic opioid therapy also frequently report a reduction in efficacy over time, presumably due to the development of pharmacologic tolerance. As a consequence of this wide variability in efficacy and safety, current guidelines recommend that patients with poor outcomes on one opioid should be tried on another opioid. This concept is referred to as "opioid rotation", (see for example, Agency for Health Care Policy and Research Clinical Practice Guidelines for Cancer Pain Management, Guideline No. 9, AHCPR Publication No. 94-0592, March 1994; American Pain Society Guideline for the Management of Cancer Pain in Adults, 2005; Hagen and Babul, Cancer 1997;79:1428-37).

Oral extended release formulations of levorphanol with their differential opioid receptor binding and clinical effects have the potential to be a drug of choice in opioid rotation regimens in patients who don't respond well to other available extended release opioids (e.g., oxycodone, morphine, oxymorphone, hydromorphone).

There are several purported challenges to the wide spread use of oral levorphanol which have until this application, provided a significant counter-argument to the development of an extended release dosage forms. These include its purported long half-life, complex dose titration, risk of significant accumulation with repeated dosing, and a long time to steady state. Such counter-arguments have heretofore proved to be a deterrent to the development and commercialization of extended release levorphanol. Long-half life drugs are considered by some to be unsuitable for extended release formulations for a number of reasons, including: (i) the lack of justification for an extended release formulation, since the drug may be administered in immediate release form with the purported benefit of an extended duration of therapeutic effect; (ii) the considerably longer time to reach steady state, which means that patients may wait an extended period of time before achieving adequate, optimum or maximum therapeutic benefit; (iii) the risk of drug accumulation and associated toxicity over time; (iv) the difficulty with dose titration, especially rapid dose titration, which leaves the patient vulnerable to accumulation and drug intoxication; (v) the need to wait until steady state before further dose titration; (vi) the risk of unexpected late onset opioid toxicity due to drug accumulation, after the clinician and patient have been "lulled" into an expectation of tolerability. Under the long half-life scenario, it has been argued by some that the use of drugs with long half-lives in extended release formulations may put patients at risk for increased nausea, vomiting, sedation, constipation, fatigue, obtundation and more seriously, respiratory depression. This in turn could require greater medical management (e.g., more frequent medical monitoring, more frequent clinic visits, slow titration and more frequent dose adjustments) and the concurrent use of drugs to manage the side effects (e.g., antiemetics, laxatives, etc). Below is a review of the purported challenges and some of the findings of the invention which provide further support the development of extended release levorphanol.

Applicant has surprisingly discovered that the mean apparent elimination half-life, pharmacokinetic variability and accumulation kinetics of oral extended release levorphanol tested of the invention are about the same or less than for commonly used extended release opioids such as morphine, hydromorphone, oxycodone and oxymorphone.

Therefore, there is now an even more compelling rationale for developing extended release dosage forms of levorphanol which provide efficacy and safety at least comparable to extended release dosage forms of morphine, hydromorphone, oxycodone and oxymorphone.

A challenge in developing opioids for once-daily administration is the documented poor bioavailability of unitary, monolithic, matrix or non-multiparticulate drug extended release opioid delivery systems. Another aspect of the invention provides for improved oral bioavailability of once-daily extended release levorphanol.

To the applicant's knowledge, there are no commercially available non-multiparticulate once-daily extended release compositions of any opioid analgesics in the USA.

Another aspect of the invention provides for resistance of the dosage forms of extended release levorphanol to alcohol induced dose dumping. This problem has been documented with several extended release opioid analgesics and can have serious adverse consequences. (Sloan and Babul, Expert Opinion on Drug Delivery 2006;3:489-97)

There is therefore a need for extended release opioids which do not evidence dose dumping in relation to alcohol intake, which do not evidence clinically significant changes in rate or extent of absorption in relation to alcohol intake, which do not evidence clinically significant pharmacodynamic variability in relation to alcohol intake, and which do not evidence bio-inequivalence of the dosage form when given with or without alcohol.

Many commercialized extended release opioids have been shown to have a significant food effect. Another aspect of the invention provides for reduced fed fasted pharmacokinetic variability. An important issue with oral extended release products is its potential for "dose dumping" in relation to food, where the active drug, intended for slow release, is instead released rapidly, resulting in toxicity on the one hand and a decreased duration of effect on the other.

There is therefore a need for extended release opioids which do not evidence dose dumping in relation to food intake, which do not evidence clinically significant changes in rate or extent of absorption in relation to food intake, which do not evidence clinically significant pharmacodynamic variability in relation to food intake, and which do not evidence bio-inequivalence of the dosage form when given in a fed or fasted state.

A pharmaceutically acceptable dosage form of oral levorphanol for the treatment of levorphanol responsive conditions beyond its short duration of action at a controlled rate over an extended period of time appears to be lacking in the pharmaceutical and medical arts.

There is therefore a need for new pharmaceutical compositions and methods for subjects in need of levorphanol which provide a prolonged duration of therapeutic effect when given orally.

An important drawback with the use of opioid analgesics is the risk of addiction, diversion and abuse. Tampering extended release opioid formulations can deliver a significant dose in immediate release form and produce a variety of potentially serious or life threatening side effects. The focus of virtually all abuse resistant technology for extended release opioid formulations has been predicated on abuse through tampering of the extended release dosage form by the recreational drug user or drug addicts. In the applicants view, the foregoing technologies are not an adequate solution to the problem of opioid abuse and fail to take into account the full spectrum of misuse, overuse and abuse of opioids. The invention also provides extended release dosage forms of levorphanol which provide a novel composition and method of deterring abuse by patients, recreational drug users and individuals with an addition disorder.

A major issue with pharmacologic management of chronic conditions is compliance with therapy. Compliance with extended releases dosage opioid analgesics is still not adequate, which defeats the now widely recommended concept of continuous suppression of pain. Another aspect of the invention provides for improved compliance with treatment.

An additional reason for the lack of commercial success of levorphanol may relate to incorrect equianalgesic and potency conversion tables in the literature for levorphanol relative to morphine. Another aspect of the invention provides for appropriate potency ratios relative to morphine.

In view of the foregoing presentation, it is immediately apparent that a serious need exists for an improvement in the delivery of oral levorphanol for its therapeutic effect. The need exists to provide a novel therapeutic composition comprising oral levorphanol, the need exists to provide a novel dosage form comprising oral levorphanol, and the need exists to provide a novel method of administering levorphanol to a patient in need of levorphanol therapy.

Therefore, the present invention has been made with a view towards solving the above problems of the prior art.

The invention provides an oral, relatively easy mode and manner of levorphanol administration.

### SUMMARY OF THE INVENTION

It is apparent from the foregoing description that levorphanol has had had little commercial success and has heretofore been viewed as unsuitable for therapeutic use in an extended release dosage form for a variety of reasons.

The present invention is therefore directed at extended release pharmaceutical compositions of oral levorphanol, it manufacture and use for the treatment for patients in need of levorphanol.

The present invention is also directed at extended release oral pharmaceutical compositions of levorphanol and methods of treatment for patients with pain, dyspnea, cough, addiction disorders and other levorphanol or opioid responsive medical conditions.

Applicant has developed a extended release dosage form of levorphanol based on numerous in vitro and in vivo findings. There are several aspects to this invention, including: (1) robust, therapeutically effective oral extended release dosage forms of levorphanol for dosing up to once-a-day (e.g., twice-a-day (BID), once-a-day (QD), Q12H or Q24H); (ii) highly bioavailable non-multparticulate oral extended release dosage forms of levorphanol for dosing once-a-day (e.g., QD or Q24H); (3) extended release dosage forms of levorphanol which provide a substantially greater temperature range of stability than claimed by the only commercially marketed oral dosage form of levorphanol; (4) extended release dosage forms of levorphanol which provide a comparable onset and peak analgeic effect to other extended release opioids, unlike commercially marketed oral dosage form of immediate release levorphanol, when compared with other immediate release opioids; (5) extended release dosage forms of levorphanol that can reduce side effects from the heretofore unknown substantially greater selectivity for serotonin reuptake inhibition over norepinephrine reuptake inhibition for levorphanol; (6) the heretofore unknown apparent elimination half-life for extended release levorphanol substantially less that suggested in the published literature from oral immediate release levorphanol data; (7) the heretofore unknown apparent elimination half-life for extended release levorphanol which is about the same or less than the apparent elimination half-life of other extended release opioids; (8) the heretofore unknown apparent time to steady state and accumulation on repeated dosing for extended release levorphanol which is about the same or less than for other extended release opioids; (9) a surprisingly slow in vitro dissolution rate corresponding to optimal (faster) in vivo release than predicted by other extended release opioids; (10) a higher therapeutic dose and wider therapeutic dose range for extended release levorphanol based on the the heretofore overestimation of levorphanol potency; (11) abuse resistance of the dosage form; (12) a method of achieving abuse resistance based on the development and use of delayed onset, extended release dosage forms of levorphanol for duodenal, jejunal, ileal and colonic delivery an drelesae of the dose; (13) extended release dosage forms of levorphanol which are resistant to alcohol associated dose dumping; (14) extended release dosage forms of levorphanol which are resistant to dose dumping and pharmacokinetic varaibilty in relation to the co-ingestion with or without food; (15) delayed onset, extended release dosage forms of levorphanol which provide for improved compliance with treatment by making the formulation less effective when taken on as as neede (PRN) basis, rather than on a scheduled (around the clock); and (16) methods to achieve efficient dose titration and therapeutic effect with reduced side effects.

Another aspect of the invention provides for improved oral bioavailability of once-daily extended release levorphanol. A challenge in developing opioids for once-daily administration is the documented poor bioavailability of unitary, monolithic, matrix or non-multiparticulate drug extended release opioid delivery systems. Matrix extended release dosage forms are a commonly used technology for development of extended release formulations. The popularity of matrix extended release drug delivery systems can be attributed to a number of factors. Such delivery systems (i) may be developed, manufactured using conventional processing and equipment; (ii) require no further capital expenditures; and (iii) have the capability to handle a wide range drug loads and drugs. However, in the case of opioids, extended release non-multiparticulate systems for once-daily administration purportedly provide poor oral bioavailability despite providing in vitro evidence (e.g., dissolution) suggestive of once-daily delivery in vivo. Without being bound by theory, this reduced bioavailability may be due to uneven or incomplete liberation or absorption of the opioid from matrix dosage forms in the distal gastrointestinal tract (necessary for once-daily extended release dosage forms) due the local environment and its interaction with the dosage form (e.g., level of hydration, pH, levels of surfactant, presystemic biotransformation).

Applicant has surprisingly discovered that extended release dosage forms of levorphanol suitable for up to once-a-day administration including non-multiparticulate dosage forms can provide surprisingly good bioavailability.

Applicant has also surprisingly discovered that certain hydrogenated vegetable oil compositions can provide a preferred bioavailability and extended release pharmacokinetic profile for levorphanol, making them suitable for up to once-a-day administration (e.g, Q12H or Q24H).

For oral extended release dosage forms, the in vitro dissolution rate and bioavailability are the two most salient properties that require consideration when assessing the performance of a dosage form. The applicant has surprisingly discovered that to provide therapeutically effective dosage forms of levorphanol suitable for dosing every 12 or 24 hours or suitable for therapeutic effects for about 12 or about 24 hours, certain extended release dosage form of the invention require an unexpectedly slow in vitro release rate of levorphanol, for example 85%, or 90%, or 95% release at about 25, 30, 35, 40, 45, 50, or 55 hours, when measured by the USP Paddle Method with a sinker at 75 rpm in 600 mL of simulated intestinal fluid (SIF) USP, pH 6.8 (without enzymes) at 37 °C.

Another aspect of the invention provides for extended release dosage forms of levorphanol which resist abuse by patients, recreational drug users and individuals with an addition disorder. Extended release opioids which do not require the incorporation of aversive and potentially unsafe excipients into the formulation, which do not require the incorporation of sequestered or unsequestered opioid antagonists, which involve multiple mechanism of abuse deterrence and/or complement other safe and effective methods of abuse deterrence provide a significant therapeutic advantage. The abuse deterrent pharmaceutical dosage forms of the invention are achieved in part through delayed onset, extended release dosage forms which provide duodenal release, jejunal release, ileal release, ileo-colonic release or colonic release of the extended release levorphanol from the dosage form.

An important drawback with the use of opioid analgesics is the risk of addiction, diversion and abuse. Tampering extended release opioid formulations can deliver a significant dose in immediate release form and produce a variety of potentially serious or life threatening side effects. The focus of virtually all abuse resistant technology for extended release opioid formulations has been predicated on abuse through tampering of the extended release dosage form by the recreational drug user or drug addicts. Such technologies purport to (i) frustrate attempts at dosage form tampering to extract the drug; (ii) nullify the effects of the drug if tampered, and/or (iii) produce an unpleasant or unwanted effect when consumed in tampered form.

In the applicants view, the foregoing technologies are not an adequate solution to the problem of opioid abuse and fail to take into account the full spectrum of misuse, overuse and abuse of opioids. Such technologies show an overreliance on the experience of addiction disorder clinics and emergency rooms which treat a very small minority of non-medical opioid users, primarily "hard core" abusers who consume the dosage form after tampering to maximize the delivered dose. In contrast to this skewed observation derived from a minority of non-medical "hard core" opioid abusers, in a vast majority of cases, the abuse of opioid analgesics is with the intact dosage form (i.e., the dosage form has not been physically manipulated or tampered with to alter its absorption profile) and the opioid is taken by the usual (oral) route of administration. For example, a vast majority of recreational drug users and patients with an addiction disorder, including iatrogenic addiction disorders will seldom or never use an opioid intended to be taken orally by any other route (e.g., intravenously after extraction and filtration, or by inhalation), nor will they physically manipulate or tamper the dosage form prior to oral ingestion. This population has a different self-image of their non-medical opioid use and attempts to distinguish or differentiate themselves and their use (abuse) from what they sometimes perceive as "reckless" and "irresponsible" use by "junkies", "addicts", "hard core addicts" or "real addicts". In addition, a considerable amount of abuse of opioids is the intact ingestion at a dose which is simply higher that the medically prescribed dose. A majority of technologies described in the art do little or nothing to deal with the abuse of intact dosage forms of opioids.

### BRIEF DESCRIPTION OF THE DRAWINGS

The included drawings are illustrative but not limiting of the methods and composition of the present invention. Other suitable modifications and adaptations of the variety of conditions and parameters normally encountered and obvious to those skilled in the art are within the spirit and scope of the invention.
Figures 1 to 53 are fully described within the specifications, within Example 6 to Example 40, inclusive.
Figure 54 shows the mean Levorphanol ER release rate obtained with a prototype over about 48 hours when measured by the USP Paddle Method with a sinker at 75 rpm in 600 mL of simulated intestinal fluid (SIF) USP, pH 6.8 (without enzymes) at 37 °C.

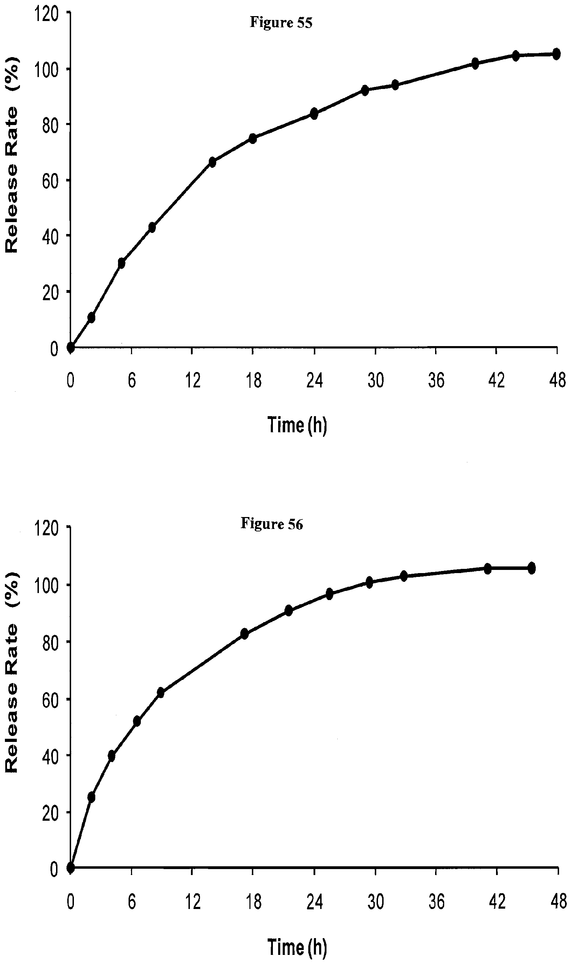

Figure 55 shows the mean Levorphanol ER release rate for obtained with a prototype over about 48 hours when measured by the USP Paddle Method with a sinker at 75 rpm in 600 mL of simulated intestinal fluid (SIF) USP, pH 6.8 (without enzymes) at 37 °C.
Figure 56 shows the mean Levorphanol ER release rate obtained with a prototype over about 48 hours when measured by the USP Paddle Method with a sinker at 75 rpm in 600 mL of simulated intestinal fluid (SIF) USP, pH 6.8 (without enzymes) at 37 °C.
Figure 57 shows the mean Levorphanol ER release rate obtained with a prototype over about 48 hours when measured by the USP Paddle Method with a sinker at 75 rpm in 600 mL of simulated intestinal fluid (SIF) USP, pH 6.8 (without enzymes) at 37 °C.
Figures 58 through 61 show the mean levorphanol plasma concentration time profile obtained with the prototypes of Levorphanol ER in Example 96.
Figures 62 through 65 show the surprising relationship between the release rate of levorphanol and the plasma concentration time profile in Example 96.
Figures 66 through 69 show a superior extended release human pharmacokinetic profile for various dosages of Levorphanol ER described in Example 96.

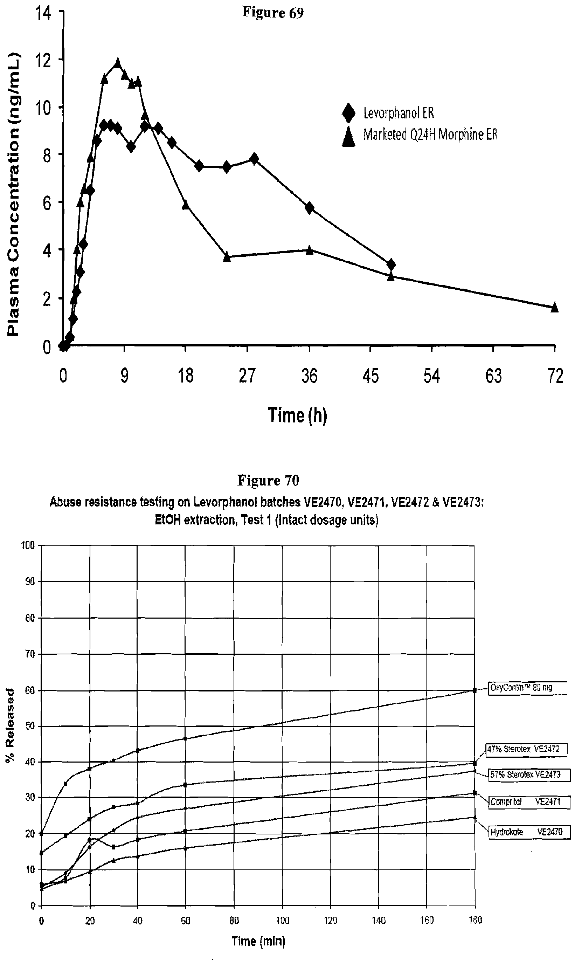

Figure 70 shows the of resistance of four Levorphanol ER dosage forms of the invention to dose dumping in a high ethanol concentration gastric environment, in comparison with a commercially available extended release dosage form of oxycodone (reference). To simulate this, representative dosage forms were put through an extreme stress test: (i) the intact dosage form was placed in 18 mL of 0.1N HCl in a 60 mL bottle; (ii) the contents were agitated at 240 rpm on an orbital shaker for 30 min; (iii) after 30 min, 12 mL of 95% ethanol was added and the contents swirled and a 1 mL aliquot obtained; (iv) agitation was continued at 240 rpm and additional aliquots obtained at 10, 20, 30, 40, 60 and 180 minutes. In this Figure, Time "0" is after 30 minutes of prior agitation in acid at 240 RPM x 30 Minutes. All four tested Levorphanol ER formulations demonstrated excellent resistance to dose dumping in the presence of ethanol. Formulation VE2470 which comprises hydrogenated palm kernel oil showed the greatest level of resistance to dose dumping in the in a high ethanol concentration gastric environment. In contrast, the reference dosage form was significantly more susceptible to the effects of ethanol and produced release rates that may be associated with toxicity, particularly in opioid naive subjects.

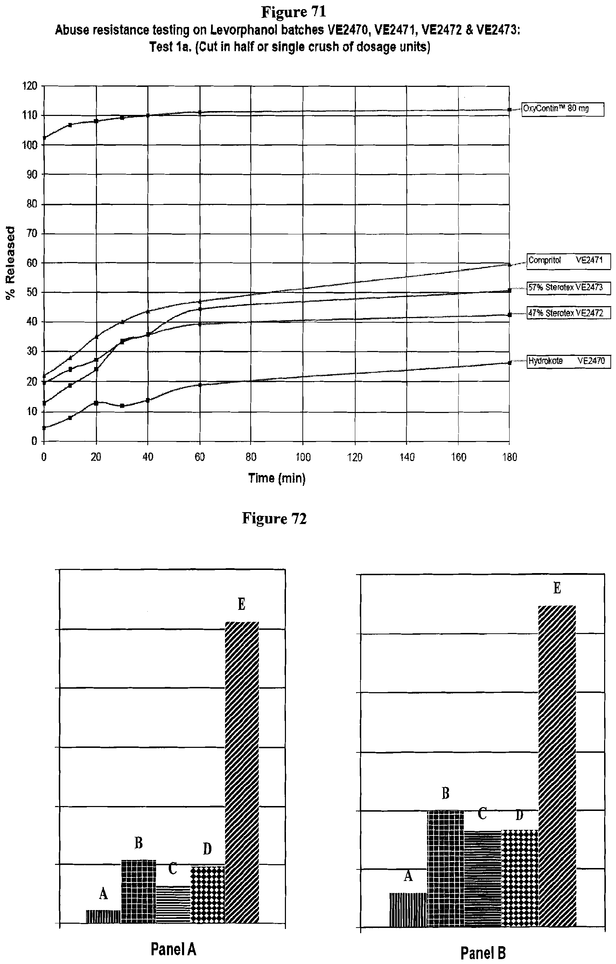

Figure 71 shows the of resistance of four Levorphanol ER dosage forms of the invention to dose dumping even in a tampered form in a high ethanol concentration gastric environment in comparison with a tampered commercially available extended release dosage form of oxycodone (reference). To simulate this, representative dosage forms were put through an extreme stress test: (i) a tampered dosage form was placed in 18 mL of 0.1N HCl in a 60 mL bottle; (ii) the contents were agitated at 240 rpm on an orbital shaker for 30 min; (iii) after 30 min, 12 mL of 95% ethanol was added and the contents swirled and a 1 mL aliquot obtained; (iv) agitation was continued at 240 rpm and additional aliquots obtained at 10, 20, 30, 40, 60 and 180 minutes. In this Figure, Time "0" is after 30 minutes of prior agitation in acid at 240 RPM x 30 Minutes. All four tested Levorphanol ER formulations demonstrated significant resistance to dose dumping in the presence of ethanol. Formulation VE2470 which comprises hydrogenated palm kernel oil showed the greatest level of resistance to dose dumping in the in a high ethanol concentration gastric environment. In contrast, the reference dosage form was significantly more susceptible to the effects of ethanol and produced release rates that may be associated with toxicity, particularly in opioid naive subjects.
Figure 72 shows resistance of four Levorphanol ER formulation batches (A, B, C and D) to dose dumping in a simulated high ethanol concentration gastric environment, when compared with a commercially available extended release dosage form of oxycodone (E), each tested in a tampered state after continuous agitation in acidic media comprising 0.1N HCl (pH 1.2) at 240 rpm for 30 minutes (Panel A), and after agitation in acidic media (0.1N HCl) for 30 minutes, followed by the addition of 95% ethanol and further agitation for 30 min at 240 rpm (Panel B). All Levorphanol ER formulations demonstrated significant resistance to dose dumping in the presence of ethanol, while the extended release oxycodone was highly susceptible to the dose dumping in simulated high ethanol gastric environment and produced active drug release rates that may be associated with significant toxicity. Formulation VE2470 which comprises hydrogenated palm kernel oil showed the greatest level of resistance to dose dumping in the in a high ethanol concentration gastric environment.
Figures 73 through 82 primarily address the delayed onset, extended release dosage Levorphanol ER dosage forms of the invention which are therapeutically effective and which also provide abuse deterrence and improved compliance as further described in the specifications.

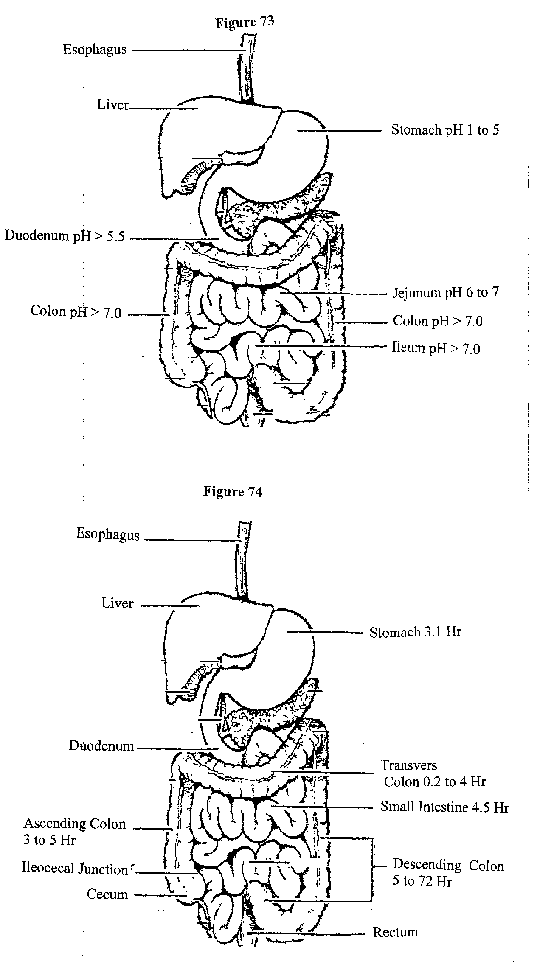

Figure 73 illustrates the average pH for dissolution of many pH sensitive polymers in the various segments of the gastrointestinal tract.
Figure 74 illustrates the average gastrointestinal transit times for various segments of the gastrointestinal tract.

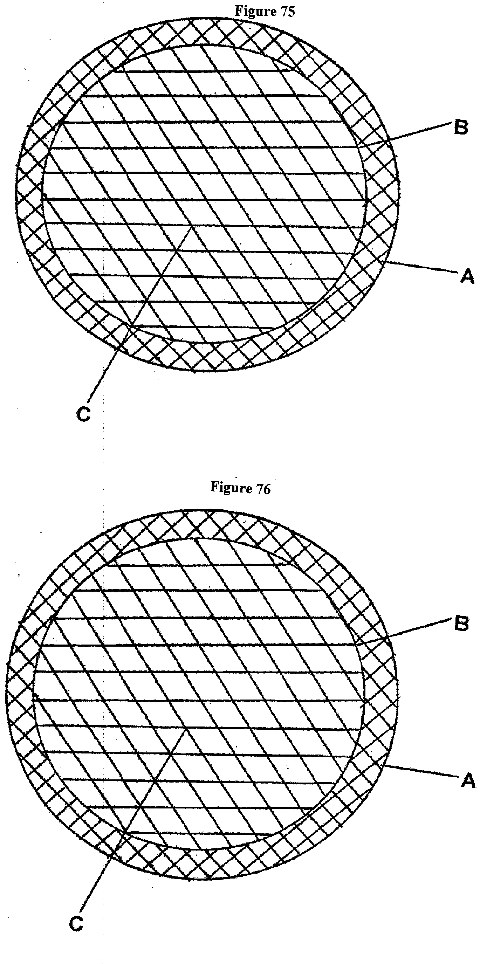

Figure 75 illustrates the cross sectional view of an oral capsule or tablet dosage form of delayed onset, extended release oral levorphanol, where (A) is a dissolution, diffusion and/or pH sensitive material to render the dosage form delayed onset, optionally further overcoated with a film coat or an immediate release active; (B) is the outer shell of the capsule, optionally sealed in the overlapping region of capsule body and cap, or the usual outer margin of the core tablet, each optionally coated with additional excipients prior to coating with said material; and (C) is a monolithic solid, or a compressed multiparticulate plug, or one or more populations of multiparticulates (e.g. beads, spheroids, pellets) comprising the active drug and controlled release material to render the dosage form extended release, and optionally, also delayed onset. The dosage form resists release of the active drug in the stomach, and optionally, also in the duodenum, jejunum and ileum, or until it reaches the desired GI pH, or until a specified amount of time has elapsed after ingestion.
Figure 76 illustrates the cross sectional view of an oral tablet dosage form of delayed onset, extended release oral Levorphanol, where (A) is a dissolution, diffusion or pH sensitive material to render the dosage form delayed onset, optionally further overcoated with a film coat or an immediate release active; (B) is the usual outer margin of the core tablet, optionally coated with controlled release material or additional excipients prior to coating with dissolution, diffusion or pH sensitive material; and (C) is the tablet core comprising the active drug and controlled release material to render the dosage form extended release (said controlled release material optional in the event the core tablet in "B" is coated with controlled release material), and optionally, also delayed onset. The dosage form resists release of the active drug in the stomach, and optionally, also in the duodenum, jejunum and ileum, or until it reaches the desired GI pH, or until a specified amount of time has elapsed after ingestion.

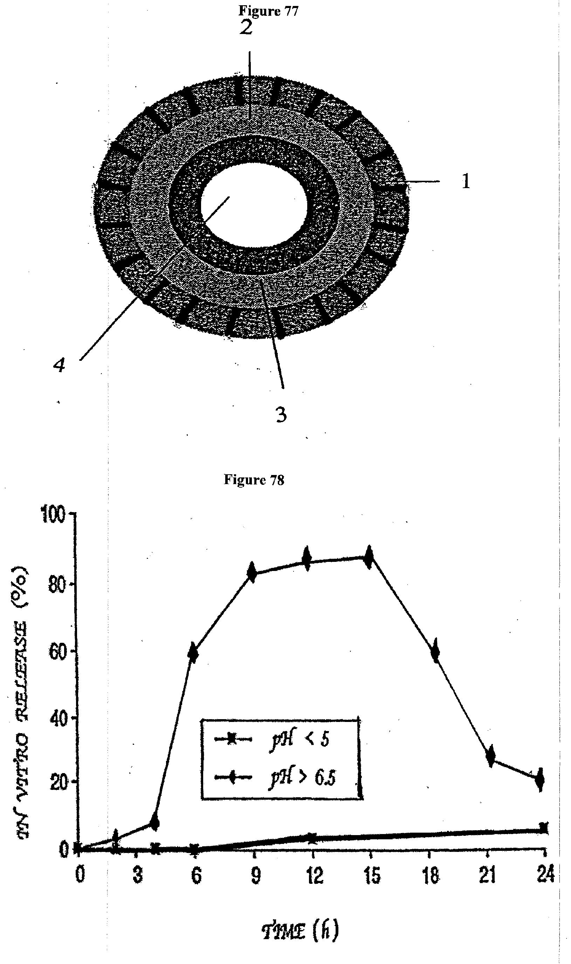

Figure 77 illustrates a dosage form for colonic release, where (1) the outer layer which dissolves at a pH of about 7; (2) a sustained release polymer coating; (3) levorphanol which has been coated onto a nonpareil core or bead (4).
Figure 78 illustrates the in vivo release of a delayed onset, extended release dosage form of Levorphanol comprising an ileo-colonic pH sensitive polymer embedded in the dosage form or coated on the dosage form. The dosage form resists release of the active drug at pH less than 5 for a prolonged period of time and gradually releases the active drug from the dosage form at a pH greater than 6.5.

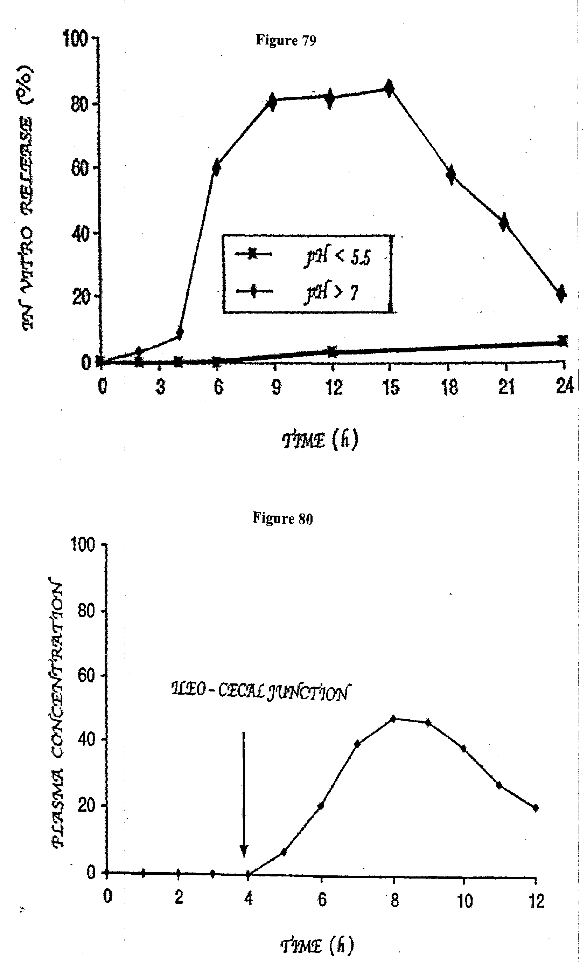

Figure 79 illustrates the in vivo release of a delayed onset, extended release dosage form of Levorphanol ER comprising an ileo-colonic pH sensitive polymer embedded in the dosage form or coated on the dosage form. The dosage form resists release of the active drug at pH less than 5.5 for a prolonged period of time and gradually releases the active drug from the dosage form at a pH greater than 7.
Figure 80 illustrates the in vivo release of a delayed onset, extended release dosage form of oral levorphanol upon reaching or traversing the ileo-cecal junction and transiting into the colon.

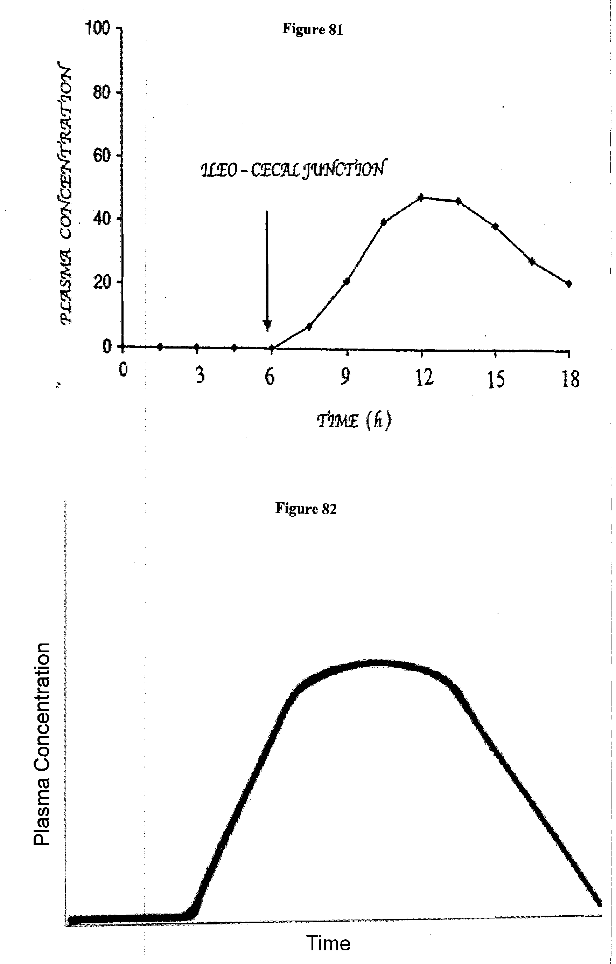

Figure 81 illustrates the in vivo release of a delayed onset, extended release dosage form of oral levorphanol upon reaching or traversing the ileo-cecal junction and transiting into the colon.
Figure 82 illustrates a delayed onset, extended release dosage form for ileo-colonic or colonic release. Following a lag period during which little or no levorphanol is released in vivo, the dosage form upon reaching a certain GI environment (e.g., desired pH, pressure, enzymes, microbial flora) or time, or a combination of variables, gradually releases the active drug.

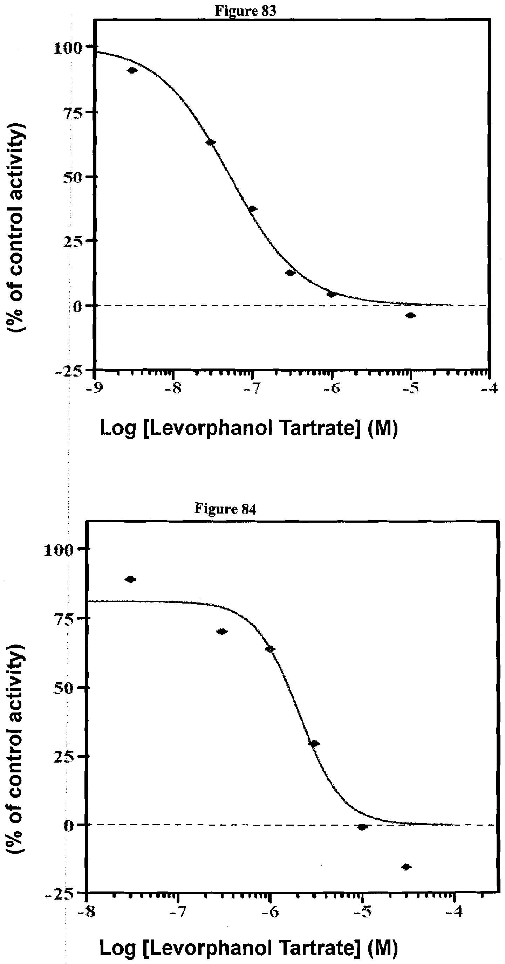

Figure 83 shows the effects of levorphanol tartrate on the [³H]5-HT incorporation into rat brain synaptosomes.
Figure 84 shows the effects of levorphanol tartrate on the [³H]NE incorporation into rat hypothalamus synaptomsomes.

### DETAILED DESCRIPTION OF THE INVENTION

The present invention is as set out in the claims. In particular, the present invention is as set out in the following clauses:
1. A dosage form for orally administering levorphanol to a human patient, the dosage form comprising
   an amount of levorphanol or a pharmaceutically acceptable salt thereof equivalent to 4 to 80 milligrams of levorphanol tartrate, functionally combined with
   a controlled release material selected from the group consisting of waxes, vegetable oils, esters of vegetable oils, and hydrogenated vegetable oils
      when the controlled release material is selected from the group consisting of waxes, vegetable oils and esters of vegetable oils, the controlled release material has been treated by application of heat at least up to its melting point and admixed with a thixotrope and a release rate modifier; and,
      when the controlled release material is hydrogenated vegetable oils, the controlled release material has been treated by application of heat at least up to its melting point and admixed with a thixotrope, a release rate modifier and medium chain triglycerides
         formulated as an orally-administrable dosage form said dosage form resistant or substantially resistant to dissolution and/or absorption in the stomach, and/or the duodenum, and/or the jejunum, and/or the ileum.
2. The dosage form of clause 1, formulated such that substantially no release of levorphanol therefrom occurs less than about 2 hours, optionally 4 hours, after oral administration of the dosage form to the patient.
3. The dosage form of any preceding clause, formulated such that, following oral administration of the dosage form to the patient, substantially no release of levorphanol therefrom occurs proximal to the stomach or in any portion of the gastrointestinal tract distal to the stomach that has a pH less than 5.
4. The dosage form of any preceding clause, further comprising at least one of i) a release rate modifier selected from the group consisting of hydroxylpropyl celluloses, cellulose acetates, powdered celluloses, cellulose acetate phthalates, hydroxyethylmethyl celluloses, carboxymethylcelluloses,hypromellose acetates, hypromellose phthalates, ethylcelluloses, and mixtures of these; and ii) a thixotrope selected from the group consisting of metal oxides, microcrystalline methylcelluloses, clays, silicates, carbon black, and combinations of these.
5. The dosage form of any preceding clause, said dosage form resistant or substantially resistant to dissolution and/or absorption in the stomach.
6. The dosage form of any preceding clause, said dosage form resistant or substantially resistant to dissolution and/or absorption in the duodenum.
7. The dosage form of any preceding clause, said dosage form resistant or substantially resistant to dissolution and/or absorption in the jejunum.
8. The dosage form of any preceding clause, said dosage form resistant or substantially resistant to dissolution and/or absorption in the ileum.
9. The dosage form of any preceding clausefor use as a medicament.

Any discussion of "embodiments" in the present specification relating to embodiments which do not fall within the claims relates to embodiments of the disclosure and not to embodiments of the invention. Methods of treatment do not form part of the present invention.

It is an object of certain embodiments of the present invention to provide bioavailable formulations of extended release levorphanol suitable for up to twice-day or up to once-a-day administration to subjects in need of levorphanol.

It is an object of certain preferred embodiments of the present invention to provide oral formulations which provide therapeutic effects for up to about 8, 10, 12, 14, 16, 18, 20, 22, 24 or 30 hours.

It is an object of the present invention to provide delayed onset, extended release dosage forms with reduced abuse which provide duodenal release, jejunal release, ileal release, ileo-colonic release or colonic release of the extended release levorphanol from the dosage form.

In some preferred embodiments, the dosage form provides an oral extended release pharmaceutical composition of levorphanol is a delayed onset, extended release of levorphanol.

The above objects and others are attained by virtue of the present invention, which in certain embodiments, provides aN oral extended release dosage form comprising an analgesically effective amount of levorphanol or a pharmaceutically acceptable salt thereof and a sufficient amount of controlled release material to render the dosage form suitable for up to once-a-day administration (e.g., Q12H, BID, QD, Q24H).

### Pharmacokinetics of Extended Release Levorphanol

In some preferred embodiments, the dosage form provides an oral extended release pharmaceutical composition comprising a therapeutically effective amount of levorphanol; and a controlled release material with gastroretentive properties to render said dosage form suitable for extended release oral administration to a human patient.

In some preferred embodiments, the composition is a liquid filled thermosoftening extended release dispersion systems which provides an alternative dosage form and method and process of manufacture of extended release levorphanol. This dosage form is advantageously utilized to prepare a robust, cost effective formulation of the invention which (i) avoids one or more or all of the excipients in commercially available oral immediate-release levorphanol tartrate tablets and their associated adverse impact on the in vivo performance of the levorphanol as discussed herein; (ii) substantially reduces or eliminates the need for pharmaceutical excipients generally recommended or considered necessary in a solid the dosage form (e.g. glidants, lubricants, diluents, fillers, binders, disintegrants and antioxidants), with associated cost, workplace safety and patient safety advantages discussed herein; (iii) is relatively easy to scale-up to manufacturing batch size; and (iv) requires a relatively simple manufacturing process, which may optionally be performed entirely in situ in a single heated mixing vessel prior to transfer to a filling machine for encapsulation. This method also overcomes many of the disadvantages of conventional extended release dosage forms (for example, matrix formulations) referred to herein.

Commercially available immediate-release dosage forms of levorphanol contain levorphanol tartrate in combination with (i) lactose, corn starch, stearic acid, magnesium stearate and talc; or (ii) lactose, stearic acid and talc; or (iii) lactose, corn starch, stearic acid and talc. When the commercially available oral immediate release levorphanol 2 mg tablets are subjected dissolution, the in-vitro release rate by weight of levorphanol by the USP Paddle Method at 50 rpm is more than 85% at about 10 minutes and more than 95% at about 20 minutes. Despite the rapid release of substantially all or all of the levorphanol from commercially available immediate-release tablet dosage form when tested in vitro using accepted dissolution methods, levorphanol provides a suboptimal biological (or in vivo) response by the oral route.

In some embodiments, an excipient in the dosage form may serve several different purposes. In some embodiments, the colloidal silicon dioxide serves both as a lubricant and as a glidant.

### Extended Release Levorphanol Dosing

In some preferred embodiments, the invention provides a method for reducing the range in daily dosages required to control pain in substantially all patients, comprising administering an extended release oral levorphanol dosage form comprising from about 4 mg to about 80 mg of levorphanol tartrate, which after first administration or single dose administration and sequential venous sampling to 48 hours provides a systemic exposure of levorphanol as assessed by the mean levorphanol area under the plasma concentration time curve from 0 to 48 hours after first administration or single dose administration (AUC₀₋₄₈) of about 14 ng.hr/mL to about 1430 ng.hr/mL, and a mean maximum plasma concentration of levorphanol from about 0.2 ng/mL to about 70 ng/mL, said dosage form suitable for administration about every 12 hours or about every 24 hours to subjects in need of extended release oral levorphanol.

In some preferred embodiments, the invention provides a method for reducing the range in daily dosages required to control pain in substantially all patients, comprising administering an extended release oral levorphanol dosage form comprising from about 4 mg to about 80 mg of levorphanol tartrate, which after first administration or single dose administration and sequential venous sampling to 48 hours provides a systemic exposure of levorphanol as assessed by the mean levorphanol area under the plasma concentration time curve from 0 to 48 hours after first administration or single dose administration (AUC₀₋₄₈) of about 20 ng.hr/mL to about 1200 ng.hr/mL, and a mean maximum plasma concentration of levorphanol from about 0.4 ng/mL to about 50 ng/mL, said dosage form suitable for administration about every 12 hours or about every 24 hours to subjects in need of extended release oral levorphanol.

In some preferred embodiments, the invention provides a method for reducing the range in daily dosages required to control pain in substantially all patients, comprising administering an extended release oral levorphanol dosage form comprising from about 4 mg to about 80 mg of levorphanol tartrate, which after first administration or single dose administration and sequential venous sampling to 48 hours, provides a mean maximum plasma concentration of levorphanol of about 0.2 ng/mL to about 70 ng/mL from a mean of about 3 hours to about 24 hours, and C₁₂ plasma concentration of levorphanol of about 0.1 ng/mL to about 60 ng/mL, said dosage form suitable for administration about every 12 hours or about every 24 hours to subjects in need of extended release oral levorphanol.

In some preferred embodiments, the invention provides a method for reducing the range in daily dosages required to control pain in substantially all patients, comprising administering an extended release oral levorphanol dosage form comprising from about 4 mg to about 80 mg of levorphanol tartrate, which after first administration or single dose administration and sequential venous sampling to 48 hours, provides a mean maximum plasma concentration of levorphanol of about 0.4 ng/mL to about 50 ng/mL from a mean of about 3 hours to about 24 hours, and C₁₂ plasma concentration of levorphanol of about 0.2 ng/mL to about 45 ng/mL, said dosage form suitable for administration about every 12 hours or about every 24 hours to subjects in need of extended release oral levorphanol.

### Extended Release Levorphanol Dosage Forms

In some embodiments, the dosage form of the invention is an oral dosage form comprising: (i) a therapeutically effective amount of levorphanol, and (ii) controlled release material to render said dosage form suitable for extended release, said dosage form suitable for dosing every 24 hours.

In some preferred embodiments, the oral dosage form comprises a plurality of pharmaceutically acceptable beads coated with drug and overcoated with controlled release material.

In one embodiment of the invention, the dosage form includes a capsule within a capsule, each capsule containing a different drug or the same drug intended for treating the same or a different malady. In some preferred embodiments, the outer capsule may be an enteric coated capsule or a capsule containing an immediate release formulation to provide rapid plasma concentrations or a rapid onset of effect or a loading dose and the inner capsule contains an extended release formulation. In some preferred embodiments, up to 3 capsules within a capsule are contemplated as part of the invention. In one embodiment of the invention, the dosage form involves one or more tablets within a capsule, wherein the levorphanol is either in the tablet and/or in one of the capsules.

In one embodiment of the invention, the formulation is ingested orally as a tablet or capsule, preferably as a capsule. In another embodiment of the invention, the formulation is administered bucally.

In some preferred embodiments, the oral dosage form comprises (i) a drug layer; and (ii) a displacement layer comprising an osmopolymer; and (b) a semipermeable wall surrounding the bilayer core having a passageway disposed therein for the release of said drug.

In some preferred embodiments, the oral dosage form comprises a compressed tablet, compressed capsule or uncompressed capsule. In some preferred embodiments, the oral dosage form comprises a liquid fill capsule.

In some preferred embodiments, the pharmacokinetic and pharmacodynamic parameters of the specifications and claims are determined under fed conditions. In other preferred embodiments, the pharmacokinetic and pharmacodynamic parameters of the specifications and claims are determined under fasted conditions.

In some preferred embodiments, the dosage form provides an oral extended release pharmaceutical composition comprising a therapeutically effective amount of levorphanol; said dosage form intended solely for the treatment of pain.

In some preferred embodiments, the invention comprises an oral pharmaceutical composition for the treatment of diseases and disorders comprising a therapeutically effective amount of levorphanol.

In some preferred embodiments, the invention comprises an oral pharmaceutical composition for the treatment of pain comprising a therapeutically effective amount of levorphanol.

It is an object of certain embodiments of the present invention to provide oral levorphanol wherein the levorphanol is dispersed within a matrix.

In certain preferred embodiments the oral dosage form of the present invention comprises a matrix which includes a controlled release material and levorphanol or a pharmaceutically acceptable salt thereof. In certain preferred embodiments, the matrix is compressed into a tablet and may be optionally overcoated with a coating that in addition to the controlled release material of the matrix may control the release of the levorphanol or pharmaceutically acceptable salt thereof from the formulation, such that blood levels of active ingredient are maintained within the therapeutic range over an extended period of time. In certain alternate embodiments, the matrix is encapsulated.

In certain preferred embodiments, the extended release oral dosage form of the present invention comprises a plurality of pharmaceutically acceptable extended release matrices comprising levorphanol, the dosage form maintaining the blood plasma levels of levorphanol within the therapeutic range over an extended period of time when administered to patients.

In some preferred embodiments, the dosage form of the invention comprises oral levorphanol formulated to release the levorphanol from the dosage form or to initiate the release of the levorphanol from the dosage form after a certain specific amount of time post-oral ingestion, or at an approximately specific anatomic location in the gastrointestinal tract, or when the dosage form is in contact with specific gastrointestinal conditions (e.g., pH range, osmolarity, electrolyte content, food content, pressure, time since first ingestion, osmotic pressure in the dosage form, osmotic pressure in the gastrointestinal tract, hydration, etc), said dosage form suitable for providing an orally effective therapeutic for a short, intermediate or extended duration of effect, said dosage form providing a rapid or delayed onset of clinical effect.

In certain preferred embodiments the extended release oral dosage form of the present invention is an osmotic dosage form which comprises a single layer or bilayer core comprising levorphanol; an expandable polymer; a semipermeable membrane surrounding the core; and a passageway disposed in the semipermeable membrane for extended release of the levorphanol or pharmaceutically acceptable salt thereof, such that blood levels of active ingredient are maintained within the therapeutic range over an extended period of time when administered to patients. Other oral osmotic delivery systems may be used for the oral administration of levorphanol, including elementary osmotic pump, multi-chamber osmotic pumps, push-pull osmotic pump, osmotic pumps with nonexpanding second chamber, controlled oral drug delivery systems, controlled porosity osmotic pump, modified osmotic pump for insoluble drugs, multiparticulate delayed-release system, monolithic osmotic systems [see Verma et al, Osmotically Controlled Oral Drug Delivery. Drug Dev Ind Pharm, 2000;26:695-708; Verma et al, Formulation aspects in the development of osmotically controlled oral drug delivery systems. J Controlled Rel. 2002;79:7-27; Xiaoling Li, Design of Controlled Release Drug Delivery Systems (page 1-430), McGraw-Hill Professional; 1 edition (November 3, 2005);Colonic Drug Delivery (page 287-294), Wilson CG, In: Modified-Release Drug Delivery Technology, Second Edition, Vol. 1, Rathbone MJ, Hadgraft J, Roberts MS, Lane ME (eds), Informa Healthcare USA Inc. 2008; Biopolymers and Colonic Delivery, Wilson CG, Mukherji G, Shah HK (pages 295-309), In: Modified- Release Drug Delivery Technology, Second Edition, Vol. 1, Rathbone MJ, Hadgraft J, Roberts MS, Lane ME (eds), Informa Healthcare USA Inc. 2008; Enteric Coating for Colonic Delivery, Shah HK, Mukherji G, Brogmann B, Wilson CG (pages 311-324), In: Modified- Release Drug Delivery Technology, Second Edition, Vol. 1, Rathbone MJ, Hadgraft J, Roberts MS, Lane ME (eds), Informa Healthcare USA Inc. 2008; Programmed Drug Delivery Systems and the Colon, Wilson CG, Shah HK, Lee WW, Brogmann B, Mukherji G (pages 325-335), In: Modified- Release Drug Delivery Technology, Second Edition, Vol. 1, Rathbone MJ, Hadgraft J, Roberts MS, Lane ME (eds), Informa Healthcare USA Inc. 2008; Targeting the Colon Using COLAL™ : A Novel Bacteria-Sensitive Drug Delivery System, McConnell EL, Basit AW (pages 343-348), In: Modified- Release Drug Delivery Technology, Second Edition, Vol. 1, Rathbone MJ, Hadgraft J, Roberts MS, Lane ME (eds), Informa Healthcare USA Inc. 2008, which are hereby incorporated in their entirety by reference for all purposes].

In some preferred embodiments of the invention, the oral levorphanol is in the form of multiparticulates.

In some preferred embodiments of the invention, the oral levorphanol is dispersed in a matrix

In some preferred embodiments of the invention, the oral levorphanol is in the form of multiparticulates can be dispersed in a matrix or contained in a capsule.

In some preferred embodiments of the invention, the oral levorphanol is in the form of multiparticulates can be dispersed in a matrix and compressed into a tablet.

In some preferred embodiments of the invention, the oral levorphanol is in a matrix that is in the form of pellets.

In some preferred embodiments of the invention, the oral levorphanol is in coated beads.

In some preferred embodiments, the dosage form of the invention comprises an oral formulation (e.g., tablet or capsule) which is coated to prevent substantial direct contact of levorphanol with oral cavity (e.g. tongue, oral mucosa), oropharyngeal mucosal surface, esophagus or stomach.In some preferred embodiments, the dosage form of the invention comprises an oral formulation which is coated with a film or polymer. In some preferred embodiments, the dosage form of the invention comprises levorphanol in an enteric coating.

In some preferred embodiments, the dosage form provides an oral extended release pharmaceutical composition comprising a therapeutically effective amount of levorphanol; said therapeutically effective amount in a reservoir comprising: (i) levorphanol or a pharmaceutically acceptable salt of levorphanol, or a mixture thereof; (ii) a membrane layer, said membrane being substantially permeable to levorphanol; wherein the dosage form substantially releases the levorphanol from the dosage form to render said dosage form suitable for extended release to a human patient.

In some preferred embodiments, the dosage form provides an oral extended release pharmaceutical composition comprising a plurality of pharmaceutically acceptable beads coated with a therapeutically effective amount of levorphanol or a pharmaceutically acceptable salt of levorphanol, or a mixture thereof; and overcoated with controlled release material to render said dosage form suitable for extended release oral administration to a human patient.

In some preferred embodiments, the dosage form provides an oral extended release pharmaceutical composition comprising (i) a drug layer comprising a therapeutically effective amount of levorphanol; and (ii) a displacement layer comprising an osmopolymer; and (b) a semipermeable wall surrounding the bilayer core having a passageway disposed therein for the release of said levorphanol or a pharmaceutically acceptable salt thereof; said dosage form suitable for extended release oral administration to a human patient.

In some preferred embodiments, the oral dosage form is a controlled release material suitable for extended release in a human patient of the dosage form comprises a matrix. In some preferred embodiments, the said matrix is a plurality of multiparticulate matrices. In some preferred embodiments, the multiparticulates are compressed into a tablet. In some preferred embodiments, the multiparticulates are disposed in a pharmaceutically acceptable capsule.

In some preferred embodiments, the extended release levorphanol dosage form of the invention is a solid dispersion. By reducing drug particle size and therefore improving drug wettability, the bioavailability may be substantially improved. Solid dispersions are usually presented as amorphous products, primarily made through two major different methods (e.g., melting and solvent evaporation). In addition, surfactants may be included to stabilize the dosage form in order to increase solubility and reduce recrystallization (see Vasconcelos et al, Drug Discovery Today, 2007; 12:1068-75, which is herein incorporated in its entirety by reference).

Applicant has now also surprisingly discovered that extended release dosage forms of levorphanol comprising waxes and vegetable oils and their esters and derivatives, when treated by application of heat at least up to their melting point and admixed with a thixotrope and a release rate modifier provides robust bioavailability and excellent extended release profiles suitable for up to once-a-day (Q24H).

Applicant has now also surprisingly discovered that extended release dosage forms of levorphanol comprising hydrogenated vegetable oils which are primarily lauric hard butter and lecithin, when treated by application of heat at least up to their melting point and admixed with a thixotrope and a release rate modifier provides robust bioavailability and excellent extended release profiles suitable for up to once-a-day (Q24H).

Applicant has now also surprisingly discovered that extended release dosage forms of levorphanol comprising other hydrogenated vegetable oils, when treated by application of heat at least up to their melting point, and admixed with a thixotrope and a release rate modifier, and further admixed medium chain triglycerides provides robust bioavailability and excellent extended release profiles suitable for up to once-a-day (Q24H).

Surprisingly, in some embodiment, oral levorphanol formulations of the invention containing Miglyol™ 812N, a fractionated coconut oil, provided more robust extended release formulations.

Surprisingly, in some embodiment, oral levorphanol formulations of the invention containing Miglyol™ 812N, a fractionated coconut oil, provided more robust extended release formulations with reduced potential for physical manipulation of the dosage form (dosage form tampering) and hence reduced potential for abuse.

Surprisingly, in some embodiment, oral levorphanol formulations of the invention containing Miglyol™ 812N, a fractionated coconut oil, imparted softness and "stickiness" to formulations that contained waxes which were naturally hard and potentially subject to grinding into powders, and hence subject both to friability and product tampering.

Surprisingly, in some embodiment, oral levorphanol formulations of the invention containing Miglyol 812N, a fractionated coconut oil added to the difficulty of attempted extraction and isolation of levorphanol using materials such as alcohols, probably by virtue of the solubility of oils in organic solvents.

In one embodiment of the present invention, the dosage form contains hydrogenated palm kernel oil (e.g., Hydrokote™ 112) in an amount not more than about 600 mg, or not more than about 500 mg, or not more than about 400 mg, or not more than about 350 mg, or not more than about 325 mg, or not more than about 300 mg, or not more than about 290 mg, or not more than about 280 mg, or not more than about 270 mg, or not more than about 260 mg, or not more than about 250 mg, or not more than about 240 mg, or not more than about 230 mg, or not more than about 220 mg, or not more than about 210 mg, or not more than about 200 mg, or not more than about 190 mg, or not more than about 180 mg, or not more than about 170 mg, or not more than about 160 mg, or not more than about 150 mg, or not more than about 140 mg, or not more than about 130 mg, or not more than about 120 mg, or not more than about 120 mg, or not more than about 110 mg, or not more than about 100 mg, or not more than about 90 mg, or not more than about 80 mg, or not more than about 70 mg, or not more than about 60 mg, or not more than about 50 mg, or not more than about 40 mg, or not more than about 30 mg, or not more than about 20 mg, or not more than about 10 mg.

In one embodiment of the present invention, the dosage form contains glyceryl behenate hydrogenated (Compritol™ 888 ATO), in an amount not more than about 600 mg, or not more than about 500 mg, or not more than about 400 mg, or not more than about 350 mg, or not more than about 325 mg, or not more than about 300 mg, or not more than about 290 mg, or not more than about 280 mg, or not more than about 270 mg, or not more than about 260 mg, or not more than about 250 mg, or not more than about 240 mg, or not more than about 230 mg, or not more than about 220 mg, or not more than about 210 mg, or not more than about 200 mg, or not more than about 190 mg, or not more than about 180 mg, or not more than about 170 mg, or not more than about 160 mg, or not more than about 150 mg, or not more than about 140 mg, or not more than about 130 mg, or not more than about 120 mg, or not more than about 120 mg, or not more than about 110 mg, or not more than about 100 mg, or not more than about 90 mg, or not more than about 80 mg, or not more than about 70 mg, or not more than about 60 mg, or not more than about 50 mg, or not more than about 40 mg, or not more than about 30 mg, or not more than about 20 mg, or not more than about 10 mg.

In one embodiment of the present invention, the dosage form contains hydrogenated cottonseed oil (e.g., Sterotex™ NF), in an amount not more than about 600 mg, or not more than about 500 mg, or not more than about 400 mg, or not more than about 350 mg, or not more than about 325 mg, or not more than about 300 mg, or not more than about 290 mg, or not more than about 280 mg, or not more than about 270 mg, or not more than about 260 mg, or not more than about 250 mg, or not more than about 240 mg, or not more than about 230 mg, or not more than about 220 mg, or not more than about 210 mg, or not more than about 200 mg, or not more than about 190 mg, or not more than about 180 mg, or not more than about 170 mg, or not more than about 160 mg, or not more than about 150 mg, or not more than about 140 mg, or not more than about 130 mg, or not more than about 120 mg, or not more than about 120 mg, or not more than about 110 mg, or not more than about 100 mg, or not more than about 90 mg, or not more than about 80 mg, or not more than about 70 mg, or not more than about 60 mg, or not more than about 50 mg, or not more than about 40 mg, or not more than about 30 mg, or not more than about 20 mg, or not more than about 10 mg.

In one embodiment of the present invention, the dosage form contains a fractionated coconut oil (e.g., Miglyol™ 812N), in an amount not more than about 600 mg, or not more than about 500 mg, or not more than about 400 mg, or not more than about 350 mg, or not more than about 325 mg, or not more than about 300 mg, or not more than about 290 mg, or not more than about 280 mg, or not more than about 270 mg, or not more than about 260 mg, or not more than about 250 mg, or not more than about 240 mg, or not more than about 230 mg, or not more than about 220 mg, or not more than about 210 mg, or not more than about 200 mg, or not more than about 190 mg, or not more than about 180 mg, or not more than about 170 mg, or not more than about 160 mg, or not more than about 150 mg, or not more than about 140 mg, or not more than about 130 mg, or not more than about 120 mg, or not more than about 120 mg, or not more than about 110 mg, or not more than about 100 mg, or not more than about 90 mg, or not more than about 80 mg, or not more than about 70 mg, or not more than about 60 mg, or not more than about 50 mg, or not more than about 40 mg, or not more than about 30 mg, or not more than about 20 mg, or not more than about 10 mg, or not more than about 5 mg.

In one embodiment of the present invention, the dosage form contains hydroxypropyl methyl cellulose (e.g, HPMC K15M), in an amount not more than about 600 mg, or not more than about 500 mg, or not more than about 400 mg, or not more than about 350 mg, or not more than about 325 mg, or not more than about 300 mg, or not more than about 290 mg, or not more than about 280 mg, or not more than about 270 mg, or not more than about 260 mg, or not more than about 250 mg, or not more than about 240 mg, or not more than about 230 mg, or not more than about 220 mg, or not more than about 210 mg, or not more than about 200 mg, or not more than about 190 mg, or not more than about 180 mg, or not more than about 170 mg, or not more than about 160 mg, or not more than about 150 mg, or not more than about 140 mg, or not more than about 130 mg, or not more than about 120 mg, or not more than about 120 mg, or not more than about 110 mg, or not more than about 100 mg, or not more than about 90 mg, or not more than about 80 mg, or not more than about 70 mg, or not more than about 60 mg, or not more than about 50 mg, or not more than about 40 mg, or not more than about 30 mg, or not more than about 20 mg, or not more than about 10 mg, or not more than about 5 mg.

In one embodiment of the present invention, the dosage form contains silicon dioxide (alone or in combination with Al₂O₃; e.g, Aerosil™, Aerosil™ 200, Aerosil™ COK84), in an amount not more than about 600 mg, or not more than about 500 mg, or not more than about 400 mg, or not more than about 350 mg, or not more than about 325 mg, or not more than about 300 mg, or not more than about 290 mg, or not more than about 280 mg, or not more than about 270 mg, or not more than about 260 mg, or not more than about 250 mg, or not more than about 240 mg, or not more than about 230 mg, or not more than about 220 mg, or not more than about 210 mg, or not more than about 200 mg, or not more than about 190 mg, or not more than about 180 mg, or not more than about 170 mg, or not more than about 160 mg, or not more than about 150 mg, or not more than about 140 mg, or not more than about 130 mg, or not more than about 120 mg, or not more than about 120 mg, or not more than about 110 mg, or not more than about 100 mg, or not more than about 90 mg, or not more than about 80 mg, or not more than about 70 mg, or not more than about 60 mg, or not more than about 50 mg, or not more than about 45 mg, or not more than about 40 mg, or not more than about 35 mg, or not more than about 30 mg, or not more than about 25 mg, or not more than about 20 mg, or not more than about 18 mg, or not more than about 15 mg, or not more than about 12 mg, or not more than about 10 mg, or not more than about 9 mg, or not more than about 8 mg, or not more than about 7 mg, or not more than about 6 mg, or not more than about 5 mg, or not more than about 4 mg, or not more than about 2 mg, or not more than about 1 mg.

In one embodiment of the present invention, release rate modifiers, including hydroxypropyl methyl cellulose (e.g, HPMC K15M) may be incorporated. Release rate modifiers can also have additional useful properties that optimize the formulation.

A variety of agents may be incorporated into the invention as thixotropes (e.g., fumed silicon dioxides, Aerosil™, Aerosil™ COK84, Aerosil™ 200, etc.). Thixotropes enhance the pharmaceutical formulations of the invention by increasing the viscosity of solutions complementing the action of HPMCs.

In some preferred embodiments, the inclusion of silicon dioxide (alone or in combination with Al₂O₃; e.g, Aerosil™, Aerosil™ 200, Aerosil™ COK84) is essential to provide the desired pharmaceutical properties of the extended release dosage form of oral levorphanol. Without being bound by theory: in some embodiments, the inclusion of silicon dioxide (alone or in combination with Al₂O₃; e.g, Aerosil™, Aerosil™ 200, Aerosil™ COK84) is essential to provide the desired pharmaceutical properties of the extended release dosage form of oral levorphanol, said silicon dioxide providing thixotropic properties, thereby providing extended release; in other embodiments, the inclusion of silicon dioxide (alone or in combination with Al₂O₃; e.g, Aerosil™, Aerosil™ 200, Aerosil™ COK84) is essential to provide the desired pharmaceutical properties of the extended release dosage form of oral levorphanol, said silicon dioxide providing thixotropic properties, said thixotrope providing a tamper or abuse deterrent dosage form; in other embodiments, the inclusion of silicon dioxide (alone or in combination with Al₂O₃; e.g, Aerosil™, Aerosil™ 200, Aerosil™ COK84) is essential to provide the desired pharmaceutical properties of the extended release dosage form of oral levorphanol, said silicon dioxide providing thixotropic properties, said thixtrope simultaneously providing a tamper or abuse deterrent dosage form and extended release; in some embodiments, the desired pharmaceutical properties of the extended release dosage form of oral levorphanol referred to in the foregoing requires the combination of hydroxypropyl methyl cellulose and silicon dioxide (alone or in combination with Al₂O₃; e.g, Aerosil™, Aerosil™ 200, Aerosil™ COK84).

In some preferred embodiments, the composition is a liquid filled thermosoftening extended release dispersion systems which provides an alternative dosage form and method and process of manufacture of extended release levorphanol suitable for dosing up to once-a-day (e.g., Q12H or Q24H) to a human patient. This dosage form is advantageously utilized to prepare a robust, cost effective formulation of the invention which (a) avoids one or more of the excipients in commercially available oral immediate-release levorphanol tartrate tablets and their associated adverse impact on the in vivo performance of the levorphanol; (b) substantially reduces or eliminates the need for pharmaceutical excipients generally recommended or considered necessary in a solid the dosage form (e.g. glidants, lubricants, diluents, fillers, binders, disintegrants and antioxidants), with associated cost, workplace safety and patient safety benefits; (c) is relatively easy to scale-up; and (d) requires a relatively simple manufacturing process, which may optionally be performed entirely in situ in a single heated mixing vessel prior to transfer to a filling machine for encapsulation.

In some preferred embodiments, said liquid filled thermosoftening extended release levorphanol dosage form comprises, in addition to levorphanol, hydrogenated vegetable oils. In some preferred embodiments, said hydrogenated vegetable oil is selected from the group comprising hydrogenated cottonseed oil, hydrogenated palm oil, hydrogenated soybean oil and hydrogenated palm kernel oil, or derivatives thereof or mixture thereof.

In some preferred embodiments, said liquid filled thermosoftening extended release levorphanol dosage form comprises, in addition to levorphanol, polyoxyethylene stearates and/or distearates. In some preferred embodiments, said polyoxyethylene stearates and/or distearates is selected from the group comprising polyoxyl 2, 4, 6, 8, 12, 20, 30, 40, 50, 100 and 150 stearates and polyoxyl 4, 8, 12, 32 and 150 distearates, or derivatives thereof or mixture thereof.

In some preferred embodiments, said liquid filled thermosoftening extended release levorphanol dosage form comprises, in addition to levorphanol, a wax. In some preferred embodiments, said wax is selected from the group comprising animal waxes, insect waxes, vegetable waxes, mineral waxes, petroleum waxes, synthetic waxes, nonionic emulsifying waxes, anionic emulsifying wax, carnauba wax, microcrystalline wax, yellow wax, white wax, cetyl esters wax, hydrogenated castor oil, lanolin alcohols, lanolin, glyceryl palmitostearate, cetostearyl alcohol, beeswax, stearoyl macrogolglycerides, Hawaiian waxes, hydrogenated jojoba oil, hydroxyoctacosanyl hydroxystearate, Japan wax, paraffin waxes and rice bran wax, or the respective esters, derivatives or mixture thereof.

In some preferred embodiments, said liquid filled thermosoftening extended release levorphanol dosage form comprises, in addition to levorphanol, a compound chosen from the group comprising glyceryl behenate, glyceryl palmitostearate and lauroyl macrogolglycerides, or mixtures thereof.

In some preferred embodiments, said liquid filled thermosoftening extended release levorphanol dosage form comprises, in addition to levorphanol, a compound chosen from the group comprising coconut oil, a coconut oil derivative, hydrogenated coconut oil, and fractionated coconut oil, or the respective esters, derivatives or mixture thereof.

In some preferred embodiments, said liquid filled thermosoftening extended release levorphanol dosage form comprises, in addition to levorphanol, a compound chosen from the group comprising glyceryl behenate, glycerol monostearate, hydrogenated vegetable oil, hydrogenated cottonseed oil, hydrogenated palm kernel oil, hydrogenated soybean oil, coconut oil, fractionated coconut oil, and beeswax, or the respective esters, derivatives or mixture thereof.

As used herein, "hydrogenated palm kernel oil" includes the composition of Hydrokote™ 112, lauric hard butter and lecithin. Hydrokote™ 112 has an INCI name of "hydrogenated palm kernel oil" and a standard lauric hard butter and lecithin derived from edible vegetable oils, which has a CAS number of 84540-04-5 or 68334-28-1 and an EINECS number of 273-627-2 or 283-093-2.

As used herein, "hydrogenated cottonseed oil" includes the composition of Sterotex™ and Sterotex™ NF. Sterotex™ are neutral organic esters (triglycerides) that conforms to the USP/NF monograph for Hydrogenated Vegetable Oil, Type I and the BP monograph for Hydrogenated Vegetable Oil, has a CAS number of 68334-00-9 and an EINECS number of 269-804-9 and goes by the synonym hydrogenated cottonseed oil and powdered vegetable stearine.

As used herein, "hydrogenated soybean oil" includes the composition of Sterotex™ HM and Sterotex™ HM, NF. Sterotex™ HM has a CAS number of 8016-70-4 and an EINECS number of 232-410-2, goes by the synonyms hydrogenated soybean oil and powdered vegetable stearine, are neutral organic esters (triglycerides), and meets the requirements of the USP/NF monograph for Hydrogenated Vegetable Oil, Type 1 and the BP monograph for Hydrogenated Vegetable Oil.

As used herein, "glycerol monostearate" includes all glyceryl monoesters, including the compositions of Cithrol™ GMS (glyceryl stearate), Cithrol™ GMS S/E (a self-emulsifying glyceryl monoester), Cithrol™ GMS N/E, Cithrol™ GMS A/S (an acid stable glyceryl monoester with PEG-100 Stearate) and Cithrol GMO (glyceryl oleate).

As used herein, "glyceryl behenate" includes the composition of Compritol™ 888 and comprises is a mixture of glycerides of fatty acids, mainly behenic acid, with a 1-monoglycerides content between 12.0 and 18.0% (USP/NF-23) or a mixture of diacylglycerols, mainly dibehenoylglycerol, together with variable quantities of mono- and triacylglycerols (PhEur 2005 (Suppl. 5.1)).

Fractionated coconut oil is a fraction in which the long-chain fatty acids are removed so that it consists substantially of medium chain saturated fatty acids. It is sometimes referred to as caprylic/capric, triglyceride oil or medium chain triglyceride oil because it is substantially comprised of the medium chain caprylic (C₈) and capric (C₁₀) acids.

Unless specifically modified, as used herein, the terms "coconut oil" and "coconut oil derivative" comprise one or more of the following, regardless of the plant source or the synthetic source: (i) the compositions of Miglyol™ 810, Miglyol™ 812, Miglyol™ 818 and Miglyol™ 829; (ii) coconut oil, hydrogenated coconut oil, fractionated coconut oil, caproic acid, caprylic acid, capric acid, lauric acid, myristic acid, palmitic acid, stearic acid, arachidic acid, oleic acid, medium-chain triglycerides or related esters, triglycerides (Akomed™ E, Akomed™ R, Miglyol™ 810, and Captex™ 355, Labrafac™ CC), propylene glycol diester of caprylic and/or capric acid (Labrafac™ PG), propylene glycol monolaurate (Lauroglycol™ FCC), medium-chain diesters of propylene glycols (Miglyol™840), partial ester of diglycerides with natural fatty acids (Softisan™ 645), and linoleic acid; and (iii) oil of any source substantially comprising medium chain triglyceride (C₆ to C₁₂). Preferably, the coconut oil is substantially consists of medium chain triglycerides (C₆ to C₁₂), more (C₆ to C₁₀).

In one preferred embodiment of the present invention, also included in the dosage form are coconut oil products, including without limitation and regardless of plant source or synthetic source, coconut oil, fractionated coconut oil, cetyl alcohol, lauric acid and medium chain triglycerides (e.g., Miglyol™ 810, Miglyol™ 812 are triglycerides of the fractionated plant fatty acids C₈ and C₁₀ which meet the requirements of the European Pharmacopeia (4.6) and USP/NF(22) as Medium Chain Triglycerides, the British Pharmacopoeia (1999) as Fractionated Coconut Oil and the Japanese Pharmaceutical Excipients (JPE) as Caprylic/Capric Triglyceride.

Miglyol™ 818 is a triglyceride of the fractionated plant fatty acids C₈ und C₁₀ and contains about 4 to 5 % linoleic acid.

Miglyol™ 829 is a glycerin ester of the fractionated plant fatty acids C₈ und C₁₀, combined with succinic acid.

Miglyol™ 840 is a propylene glycol diester of saturated plant fatty acids with chain lengths of C₈ and C₁₀.

In some preferred embodiments, the extended release levorphanol dosage form comprises, in addition to levorphanol, a vegetable oil or an esters or derivative of the vegetable oil, or mixture thereof. Any pharmaceutically acceptable ester of a vegetable oil may be used to practice the invention.

In some preferred embodiments, the extended release levorphanol dosage form comprises, in addition to levorphanol, a vegetable oil which is a hydrogenated vegetable oil, or an esters or derivative of the vegetable oil derived made from andiroba seed oil, apricot kernel oil, arachis oil, argane oil, artemisia oil, avocado butter, avocado oil, babassu oil, baobab oil, bitter cherry kernel oil, black cumin seed oil, black currant seed oil, black walnut oil, borage (starflower) oil, brazil nut oil, buriti oil, camellia oil, cape chestnut oil, cashew nut oil, castor oil, castor oil (sulfated), chaulmoogra oil, chia oil, chufa oil, cocoa butter, coconut oil, coffee bean oil, corn oil, cotton seed oil, cranberry seed oil, cupuacu butter, echium seed oil, evening primrose oil, gold of pleasure oil, grape seed oil, grapefruit seed oil, hazelnut oil, hemp oil, horseradish tree (Moringa) oil, illipe butter, kiwi seed oil, kokum butter, kukui nut oil, lime seed oil, linseed oil, london rocket oil, macadamia nut oil, mango butter, manketti nut oil, marigold seed oil, marula oil, meadowfoam seed oil, mobola plum oil, murumuru butter, neem oil, ngali nut oil, olive oil, palm kernel oil, palm oil, papaya seed oil, passion fruit, seed oil, peach kernel oil, peanut oil, pecan oil, pequi oil, perilla seed oil, pistachio nut oil, plum kernel oil, pomegranate seed oil, poppy seed oil, pumpkin seed oil, rape seed oil, raspberry seed oil, rice bran oil, rose hip oil (dog rose), rose hip oil (sweet brier), safflower oil, scarlet strawberry seed oil, sea buckthorn oil, seaside plum oil, sesame oil, shea butter, shorea robusta butter, soybean oil, sunflower oil, sweet almond oil, sweet cherry kernel oil, sweet orange seed oil, tamanu oil, walnut oil, watermelon seed oil (ootanga), wheatgerm oil and white mustard seed oil.

In some preferred embodiments, the extended release levorphanol dosage form comprises, in addition to levorphanol, a fatty acid derived from any vegetable or synthetic oil or fat, including caproic, caprylic, coconut, isostearic, lauric, myristic, oleic, palmitic and stearic acids.

In some preferred embodiments, the extended release levorphanol dosage form comprises, in addition to levorphanol, a fatty acid ester derived from any vegetable or synthetic oil or fat.

In some preferred embodiments, the extended release levorphanol dosage form comprises, in addition to levorphanol, a fat alcohol derived from any vegetable or synthetic fat, including behenyl, cetearyl, cetyl, isopropyl, isostearyl, lanolin, oleyl and stearyl alcohol.

Any amount or combination of vegetable oil, hydrogenated vegetable oils, coconut oil, polyoxyethylene stearates and/or distearates, wax, or wax like compound may be included in the dosage form. Preferably, said compound represents about 0.001% to about 99% of the dosage form, more preferably, about 5% to about 95%, or about 10% to about 95%, or about 15% to about 95%, or about 20% to about 95%, or about 5% to about 85%, or about 5% to about 80%, or about 5% to about 70%, or about 10% to about 80%, or about 20% to about 80%, or about 30% to about 80%, or about 40% to about 80%, or about 50% to about 80%, each on on a %w/w basis. In one preferred embodiment of the present invention, also included in the dosage form are solubilizing agents, surfactants, emulsifying agents and other excipients selected from the group comprising Polyglycerol dioleate (Plurol Oleique™ CC 497), polyoxyethylene-polyoxypropylene copolymer (Poloxamer™ 124 and 188), partial glycerides of hydroxylated unsaturated fatty acids (Softigen™ 701), PEG-6 caprylic/capric glycerides (Softigen™ 767), polyoxyethylene glyceryl trioleate (Tagat™ TO), polyoxyethylene(20)sorbitan monooleate (Tween™ 80), propylene glycol monocaprylate (Capryol™ 90), polyglycolized glycerides (Gelucire™ 44/14 and 50/13), polyoxyl-40 hydrogenated castor oil (Cremophor™ RH 40), glycerol monostearate/di-triglycerides with glycerin(Imwitor™ 191), isosteryl diglyceryl succinate (Imwitor™ 780 K), glyceryl cocoate (Imwitor™ 928), glyceryl caprylate (Imwitor™ 988), oleoyl macrogol-8 glycerides (Labrafil™ M 1944 CS), linoleoyl macrogolglycerides (Labrafil™ M 2125 CS), PEG-8 caprylic/capric glycerides (Labrasol™), glyceryl monocaprylate (Imwitor® 308), glyceryl cocoate/citrate/lactate (Imwitor™ 380), glyceryl mono-di-caprylate/caprate (Imwitor™ 742), lauric acid, propylene glycol laurate (Lauroglycol™ 90), oleic acid and PEG MW > 4000.

In some embodiments, the invention comprises a liquid filled oral pharmaceutical composition comprising: (a) levorphanol; (b) one or more material selected from the group consisting of glycerol monostearate, hydrogenated vegetable oil, hydrogenated cottonseed oil, hydrogenated palm kernel oil, hydrogenated soybean oil, hydrogenated coconut oil, fractionated coconut oil, and beeswax; (c) a thixotrope selected from the group consisting of amorphous anhydrous colloidal silicon dioxide, silicon dioxide, silca, fumed silicon dioxides, or a mixture of silicon dioxide and aluminum oxide, aluminum oxide, microcrystalline methylcellulose, bentonite clay, hectorite clay, magnesium aluminum silicate, lithium magnesium silicate, magnesium silicate, silicates, clays, metal oxides, aluminum hydroxide, magnesium hydroxide, carbon black, stearalkonium hectorite, kaolin, and montmorillonite; (d) a release rate modifier selected from the group consisting of hydroxypropyl methylcellulose, cellulose acetate, microcrystalline cellulose, powdered cellulose, cellulose acetate phthalate, hydroxyethyl cellulose, silicified microcrystalline cellulose, hydroxypropyl cellulose, hydroxyethylmethyl cellulose, low-substituted hydroxypropyl cellulose, carboxymethylcellulose, carboxymethylcellulose calcium, hypromellose acetate succinate, hypromellose phthalate and ethylcellulose; and optionally, (e) one or more pharmaceutically acceptable excipients; wherein (b) is dispensed into a mixer heated until fully melted, (d) is dispensed into the same heated mixer and mixed with (b) until dispersed, (c) is dispensed into the same heated mixer and mixed with (b) and (d) until dispersed, (e) is dispensed into the same heated mixer and mixed with (b), (c) and (d) until dispersed, (a) is dispensed into the same heated mixer with (b), (c), (d) and (e) and mixed thoroughly with a high shear mixer; wherein said mixture is substantially uniformly dispersed; wherein the mixed liquid mass comprising (a), (b), (c), (d) and (e) are transferred into a liquid filling machine and filled into capsules; wherein the liquid mass becomes a solid when allowed to cool to room temperature following encapsulation; wherein the liquid mass comprising (a), (b), (c), (d) and (e) is optionally prepared *in situ* in a single heated mixing vessel prior to transfer to a filling machine for encapsulation. In some embodiments, the capsule is coated with or incorporates controlled release material (e.g., a pH sensitive polymer) to render it delayed onset, extended release.

In some embodiments, said liquid filled thermosoftening extended release levorphanol dosage form comprises, in addition to levorphanol and hydrogenated vegetable oils, polyoxyethylene stearates and/or distearates, wax, or wax like compound, the coconut oil or a coconut oil derivative in amount of up to about 50%, or 40%, or 35%, or 30%, or 25%, or 20%, or 15%, or 10%, or 5% of the dosage form on a w/w basis. The benefit of incorporating a coconut oil or a coconut oil derivative as an additional compound are described herein.

In some embodiments of the present invention, a release rate modifier may be incorporated into the dosage form to impart optimized release rate properties. In some embodiments of the invention, the release rate modifiers is selected from the group comprising hydroxypropyl methylcellulose, cellulose acetate, powdered cellulose, cellulose acetate phthalate, hydroxyethyl cellulose, silicified microcrystalline cellulose, hydroxypropyl cellulose, hydroxyethylmethyl cellulose, low-substituted hydroxypropyl cellulose, carboxymethylcellulose, carboxymethylcellulose calcium, hypromellose acetate succinate, hypromellose phthalate, and ethylcellulose, and mixture thereof. Release rate modifiers can also have additional useful properties that optimize the formulation, including the surprising property of tamper resistance.

In some embodiments, the release rate modifier is in an amount of up to 80%, or 70%, or 60%, or 50%, or 40%, or 30%, or 20%, or 10% on %w/w basis, preferably, up to 45%, or 40%, or 35%, or 30%, or 25%, or 20%, or 15% on %w/w basis.

In some embodiments, the release rate modifier is in an amount of up to about 200 mg, or 150 mg, or 120 mg, or 100 mg, or 80 mg, or 60 mg, or 40 mg or 20 mg.

In some embodiments, a preferred release rate modifier is hydroxypropyl methyl cellulose (e.g, HPMC K15M). Preferably, the HPMC is of a grade containing 16.5% to 30% methoxy and 4% to 32% hydroxypropyl groups. Preferably, a 2% (w/v) aqueous solutions of the hydroxypropyl methylcellulose has a viscosity of about 4 to about 300,000 Centipoise at 20 °C, said viscosity is more preferably about 2000 to about 150,000 Centipoise, even more preferably, about 4000 to about 120,000 Centipoise, and most preferably, about 10000 to about 120,000 Centipoise.

In some embodiments, the HPMC is in an amount of up to 80%, or 70%, or 60%, or 50%, or 40%, or 30%, or 20%, or 10% on %w/w basis, preferably, up to 45%, or 40%, or 35%, or 30%, or 25%, or 20%, or 15% on %w/w basis.

In some embodiments, the HPMC is in an amount of up to about 200 mg, or 150 mg, or 120 mg, or 100 mg, or 80 mg, or 60 mg, or 40 mg or 20 mg.

A variety of agents may be incorporated into the invention as thixotropes (e.g., amorphous anhydrous colloidal silicon dioxide, silicon dioxide, silca, fumed silicon dioxides, or a mixture of silicon dioxide and aluminum oxide, aluminum oxide, microcrystalline methylcellulose, bentonite clay, hectorite clay, magnesium aluminum silicate, lithium magnesium silicate, magnesium silicate, aluminum hydroxide, magnesium hydroxide, carbon black, stearalkonium hectorite, kaolin, and montmorillonite which have silica, silicate or silicon dioxide groups on their surface.

### Thixotropes

Fluids can generally be described as Newtonian or non-Newtonian. Liquid dispersions containing thixotropes or thixotropic material are a particular case of non-Newtonian fluids. Newtonian fluids are simple liquids that generally consist of small molecules and solutions where solute and solvent are show a linear relationship between the rate of flow or shear and the applied shear stress at a constant viscosity. In other words, the viscosity of Newtonian fluids depends only on composition, temperature and pressure. The viscosity increases moderately with increasing pressure and considerably with decreasing temperature. Fluids that deviate from Newton's law are called non-Newtonian fluids. Such fluids may be generally categorized as plastic, pseudoplastic or dilatant. Plastic fluids do not move until the applied shear stress exceeds a certain minimum value. Fluids are generally plastic due to the formation of a network structure throughout the fluid. This network needs to be disrupted before flow can begin. Pseudoplastic fluids (or shear thinning fluids) have flow immediately after application of shear stress and their apparent viscosity decreases with increased application of shear stress. Unlike fluids that exhibit Pseudoplastic flow, dilatant fluids are shear thickening, i.e., they show increased apparent viscosity with increasing shear stress. In the foregoing description of Newtonian and non-Newtonian fluids, repeated testing can be expected to produce the same results, i.e., the same rate of shear or viscosity with the same shear stress. In other words, the viscosity is independent of the prior history of the solution. In contrast, thixotropes do not follow this general rule. When mixed with fluids, thixotropes are material whose aqueous dispersions show non-Newtonian behavior but whose consistency depends on the duration of shear as well as on the rate of shear. Their apparent viscosity depends, in addition to temperature, composition and rate of shear stress, on the previous shear history and time under shear. For example, an aqueous dispersion containing an effective amount of a thixotropic material may set to gel within three hours after it is has been prepared when undisturbed, but may flow and be pourable within minutes after it has been stirred above a particular yield value. After a period of prolonged rest, the aqueous dispersion reverts to a gel as it rebuilds a three-dimensional gel-like structure. As used herein, a "thixotrope" is a non-toxic compound which when tested at one or more concentrations or in one or more amounts after incorporation into a fluid (e.g., a liquid dispersion) exhibits thixotropy, including, without limitation and without being bound by theory, compounds chosen from the group comprising a thixotrope selected from the group consisting of amorphous anhydrous colloidal silicon dioxide, silicon dioxide, silca, fumed silicon dioxides, or a mixture of silicon dioxide and aluminum oxide, aluminum oxide, microcrystalline methylcellulose, bentonite clay, hectorite clay, magnesium aluminum silicate, lithium magnesium silicate, magnesium silicate, silicates, clays, metal oxides, aluminum hydroxide, magnesium hydroxide, carbon black, stearalkonium hectorite, kaolin, and montmorillonite. As used herein, "thixotropy" is a property exhibited by a liquid material if, when sheared at a constant rate, its apparent viscosity (or corresponding shear stress) decreases with time of shearing (see, Remington: The Science and Practice of Pharmacy, 21st ed, Lippincott Williams & Wilkins (2005); Kim C-J. Advanced Pharmaceutics: Physicochemical Principles, CRC Press (2004); Chhabra RP and Richardson JF, Non-Newtonian Flow and Applied Rheology: Engineering Applications, Butterworth-Heinemann (2008). Thixotropy may be measured using established equipment, software and methods, for example, certain viscometers such as R/S Plus™ Rheometers with a mounted spiral adapter running Rheo300™ software from Brookfield Engineering Laboratories, Inc., Middleboro, MA.

A variety of agents may be incorporated into the invention as thixotropes (e.g., amorphous anhydrous colloidal silicon dioxide, silicon dioxide, silca, fumed silicon dioxides, or a mixture of silicon dioxide and aluminum oxide, aluminum oxide, microcrystalline methylcellulose, bentonite clay, hectorite clay, magnesium aluminum silicate, lithium magnesium silicate, magnesium silicate, silicates, clays, metal oxides, aluminum hydroxide, magnesium hydroxide, carbon black, stearalkonium hectorite, kaolin, and montmorillonite which have silica, silicate or silicon dioxide groups on their surface.

Thixotropes enhance the pharmaceutical formulations of the invention by increasing the viscosity of solutions during attempted extraction, complementing the action of HPMCs. They may also provide a tamper resistance by helping to retain the structure of dosage units that have been heated to temperatures greater than the melting point of the base excipient (Aerosils™ are unaffected by heat). Preferably, the thixotrope is silicon dioxide, silca, fumed silicon dioxides, a mixture of silicon dioxide and aluminum oxide, or aluminum oxide.

In some embodiments, the thixotrope is in an amount of up to about 200 mg, or 150 mg, or 120 mg, or 100 mg, or 80 mg, or 60 mg, or 40 mg or 20 mg.

In some embodiments, the thixotrope is in an amount of up to about 40%, or 30%, or 20%, or 15%, or 12%, or 10%, or 8%, or 6%, or 5%, or 4%, or 3%, or 2%, or 1%, or 0.1%, or 0.1% of the dosage form on a % w/w basis, preferably up to about 10% or 8% on a % w/w basis, more preferably, up to about 6%, or 5%, or 4%, or 3% on a % w/w basis.

In some embodiments, the silicon dioxide, silca, fumed silicon dioxides, mixture of silicon dioxide and aluminum oxide, or aluminum oxide is in an amount of up to about 200 mg, or 150 mg, or 120 mg, or 100 mg, or 80 mg, or 60 mg, or 40 mg or 20 mg.

In some embodiments, the silicon dioxide, silca, fumed silicon dioxides, mixture of silicon dioxide and aluminum oxide, or aluminum oxide is in an amount of up to about 40%, or 30%, or 20%, or 15%, or 12%, or 10%, or 8%, or 6%, or 5%, or 4%, or 3%, or 2%, or 1%, or 0.1%, or 0.1% of the dosage form on a % w/w basis, preferably up to about 10% or 8% on a % w/w basis, more preferably, up to about 6%, or 5%, or 4%, or 3% on a % w/w basis.

In some embodiments, the thixotrope is in an amount of up to about 200 mg, or 150 mg, or 120 mg, or 100 mg, or 80 mg, or 60 mg, or 40 mg or 20 mg.

### Delayed Onset Extended Release Levorphanol Formulations

In some preferred embodiments, the dosage form provides a pharmaceutical dosage form comprising a therapeutically effective amount of levorphanol, said dosage form resistant or substantially resistant to dissolution and/or absorption in the stomach, and/or in the duodenum, and/or in the jejunum, and/or in the ileum, or in the small intestine, or in the stomach and duodenum, or in the stomach, duodenum and jejunum, or in the stomach, duodenum, jejunum and terminal ileum, or in the stomach and small intestine, or before it reaches the ileo-cecal junction, or until it crosses the ileo-cecal junction, or until it reaches the colon; said levorphanol in the dosage form released rapidly or slowly upon reaching a the desired anatomic region of the GI tract (e.g., ileum or colon) or upon reaching the desired gastrointestinal conditions conducive to release from the dosage form (e.g., osmotic pressure, pH, time after ingestion, microbial flora); said dosage form in some embodiments providing immediate release of levorphanol following the expected lag time; said dosage form in some other embodiments providing sustained release of levorphanol following the expected lag time.

In embodiments, the delayed onset, extended release levorphanol dosage form provides abuse resistant properties in opioid abusers and in individuals with an opioid addiction disorder or a polysubstance addiction disorder.

In some embodiments, the delayed onset, extended release levorphanol dosage form is deters against PRN or as needed use of the drug by providing suboptimal efficacy upon PRN use but robust efficacy upon scheduled, around the clock of continuous use.

In embodiments, the delayed onset, extended release levorphanol dosage form provides resistance to alcohol associated dose dumping.

In embodiments, the delayed onset, extended release levorphanol dosage form provides minimizes pharmacokinetic when taken in the fed versus fasted state.

In some embodiments, the delayed onset, extended release levorphanol dosage form is substantially non-releasable until up to about 1, or 1.5, 2, or 2.25, or 2.5, or 2.75, or 3, or 3.25, or 3.5, or 3.75, or 4, or 4.25, or 4.5, or 4.75, or 5, or 5.25, or 5.5, or 5.75, or 6, or 6.25, or 6.5, or 7.75, or 7, or 7.25, or 7.5, or 7.75, or 8, or 8.25, or 8.5, or 8.75, or 9, or 9.25, or 9.5, or 9.75, or 10, or 10.25, or 10.5, or 10.75, or 11, or 11.25, or 1.5, or 11.75, or 12, or 14, or 16, or 18, or 20 hours after oral ingestion of the oral dosage form. In some particularly preferred embodiments, said dosage form is substantially non-releasable until up to about 2.5, or 2.75, or 3, or 3.25, or 3.5, or 3.75, or 4, or 4.25, or 4.5, or 4.75, or 5, or 5.25, or 5.5, or 5.75, or 6, or 6.25, or 6.5, or 7.75, or 7 hours after oral ingestion of the oral dosage form.

In some preferred embodiments, the delayed onset, extended release levorphanol dosage form is a coated capsule or tablet wherein the coating comprises material which dissolves at a pH ≥ 5, or ≥ 5.5, or ≥ 5.7, or ≥ 6, or ≥ 6.2, or ≥ 6.4, or ≥ 6.6, or ≥ 6.8, ≥ 7, or ≥ 7.2. In some other preferred embodiments, the delayed onset, extended release levorphanol dosage form includes material incorporated in dosage form, wherein the material substantially resists dissolution for at least about 1, or 1.5, or 2, or 2.5, or 3, or 3.25, or 3.5, or 3.75, or 4, or 4.25, or 4.5, or 4.75, or 5, or 5.25, or 5.5, or 5.75, or 6, or 6.25, or 6.5, or 7.75, or 7 31 hours at a pH of about ≤ 5, or ≤ 5.5, or ≤ 5.7, or ≤ 6, or ≤ 6.2, or ≤ 6.4, or ≤ 6.6, or ≤ 6.8, ≤ 7, or ≤ 7.2.

In some embodiments, the delayed onset, extended release levorphanol dosage form is coated with or includes incorporated one or more of the following: (i) cellulose acetate trimellitiate (CAT); (ii) hydroxypropylmethyl cellulose phthalate (HPMCP); (iii) polyvinyl acetate phthalate (PVAP); (iv) shellac; (v) a copolymer of methacrylic acid and methylmethacrylate; (vi) a material which is redox-sensitive; (vii)an azopolymer or a disulphide polymer; (viii) a material which is degraded by enzymes or bacteria present in the colon; (ix) a copolymer of methacrylic acid and methylmethacrylate to which has been added during polymerization the monomer methyl acrylate; (x) a cellulose ester; (xi) polyvinyl acetate phthalate.

In some embodiments, the dosage form consists of a coated capsule wherein the coating is applied separately to empty capsule body and cap. In some embodiments, the dosage form consists of a coated capsule filled with a caplet or tablet.

In some embodiments, the dosage form is coated with a film or incorporates material which makes the delayed onset, extended release the dosage form: (i) is non-dissolving at pH<3 to 4 and dissolving at pH>5; or (ii) non-dissolving at pH<3 to 4 and dissolving at pH>5.5; or (iii) non-dissolving at pH<3 to 4 and dissolving at pH>6; or (iv) non-dissolving at pH<3 to 4.5 and dissolving at pH>6; or (v) non-dissolving at pH<3 to 4 and dissolving at pH>6.5; or (vi) non-dissolving at pH<3 to 4.5, and dissolving at pH>6.5; or (vii) non-dissolving at pH<3 to 4 and dissolving at pH>7; or (viii) non-dissolving at pH<3 to 4.5 and dissolving at pH>7; or (ix) is non-dissolving at pH<3 to 5 and dissolving at pH>7; or (x) non-dissolving at pH<3 to 5.5 and dissolving at pH>7.

In some embodiments, the delayed onset, extended release levorphanol dosage form is non-dissolving or substantially non-dissolving at pH < 5.5, or at pH < 6.0, or at pH < 6.2, or at pH < 6.5, or at pH < 6.8, or at pH < 7.0, when measured by USP Basket Method or USP Paddle Method at 100 rpm in 900 mL of water at 37 °C (adjusted to the required pH with hydrochloric acid or sodium hydroxide) for up to about 2, 2.5, 3, 3.5, 4, 4.5, or 5.

In some embodiments, the delayed onset, extended release levorphanol dosage form is non-releasing or substantially non-releasing at pH < 5.5, or at pH < 6.0, or at pH < 6.2, or at pH < 6.5, or at pH < 6.8, or at pH < 7.0, when measured by USP Basket Method or USP Paddle Method at 100 rpm in 900 mL of water at 37 °C (adjusted to the required pH with hydrochloric acid or sodium hydroxide) for up to about 2, 2.5, 3, 3.5, 4, 4.5, or 5.

In some embodiments, the delayed onset, extended release levorphanol dosage form comprises material which is non-dissolving or substantially non-dissolving at a particular pH or range of pH and is dissolving or substantially dissolving at another pH or another range of pH, said material (i) commingled with the levorphanol API or with the granulation containing levorphanol API; or (ii) commingled with the levorphanol API or with the granulation containing levorphanol API, in addition to being coated on the dosage form.

In some embodiments, the specifications regarding coating of the delayed onset, extended release the dosage form of the invention with controlled release material or pH sensitive material are also applicable to dosage forms where said material is commingled with the levorphanol API or with the granulation containing levorphanol API, instead of or in addition to coating the dosage form.

In some embodiments, the specifications regarding coating of the delayed onset, extended release the dosage form of the invention with controlled release material or pH sensitive material are also applicable to dosage forms where the coating is applied to multiparticulate matrices or to subunits of the dosage form e.g., beads incorporating drug), instead of or in addition to coating the dosage form.

In some embodiments, the delayed onset, extended release the dosage form consists of a coated capsule wherein the coating is applied to capsules having a seal on the gap between capsule body and cap.

In some embodiments, the delayed onset, extended release the dosage form consists of a coated capsule containing levorphanol, wherein the capsule is coated with a material selected from the group comprising cellulose acetate trimellitiate, hydroxypropylmethyl cellulose phthalate, polyvinyl acetate phthalate, shellac, and a copolymer of methacrylic acid and ethyl acrylate, azopolymers, disulfide polymers and amylose.

In some embodiments, the delayed onset, extended release levorphanol dosage form releases less than about 0.1%, or 0.5%, or 1%, or 1.5%, or 2%, or 2.5%, or 3%, or 3.5%, or 4%, or 4.5%, or 5%, or 6%, or 7%, or 8%, or 9%, or 10%, or 12%, or 14%, or 15%, or 16%, or 17%, or 18%, or 20% of levorphanol in vitro from the dosage form when measured at about 1, 1.5, 2, 2.5, 3, 3.25, 3.5, 3.75, 4, 4.25, 4.5, 4.75, 5, 5.5, 6, 6.5, 7, 7.5, 8, 8.5, 9, 9.5, 10, 10.5, 10.5, 11, 11.5, or 12 hours, said in vitro release measured by the USP Basket or Paddle Method at 100 rpm in 100 to 900 mL in one or more of the following: (a) water at 37 °C at a pH of 4.5, adjusted with HCl; (b) water at 37 °C at a pH of 5, adjusted with HCl; (c) water at 37 °C at a pH of 5.5, adjusted with HCl; (d) simulated gastric fluid at 37 °C; (e) simulated intestinal fluid at 37 °C; (f) simulated gastric fluid at 37 °C for one hour followed by a switch to simulated intestinal fluid; (g) Phosphate buffer 0.067M (pH 7.0) at 37 °C; and (h) Phosphate buffer 0.067M (pH 7.0) containing Tween 80 at 37 °C. In some embodiments, said in vitro dissolution is measured by the USP Apparatus III (Reciprocating Cylinder) Method instead of the Basket or Paddle Method. In some embodiments, most preferably, the dosage form releases less than about 0.1%, or 0.5%, or 1%, or 1.5%, or 2%, or 2.5%, or 3%, or 3.5%, or 4%, or 4.5%, or 5%, or 6%, or 7%, or 8%, or 9%, or 10 of levorphanol in vitro from the dosage form when measured at about 1.5, 2, 2.5, 3, 3.5, 4, 4.5, 5, 5.5, 6, 6.5, or 7 hours.

In some embodiments, the delayed onset, extended release levorphanol dosage form releases 0%, or less than about 0.1%, or 0.5%, or 1%, or 1.5%, or 2%, or 2.5%, or 3%, or 3.5%, or 4%, or 4.5%, or 5%, or 6%, or 7%, or 8%, or 9%, or 10%, or 12%, or 14%, or 15%, or 16%, or 17%, or 18%, or 20% or 25% or 28% or 30% or 35% of levorphanol in vivo from the dosage form prior to reaching the duodenum, or jejunum, or ileum, or terminal ileum, or ileo-cecal junction, or ascending colon, or transverse colon, or descending colon, or colon

In some embodiments, the targeted gastrointestinal delivery of the levorphanol from the oral extended release levorphanol dosage form into the lower segments of the gastrointestinal tract can be achieved through a variety of approaches, including but limited to incorporation of material or processes to achieve one or more of the following: time-controlled, pH-controlled, pressure-controlled, enzyme-controlled and hydration-controlled. Since the gastrointestinal tract is a complex, variable and highly dynamic environment and further complicated by the volume, content and location of food and beverages, in some embodiments, incorporation of material to achieve more than one of the above approaches is preferred.

In some embodiments, the targeted gastrointestinal delivery of the levorphanol from the oral extended release levorphanol dosage form into the lower segments of the gastrointestinal tract can be achieved through encapsulation of the levorphanol, preferably with excipients or functional excipients, said capsule incorporating, coated with or overcoated with material or processes to achieve targeted gastrointestinal delivery.

In some embodiments, the oral extended release levorphanol dosage form is coated with a material or incorporates material which is non-dissolving or substantially resistant to dissolution, each when measured by USP Basket Method or USP Paddle Method at 100 rpm in 900 mL of water at 37 °C (adjusted to the required pH with hydrochloric acid or sodium hydroxide) at about pH 2, pH 2.2, pH 2.4, pH 2.6, pH 2.8, pH 3, pH 3.2, pH 3.4, pH 3.6, pH 3.8, pH 4, pH 4.2, pH 4.4, pH 4.6, pH 4.8, pH 5, pH 5.2, pH 5.4, pH 5.6, pH 5.8, pH 6, pH 6.2, pH 6.4, pH 6.6, pH 6.8, pH 7, pH 7.2, pH 7.4, for up to about 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 14, 16, 18, or 20 hours.

In some embodiments, the oral extended release levorphanol dosage form is coated with a material or incorporates material which is non-releasing or substantially non-releasing, each when measured by USP Basket Method or USP Paddle Method at 100 rpm in 900 mL of water at 37 °C (adjusted to the required pH with hydrochloric acid or sodium hydroxide) at about pH 2, pH 2.2, pH 2.4, pH 2.6, pH 2.8, pH 3, pH 3.2, pH 3.4, pH 3.6, pH 3.8, pH 4, pH 4.2, pH 4.4, pH 4.6, pH 4.8, pH 5, pH 5.2, pH 5.4, pH 5.6, pH 5.8, pH 6, pH 6.2, pH 6.4, pH 6.6, pH 6.8, pH 7, pH 7.2, pH 7.4, for up to about 0.5, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 14, 16, 18, or 20 hours.

In some embodiments, the invention provides a delayed onset, extended release oral pharmaceutical compositions of levorphanol, said compositions in extended release form, said dosage form releasing most, substantially all or all of the releasable levorphanol in the lower segment of the gastrointestinal tract (e.g., distal to the duodenum, or jejunum, or ileum, or terminal ileum, or ileo-cecal junction, ascending colon, or transverse colon), said release occurring over about 4, 5, 6, 7, 8, 9, 10, 12, 14, 15, 16, 17, 18, 19, 20, 21, 22, 23, 24, 25, 26, 30, 32, 36 or 40 hours.

In some embodiments, the invention provides a delayed onset, extended release oral pharmaceutical compositions of levorphanol, said compositions in extended release form, said dosage form releasing most, substantially all or all of the releasable levorphanol in the lower segment of the gastrointestinal tract (e.g., distal to the duodenum, or jejunum, or ileum, or terminal ileum, or ileo-cecal junction, ascending colon, or transverse colon), said release occurring over about 0.25, 0.5, 0.75, 1, 1.5, 2, 2.5, 3, 3.5 or 4 hours.

Although dosage forms that provide pH independent in vitro dissolution and in vivo release are frequently sought after and viewed favorably, particularly many extended release dosage forms, in some embodiments, pH independent dissolution and release can work against the objectives of the some oral dosage forms of levorphanol (e.g., delayed onset, extended release) which are intended to provide delivery or release of the dosage form in the proximal to the stomach, duodenum, or ileum (i.e., duodenal release, jejunal release, ileal release, ileo-colonic release or colonic release). Indeed, certain controlled release material used to achieve delayed onset, duodenal release, jejunal release, ileal release, ileo-colonic release or colonic release exploit the pH difference in the GI tract to achieve some or all of its objectives.

Therefore, in some preferred embodiments, where the oral levorphanol pharmaceutical composition is intended to provide delayed onset, extended release, through use of a pH sensitive controlled release material, the in-vitro release rate is substantially dependent on pH in that the amount of levorphanol released at an undesirable pH (e.g., pH 1.2) and the amount released at a desirable pH (e.g., depending on the controlled release material and delivery and release objectives, pH 5.5, 6, 6.5, 7, 7.2, 7.4), when measured in-vitro at 1.5 or 2 hours using the USP Basket or Paddle Method of USP Drug Release test of U.S. Pharmacopeia (2003) at 100 rpm in 900 ml aqueous buffer is significantly different. For example, in some preferred embodiments, the in-vitro release rate difference is greater than about 35%, or 40%, or 45%, or 50%, or 55%, or 60% or 70%, or 80%, with the release rate higher at the desirable pH compared with the undesirable pH.

In some preferred embodiments of the invention, the dosage form of the invention is a duodenal delivery, or jejunal delivery, or ileal delivery, or ileo-colonic delivery or colonic delivery dosage form.

In some preferred embodiments, the levorphanol in the dosage form is non-releasable or substantially non-releasable until (i) after a particular time following oral ingestion, when the dosage form can be anticipated to have reached the duodenum, jejum, ileum, ileo-cecal junction, cecum, or colon; or (ii) the dosage form has come in contact or substantial contact or sustained contact with a desired gastrointestinal pH environment (e.g., pH > 3, or pH > 3.5, or pH > 4, or pH >4.5, or pH, > 5, or pH > 5.5, or pH > 6, or pH > 7, or pH > 7.5, or pH > 7.8); or (iii) the dosage form has come in contact with desired microbial flora (e.g., colonic microbial flora).

In certain situations involving pharmacokinetic evaluations, it may not be possible to provide the same amount of drug by different routes of administration due to the lack of commercially available dosage strengths or because such administration would require testing outside the approved method of administration (e.g., oral administration of high doses of immediate release levorphanol). Under such circumstances, the term "after the same amount" of levorphanol or "after an equal amount" of levorphanol may be modified and different amounts of drug may be evaluated, provided the data are dose normalized using pharmacokinetic approaches well known in the art.

Also disclosed are methods for the treatment of a human patient suffering from a medical condition amenable to treatment with levorphanol comprising administering a therapeutically effective amount of oral levorphanol and a controlled release material to render said dosage form extended release.

Also disclosed are methods for the treatment of a human patient suffering from pain with levorphanol comprising administering a therapeutically effective amount of oral levorphanol, and a controlled release material to render said dosage form extended release.

Also disclosed are methods for the treatment of medical conditions amenable to treatment with levorphanol in patients who are at higher risk for nausea, vomiting, sedation or other opioid agonist side effects or who have a prior history of said side effects on other opioids comprising administering a therapeutically effective amount of oral levorphanol, and a controlled release material to render said dosage form extended release.

The treatment of all pain states is contemplated by this invention, regardless of etiology, mechanisms, duration, prior treatment response and anatomic location, including acute pain, inflammatory pain, chronic pain, cancer pain, visceral pain and neuropathic pain. Particularly preferable pain states are those requiring treatment for more than a few days, more than a few weeks or more than a few months, e.g., chronic (non-cancer) pain, chronic cancer pain and neuropathic pain.

Also disclosed are methods of providing relief in a human patient suffering from neuropathic pain and chronic. In some preferred embodiments, the dosage form of the invention is intended for the treatment of neuropathic pain, peripheral neuropathic pain, central neuropathic pain, chronic pain, osteoarthritis, back pain, cancer pain, and chronic inflammatory pain.

The invention is also directed to methods of preparing the dosage forms disclosed herein.

The invention is also directed to a process for the preparation and manufacture of the dosage form.

Also disclosed are methods of providing relief in a human patient suffering from acute pain.

All kinds of kits of the present invention are contemplated. In some preferred embodiments, also provided are kits for use in treating or preventing the pain with the oral administration of levorphanol or a pharmaceutically acceptable salt of levorphanol, or a mixture thereof for a subject in need of such treatment, comprising: (i) a dosage form of the invention; (ii) a container for the dosage form; and optionally, any of (iii) to (vi): (iii) a container for individual units of the dosage form (e.g., individual tablets or capsules in blisters); (iv) educational instructions in any media about various medical conditions, their etiology, pathophysiology, consequences and treatment, including information on the potential for abuse and diversion and methods for prevention of same and information on the proper use and disposal of the medication; (v) containers or bags for the safe disposal of any used or remaining unused dosage form, preferably child proof and flushable; (vi) tamper evident and child proof packaging for the kit and its contents.

### ACTIVE TREATMENTS

### Levorphanol

The amount of levorphanol in the oral dosage form will vary depending on variety of physiologic, pharmacologic, pharmacokinetic, pharmaceutical and physicochemical factors, including, without limitation: (i) the choice of levorphanol as the base, pharmaceutically acceptable salt or mixtures therof; (ii) the nature of the oral dosage form (e.g, immediate release or extended release); (iii) the anatomical location of the pain relieving target; (iv) the intensity and intractability of the pain; (v) the contribution of different mechanism to the initiation, propagation, summation and maintenance of the pain; (vi) the absorption, metabolism, distribution and excretion of orally administered levorphanol in healthy subjects and in patients with various diseases and disorders, including renal and hepatic impairment; (vii) the presence of comorbid pathology; (viii) the patient's risk of iatrogenic side effects; (ix) the tolerability of the dose, including the patient's propensity for levorphanol associated CNS and gastrointestinal side effects; (x) use of concurrent analgesics; (xi) the efficiency of the dosage form; and (xii) prior opioid exposure, including the type of opioid, the dose, the duration and the recency of use.

In certain embodiments, the amount of levorphanol in the oral dosage form of the invention for use in an adult is about 0.5 mg to about 5 grams. In other embodiments, the amount of levorphanol in the dosage form is about 1 mg to about 800 mg, or about 1 mg to about 600 mg, or about 1 mg to about 400 mg, or about 1 mg to about 300 mg, or about 1 mg to about 250 mg, or about 1 mg to about 200 mg, or about 1 mg to about 180 mg, or about 1 mg to about 160 mg, or about 1 mg to about 140 mg, or about 1 mg to about 120 mg, or about 1 mg to about 100 mg, or about 1 mg to about 90 mg, or about 1 mg to about 80 mg, or about 1 mg to about 70 mg, or about 1 mg to about 60 mg, or about 1 mg to about 55 mg, or about 1 mg to about 50 mg, or about 1 mg to about 45 mg, or about 1 mg to about 40 mg, or about 1 mg to about 38 mg, or about 1 mg to about 35 mg, or about 1 mg to about 32 mg, or about 1 mg to about 30 mg, or about 1 mg to about 28 mg, or about 1 mg to about 26 mg, or about 1 mg to about 25 mg, or about 1 mg to about 23 mg, or about 1 mg to about 20 mg, or about 2 mg to about 100 mg, or about 2 mg to about 80 mg, or about 2 mg to about 50 mg, or about 2 mg to about 40 mg, or about 2 mg to about 30 mg, or about 4 mg to about 100 mg, or about 4 mg to about 80 mg, or about 4 mg to about 50 mg, or about 4 mg to about 40 mg, or about 4 mg to about 30 mg, or about 5 mg to about 100 mg, or about 5 mg to about 80 mg, or about 5 mg to about 50 mg, or about 5 mg to about 40 mg, or about 5 mg to about 30 mg, or about 6 mg to about 100 mg, or about 6 mg to about 80 mg, or about 6 mg to about 50 mg, or about 6 mg to about 40 mg, or about 6 mg to about 30 mg, or about 8 mg to about 100 mg, or about 8 mg to about 80 mg, or about 8 mg to about 50 mg, or about 8 mg to about 40 mg, or about 8 mg to about 30 mg, or about 8 mg to about 100 mg, or about 8 mg to about 80 mg, or about 8 mg to about 50 mg, or about 8 mg to about 40 mg, or about 8 mg to about 30 mg. In more preferred embodiments, the amount of levorphanol in the dosage form is about 2 mg to about 80 mg, or about 2 mg to about 60 mg, or about 2 mg to about 40 mg, or about 4 mg to about 60 mg, or about 4 mg to about 50 mg, or about 5 mg to about 30 mg.

In other embodiments, the amount of levorphanol in a single unit dose (e.g., one tablet or capsule) of the invention for use in an adult the exceeds the maximum unit dose of oral immediate release levorphanol by at least about 20%, or 30%, or 40%, or 50%, or 60%, or 70%, or 80%, or 90%, or 100%, or 120%, or 140%, or 160%, or 180%, or 200%, or 220%, or 240%, or 260%, or 280%, or 300%, or 320%, or 340%, or 360%, or 380%, or 400%, or 450%, or 500%, or 550%, or 600%, or 650%, or 700%.

Suitable pharmaceutically acceptable salts of levorphanol include levorphanol bitartrate, levorphanol bitartrate hydrate, levorphanol hydrochloride, levorphanol p-toluenesulfonate, levorphanol phosphate, levorphanol thiosemicarbazone, levorphanol sulfate, levorphanol trifluoroacetate, levorphanol hemipentahydrate, levorphanol pentafluoropropionate, levorphanol p-nitrophenylhydrazone, levorphanol o-methyloxime, levorphanol semicarbazone, levorphanol hydrobromide, levorphanol mucate, levorphanol oleate, levorphanol phosphate dibasic, levorphanol phosphate monobasic, levorphanol inorganic salt, levorphanol organic salt, levorphanol acetate trihydrate, levorphanol bis(heptafuorobutyrate), levorphanol bis(methylcarbamate), levorphanol bis(pentafluoropropionate), levorphanol bis(pyridine carboxylate), levorphanol bis(trifluoroacetate), levorphanol chlorhydrate, and levorphanol sulfate pentahydrate. Preferably, the levorphanol is present as the tartrate salt or the base.

### Definitions

As used herein the terms: (i) "AUC₀₋ₜ", and "AUC_{0-τ}" (or "AUC_{0-Tau}") mean the area under the plasma drug concentration-time curve from time zero to the intended dosing frequency of the dosage form after first administration (e.g., 8 hours, 12 hours or 24 hours) and to the end of the dosing interval after repeated dosing or at steady-state, respectively; (ii) "AUC_{0-inf}" (means area under the plasma drug concentration-time curve from time zero to infinity first administration; (iii) "AUC₀₋₁", "AUC₀₋₂", "AUC₀₋₃", "AUC₀₋₄", "AUC₀₋₆", "AUC₀₋₈", "AUC₀₋₁₂" "AUC₀₋₂₄" means area under the plasma drug concentration-time curve from time zero to the specified time in hours, for example, AUC₀₋₁₂ is the area under the plasma drug concentration-time curve from time zero to 12 hours after dosing; (iv) "C_{max"} means the maximum observed plasma drug concentration; (v) "Cₘᵢₙ" means the minimum plasma concentration of the drug after the occurrence of the Cₘₐₓ over a dosing interval at steady state or minimum plasma concentration of the drug at a specified time or time interval following dosing and after the occurrence of the Cₘₐₓ or means the minimum plasma concentration of the drug at the end of the intended dosing interval; (vi) "tₘₐₓ" or "Tₘₐₓ" means the time of the observed maximum drug concentration (also known as time to achieve Cₘₐₓ); (vii) "half value duration" or "HVD" means the duration after dosing during which plasma concentration of drug are greater than or equal to one-half of Cₘₐₓ, obtained by calculating the time interval beginning when the actual or interpolated plasma concentration first equals or exceeds one-half of Cₘₐₓ and ending at the first time point for which the actual or interpolated plasma concentration falls below one-half of Cₘₐₓ; (viii) "W₅₀" for purposes of the present invention means the width of the plasma concentration time curve at 50% of the height of the Cₘₐₓ over the dosing interval; (ix) "steady state" is a state of equilibrium wherein the amount of the drug reaching the system is approximately the same as the amount of the drug leaving the system or put another way, the patient's body eliminates the drug at approximately the same rate that the drug becomes available to the patient's system through absorption into the blood stream, said "time to steady state" measured by calculating (a) the Cₘᵢₙ after each sequential dosing of drug administered at the intended dosing frequency until two consecutive Cₘᵢₙ's are not statistically different at a 10% significance level (p = 0.10), or (b) calculated using pharmacokinetic data generated from well conducted single studies, said data from the mean population plasma concentration time data or the individual plasma concentration time data from which a mean value is derived; (x) "percent fluctuation", "% fluctuation" and "% FL" means the variation in plasma concentrations of the drug computed as: (a) (Cₘₐₓ-Cₘᵢₙ)/Cₘᵢₙ x 100 (for an individual patient) and (mean Cₘₐₓ-mean Cₘᵢₙ)/mean Cₘᵢₙ x 100 (for a population), or (b) (Cmax-Cmin)/Cav x 100 (for an individual patient) and (mean Cmax-mean Cₘᵢₙ)/mean Cₐᵥ x 100 (for a population), where Cₘᵢₙ is the plasma concentration at the end of the dosing interval; (xi) "accumulation index" or "AI" means (a) the ratio of the minimum plasma concentration of the drug at the end of the intended dosing interval (i.e., 12 hours for a Q12H dosage form and 24 hours for a Q24H dosage form) after steady-state (Cₛₛₘᵢₙ) administration, to the plasma concentration of the drug at the end of the intended dosing interval determined at first administration, or (b) the ratio of the mean plasma concentration of the drug over the dosing interval (i.e., over 12 hours for a Q12H dosage form, and over 24 hours for a Q24H dosage form) after steady-state (Cₛₛₐᵥₑ) administration, to the mean plasma concentration of the drug over the first dosing interval, or (c) 1 / (1 - exp(-lambda Z^{∗}tau), each of (a), (b) and (c) calculated using observed data or using pharmacokinetic data generated from well conducted single studies and each using the mean plasma concentration time data or using individual plasma concentration time data from which a mean value is derived; (xii) "AUC₀₋ₙ" means the area under the plasma drug concentration-time curve from time zero to the specified time point ("n"), where n is the time in hours (unless specified otherwise); (xiii) "Cₐᵥ" or "Cₘₑₐₙ" is the average plasma concentration of the drug over the dosing interval; (xiv) "AUC_{0-inf}" (means area under the plasma drug concentration-time curve from time zero to infinity; (xiv) "AUMCINF" is the area under the moment curve extrapolated to infinity; (xv) "AUMClast" is the area under the moment curve computed to the last observation; (xvi) "MRT" or "Mean Residence Time" is the average amount of time a particle remains in a compartment or system, calculated by dividing the AUMC by the AUC; (xvii) "MRTₗₐₛₜ" or "mean unextrapolated mean residence time" is mean residence time when the plasma concentration profile is not extrapolated to infinity, but rather is based on values up to and including the last measured concentration, calculated by dividing the AUMClast by the AUClast, where AUClast the AUC computed to the last observation; (xviii) "MRTINFO", "MRTINF_obs" and "MRT Infinity Observed" is the observed mean residence time extrapolated to infinity, calculated by dividing the AUMCINF by the AUCinf; (xix) "absorption rate constant" the rate at which the drug is absorbed into the central compartment from outside the system, often denoted as K01 and determined by curve fitting plasma concentration-time date for individual subjects to appropriate one or two compartment pharmacokinetic models in WinNonlin™ (Pharsight Corporation), with a first-order extravascular input function, a lag-time and micro-constants; (xx) "T_{lag}" is an empirical pharmacokinetic parameter which estimates the time delay prior to commencement of first order absorption, calculated by in WinNonlin™ (Pharsight Corporation). T_{lag} may also be calculated using other suitable methods, such as those described as suitable by Csizmadia and Endrenyi (Journal of Pharmaceutical Sciences, 1998; 87:608-12); (xxi) "mean absorption time" or "MAT" is the sum of the inverse of the absorption rate constant and T_{lag}; (xxii) "cumulative" area under the plasma concentration time curve", or "cumulative AUC" means the sum of the AUC from the time of drug administration (time zero) to a specified time point, for example, the mean cumulative AUC at 48 hours means the entire AUC from time of drug administration (time zero) to 48 hours; (xxiii) "AUC" means the mean the area under the plasma drug concentration-time curve, wherein the plasma is harvested from blood collected through a peripheral vein.

In some preferred embodiments, the pharmacokinetic and pharmacodynamic parameters are determined from first administration or single dose administration. In other preferred embodiments, the pharmacokinetic and pharmacodynamic parameters are determined from steady state administration. In yet other preferred embodiments, the pharmacokinetic and pharmacodynamic parameters are determined from at a time between first administration and steady state administration.

In some preferred embodiments, the pharmacokinetic and pharmacodynamic parameters are determined from an individual subject. In other preferred embodiments, the pharmacokinetic and pharmacodynamic parameters are determined from a population of subjects, said population comprising two or more subjects.

In some preferred embodiments, the in vivo specifications and claims of the invention are determined after administration of a few or some doses of the invention. In other preferred embodiments, the in vivo specifications and claims of the invention are determined after administration of most, substantially all or all doses of the invention.

In some preferred embodiments, the pharmacokinetic and pharmacodynamic parameters of the specifications and claims are determined in subjects having a Body Mass Index (BMI) between 18 and 26 kg/m², inclusive (BMI = [weight in kg/ height in m²] x 10,000). In some other preferred embodiments, the pharmacokinetic parameters of the specifications and claims are determined in subjects having a Body Mass Index (BMI) ≥ 38 kg/m².

The term "semipermeable wall" for purposes of the present invention means that the wall is permeable to the passage of an exterior fluid, such as aqueous or biological fluid, in the environment of use, including the gastrointestinal tract, but impermeable to drug.

The term "first administration", "single administration" or "single dose administration" means administration of a dose of the present invention at the initiation of therapy to an individual patient or a patient population. First administration also includes administration of an initial dose of the present invention to subjects who are not in need of treatment but who are volunteers participating in experimentation and testing.

The term "steady state" means that the amount of the drug reaching the system is approximately the same as the amount of the drug leaving the system. Thus, at "steady-state", the patient's body eliminates the drug at approximately the same rate that the drug becomes available to the patient's system through absorption into the blood stream.

As used herein, "dose proportionality", "dose-proportional" and "dose proportional bioavailability" means that change does by a particular percentage changes the drug exposure by a similar percentage, as assessed by the area under the plasma concentration time curve and maximum plasma concentration (Cₘₐₓ). For example, if a dosage form provides dose proportional bioavailability, doubling the dose doubles the AUC and Cₘₐₓ. Some drugs or dosage forms may be dose proportional bioavailability over a narrow range of doses (e.g., 2 fold, or 3 fold), but may not be dose proportional over a wider range of doses (e.g., 4, 5, 6, or 7 fold). Dose proportionality may be assessed by methods well known in the art. A particularly preferred method is described by Smith BP et al (Confidence interval criteria for assessment of dose proportionality. Pharm Res 17:1278-1283), which is hereby incorporated in its entirety by reference.

As used herein, "population of patients" or "patient population" means at least two patients or subjects.

For purposes of the invention, the term a "patient" or a "subject" in reference to pharmacokinetic and pharmacodynamic parameters means that the specification or claim is directed to the pharmacokinetic or parameters of an individual patient or subject, or to population of patients.

The term "USP Paddle Method", "USP Basket Method" and "USP Paddle or Basket Method" are the respective Paddle and Basket Methods described, e.g., as specified in the United States Pharmacopeia, USP-28 NF-23 (2005), published by the United States Pharmacopeial Convention, Inc, herein incorporated by reference.

In some embodiments of the invention, pH adjustments of the dissolution media may be achieved by adjustment as required with hydrochloric acid or other acids, sodium hydroxide or other bases, other pharmaceutical excipients and buffers known in the art.

In some embodiments, the dissolution specifications may be assessed at 50 rpm or 75 rpm instead of 100 rpm. In some embodiments, the dissolution specifications may be assessed in 100 ml, 250 ml, 500 mL or 600 mL of dissolution media instead of 900 mL. In some embodiments, the dissolution specifications may be assessed with the aid of a sinker (e.g., a nonreactive stainless steel wire helix, other inert material, cork borers, cylinders, see, for example, USP 32-NF 27. In some embodiments, the dissolution specifications may be assessed using Simulated Intestinal Fluid (SIF) USP, with or without the inclusion of enzyme, or Simulated Gastric Fluid (SGF) USP instead of distilled water.

The term "pH-independent" for purposes of the present invention is defined as having characteristics (e.g., dissolution) which are substantially unaffected by pH.

The term "pH-dependent" for purposes of the present invention is defined as having characteristics (e.g., dissolution) which are substantially affected by pH.

The term "bioavailability" is defined for purposes of the present invention as the extent to which the drug (e.g., levorphanol) is absorbed from the unit dosage forms.

In certain situations involving pharmacokinetic evaluations, it may not be possible to provide the same amount of drug for the purposes of a pharmacokinetic comparison, for example, due to a lack of commercially available dosage strengths, or for safety reasons or because such administration would require testing outside the approved method of administration. Under such circumstances, the term "after the same amount of an oral immediate release formulation of levorphanol", and the like may be waived and different amounts of drug may be evaluated, provided the data are dose normalized using pharmacokinetic approaches well known in the art.

The term "agonist" means a ligand that binds to a receptor and alters the receptor state resulting in a biological response. Conventional agonists increase receptor activity, whereas inverse agonists reduce it (See Neubig et al, IUPHAR Committee on Receptor Nomenclature and Classification, Pharmacol Rev, 2003; Howlett et al., Mol Pharmacol, 1988).

The term "opioid agonist" means a molecule that causes a specific physiologic, pathophysiologic or pharmacologic effect after binding to an opioid receptor. Except when specifically modified, the term "opioid" has the same meaning as "opioid agonist". For example, the phrases "a wide variety of opioids are used to treat pain" and "opioids can cause side effects" have the same meaning as "a wide variety of opioid agonists are used to treat pain" and "opioid agonists can cause side effects", respectively. Similarly, the phrases "opioid antagonists may be given in sequestered form" and "a suitable opioid antagonist is may be given" do not refer to "opioid agonists". For the purposes of the present invention, any drug having exhibiting agonism at the opioid receptor [e.g., agonism at the mu (µ), delta (δ) and kappa (κ) opioid receptors] is an "opioid agonist", even if it possesses other pharmacologic activity (e.g., NMDA antagonism, opioid antagonism, monoaminergic reuptake inhibition). For example, a drug that exhibits mixed agonist-antagonist effects at one or more opioid receptor is an opioid agonist. Similarly, a drug that exhibits partial agonism at one or more opioid receptor is an opioid agonist.

Opioid agonists include alfentanil, allylprodine, alphaprodine, anileridine, apomorphine, apocodeine, benzylmorphine, bezitramide, buprenorphine, butorphanol, carfentanil, clonitazene, codeine, cyclazocine, cyclorphen, cyprenorphine, desomorphine, dextromoramide, dezocine, diampromide, dihydrocodeine, dihydromorphine, dimenoxadol, dimepheptanol, dimethylthiambutene, dioxyaphetyl butyrate, dipipanone, eptazocine, ethoheptazine, ethylmethylthiambutene, ethylmorphine, etonitazene, fentanyl, heroin, hydrocodone, hydroxymethylmorphinan, hydromorphone, hydroxypethidine, isomethadone, ketobemidone, levallorphan, levorphanol, levophenacylmorphan, lofentanil, meperidine, meptazinol, metazocine, methadone, methylmorphine, metopon, morphine, myrophine, nalbuphine, narceine, nicomorphine, norlevorphanol, normethadone, nalorphine, nociceptin/orphanin FQ (N/OFQ), normorphine, norpipanone, ohmefentanyl, opium, oxycodone, oxymorphone, papaveretum, pentazocine, phenadoxone, phenomorphan, phenazocine, phenoperidine, pholcodine, piminodine, piritramide, propheptazine, promedol, profadol, properidine, propiram, propoxyphene, racemorphan, remifentanil, sufentanil, tapentadol, tramadol, tilidine, methylnaltrexone, naloxone methiodide, naloxonazine, nalmexone, nalbuphine, nalorphine dinicotinate, naltrindole (NTI), naltrindole isothiocyanate, (NTII), naltriben (NTB), nor-binaltorphimine (nor-BNI), beta-funaltrexamine (b-FNA), BNTX, cyprodime, ICI-174,864, LY117413, MR2266, etorphine, DAMGO, CTOP, diprenorphine, naloxone benzoylhydrazone, bremazocine, ethylketocyclazocine, U50,488, U69, 593, spiradoline, DPDPE, [D-Ala2,Glu4] deltorphin, DSLET, Met-enkephalin, Leu-enkephalin, (3-endorphin, dynorphin A, dynorphin B, a-neoendorphin, or an opioid having the same pentacyclic nucleus as nalmefene, naltrexone, buprenorphine, levorphanol, meptazinol, pentazocine, dezocine, or their pharmaceutically acceptable salts, prodrugs, esters, analogs, derivatives, solvates, complexes, polymorphs, hydrates and metabolites, as racemates or an individual

An "antagonist" is a drug or ligand that reduces the action of another drug or ligand, generally an agonist. Many antagonists act at the same receptor macromolecule as the agonist. (See Neubig et al, IUPHAR Committee on Receptor Nomenclature and Classification, Pharmacol Rev, 2003; Howlett et al., Mol Pharmacol, 1988).

The term "receptor" means a molecule within a cell, on a cell surface, on a membrane, in tissue, in fluid or otherwise found in humans that serve as a recognition or binding site to cause specific physiologic, pathophysiologic or pharmacologic effects. The term "receptor" also means a cellular macromolecule, or an assembly of macromolecules, that is concerned directly and specifically in chemical signaling between and within cells. Combination of a hormone, neurotransmitter, drug, ligand, or intracellular messenger with its receptor(s) initiates a change in cell function (Neubig et al, IUPHAR Committee on Receptor Nomenclature and Classification, Pharmacol Rev, 2003).

The term "opioid receptor" includes mu (µ), delta (δ) and kappa (κ) opioid receptors, their subtypes and splice variants such as mu₁, mu₂, delta₁, delta₂, kappa₁, kappa₂ and kappa₃, etc.

Opioid antagonists are known or readily determined by individuals who practice the art. Preferably, the opioid antagonists useful for the present invention may be selected from the group consisting of naltrexone, methylnaltrexone, nalbuphine, naloxone, nalmefene, cyclazocine, cyclorphan, oxilorphan nalorphine, nalorphine dinicotinate, nalmefene, nadide and levallorphan.

In certain preferred embodiments of the present invention, the invention allows for the use of lower doses of levorphanol by virtue of the inclusion or co-administration of an additional drug for the prevention or treatment of pain. By using lower amounts of either or both drugs, the side effects associated with treatment in humans are reduced.

The term "levorphanol" or "/ 3-hydroxy-N-methylmorphinan" is defined for purposes of the invention as comprising: (i) unsalified levorphanol (also known as levorphanol base), its pharmaceutically acceptable salts and mixtures thereof; (ii) the racemate of levorphanol (racemorphan or *d,l* 3-hydroxy-N-methylmorphinan), its pharmaceutically acceptable salts and mixtures thereof; (iii) non-stoichiometric ratios of *l* 3-hydroxy-N-methylmorphinan and *d* 3-hydroxy-N-methylmorphinan, their pharmaceutically acceptable salts and mixtures thereof; (iv) morphinan-3-ol, 17-methyl-, [*R*-(*R**,*R**)]-2,3-dihydroxybutane-dioate (1:1) (salt) dihydrate; or (v) 17-Methylmorphinan-3-ol, tartrate (1:1) (salt) dihydrate; or (vi) esters, solvates, complexes, polymorphs, hydrates or prodrugs of (i) to (v); or (vii) mixtures of one or more of (i) to (vi). Unless specifically modified, (for example, by reference to "levorphanol base", or "unsalified levorphanol" or "levorphanol hydrobromide" or "levorphanol tartrate"), as used herein, "levorphanol" means one or more of the items in the foregoing (i) to (iv). Preferably, the levorphanol in the composition is unsalified *l* 3-hydroxy-N-methylmorphinan, a pharmaceutically acceptable salt thereof, or a mixture thereof.

Levorphanol tartrate dihydrate has a Chemical Abstract Services No. of 5985-38-6 dihydrate.

As used herein, the mass of levorphanol in any dosage form of the invention or of a test, reference, control or comparator dosage form comprising levorphanol refers to the amount (mass) of unsalified levorphanol, or a pharmaceutically salt of levorphanol, or a mixture thereof. When any dosage form of the invention or of a test, reference, control or comparator dosage form comprises the racemate of levorphanol (i.e., racemorphan or *d,l* 3-hydroxy-N-methylmorphinan), the mass of levorphanol in the specifications and claims refer to mass of the levo isomer of racemorphan (i.e., levorphanol or *l* 3-hydroxy-N-methylmorphinan).

Levorphanol tartrate is commercially sold as levorphanol tartrate dihydrate having a molecular formula of C₁₇H₂₃NO . C₄H₆O₆ . 2H₂O, a molecular weight of about 443.5.

In some preferred embodiments, the levorphanol dose of the specifications and claims is the dose of unsalified levorphanol (levorphanol base). In other preferred embodiments, the levorphanol dose of the specifications and claims is the dose of salified levorphanol (a levorphanol salt, e.g., levorphanol tartrate).

As used herein with respect to the levorphanol dosage form of the invention, the term "oral", "oral dosage form", "oral pharmaceutical dosage form", "oral administration", "oral compositions" "oral pharmaceutical compositions", "oral tablets", "oral capsules", "orally ingested", "orally", "oral route" and the like all refer to any method of administration through the mouth. The oral dosage form of the invention is usually ingested intact, although it may be ingested tampered (e.g., crushed) and usually with the aid of water or a beverage to hasten passage through the mouth.

As used herein, "controlled release material", "controlled release means", "rate controlling means", "rate controlling excipient", "rate controlling ingredient", "rate controlling material", "release rate controlling means", "release rate controlling excipient", "release rate controlling ingredient", "release rate controlling material", and "material to provide controlled release" means an in vitro or in vivo release rate controlling excipient or material incorporated in the dosage form whose function or primary function is to modify release (e.g, onset of release, rate of release, duration of release) of an active drug (e.g., levorphanol) from a dosage form or a portion (i.e., cause the dosage form to release in other than an immediate release fashion). In more preferred embodiments of the invention, the controlled release material functions to provide one or more of the following, compared to immediate release levorphanol: (1) change in the onset of release; (2) change in the rate of release; (3) change in the duration of release; (4) change in the time of peak plasma concentration; (5) change in the peak plasma concentration; (6) change in the extent of absorption; (7) change in the onset of therapeutic effect; (8) change in the duration of therapeutic effect; and (9) change in the GI anatomic location of release.

As used herein, the term "extended release" dosage forms mean pharmaceutical preparations which release an active ingredient from a dosage form or a portion thereof in other than an immediate release fashion. Extended release pharmaceutical compositions are made by incorporating a controlled release material in the dosage form. Extended release dosage forms are sometimes designed to accomplish pharmaceutical, pharmacokinetic, pharmacodynamic, therapeutic or convenience objectives not offered by conventional dosage forms such as a solution or an immediate release dosage form.

As used herein, the term "extended release" is interchangeably with "controlled release", "prolonged release", "slow release", "sustained release", "retarded release", "long acting" and the like. Extended release dosage forms release the active ingredient from a dosage form or a portion thereof over an extended period of time (over a period of time greater than 4 or 6 hours, preferably over for period greater than about 8 hours, and most preferably over for period greater than about 10 hours, 12, 14, 16, 18, 20, 22 or 24 hours. Extended release dosage forms may be either delayed onset formulations, i.e., "delayed onset, extended release" (e.g., a delay in release of 1, 1.5, 2, 2.5, 3, 3.5, 4, 4.5, 5, 5.5, 6, 6.5, 7, 7.5 or 8 hours after ingestion, preferably at least 2 hours after ingestion) or "extended release" (i.e., without a significant initial delay in release). In some embodiments, particularly preferred extended release dosage forms are suitable for or intended to be used twice-a-day (e.g., Q12H or Q12H PRN) or once-a-day (e.g., QD, Q24H or Q24H PRN), with once-a-day preferred over twice-a-day.

As used herein, unless specifically modified, the term "extended release levorphanol", "extended release dosage forms of levorphanol", "extended release compositions of levorphanol", "extended release pharmaceutical compositions of levorphanol", "extended release dosage forms of levorphanol", "Levorphanol ER", "pharmaceutical compositions of the invention", "compositions of the invention", "dosage forms of the invention", "pharmaceutical dosage forms of the invention", or use of the term "extended release" in association with levorphanol means "extended release levorphanol", or "delayed onset, extended release" oral levorphanol dosage forms of the invention.

As used herein, "delayed onset" and "delayed release" dosage forms mean pharmaceutical preparations which release begin the first release of an active ingredient from a dosage form or a portion thereof (i) at time other than immediately following oral administration; and/or (ii) after a lag period lasting from minutes to hours (e.g., 0.5, 1, 1.5, 2, 2.5, 3, 3.5, 4, 4.5, 5, 5.5, 6, 6.5, 7, 7.5 or 8 hours); and/or (iii) upon reaching the desired GI anatomic location distal to the stomach (e.g., distal to the duodenum, jejunum, ileum, ileo-cecal junction or colon) or and/or (iii) upon reaching the desired GI environment distal to the stomach (e.g., pH at the point of release, osmotic pressure at the point of release, hydration, microbial flora). Delayed onset, extended release dosage forms are dosage forms that provide duodenal delivery, jejunal delivery, ileal delivery, ileo-colonic delivery or colonic delivery of at least the levorphanol in the dosage form.

As used herein, "delayed onset, extended release" means dosage forms which after a desired lag period post-ingestion (e.g., 0.5, 1, 1.5, 2, 2.5, 3, 3.5, 4, 4.5, 5, 5.5, 6, 6.5, 7, 7.5 or 8 hours), slowly release the active drug from the dosage form over an extended period of time (e.g., over about 4, 4.5, 5, 6, 7, 8, 9, 10, 12, 14, 16, 18, 20, 22, 24, 26, or 30 hours).

As used herein, "controlled release material", "controlled release means", and "material to provide controlled release" means an in vitro or in vivo release rate controlling excipient or material incorporated in the dosage form whose function or primary function is to modify release (e.g, onset of release, rate of release, duration of release) of an active drug (e.g., levorphanol) from a dosage form or a portion (i.e., cause the dosage form to release in other than an immediate release fashion), in other words to cause the dosage form to be extended release dosage form. In particularly preferred embodiments of the invention, the controlled release material functions to provide one or more of the following: (1) delay in the onset of release; (2) delay in the rate of release; (3) prolonged or extended duration of release; (4) delay in the onset of therapeutic effect; (5) delay in onset of side effects; (6) delay in the onset of psychic or mood altering effects; (7) reduced abuse liability; (8) prolonged or extended duration of therapeutic effect; or (9) more robust therapeutic effect; (10) a pharmacokinetic profile consistent with dosage forms which are extended release or delayed onset, extended release; (11) a pharmacodynamic profile consistent with dosage forms which are extended release or delayed onset, extended release; and (12) reduced potential for misuse, abuse, addiction and drug diversion.

In some preferred embodiments, one or more of the specifications for extended release dosage forms of levorphanol also apply to delayed onset, extended release dosage forms of levorphanol.

As used herein with respect to the levorphanol dosage form of the invention, "duodenal release" and "duodenal delivery" are interchangeable and refer to in vivo release of all, substantially all or most levorphanol from the dosage form into the portion of gastrointestinal tract distal to the stomach. In some embodiments, duodenal release or duodenal delivery dosage forms of the invention provide in vivo release of all, substantially all or most levorphanol from the dosage form rapidly upon reaching the portion of gastrointestinal tract distal to the stomach. In other embodiments, duodenal release and duodenal delivery dosage forms of the invention provide in vivo release of all, substantially all or most levorphanol from the dosage form slowly upon reaching the portion of gastrointestinal tract distal to the stomach.

As used herein with respect to the levorphanol dosage form of the invention, "jejunal release" and "jejunal delivery" are interchangeable, and refers to in vivo release of all, substantially all or most levorphanol from the dosage form into the portion of gastrointestinal tract distal to the duodenum. In some embodiments, jejunal release" or "jejunal delivery dosage forms of the invention provide in vivo release of all, substantially all or most levorphanol from the dosage form rapidly upon reaching the portion of gastrointestinal tract distal to the duodenum. In other embodiments, duodenal release and duodenal delivery dosage forms of the invention provide in vivo release of all, substantially all or most levorphanol from the dosage form slowly upon reaching the portion of gastrointestinal tract distal to the duodenum.

As used herein with respect to the levorphanol dosage form of the invention, "ileal release" and "ileal delivery" are interchangeable and refers to in vivo release of all, substantially all or most levorphanol from the dosage form into the portion of gastrointestinal tract distal to the jejunum. In some embodiments, ileal release or ileal delivery dosage forms of the invention provide in vivo release of all, substantially all or most levorphanol from the dosage form rapidly upon reaching the portion of gastrointestinal tract distal to the jejunum. In other embodiments, ileal release and ileal delivery dosage forms of the invention provide in vivo release of all, substantially all or most levorphanol from the dosage form slowly upon reaching the portion of gastrointestinal tract distal to the jejunum.

As used herein with respect to the levorphanol dosage form of the invention, "ileo-colonic release" and "ileo-colonic delivery" are interchangeable and are interchangeable and refer to in vivo release of all, substantially all or most levorphanol from the dosage form into the portion of gastrointestinal tract distal to the jejunum and/or distal to the ileum. In some embodiments, ileo-colonic release or ileo-colonic delivery dosage forms of the invention provide in vivo release of all, substantially all or most levorphanol from the dosage form rapidly upon reaching the portion of gastrointestinal tract distal to the jejunum or distal to the ileum. In other embodiments, ileo-colonic release and ileo-colonic delivery dosage forms of the invention provide in vivo release of all, substantially all or most levorphanol from the dosage form slowly upon reaching the portion of gastrointestinal tract distal to the jejunum or distal to the ileum.

As used herein with respect to the levorphanol dosage form of the invention, "colonic release" and "colonic delivery" are interchangeable and refer to in vivo release of all, substantially all or most levorphanol from the dosage form into the portion of gastrointestinal tract distal to the ileum. In some embodiments, colonic release and colonic delivery dosage forms of the invention provide in vivo release of all, substantially all or most levorphanol from the dosage form rapidly upon reaching the portion of gastrointestinal tract distal to the ileum. In other embodiments, colonic release and colonic delivery dosage forms of the invention provide in vivo release of all, substantially all or most levorphanol from the dosage form slowly upon reaching the portion of gastrointestinal tract distal to the ileum.

Duodenal delivery, jejunal delivery, ileal delivery, ileo-colonic delivery or colonic delivery dosage forms are in the form of delayed onset extended release dosage forms

When applied to the present invention, the term "immediate release", "immediate release dosage forms", "immediate release composition", "immediate release tablet", "immediate release capsule", "immediate release formulation", immediate release forms" and the like is a dosage form which is formulated to release the active drug from the dosage form immediately (i.e., without an attempt to delay or prolong the release of the active drug from the dosage form as is the case, for example, with extended release dosage forms) or a dosage form which allows the drug to dissolve in the gastrointestinal contents, with no intention of delaying or prolonging the dissolution or absorption of the drug). Immediate release dosage forms may be in any form, including tablet, capsule, solution, suspension, powder, micronized, granulated etc. When applied to levorphanol dosage forms of the invention, unless further modified to alter the meaning, "immediate release" refers to oral dosage forms. In the absence of a commercially available oral immediate release levorphanol product, an available parenteral or intranasal formulation of levorphanol or a salt thereof may be used orally, or a solution of levorphanol or a salt thereof may be prepared or an immediate release tablet may be prepared for the purpose of in vivo testing requiring immediate release levorphanol. Alternatively, an immediate release formulation of levorphanol may be prepared by encapsulating liquid or uncompressed solid levorphanol, or by compressing levorphanol into tablet form without excipients or material that impart a delay or retardation to its release. Immediate release dosage forms generally disintegrate in ≤ about 0.5 hours, and generally substantially or completely dissolve in ≤ about 0.25, or ≤ about 0.5, or ≤ about 0.75, or ≤ about 1 hour, when measured by the recommended or appropriate USP compendial methods (for example some dosage forms may be tested by USP Basket Method or USP Paddle Method at 100 rpm in 900 mL of water at 37 °C).

For purposes of the invention, the oral extended release and oral immediate release formulations are pharmacokinetically dose proportional. In such formulations, the pharmacokinetic parameters (e.g., AUC and Cₘₐₓ) generally increase linearly from one dosage strength to another. Therefore the pharmacokinetic parameters of a particular dose can be inferred from the parameters of a different dose of the same formulation.

In some preferred embodiments, extended release dosage forms and delayed onset, extended release dosage forms release levorphanol at such a rate that plasma concentrations and/or therapeutic effects are maintained within the therapeutic range (above the minimum effective therapeutic concentration) but below toxic levels for most, substantially all or all of the intended duration (e.g., over a period of about 6 to about 30 hours, preferably over a period of time indicative of a Q12H, Q12H PRN, Q24H or Q24H PRN administration, more preferably over a period of time indicative of Q24H or Q24H PRN administration).

The phrase "comprising a therapeutically effective amount of levorphanol" means "comprising a therapeutically effective amount of levorphanol or a pharmaceutically acceptable salt of levorphanol, or prodrugs, esters, analogs, derivatives, solvates, complexes, polymorphs and hydrates thereof, as racemates or an individual diastereoisomers or enantiomeric isomers thereof or mixtures thereof'.

The singular forms "a", "an" and "the" include plural references unless the context clearly dictates otherwise. Thus, for example, reference to "a polymer" includes a single polymer as well as a mixture of two or more different polymers, reference to "a permeation enhancer" includes a single permeation enhancer as well as two or more different permeation enhancer in combination, and the like.

The mean drowsiness score is the score in mm on a 100 mm VAS scale bounded on the left by "no drowsiness" and on the right by "extreme drowsiness".

The mean nausea score is the score in mm on a 100 mm VAS scale bounded on the left by "no nausea" and on the right by "extreme nausea".

The mean dizziness score is the score in mm on a 100 mm VAS scale bounded on the left by "no dizziness" and on the right by "extreme dizziness".

The mean vomiting score is the number of episodes of vomiting or retching.

The "NNH" or "number needed to harm" is a measure that indicates how many patients would require a specific treatment to cause harm in one patient. As used herein, the "NNH or "number needed to harm" is a measure that includes: (i) how many opioid naive healthy subjects would require treatment to cause moderate or severe sedation (or drowsiness) in one subject, where moderate to severe drowsiness is defined as a VAS score of ≥ 50 mm on a 100 mm scale bounded on the left by "no sedation or drowsiness" and on the right by "extreme sedation or drowsiness"; (ii) how many opioid naive healthy subjects would require treatment to cause moderate or severe nausea in one subject, where moderate to severe nausea is defined as a VAS score of ≥ 50 mm on a 100 mm scale bounded on the left by "no nausea" and on the right by "extreme nausea"; (iii) how many opioid naive healthy subjects would require treatment to cause dizziness in one subject, where dizziness is defined as unsteadiness, imbalance, lightheadedness, spinning sensation or sensation that one is falling.

The "drug effects" questionnaire assesses the extent to which subjects currently felt a drug effect, on a scale of 1 to 5 (1 = "I feel no effect from it at all"; 2 = "I think I feel a mild effect, but I'm not sure"; 3 = "I feel an effect, but it is not real strong"; 4 = "I feel a strong effect"; 5 = "I feel a very strong effect"). This questionnaire can be used to examine the overall drug effects, preferably in drug abusers and recreational drug users.

The "drug liking" questionnaire assesses the extent to which subjects currently like the effects of the drug on a 100-mm VAS, bounded on the left by "0 = dislike a lot", bounded on the right by "100 = like a lot". This questionnaire can be used to examine the overall drug liking of, preferably in drug abusers and recreational drug users.

The "take again" questionnaire assesses whether subjects would take the drug again if given the opportunity. The patient is asked "If given an opportunity, would you take this drug again? (circle one: YES or NO). This questionnaire can be used to examine the overall desirability of the drug experience, preferably in drug abusers and recreational drug users.

On the "coasting" questionnaire the patient is asked to put a mark on a horizontal line that best describes their response to the question: "Do you feel like you are coasting or spaced out? The horizontal line is a visual analog scale (VAS) bounded on the left by "not at all" and on the right by "extremely". This questionnaire can be used to examine the degree to which subjects feel like they are coasting or spaced out, preferably in drug abusers and recreational drug users.

Three performance tasks may be employed for measuring skills related to driving.

The "critical tracking task" measures the patient's ability to control a displayed error signal in a first-order compensatory tracking task. The error is displayed as a horizontal deviation of a cursor from the midpoint on a horizontal, linear scale. Compensatory joystick movements correct the error by returning the cursor to the midpoint. The frequency at which the patient loses the control is the critical frequency. The critical tracking task measures the psychomotor control during a closed loop operation. It is a laboratory analog to on-the-road tracking performance.

The "stop signal task" measures motor impulsivity, which is defined as the inability to inhibit an activated or pre-cued response leading to errors of commission. The task requires patients to make quick key responses to visual go signals, i.e. the letters ABCD presented one at a time in the middle of the screen, and to inhibit any response when a visual stop signal, i.e. "*" in one of the four corners of the screen, is presented at predefined delays. The main dependent variable is the stop reaction time on stop signal trials that represents the estimated mean time required to inhibit a response.

The "Tower of London" (TOL) is a decision-making task that measures executive function and planning. The task consists of computer generated images of begin- and end-arrangements of three colored balls on three sticks. The subject's task is to determine as quickly as possible, whether the end-arrangement can be accomplished by "moving" the balls in two to five steps from the beginning arrangement by pushing the corresponding number coded button. The total number of correct decisions is the main performance measure.

As used herein, "plasma" is the virtually cell-free supernatant of venous blood containing anticoagulant (such as citrated, EDTA or heparinized blood) obtained after centrifugation. For example, venous whole blood may be centrifuged at about 3000 rpm for 15 minutes at 4 °C and the resulting supernatant (plasma) is harvested and stored in a freezer, at about -20 °C or lower (e.g., - 70 °C) until assayed.

As used herein, "serum" is the undiluted, extracellular portion of venous blood after adequate coagulation is complete. For example, venous whole blood is collected and allowed to coagulate. When coagulation is complete, the sample is centrifuged for at least about 10 minutes at about 3000 rpm.

Although plasma is usually preferred over serum for a variety of reasons (e.g, time savings, higher yield, prevention of coagulation-induced interferences and prevention of coagulation-induced changes), there are a few conditions under which serum may be preferred over plasma (e.g., avoiding analyte assay interference from anticoagulants, avoiding contamination with cations). For the purposes of this invention, the some or all of the specifications and claims relating to plasma are also applicable to serum.

As used herein, "plasma T_{lag}" refers to a time period from first administration (or first dosing) of levorphanol to the occurrence of first of two consecutive mean plasma levorphanol concentrations from the higher of the following: (i) a concentration at least equal to four times the lower limit of quantification (LLOQ) for the levorphanol; or (ii) a concentration at least equal to 5% of the mean Cₘₐₓ of the levorphanol; provided however, that the second consecutive levorphanol plasma concentration is obtained not less than about 15 minutes and not more than about 30 minutes after the first plasma levorphanol concentration is also at least equal to the higher of (i) or (ii); and provided however, that the plasma concentrations of levorphanol are determined by sequential venous blood sampling not less than about every 15 minutes and not more than about every 30 minutes after first administration and the mean concentration is determined from not less than 18 healthy subjects receiving the levorphanol in a fasted or fed state.

As used herein, the "lower limit of quantification" "LLOQ", "lower limit of quantitation" and "LLQ" are interchangable and mean the lowest concentration of the standard curve that can be measured with acceptable accuracy and precision as defined in Bioanalytical Method Validation, Guidance for Industry, Food and Drug Administration, May 2001, which is hereby incorporated in its entirety by reference, where (i) accuracy and precision are determined by replicate analysis using a minimum of five determinations per concentration level (excluding blank samples); and (ii) accuracy is within ±20% of the theoretical value; and (iii) precision around the mean value is not in excess of 20% of the coefficient of variation (CV); and (iv) the analyte response at the LLOQ is at least 5 times the response compared to blank response; and (v) the analyte peak (response) should is identifiable and discrete.

As used herein, "T_{lagD(x)}" refers to the time from the start of dissolution testing to the first attainment of an in-vitro release of about 5% by weight of the active drug from the dosage form when measured by the USP Basket or Paddle Method at 100 rpm in 900 mL of distilled water at 37 °C, where "₍ₓ₎" is the pH of the dissolution media.

As used herein, "cough" includes acute cough, chronic cough, iatrogenic cough, post-infectious cough, and cough secondary to asthma, COPD, lung cancer, gastroesophageal reflux disease, respiratory bacterial and viral infections, and upper airway cough syndrome.

As used herein, the term "pain" includes: (i) peripheral neuropathic pain, e.g., acute and chronic inflammatory demeyelinating polyradiculopathy, alcoholic polyneuropathy, chemotherapy-induced polyneuropathy, complex regional pain syndrome (CRPS) Type I and Type II, entrapment neuropathies (e.g., carpal tunnel syndrome), HIV sensory neuropathy, iatrogenic neuralgias (e.g., postthoracotomy pain, postmastectomy pain), idiopathic sensory neuropathy, painful diabetic neuropathy, phantom limb pain, postherpetic neuralgia, trigeminal neuralgia, radiculopathy (e.g., cervical thoracic, lumbosacral), sciatica, acute herpes zoster pain, temporomandibular joint disorder pain and postradiation plexopathy; and (ii) central neuropathic pain, e.g., compressive myelopathy from spinal stenosis, HIV myelopathy, multiple sclerosis pain, Parkinson's disease pain, postischemic myelopathy, post postradiation myelopathy, poststroke pain, posttraumatic spinal cord injury and syringomyelia; and (iii) cancer associated neuropathic pain, e.g., chemotherapy induced polyneuropathy, neuropathy secondary to tumor infiltration or nerve compression, phantom breast pain, postmastectomy pain, postradiation plexopathy and myelopathy; (iv) chronic pain, e.g., back pain, rheumatoid arthritis, osteoarthritis, inflammatory pain, non-inflammatory pain, myofascial pain, cancer pain, visceral pain, somatic pain, pelvic pain, musculoskeletal pain, post-traumatic pain, bone pain and idiopathic pain; (v) acute pain, e.g, acute postsurgical pain (including laparoscopic, laparatomy, gynecologic, urologic, cardiothoracic, arthroscopic, gastrointestinal, neurologic, orthopedic, oncologic, maxillofacial, ophthalmic, otolaryngologic, soft tissue, plastic, cosmetic, vascular and podiatric surgery, including abdominal surgery, abdominoplasty, adenoidectomy, amputation, angioplasty, appendectomy, arthrodesis, arthroplasty, arthroscopy, bilateral cingulotomy, biopsy, brain surgery, breast biopsy, cauterization, cesarean section, cholecystectomy, circumcision, commissurotomy, cordotomy, corneal transplantation, cricothoracotomy, discectomy, diverticulectomy, episiotomy, endarterectomy, endoscopic thoracic sympathectomy, foreskin restoration, fistulotomy, frenectomy, frontalis lift, fundectomy, gastrectomy, grafting, heart transplantation, hemicorporectomy, hemorrhoidectomy, hepatectomy, hernia repair, hypnosurgery, hysterectomy, kidney transplantation, laminectomy, laparoscopy, laparotomy, laryngectomy, lithotripsy, lobotomy, lumpectomy, lung transplantation, mammectomy, mammoplasty, mastectomy, mastoidectomy, mentoplasty, myotomy, mryingotomy, nephrectomy, nissen fundoplication, oophorectomy, orchidectomy, parathyroidectomy, penectomy, phalloplasty, pneumotomy, pneumonectomy, prostatectomy, psychosurgery, radiosurgery, ritidoplasty, rotationplasty, sigmoidostomy, sphincterotomy, splenectomy, stapedectomy, thoracotomy, thrombectomy, thymectomy, thyroidectomy, tonsillectomy, tracheotomy, tracheostomy, tubal ligation, ulnar collateral ligament reconstruction, ureterosigmoidostomy, vaginectomy, vasectomy, vulvectomy; renal colic; incisional pain; inflammatory incisional pain; nociceptive incisional pain; acute neuropathic incisional pain following surgery), renal colic, trauma, acute back pain, burn pain, burn dressing change pain, migraine pain, tension headache pain, acute musculoskeletal pain, acute exacerbation or flare of chronic back pain, acute exacerbation or flare of osteoarthritis, acute exacerbation or flare of chronic pain, breakthrough chronic non-cancer pain, breakthrough cancer pain, acute exacerbation or flare of rheumatoid arthritis, acute exacerbation or flare of myofacsial pain, acute exacerbation or flare of chronic idiopathic pain, acute exacerbation or flare of neuropathic pain, procedure related pain (e.g., arthroscopy, laparoscopy, endoscopy, intubation, bone marrow biopsy, soft tissue biopsy, catheterization), and other self-limiting pain states.

As used herein, the term "acute pain" refers to self-limiting pain that subsides over time and usually lasting less that about 30 days and more preferably lasting less than about 21 days. Acute pain does not include chronic conditions such as chronic neuropathy, chronic neuropathic pain and chronic cancer and non-cancer pain.

As used herein, "neuropathic pain" is pain initiated or caused by a primary lesion or dysfunction of the nervous system and includes (i) peripheral neuropathic pain and (ii) central neuropathic pain.

As used herein, the term "chronic pain" includes all non-neuropathic pain usually lasting more than 30 days, including inflammatory pain, non-inflammatory pain, muscle pain, joint pain, fascia pain, visceral pain, bone pain and idiopathic pain.

The term "therapeutic effectiveness" is defined for purposes of the present invention as a satisfactory prevention, reduction in or elimination of signs and symptoms of the medical disorder, disease or syndrome (e.g., pain), along with a tolerable level of side effects, as determined by the human patient.

The term "analgesic effectiveness" is defined for purposes of the present invention as a satisfactory prevention, reduction in or elimination of pain, along with a tolerable level of side effects, as determined by the human patient.

The analgesic effects of the dosage form of the present invention may be evaluated as described below. The choice of doses, patient population, type of pain and study design will vary depending on the needs of the study. Evaluation in acute and chronic pain is contemplated. The increased therapeutic benefits associated with the administration of the dosage form may in some embodiments be evaluated after third molar surgery and bunionectomy surgery.

In the case of the third molar extraction model, male and female patients with acute postsurgical pain following the removal of one or more bony impacted third molars are participants. In the case of the bunionectomy surgery model, male or female patients requiring primary unilateral first metatarsal bunionectomy surgery alone or with ipsilateral hammertoe repair (without additional collateral procedures) under regional anesthesia (e.g., Mayo block) are participants. Within 4 to 6 hours after completion of either surgery, patients who are experiencing moderate or severe pain, as measured by a visual analog pain intensity scale (VAS ≥ 50 mm) and by a categorical pain intensity scale (moderate or severe pain descriptor), and who meet all other inclusion/exclusion criteria are admitted to the study. Patients are randomly assigned to receive test and reference or control treatments. Pain intensity (VAS and categorical), pain relief (categorical) and whether pain is half-gone is recorded by the patient under the supervision of the investigator or study coordinator at the various time points: Baseline (0 hour - pain intensity only), 15, 30 and 45 minutes, and at 1, 1.5, 2, 3, 4, 5, 6, 7, and 8 hours after administration of study medication, and immediately prior to the first rescue dose. Time to onset of perceptible and meaningful pain relief is evaluated using the two stopwatch method. Patients record their global evaluation of study medication at the completion of the 8-hour assessment or at the time of first rescue medication use. Efficacy endpoints include Total Pain Relief (TOTPAR), Sum of Pain Intensity Difference (SPID) and Sum of Pain Relief Intensity Difference (SPRID) at various time points, Time to First Rescue, Time Specific Pain Intensity Difference (PID), Time Specific Pain Relief (PR), Peak Pain Intensity Difference (PPID), Peak Pain Relief (PPR), Time to Confirmed Perceptible Pain Relief (stopwatch) and Time to Meaningful Pain Relief (stopwatch) and Patient Global Evaluation.

In the case of chronic pain of osteoarthritis, the therapeutic benefits associated with the administration of the dosage form in some embodiments is demonstrated in repeated dose randomized, double-blind, controlled studies are assigned to receive test and reference or control treatments. Typically, patients who meet the American College of Rheumatology criteria for knee and/or hip OA are washed off their analgesics for 2 to 7 days to allow for pain of moderate to severe intensity to return (Babul et al. Journal of Pain and Symptom Management 2004;28:59-71). Once a stable baseline pain score is established, patients are randomized to treatment, usually for a period of one to 12 weeks. Pain, joint stiffness and physical function can be measured with a multidimensional instrument, such as the WOMAC, quality of life with the SF-12 or SF-36 and adverse events with a non-directed questionnaire at baseline and at post-baseline return visits. Response to pain, stiffness, physical function, quality of life and adverse events are calculated as change from baseline and compared between treatments. Sample sizes in the studies are sufficient to demonstrate the increased therapeutic benefit of the invention.

In the case of postherpetic neuralgia, the analgesic efficacy of the invention may be demonstrated in repeated dose randomized double-blind, controlled studies. Patients are randomized to receive are assigned to receive test and reference or control treatments. Patients with a history of postherpetic neuralgia ≥ 3 months and pain of at least moderate intensity are enrolled in the study. Patients with hypersensitivity to study medications, a history of drug or alcohol abuse and significant pain of alternate etiology are generally excluded. Patients meeting study eligibility criteria are "washed off their analgesics in some embodiments, generally for 2 to 7 days to allow for pain of moderate to severe intensity to return. Once a stable baseline pain score is established, patients are randomized to treatment, usually for a period of 4 to 12 weeks. Pain intensity is assessed one to several times a day and in some cases only once weekly using VAS, categorical or numerical rating scales. Various dimensions of neuropathic pain may be assessed, including steady pain (ongoing pain), brief pain (paroxysmal pain) and skin pain (allodynia). Pain may also be assessed at scheduled clinic study visits. Pain may also be assessed using standardized pain scales such as the Neuropathic Pain Symptom Inventory (Bouhassira et al., Pain 2004;108:248-57), or interference measures of the Brief Pain Inventory (Cleeland, CRC Press, 1991:293-305 & Ann Acad Med Singapore 1994;23:129-38). Patient Global Impression of Change (Farrar et al., Pain 2001;94:149-580) and quality of life may be assessed. Examples of randomized, placebo or active studies conducted in postherpetic neuralgia are known in the art (e.g., Watson and Babul, Neurology 1998;50:1837-41). Adverse events may be assessed using a non-directed questionnaire or a symptom checklist. Response to pain, function, quality of life and adverse events are calculated as change from baseline and compared between treatments.

As used herein, the "Orange Book" as it is commonly known is the database of Approved Drug Products with Therapeutic Equivalence Evaluations maintained by or on behalf of the US Food and Drug Administration, (http://www.fda.gov/cder/ob/default.htm, accessed February 15, 2008), the content of which is hereby incorporated by reference.

"Drug", "drug substance", "substance", "therapeutic", "therapeutic agent", "pharmacological agent", "pharmaceutical agent", "active agent", "active ingredient", "agent" "active pharmaceutical ingredient" or "API" are used interchangeably and are intended to have their broadest interpretation as to any therapeutically active substance which is delivered to a living organism to furnish pharmacological activity or other direct effect in the diagnosis, cure, mitigation, treatment, or prevention of a disease, or to affect the structure or any function of the human body. In general, this includes therapeutic agents in all of the major therapeutic areas.

As used herein, "dosage forms" is interchangeable with "formulations", "compositions", "pharmaceutical compositions", "pharmaceutical preparations", "preparations" or "doses". The foregoing compositions, when referring to levorphanol, means extended release dosage forms comprising unsalified levorphanol (/ 3-hydroxy-N-methylmorphinan base), a pharmaceutically acceptable salt of levorphanol, the racemate of levorphanol (*d,l* 3-hydroxy-N-methylmorphinan), non-stoichiometric ratios of *l* 3-hydroxy-N-methylmorphinan and *d* 3-hydroxy-N-methylmorphinan, pharmaceutically acceptable salts thereof, or esters, solvates, complexes, polymorphs and hydrates thereof, or mixture thereof.

The term "subject" for purposes of treatment is used interchangeably with "patient", "male", "female" and "human", and includes any human subject.

"Pharmaceutically or therapeutically acceptable excipient or carrier" or "excipient" refers to a substance which does not interfere with the effectiveness or the biological activity of the active ingredients and which is not toxic to the subject. In some embodiments of the present invention, pharmaceutically or therapeutically acceptable excipients or carriers may play a role in imparting or optimizing the rate and extent of absorption or levorphanol or additional drugs in the pharmaceutical composition. In some embodiments of the present invention, pharmaceutically or therapeutically acceptable excipients or carriers may play a role in stabilizing the levorphanol or additional drugs in the pharmaceutical composition. Excipients are widely known in the art (see, for example, FDA EAFUS database; FDA Food Additives Status List; FDA GRAS list and database; FDA Color Additive Status List; FDA Inactive Ingredients Database; Rowe, Sheskey and Owen, Handbook of Pharmaceutical Excipients, APhA Publications; 5th edition (2006); Goodman & Gilman's The Pharmacological Basis of Therapeutics (Brunton, Lazo and Parker, eds, 11th ed., McGraw Hill (2005); Remington: The Science and Practice of Pharmacy, 21st ed, Lippincott Williams & Wilkins (2005); Martindale: The Complete Drug Reference, 35th Edition, Pharmaceutical Press (2007); United States Pharmacopeia-National Formulary (USP-NF), (USP 30 - NF 25, 2007), the International Programme on Chemical Safety and Health Canada's List of Acceptable Non-medicinal Ingredients).

As used herein, the term "aversive", "aversive agents", "aversion producing agents" and "aversive compounds" means to compounds contained within the dosage form that produce an aversive, undesirable, repugnant, distasteful, unpleasant, unacceptable physiologic effect, unacceptable psychic effect, or that pharmacologically block or reduce physiologic effects sought by recreational drug users, addicts and drug abusers, including one or more of the following effects: mood alterations; euphoria, pleasure; a feeling of high; a feeling of drug liking; anxiolysis; sedation; calmness; a state of relaxation; psychotomimesis; hallucinations; alterations in perception, cognition and mental focus; drowsiness; and psychological reinforcement.

The term "tampering" or "tamper" means any manipulation by mechanical, thermal, chemical and/or pharmacologic means which changes the physical or chemical properties of the dosage form, e.g., to liberate the levorphanol for immediate release if it is in extended release form, or to make the levorphanol available for inappropriate use such as administration by an alternate route, e.g., parenterally. The tampering can be, e.g., by means of crushing, shearing, grinding, mechanical extraction, solvent extraction, solvent immersion, combustion, heating or any combination thereof.

The term "abuse", "drug abuse", "opioid abuse", "recreational drug use" and "drug misuse" in the context of the present invention means, use: (i) in quantities or by methods and routes of administration that do not conform to standard medical practice; (ii) outside the scope of specific instructions for use provided by a qualified medical professional; (iii) outside the supervision of a qualified medical professional; (iv) outside the approved instructions on proper use provided by the drug's legal manufacturer; (v) which is not in specifically approved dosage forms for medical use as pharmaceutical agents; (vi) where there is an intense desire for and efforts to procure same; (vii) compulsive use; (viii) through acquisition by manipulation of the medical system, including falsification of medical history, symptom intensity, disease severity, patient identity, doctor shopping, prescription forgeries; (ix) where there is impaired control over use; (x) despite harm; (xi) by procurement from non-medical sources; (xii) by others through sale or diversion by the individual into the non-medical supply chain; (xiii) for medically unapproved or mood altering purposes.

The term "mood altering" is defined for purposes of the present invention to mean that the "high", "liking", pleasurable, euphoric, alerting, calming, anxiolytic, auditory and visual perception altering, relaxing, psychotomimetic, rewarding and reinforcing following use of levorphanol.

The term "abuse resistant", "abuse deterrent", "tamper resistant", "deter abuse" and "resist abuse" (as well of the words "resist" or "deter" when applied levorphanol) are used interchangeably in the context of the present invention and include pharmaceutical compositions, methods and processes that resist, deter, discourage, diminish, delay and/or frustrate: (i) the physical, chemical, thermal or pharmacologic manipulation or tampering of the dosage form (e.g., crushing, shearing, grinding, chewing, dissolving, melting, needle aspiration, inhalation, insufflation, extraction by mechanical, thermal and chemical means, and/or filtration); (ii) use or misuse of the dosage form outside the scope of specific instructions for use provided by a qualified medical professional; (iii) use outside the supervision of a qualified medical professional; (iv) use outside the approved instructions on proper use provided by the drug's legal manufacturer (e.g., intravenous use, intranasal use, inhalational use and oral ingestion to provide high peak concentrations, use in excess quantities, etc.); (v) the conversion of an extended release dosage form of the invention into a more immediate release form; (vi) the intentional and iatrogenic increase in physical and psychic effects sought by recreational drug users, addicts, and patients with pain who have an addiction disorder; (vii) attempts to procure the dosage form by manipulation of the medical system and from non-medical sources; (viii) the sale or diversion of the dosage form into the non-medical supply chain and for medically unapproved or unintended mood altering purposes; (ix) the intentional, unintentional or accidental attempts at otherwise changing the physical, pharmaceutical, pharmacological and/or medical properties of the dosage form from what was intended by the manufacturer; (x) the psychic, pleasurable, reinforcing or rewarding effects of the dosage form when used as directed or when used outside the approved instructions on proper use provided by the drug's legal manufacturer.

"Therapeutically effective amount" or "therapeutically-effective" refers to the amount of an active agent sufficient to induce a desired biological result. That result may be alleviation of the signs, symptoms, or causes of a disease, or any other desired alteration of a biological system.

"Therapeutically effective amount of levorphanol" refers to the amount of oral levorphanol sufficient to prevent, to cure, or at least partially arrest a medical disorder, disease, sign or symptom for which the levorphanol has been prescribed to a subject.

The term "effective amount" means the quantity of a compound according to the invention necessary to prevent, to cure, or at least partially arrest a medical disorder, disease, sign or symptom for which the levorphanol has been prescribed to a subject.

The term "pharmaceutically acceptable salt" as used herein refers to a salt which is toxicologically safe for human and animal administration. Nonlimiting examples of salts include hydrochlorides, hydrobromides, hydroiodides, sulfates, bisulfates, nitrates, citrates, tartrates, bitartrates, phosphates, malates, maleates, napsylates, fumarates, succinates, acetates, terephlhalates, pamoates and pectinates. In some embodiments, the pharmaceutical composition is a salt or complex of inorganic cation salts, organic salts such primary, secondary, tertiary and quaternary amines include substituted amines

As used herein, "levorphanol responsive conditions", levorphanol responsive medical conditions", "opioid responsive conditions", "opioid responsive medical conditions", "levorphanol or opioid responsive medical conditions", "in need to levorphanol", and the like refer to any medical condition in which levorphanol can be employed for a therapeutically beneficial outcome.

The terms "medical condition", "malady", "disease", "disorder" and "pathological states" are used interchangeably and are intended to have their broadest interpretation to refer to any physiologic, pathologic or pathophysiologic state in a human or other mammal that can be prevented, treated, managed or altered to produce a desired, usually beneficial effect.

In some embodiments, the oral levorphanol is intended to prevent or treat pain. A co-administered drug (in the same or different dosage form, by any route of administration) may be used to provide additive, complementary, superadditive or synergistic therapeutic analgesic effects, including other NSAIDs, NO-NSAIDs, COX-2 selective inhibitors, acetaminophen, tramadol, local anesthetics, antidepressants, beta adrenergic agonists, alpha-2 agonists, selective prostanoid receptor antagonists, cannabinoid agonists, other opioid receptor agonists, NMDA receptor antagonists, gabapentin, pregabalin, gabapentinoids, neuronal nicotinic receptor agonists, calcium channel antagonists, sodium channel blockers, superoxide dismutase mimetics, p38 MAP kinase inhibitors, TRPV1 agonists, dextromethorphan, dextrorphan, ketamine, glycine receptor antagonists, antiepileptics, and any other drugs that can be shown by a person proficient in the art to prevent or treat pain.

In some embodiments, a preferred combination includes levorphanol with acetaminophen.

In other embodiments, particularly preferred combinations include levorphanol with an NSAID. Nonsteroidal anti-inflammatory drugs typically have analgesic, anti-inflammatory, and antipyretic properties. Their mode of action appears to involve inhibition of cyclooxygenases (COX-1 and COX-2), leukotriene biosynthesis, and antibradykinin activity. NSAIDs may be non-selective (inhibit COX-1 and COX-2 isozymes) or COX-2 selective (preferentially inhibit the COX-2 isozymes).

In other embodiments, more preferred combinations include levorphanol with other opioids.

In certain preferred embodiments of the present invention, an effective amount of another drug to treat the levorphanol responsive condition, a levorphanol related side effect (e.g., laxative, CNS stimulant or anti-emetic) or a co-existing medical condition may be incorporated into the dosage form. Such a coadministered drug may be in any form, including immediate release, controlled release and delayed release. The co-administered drug may be incorporated at a therapeutic dose or a subtherapeutic dose. In certain preferred embodiments of the present invention where the dosage form is a delayed onset, extended release, an effective amount of another drug to treat the levorphanol responsive condition in immediate release form may be particularly advantageous. In certain preferred embodiments, an NSAID, acetaminophen or a COX-2 inhibitor in immediate release form may be advantageously incorporated into the dosage form. In some embodiments, another drug to treat the same condition as the oral levorphanol or to treat a different condition may be incorporated into the oral dosage form, where the other drug shares one, or more of the dissolution rate specifications, GI delivery and release specifications and pharmacokinetic parameter specifications as the oral levorphanol in the dosage form.

### METHODS OF CARRYING OUT THE INVENTION

### CONTROLLED-RELEASE DOSAGE FORMS

In some preferred embodiments, the dosage form comprises a therapeutically effective amount of levorphanol or a pharmaceutically acceptable salt of levorphanol, or a mixture thereof and controlled release material to render said dosage form suitable for extended release in a human patient; said material substantially providing said release through diffusion-control from a matrix or a reservoir. A matrix diffusion-control system consists of a release rate controlling material such as a polymer with drug uniformly dissolved or dispersed in it. A reservoir diffusion-control system separates the drug compartment from a polymer membrane that presents a barrier to diffusion to yield a drug flux which may be either zero order or first order. Osmotic delivery extended release systems are a subset of diffusion-controlled dosage forms

Extended release levorphanol may be formulated as a hydrophobic matrix dosage forms. Such compositions do not necessarily require a polymer as controlled release material to provide an extended release profile. In such dosage forms, the rate controlling means are the water insoluble material described herein. Release rate modifiers are usually incorporated to modulate the rate of release of drug.

Extended release levorphanol may be formulated as a hydrophilic matrix, where swellable polymers serve as the controlled release material. The polymers are usually swellable polymers which expand upon contacting aqueous media for prolonged periods to form a gel layer which then modulates drug release. Such dosage forms control release rate of active through erosion and diffusion processes. Among the most widely used polymers are hypromellose, sodium alginate, polyethylene oxide and xantham gum.

Extended release levorphanol may be formulated as a reservoir dosage forms, which usually comprise a reservoir (which is the core tablet) often devoid of controlled release material and a coating layer which provides the controlled release means to the dosage form. The general method of release of active from such dosage forms is by diffusion. Reservoir dosage forms may comprise multiparticulates or may be a tablet core which is coated. The controlled release material is commonly acrylic copolymers or a cellulose, with pore formation provided by compounds such as hypromellose, as described herein

Extended release levorphanol may be coated with controlled release polymer over a core, with the core itself in immediate release form or incorporating additional controlled release means of the active drug.

### Matrix-Based Dosage Forms

A matrix system comprises homogeneously mixed active drug and pharmaceutically acceptable excipients. Matrix based extended release dosage forms can have a number of advantages, including, manufacture with conventional equipment, reduced development costs, the ability to accommodate active drugs with a variety of physicochemical attributes and varying drug loads.

In some embodiments, the extended release formulations of the present invention are provided as matrix-based dosage forms. Matrix formulations according to the invention may include hydrophilic, e.g., water-soluble, and/or hydrophobic, e.g., water-insoluble, polymers. The matrix formulations of the present invention may optionally be prepared with functional coatings, which may be enteric, e.g., exhibiting a pH-dependent solubility, or non-enteric, e.g., exhibiting a pH-independent solubility.

Matrix formulations of the present invention may be prepared by using, for example, direct compression or wet granulation. A functional coating, as noted above, may then be applied in accordance with the invention. Additionally, a barrier or sealant coat may be applied over a matrix tablet core prior to application of a functional coating. The barrier or sealant coat may serve the purpose of separating an active ingredient from a functional coating, which may interact with the active ingredient, or it may prevent moisture from contacting the active ingredient.

In a matrix-based dosage form in accordance with the present invention, the levorphanol and optional pharmaceutically acceptable excipient(s) are dispersed within a polymeric matrix, which typically comprises one or more water-soluble polymers and/or one or more water-insoluble polymers. The drug may be released from the dosage form by diffusion and/or erosion.

In one embodiment, a matrix-based dosage form comprises levorphanol, a filler such as starch, lactose, or microcrystalline cellulose; a controlled-release polymer, such as hydroxypropyl methylcellulose or polyvinyl pyrrolidone; a disintegrant, such crospovidone, or starch; a lubricant, such as magnesium stearate or stearic acid; a surfactant, such as sodium lauryl sulfate or polysorbates; and a glidant, such as colloidal silicon dioxide or talc. The amounts and types of polymers, and the ratio of water-soluble polymers to water-insoluble polymers in the inventive formulations are generally selected to achieve a desired release profile of levorphanol. For example, by increasing the amount of water insoluble-polymer relative to the amount of water soluble-polymer, the release of the drug may, in some embodiments, be delayed or slowed. This is due, in part, to an increased impermeability of the polymeric matrix, and, in some cases, to a decreased rate of erosion during transit through the GI tract.

The controlled-release dosage form may optionally include a controlled release material which is incorporated into a matrix along with the levorphanol, or which is applied as a controlled release material coating over a substrate comprising the drug (the term "substrate" encompassing beads, pellets, spheroids, tablets, tablet cores, etc). The controlled release material may be hydrophobic or hydrophilic as desired. The oral dosage form according to the invention may be provided as, for example, granules, spheroids, pellets or other multiparticulate formulations. An amount of the multiparticulates which is effective to provide the desired dose of levorphanol over time may be placed in a capsule or may be incorporated in any other suitable oral solid form, e.g., compressed into a tablet. On the other hand, the oral dosage form according to the present invention may be prepared as a tablet core coated with a controlled-release coating, or as a tablet comprising a matrix of drug and controlled release material, and optionally other pharmaceutically desirable ingredients (e.g., diluents, binders, colorants, lubricants, etc.). The controlled release dosage form of the present invention may also be prepared as a bead formulation or an osmotic dosage formulation.

In certain preferred embodiments of the present invention, the controlled-release formulation is achieved via a matrix (e.g. a matrix tablet) which includes a controlled-release material as set forth below. A dosage form including a controlled-release matrix provides in-vitro dissolution rates of levorphanol within the preferred ranges and that releases the levorphanol in a pH-dependent or pH-independent manner. The materials suitable for inclusion in a controlled-release matrix will depend on the method used to form the matrix. The oral dosage form may contain between 1% and 99% (by weight) of at least one hydrophilic or hydrophobic controlled release material.

Any pharmaceutically acceptable hydrophobic or hydrophilic controlled-release material which is capable of imparting controlled-release of the levorphanol may be used in accordance with the present invention. Preferred controlled-release polymers include alkylcelluloses such as ethylcellulose, acrylic and methacrylic acid polymers and copolymers, and cellulose ethers, especially hydroxyalkylcelluloses (e.g., hydroxypropylmethylcellulose) and carboxyalkylcelluloses. Preferred acrylic and methacrylic acid polymers and copolymers include methyl methacrylate, methyl methacrylate copolymers, ethoxyethyl methacrylates, cynaoethyl methacrylate, aminoalkyl methacrylate copolymer, poly(acrylic acid), poly(methacrylic acid), methacrylic acid alkylamine copolymer, poly(methyl methacrylate), poly(methacrylic acid)(anhydride), polymethacrylate, polyacrylamide, poly(methacrylic acid anhydride), glycidyl methacrylate copolymers, ethylcellulose, cellulose acetate cellulose propionate, cellulose acetate propionate, cellulose acetate butyrate, cellulose acetate phthalate, cellulose triacetate, poly (methyl methacrylate), poly (ethyl methacrylate), poly (butyl methacrylate), poly (isobutyl methacrylate), and poly (hexyl methacrylate), poly (isodecyl methacrylate), poly (lauryl methacrylate), poly (phenyl methacrylate), poly (methyl acrylate), poly (isopropyl acrylate), poly (isobutyl acrylate), poly (octadecyl acrylate), poly (ethylene), poly (ethylene) low density, poly (ethylene) high density, poly (ethylene oxide), poly (ethylene terephthalate), poly (vinyl isobutyl ether), poly (vinyl acetate), poly (vinyl chloride) or polyurethane, and/or mixtures thereof. Certain preferred embodiments utilize mixtures of any of the foregoing controlled-release materials in the matrices of the invention.

The matrix also may include a binder. In such embodiments, the binder preferably contributes to the controlled-release of the levorphanol from the controlled-release matrix. Preferred hydrophobic binder materials are water-insoluble with more or less pronounced hydrophilic and/or hydrophobic trends. Preferred hydrophobic binder materials which may be used in accordance with the present invention include digestible, long chain (C₈-C₅₀, especially C₁₂-C₄₀), substituted or unsubstituted hydrocarbons, such as fatty acids, fatty alcohols, glyceryl esters of fatty acids, mineral and vegetable oils, natural and synthetic waxes and polyalkylene glycols. Preferably, the hydrophobic binder materials useful in the invention have a melting point from about 30 to about 200 °C, preferably from about 45 to about 90 °C. When the hydrophobic material is a hydrocarbon, the hydrocarbon preferably has a melting point of between 25 and 90 °C. Of the long chain (C₈-C₅₀) hydrocarbon materials, fatty (aliphatic) alcohols are preferred. The oral dosage form may contain up to 98% (by weight) of at least one digestible, long chain hydrocarbon.

The oral dosage form contains up to 98% (by weight) of at least one polyalkylene glycol. The hydrophobic binder material may comprise natural or synthetic waxes, fatty alcohols (such as lauryl, myristyl, stearyl, cetyl or preferably cetostearyl alcohol), fatty acids, including but not limited to fatty acid esters, fatty acid glycerides (mono-, di-, and tri-glycerides), hydrogenated fats, hydrocarbons, normal waxes, stearic acid, stearyl alcohol and hydrophobic and hydrophilic materials having hydrocarbon backbones. Suitable waxes include, for example, beeswax, glycowax, castor wax and carnauba wax. For purposes of the present invention, a wax-like substance is defined as any material which is normally solid at room temperature and has a melting point of from about 30 to about 100 °C.

In certain preferred embodiments, a combination of two or more hydrophobic binder materials are included in the matrix formulations. If an additional hydrophobic binder material is included, it is preferably selected from natural and synthetic waxes, fatty acids, fatty alcohols, and mixtures of the same. Examples include beeswax, carnauba wax, stearic acid and stearyl alcohol. This list is not meant to be exclusive.

One particular suitable controlled-release matrix comprises at least one water soluble hydroxyalkyl cellulose, at least one C₁₂-C₃₆, preferably C₁₄-C₂₂, aliphatic alcohol and, optionally, at least one polyalkylene glycol. The hydroxyalkyl cellulose is preferably a hydroxy (C₁ to C₆) alkyl cellulose, such as hydroxypropyl cellulose, hydroxypropyl methylcellulose and, especially, hydroxyethyl cellulose. The amount of the at least one hydroxyalkyl cellulose in the present oral dosage form will be determined, inter alia, by the precise rate of the levorphanol release required. The aliphatic alcohol may be, for example, lauryl alcohol, myristyl alcohol or stearyl alcohol. In more preferred embodiments of the present oral dosage form, however, the at least one aliphatic alcohol is cetyl alcohol or cetostearyl alcohol. The amount of aliphatic alcohol in the present oral dosage form will be determined, as above, by the precise rate of the levorphanol release required. It will also depend on whether at least one polyalkylene glycol is present in or absent from the oral dosage form. In the absence of at least one polyalkylene glycol, the oral dosage form preferably contains between 20% and 50% (by wt) of the aliphatic alcohol. When a polyalkylene glycol is present in the oral dosage form, then the combined weight of the aliphatic alcohol and the polyalkylene glycol preferably constitutes between 20% and 50% (by wt) of the total dosage.

In one preferred embodiment, the ratio of, e.g., the at least one hydroxyalkyl cellulose or acrylic resin to the at least one aliphatic alcohol/polyalkylene glycol determines, to a considerable extent, the release rate of the levorphanol from the formulation. A ratio of the hydroxyalkyl cellulose to the aliphatic alcohol/polyalkylene glycol of between 1:2 and 1:4 is preferred, with a ratio of between 1:3 and 1:4 being more preferred.

The polyalkylene glycol maybe, for example, polypropylene glycol or, which is preferred, polyethylene glycol. The number average molecular weight of the at least one polyalkylene glycol is preferred between 1,000 and 15,000 especially between 1,500 and 12,000.

Another suitable controlled-release matrix comprises an alkylcellulose (especially ethylcellulose), a C₁₂ to C₃₆ aliphatic alcohol and, optionally, a polyalkylene glycol.

In one embodiment of the present invention, one or more release rate modifiers may be incorporated into the formulation, including, without limittauion, hydroxypropyl methylcellulose, cellulose acetate, powdered cellulose, cellulose acetate phthalate, hydroxyethyl cellulose, silicified microcrystalline cellulose, hydroxypropyl cellulose, hydroxyethylmethyl cellulose, low-substituted hydroxypropyl cellulose, carboxymethylcellulose, carboxymethylcellulose calcium, hypromellose acetate succinate, hypromellose phthalate, and ethylcellulose, and mixture thereof. Release rate modifiers can also have additional useful properties that optimize the formulation. In addition to the above ingredients, a controlled-release matrix may also contain suitable quantities of other materials, e.g., diluents, lubricants, binders, granulating aids, colorants, flavorants and glidants that are conventional in the pharmaceutical art.

In order to facilitate the preparation of a solid, controlled-release oral dosage form according to the invention there is provided, in a further aspect of the present invention, a process for the preparation of a solid, controlled-release oral dosage form according to the present invention comprising incorporating the levorphanol or a salt thereof in a controlled-release matrix. Incorporation in the matrix may be effected, for example, by (a) forming granules comprising at least one hydrophobic and/or hydrophilic material as set forth above (e.g., a water soluble hydroxyalkyl cellulose) together with the levorphanol; (b) mixing the at least one hydrophobic and/or hydrophilic material-containing granules with at least one C₁₂-C₃₆ aliphatic alcohol, and (c) optionally, compressing and shaping the granules. The granules may be formed by any of the procedures well-known to those skilled in the art of pharmaceutical formulation. For example, in one preferred method, the granules may be formed by wet granulating hydroxyalkyl cellulose/levorphanol with water. In a particular preferred embodiment of this process, the amount of water added during the wet granulation step is preferably between 1.5 and 5 times, especially between 1.75 and 3.5 times, the dry weight of the levorphanol.

In certain embodiments, the dosage form comprises a plurality of matrices described above.

The matrices of the present invention may also be prepared via a melt pellitization technique. In such circumstance, the levorphanol in finely divided form is combined with a binder (also in particulate form) and other optional inert ingredients, and thereafter the mixture is pelletized, e.g., by mechanically working the mixture in a high shear mixer to form the pellets (granules, spheres). Thereafter, the pellets (granules, spheres) may be sieved in order to obtain pellets of the requisite size. The binder material is preferably in particulate form and has a melting point above about 40 °C. Suitable binder substances include, for example, hydrogenated castor oil, hydrogenated vegetable oil, other hydrogenated fats, fatty alcohols, fatty acid esters, fatty acid glycerides, and the like.

Controlled-release matrices can also be prepared by, e.g., melt-granulation or melt-extrusion techniques. Generally, melt-granulation techniques involve melting a normally solid hydrophobic binder material, e.g. a wax, and incorporating a powdered drug therein. To obtain a controlled release dosage form, it may be necessary to incorporate a hydrophobic controlled release material, e.g. ethylcellulose or a water-insoluble acrylic polymer, into the molten wax hydrophobic binder material.

The hydrophobic binder material may comprise one or more water-insoluble wax-like thermoplastic substances possibly mixed with one or more wax-like thermoplastic substances being less hydrophobic than said one or more water-insoluble wax-like substances. In order to achieve controlled release, the individual wax-like substances in the formulation should be substantially non-degradable and insoluble in gastrointestinal fluids during the initial release phases. Useful water-insoluble wax-like binder substances may be those with a water-solubility that is lower than about 1:5,000 (w/w).

Specific examples of pharmaceutically acceptable carriers and excipients that may be used to formulate oral dosage forms of the present invention are described in the Handbook of Pharmaceutical Excipients, APhA Publications; 5 edition (January 5, 2006) incorporated by reference herein.

The multiparticulates may be overcoated with a controlled-release coating including a controlled-release material such as those described hereinabove. The controlled-release coating is applied to a weight gain of from about 5 to about 30%. The amount of the controlled-release coating to be applied will vary according to a variety of factors, e.g., the composition of the multiparticulate and the chemical and/or physical properties of the drug.

### Preparation of Coated Bead Formulations

In certain preferred embodiments of the present invention the oral solid controlled release dosage form of the present invention comprises a plurality of coated substrates, e.g., inert pharmaceutical beads such as nu pariel 18/20 beads. An aqueous dispersion of hydrophobic material is used to coat the beads to provide for the controlled release of the levorphanol. In certain preferred embodiments a plurality of the resultant stabilized solid controlled-release beads may be placed in a gelatin capsule in an amount sufficient to provide an effective controlled-release dose when ingested and contacted by an environmental fluid, e.g., gastric fluid or dissolution media.

The stabilized controlled-release bead formulations of the present invention slowly release the levorphanol, e.g., when ingested and exposed to gastric fluids, and then to intestinal fluids. The controlled-release profile of the formulations of the invention can be altered, for example, by varying the amount of overcoating with the aqueous dispersion of hydrophobic controlled release material, altering the manner in which the plasticizer is added to the aqueous dispersion of hydrophobic controlled release material, by varying the amount of plasticizer relative to hydrophobic controlled release material, by the inclusion of additional ingredients or excipients, by altering the method of manufacture, etc. The dissolution profile of the ultimate product may also be modified, for example, by increasing or decreasing the thickness of the controlled release coating.

Substrates coated with a therapeutically active agent are prepared, e.g. by dissolving the therapeutically active agent in water and then spraying the solution onto a substrate, for example, nu pariel 18/20 beads, using a Wuster insert. Optionally, additional ingredients are also added prior to coating the beads in order to assist the binding of the levorphanol to the beads, and/or to color the solution, etc. For example, a product which includes hydroxypropyl methylcellulose, etc. with or without colorant (e.g., Opadry™) may be added to the solution and the solution mixed (e.g., for about 1 hour) prior to application of the same onto the substrate. The resultant coated substrate may then be optionally overcoated with a barrier agent, to separate the therapeutically active agent from the hydrophobic controlled-release coating.

An example of a suitable barrier agent is one which comprises hydroxypropyl methylcellulose. However, any film-former known in the art may be used. It is preferred that the barrier agent does not affect the dissolution rate of the final product.

The substrates may then be overcoated with an aqueous dispersion of the hydrophobic controlled release material as described herein. The aqueous dispersion of hydrophobic controlled release material preferably further includes an effective amount of plasticizer, e.g. triethyl citrate. Pre-formulated aqueous dispersions of ethylcellulose, such as Aquacoat™ or Surelease™, may be used. If Surelease™ is used, it is not necessary to separately add a plasticizer. Alternatively, pre-formulated aqueous dispersions of acrylic polymers such as Eudragit™ can be used.

### Coatings

The dosage forms of the present invention may optionally be coated with one or more coatings suitable for the regulation of release or for the protection of the formulation. In one embodiment, coatings are provided to permit either pH-dependent or pH-independent release, e.g., when exposed to gastrointestinal fluid. When a pH-independent coating is desired, the coating is designed to achieve optimal release regardless of pH-changes in the environmental fluid, e.g., the GI tract. Other preferred embodiments include a pH-dependent coating that releases the levorphanol in desired areas of the gastrointestinal (GI) tract, e.g., the stomach or small intestine, such that an absorption profile is provided which is capable of providing at least about twelve hour and preferably up to twenty-four hour analgesia to a patient. It is also possible to formulate compositions which release a portion of the dose in one desired area of the GI tract, e.g., the stomach, and release the remainder of the dose in another area of the GI tract, e.g., the small intestine.

Formulations according to the invention that utilize pH-dependent coatings may also impart a repeat-action effect whereby unprotected drug is coated over an enteric coat and is released in the stomach, while the remainder, being protected by the enteric coating, is released further down the gastrointestinal tract. Coatings which are pH-dependent may be used in accordance with the present invention include a controlled release material such as, e.g., shellac, cellulose acetate phthalate (CAP), polyvinyl acetate phthalate (PVAP), hydroxypropyl methylcellulose phthalate, and methacrylic acid ester copolymers, zein, and the like.

In another preferred embodiment, the present invention is related to a stabilized solid controlled dosage form comprising the levorphanol coated with a hydrophobic controlled release material selected from (i) an alkylcellulose; (ii) an acrylic polymer; or (iii) mixtures thereof. The coating may be applied in the form of an organic or aqueous solution or dispersion.

In certain preferred embodiments, the controlled release coating is derived from an aqueous dispersion of the hydrophobic controlled release material. The coated substrate containing the levorphanol (e.g., a tablet core or inert pharmaceutical beads or spheroids) is then cured until an endpoint is reached at which the substrate provides a stable dissolution. The curing endpoint may be determined by comparing the dissolution profile (curve) of the dosage form immediately after curing to the dissolution profile (curve) of the dosage form after exposure to accelerated storage conditions of, e.g., at least one month at a temperature of 40 °C and a relative humidity of 75%.

In preferred embodiments, the controlled release coatings include a plasticizer such as those described herein.

In certain embodiments, it is necessary to overcoat the substrate comprising the levorphanol with a sufficient amount of the aqueous dispersion of e.g., alkylcellulose or acrylic polymer, to obtain a weight gain level from about 2 to about 50%, e.g., about 2 to about 25% in order to obtain a controlled-release formulation. The overcoat may be lesser or greater depending upon the physical properties of the therapeutically active agent and the desired release rate, the inclusion of plasticizer in the aqueous dispersion and the manner of incorporation of the same, for example.

### Targeted Gastrointestinal Delivery

Targeted delivery of the levorphanol dosage form of the present invention for release and subsequent absorption are achieved to provide delayed onset, extended release dosage forms and other extended release dosage forms. In addition, conventional extended release products which release the active drug rapidly on ingestion may be coated or embedded with further controlled release material designed to provide a lag time before release of drug upon ingestion.

A wide variety of methods for the preparation of delayed onset dosage form are known in the art. These methods may be employed for the preparation of delayed onset dosage forms of the invention, including but not limited to: (i) Prodrug approach: in some embodiments such products control the rate of release of active drug by azo-bond conjugates, glycoside conjugates, glucuronide conjugates, cyclodextrin conjugates, dextran conjugates, polypeptide conjugates; (ii) Polymeric coating: in some embodiments, such products control the release of active drug by coating with pH sensitive polymers and coating with biodegradable polymers; (iii) Embedding in matrices: in some embodiments, such products control the release of active drug by embedding in pH sensitive matrices, embedding in biodegradable hydrogels and matrices (e.g., amylose, chondroitin sulfate, chitosan, inulin, dextran, guar gum, pectin). Other approaches include the use of time dependent systems, Pulsincap™, CODDES™ and intestinal pressure controlled delivery systems.

In some embodiments, the need for a rapid initial dose may require that a portion of the dose (e.g., up to about 1%, or 3%, or 5%, or 7%, or 10%, or 12%, or 15%, or 17%, or 20%, or 22%, or 25%, or 30%) is provided without delay as an immediate release dosage form (e.g., without limitation, a capsule within a capsule, a tablet within a capsule, an immediate release overcoat on a tablet or a capsule, an immediate release solution, suspension, powder or matrix within an extended release capsule) in order to achieve, for example, immediate symptom relief

An extended release tablet or capsule formulation may be overcoated with one or more polymers to provide levorphanol release in the appropriate gastrointestinal environment (defined, in some embodiments by location in the GI tract, pH at the point of release, osmotic pressure at the point of release, hydration, microbial flora, and/or the time after ingestion at the point of release).

In some embodiments, the dosage form of the invention is in the form of a compressed tablet, or a capsule, said tablet or capsule coated with a polymer to retard or delay its release to achieve the objectives of the invention, said polymers including polymethacrylates (copolymerisate of methacrylic acid and either methylmethacrylate or ethyl acrylate (Eudragit™), cellulose based polymers e.g. cellulose acetate phthalate (Aquateric™) or polyvinyl derivatives e.g. polyvinyl acetate phthalate (Coateric™).

In some embodiments, the dosage form of the invention is in the form of a compressed tablet or a capsule, said tablet or capsule coated with one or more anionic polymers with methacrylic acid as a functional group (Eudragit™ polymer, Evonik Degussa, Darmstadt, Germany) to retard or delay its release to achieve the objectives of the invention, said polymers including Eudragit™ L 30 D-55 or Eudragit™ L 100-55 which dissolve in the duodenum or at about pH > 5.5, or Eudragit™ L 100 which dissolves in the jejunum or at a pH of about 6, or Eudragit™ S100, which dissolves in the ileum or at a pH o > 7.0, or Eudragit™ FS 30D, which dissolves in the colon or at a pH of about 6, which dissolve at a pH> 7.0.

In some embodiments, the dosage form of the invention can provide delayed and subsequently ileo-colonic or colonic release over an extended period of time (an extended release) by use of multiple polymers. In one embodiment, at the center of the dosage form is a core containing the levorphanol. The levorphanol is then coated with one or more layers of polymers that permit the drug to pass through the stomach, duodenum and jejunum (and optionally the ileum) without substantial absorption. As the dosage form reaches the alkaline pH of the ileum and colon (or optionally, upon arrival near or in the colon, for example upon traversing the ileo-cecal junction), the polymer allows permeability to water and thereby allows drug to diffuse from the dosage form and be available for systemic absorption in the terminal ileum and/or in the colon.

In some embodiments, the dosage form of the invention is in the form of a capsule, said capsule made from materials known in the art, including gelatin, plasticizers, starch, hydroxypropylmethyl cellulose (HPMC), pullulan capsule.

In some embodiments of the invention, the levorphanol if formulated as an immediate release or controlled release tablet or capsule formulation. If used as prepared, the dosage form would usually release some of the levorphanol from the dosage form in the stomach, duodenum, jejunum and ileum. Some suitable coatings include US Patent No. 4,311,833; 4,377,568; 4,385,078; 4,457,907; 4,462,839; 4,518,433; 4,556,552; 4,606,909; 4,615,885; 4,670,287; 5,536,507; 5,567,423; 5,591,433; 5,597,564; 5,609,871; 5,614,222; 5,626,875; 5,629,001; and 6,608,075, all of which are incorporated herein in their entirety by reference.

In some embodiments of the invention, preferred coating compositions include alkyl and hydroxyalkyl celluloses and their aliphatic esters, e.g., methylcellulose, ethylcellulose, hydroxyethyl cellulose, hydroxypropyl cellulose, hydroxybutyl cellulose, hydroxyethylethyl cellulose, hydroxyprophymethyl cellulose, hydroxybutylmethyl cellulose, hydroxypropyl cellulose phthalate, hydroxypropylmethyl cellulose phthalate and hydroxypropylmethyl cellulose acetate succinate; carboxyalkyl celluloses and their salts, e.g., carboxymethylethyl cellulose; cellulose acetate phthalate; cellulose acetate trimellitate, polycarboxymethylene and its salts and derivatives; polyvinyl alcohol and its esters: polyvinyl acetate phthalate; polycarboxymethylene copolymer with sodium formaldehyde carboxylate; acrylic polymers and copolymers, e.g., methacrylic acid-methyl methacrylic acid copolymer and methacrylic acid-methyl acrylate copolymer; edible oils such as peanut oil, palm oil, olive oil and hydrogenated vegetable oils; polyvinylpyrrolidone; polyethylene glycol and its esters: natural products such as shellac, and zein.

In some embodiments of the invention, other preferred coatings include polyvinylacetate esters, e.g., polyvinyl acetate phthalate; alkyleneglycolether esters of copolymers such as partial ethylene glycol monomethylether ester of ethylacrylate-maleic anhydride copolymer or diethyleneglycol monomethylether ester of methylacrylate-maleic anhydride copolymer, N-butylacrylate-maleic anhydride copolymer, isobutylacrylate-maleic anhydride copolymer or ethylacrylate-maleic anhydride copolymer; and polypeptides resistant to degradation in the gastric environment, e.g., polyarginine and polylysine. Other suitable coatings and methods to make and use delayed onset, extended release, extended release, duodenal release, jejunal release, ileal release, ileo colonic release and colonic release pharmaceutical compositions and dosage forms of oral levorphanol are well known in the [see for example, Colonic Drug Delivery (page 287-294), Wilson CG, In: Modified- Release Drug Delivery Technology, Second Edition, Vol. 1, Rathbone MJ, Hadgraft J, Roberts MS, Lane ME (eds), Informa Healthcare USA Inc. 2008; Biopolymers and Colonic Delivery, Wilson CG, Mukherji G, Shah HK (pages 295-309), In: Modified- Release Drug Delivery Technology, Second Edition, Vol. 1, Rathbone MJ, Hadgraft J, Roberts MS, Lane ME (eds), Informa Healthcare USA Inc. 2008; Enteric Coating for Colonic Delivery, Shah HK, Mukherji G, Brogmann B, Wilson CG (pages 311-324), In: Modified- Release Drug Delivery Technology, Second Edition, Vol. 1, Rathbone MJ, Hadgraft J, Roberts MS, Lane ME (eds), Informa Healthcare USA Inc. 2008; Programmed Drug Delivery Systems and the Colon, Wilson CG, Shah HK, Lee WW, Brogmann B, Mukherji G (pages 325-335), In: Modified- Release Drug Delivery Technology, Second Edition, Vol. 1, Rathbone MJ, Hadgraft J, Roberts MS, Lane ME (eds), Informa Healthcare USA Inc. 2008; Targeting the Colon Using COLAL™ : A Novel Bacteria-Sensitive Drug Delivery System, McConnell EL, Basit AW (pages 343-348), In: Modified- Release Drug Delivery Technology, Second Edition, Vol. 1, Rathbone MJ, Hadgraft J, Roberts MS, Lane ME (eds), Informa Healthcare USA Inc. 2008; Remington: the science of Pharmacy Practice, 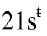 Edition, 2006, Lippincott, Williams & Wilkins, Baltimore, MD; Pharmaceutical Preformulation and Formulation: A Practical Guide from Candidate Drug Selection to Commercial Dosage Form. Gibson, M (ed). CRC Press, 2001; Niazi, S. Handbook of Pharmaceutical Manufacturing Formulations: Compressed Solid Products (Volume 1 of 6), CRC Press, 2004; Niazi, S. Mollet, H, Grubenmann A, Payne H. Formulation Technology: Emulsions, Suspensions, Solid Forms, Wiley-VCH, 2001; FDA list and database; FDA Color Additive Status List; FDA Inactive Ingredients Database; Rowe, Sheskey and Owen, Handbook of Pharmaceutical Excipients, APhA Publications; 5th edition (2006); Rowe, Sheskey and Quinn, Handbook of Pharmaceutical Excipients, 6 edition, Pharmaceutical Press; APhA Publications; 2009; Pharmaceutical Additives Electronic Handbook, Third Edition, Michael Ash (compiler), Synapse Information Resources, Inc.; 3 Cdr edition (February 19, 2007); and Health Canada's List of Acceptable Non-medicinal Ingredients; Patel et al. Therapeutic Opportunities in Colon-Specific Drug-Delivery System, Therapeutic Drug carrier Systems, 24(2), 147-202 (2007); Van den Mooter, Colon Drug Delivery, Expert Opin Drug Deliv (2006) 3(1):111-125; Singh. Modified-Release Solid Formulation for Colonic Delivery, Drug Deliv & Formulation 2007,1, 53-56; Kumar et al. Colon Targeted Drug System-An Overview, Current Drug Deliv, 2008, 5, 186-198; Jain et al. Target-Specific Drug Release to the Colon, Expert Opin Drug Deliv (2008) 5(5): 483-498; Wei et al. Selective Drug Delivery to the Colon Using Pectin-Coated Pellets, PDA J Pharm Sci Tech Vol. 62, No. 4, 2008; Schellekens et al. Pulsatile Drug Delivery to Ileo-Colonic Segments by Structured Incorporation of Disintegrants in pH-Responsive Polymer Coatings, J Controlled Release 132 (2008) 91-98; Coviello et al. Polysaccharide Hydrogels for Extended release Formulations, J Controlled Release 119 (2007) 5-24; McConnell et al. An in Vivo Comparison of Intestinal pH & Bacteria as Physiological Trigger Mechanisms for Colonic Targeting in Man, J Controlled Release 130 (2008) 154-160; Wei et al. Chitosan/Kollicoat SR 30D film-coated pellets of aminosalicylates for colonic drug delivery J Pharm Sci, 2009 Aug 4; Yassin et al. New targeted-colon delivery system: in vitro and in vivo evaluation using X-ray imaging. J Drug Target, 2009 Aug 5; Fan et al. Studies of chitosan/Kollicoat SR 30D film-coated tablets for colonic drug delivery. Int J Pharm, 2009 Jun 22;375(1-2):8-15; Yehia et al. Optimization of budesonide compression-coated tablets for colonic delivery. AAPS PharmSciTech, 2009;10(1):147-57; Chandran et al. Microspheres with pH modulated release: Design and characterization of formulation variables for colonic delivery J Microencapsul, 2008 Sep 22:1-11; Gohel et al. Design of a potential colonic drug delivery system of mesalamine. Pharm Dev Technol 2008;13(5):447-56; Maestrelli et al. Microspheres for colonic delivery of ketoprofen-hydroxypropyl-beta-cyclodextrin complex. Eur J Pharm Sci 2008 May 10;34(1):1-11; Maestrelli et al. Development of enteric-coated calcium pectinate microspheres intended for colonic drug delivery. Eur J Pharm Biopharm 2008 Jun;69(2):508-18; Fude et al. Preparation and in vitro evaluation of pH, time-based and enzyme-degradable pellets for colonic drug delivery. Drug Dev Ind Pharm 2007 Sep;33(9):999-1007; Oosegi et al. Novel preparation of enteric-coated chitosan-prednisolone conjugate microspheres and in vitro evaluation of their potential as a colonic delivery system. Eur J Pharm Biopharm, 2008 Feb;68(2):260-6; Nunthanid et al. Development of time-, pH-, and enzyme-controlled colonic drug delivery using spray-dried chitosan acetate and hydroxypropyl methylcellulose. Eur J Pharm Biopharm, 2008 Feb;68(2):253-9; Singh Characterization and relevance of physicochemical interactions among components of a novel multiparticulate formulation for colonic delivery. Int J Pharm, 2007 Aug 16;341(1-2):143-51; Gazzaniga et al. Oral Colon Delivery: Rationale & Time-based Drug Design Strategy. Discov Med, 2006 Dec;6(36):223-8; Singh Modified-release solid formulations for colonic delivery. Recent Pat Drug Deliv Formul, 2007;1(1):53-63; Akhgari et al. Combination of time-dependent & pH-dependent polymethacrylates as a single coating formulation for colonic delivery of indomethacin pellets. Int J Pharm, 2006 Aug 31;320(1-2):137-42; Ibekwe et al. A comparative in vitro assessment of the drug release performance of pH-responsive polymers for ileo-colonic delivery. Int J Pharm. 2006 Feb 3;308(1-2):52-60; Al-Saidan et al. In vitro and in vivo evaluation of guar gum-based matrix tablets of rofecoxib for colonic drug delivery. Curr Drug Deliv, 2005 Apr;2(2):155-63; Basit. Advances in colonic drug delivery. Drugs. 2005;65(14):1991-2007; Bott et al. In vivo evaluation of a novel pH- & time-based multiunit colonic drug delivery system. Aliment Pharmacol Ther. 2004 Aug 1;20(3):347-53; Qi et al. A novel pH- and time-dependent system for colonic drug delivery. Drug Dev Ind Pharm. 2003 Jul;29(6):661-7; 21: Shareef et al. Colonic drug delivery: an updated review. AAPS PharmSci. 2003;5(2):E17. Review. PubMed PMID: 12866944; Tuleu et al. Colonic delivery of sodium butyrate via oral route: acrylic coating design of pellets and in vivo evaluation in rats. Methods Find Exp Clin Pharmacol. 2001 Jun;23(5):245-53; Gupta et al. A novel pH- and time-based multi-unit potential colonic drug delivery system. I. Development. Int J Pharm. 2001 Feb 1;213(1-2):83-91; Gupta et al. A novel pH-& time-based multi-unit potential colonic drug delivery system. II. Optimization of multiple response variables. Int J Pharm. 2001 Feb 1;213(1-2):93-102; Muraoka et al. Evaluation of intestinal pressure-controlled colon delivery capsule containing caffeine as a model drug in human volunteers. J Control Release. 1998 Mar 2;52(1-2): 119-29; Niwa et al. Preparation & evaluation of a time-controlled release capsule made of ethylcellulose for colon delivery of drugs. J Drug Target. 1995;3(2):83-9. Erratum in: J Drug Target 96;3(6):478; Ashford et al. Targeting drugs to the colon: delivery systems oral administration. J Drug Target. 1994;2(3):241-57], all hereby incorporated by reference in their entirety.

In some embodiments of the invention, the coating material may be mixed with various excipients including plasticizers such as triethyl citrate, acetyl triethyl citrate, diethyl phthalate, dibutyl phthalate, dibutyl subacute, dibutyl tartrate, dibutyl maleate, dibutyl succinate and diethyl succinate and inert fillers such as chalk or pigments.

The composition and thickness of the coating may be selected to dissolve immediately upon contact with the digestive juice of the intestine. Alternatively, the composition and thickness of the external coating may be selected to be a time-release coating which dissolves over a selected period of time, as is well known in the art.

The amount of enteric coating depends on the particular coating composition used and is preferably sufficient to substantially prevent the absorption of in the stomach, duodenum, jejunum and, in some embodiments, the ileum as well.

In some embodiments of the invention, hydroxyalkyl celluloses and their aliphatic esters, carboxyalkyl celluloses and their salts, polycarboxymethylene and its salts and derivatives, polyvinyl alcohol and its esters, polycarboxymethylene copolymer with sodium formaldehyde carboxylates, poly-vinylpyrrolidone, and polyethylene glycol and its esters can be applied as coatings by first dissolving the compound in a minimum amount of water. Alcohol is then added to the point of incipient cloudiness. The mixture can then be applied by conventional techniques.

In some embodiments, application of cellulose acetate phthalate may be accomplished by simply dissolving the cellulose acetate phthalate in a minimum amount of alcohol and then applying by conventional techniques. Hydrogenated vegetable oils may be applied by first dissolving the oil in a minimal amount of a non-polymer solvent, such as methylene chloride, chloroform or carbon tetrachloride, then adding alcohol to the point of incipient cloudiness and then applying by conventional techniques.

In some embodiments, the dosage form of is a capsule or tablet, said dosage form pre-coated with an excipient, prior to coating with a polymer.

In some embodiments, the capsule dosage form is sealed after filling in the overlapping region of capsule body and cap by commonly known sealing techniques like banding or applying a sealing liquid and/or heat to the gap between capsule body and cap. Preferred is a sealing process, in which a sealing liquid which may include a solvent applied individually and uniformly to the external edge of the gap of a capsule to be sealed to form a liquid ring around the circumference of the capsule, removing excess sealing liquid from the exterior of the capsule and drying the capsule by applying thermal energy from outside. Such a sealing before coating can prevent problems e.g. with nonuniformity of the coating at the gap or development of fissures during storage under stressing conditions, which can lead to early leaking of the capsule content into the stomach. In some embodiments, the banding is achieved through the application of a ring of liquid gelatin or hypromellose on the external surface of the capsule. In some embodiments, a double-band sealing technique is used to ensure ensures that if an air bubble or unevenness occurs in the first band, it will be eliminated by the second application. Capsule banding can provide tamper evidence, deter counterfeiting, improve mechanical strength, provide color brand differentiation, improve product stability and reduce oxygen diffusion.

For controlled or slow release of the drug from the dosage form in some embodiments, the composition of the pH sensitive coating will dissolve, but other controlled release mechanisms will provide for slow release of the drug in the preferred anatomic location and/or at the preferred time after oral ingestion and/or in the preferred gastrointestinal environment, including preferred pH, preferred osmotic pressure in the lumen, preferred osmotic pressure in the dosage form, preferred hydration level, preferred microbial environment, preferred level of GI peristalsis or agitation.

In some embodiments, preferred coating materials are those which dissolve at a pH between 5 and 7.6, for example, > about 5, or > about 5.2, or > about 5.5, or > about 5.7, or > about 6, or > about 6.1, or > about 6.2, or > about 6.3, or > about 6.4, or > about 6.5, or > about 6.6, or > about 6.7, or > about 6.8, or > about 6.9, or > about 7, or > about 7.2, or > about 7.4, or > about 7.6.

In some embodiments, preferred coating materials include polymers such as cellulose acetate trimellitiate (CAT), hydroxypropylmethyl cellulose phthalate (HPMCP), polyvinyl acetate phthalate (PVAP), cellulose acetate phthalate (CAP) and shellac. In some embodiments, especially preferred materials for aqueous film coating are copolymers of methacrylic acid and ethyl acrylate, Eudragit™ L30D-55 (Evonik Degussa, Darmstadt, Germany).

In some embodiments, for release in the terminal ileum or colon any coating can be used which ensures that the dosage form does not disintegrate until it is reaches the desired location. In some embodiments, the coating may be one which is pH-sensitive, redox-sensitive or sensitive to particular enzymes or bacteria, such that the coating only dissolves or finishes dissolving in the colon. Thus the capsules will not release the drug until it is in the terminal ileum or colon.

In some embodiments, preferred coating materials are those which dissolve at a pH of 7 or above. Generally, such coatings only start to dissolve when they have left the stomach and passed through the duodenum and in cases the jejunum and/or terminal ileum. Generally, by the time the dosage form has reached the terminal ileum or colon the coating will have completely dissolved. Such a coating can be made from a variety of polymers including, without limitation, cellulose acetate trimellitiate (CAT) hydroxypropylmethyl cellulose phthalate (HPMCP), polyvinyl acetate phthalate (PVAP), cellulose acetate phthalate (CAP), shellac and copolymers of methacrylic acid and ethyl acrylate. In some embodiments, especially preferred materials for aqueous film coating are copolymers of methacrylic acid and ethyl acrylate to which a monomer of methylacrylate has been added during polymerization. (Eudragit™ FS 30 D, Evonik Degussa, Darmstadt, Germany). Due to the free carboxylic acid group the polymer dissolves at pH 7 or above making it particularly suitable for delivery into the colon.

It should be noted that when delayed but (subsequently) extended release of levorphanol is desired, upon dissolution or disintegration of the pH sensitive coating or material, a variety of mechanisms can provide extended release of the drug, including diffusion from matrix, membranes or pores, osmotic pressure, hydration, etc)

Using preparation Eudragit™ FS 30D a coating thickness of 5 to 15 mg polymer per cm² of capsule surface is preferred in some embodiments.

The colonic region is rich in microbial anaerobic organisms providing reducing conditions. Thus the coating may suitably comprise a material which is redox-sensitive. Such coatings may comprise azopolymers which can for example consist of a random copolymer of styrene and hydroxyethyl methacrylate, cross-linked with divinylazobenzene synthesized by free radical polymerization, the azopolymer being broken down enzymatically and specifically in the colon or may consist of disulphide polymers.

Other materials providing release in the colon are amylose, for example a coating composition can be prepared by mixing amylose-butan-1-ol complex (glassy amylose) with an aqueous dispersion of Ethocel or a coating formulation comprising an inner coating of glassy amylose and an outer coating of cellulose or acrylic polymer material, calcium pectinate, pectin, a polysaccharide which is totally degraded by colonic bacterial enzymes, chondroitin sulfate and resistant starches, dextran hydrogels, modified guar gum such as borax modified guar gum, cyclodextrins, beta.-cyclodextrin, saccharide containing polymers, which can include a polymeric construct comprising a synthetic oligosaccharides-containing biopolymer including methacrylic polymers covalently couples to oligosaccharides such as cellobiose, lactalose, raffinose, and stachyose, or saccharide-containing natural polymers including modified mucopolysaccharides such as cross-linked chondroitin sulfate and metal pectin salts, for example calcium pectate, methacrylate-galactomannan and pH sensitive hydrogels.

Pharmaceutical compositions of the present invention can be prepared using methods described in the art. There is a wide body of literature and other prior art on the delivery, release and absorption of drug from oral dosage forms wherein said delivery, release and absorption is "targeted", i.e., where said delivery, release and absorption is: (i) achieved at the desired anatomic location of the GI tract; (ii) substantially avoided at certain anatomic locations of the GI tract; (iii) achieved after a particular amount of time has elapsed post-ingestion; (iv) achieved when the GI environment meets certain conditions (e.g., pH, electrolyte concentration, enzymes, hydration, bacterial flora, and the like).

Extended release pharmaceutical compositions are disclosed in U.S. Patent No. 7,196,059, 7,189,414, 7,163,696, 7,157,444, 7,119,079, 7,112,578, 7,109,239, 7,094,425, 7,041,651, 7,030,082, 7,022,683, 6,930,093, 6,919,348, 6,916,791, 6,897,205, 6,893,662, 6,867,183, 6,852,693, 6,824,790, 6,777,000, 6,770,625, 6,761,901, 6,747,014, 6,743,445, 6,734,170, 6,727,287, 6,699,848, 6,692,766, 6,677,321, 6,669,951, 6,632,454, 6,632,451, 6,630,453, 6,555,136, 6,552,072, 6,531,152, 6,525,078, 6,432,967, 6,428,968, 6,346,547, 6,340,476, 6,326,364, 6,277,411, 6,238,689, 6,231,888, 6,228,396, 6,217,904, 6,200,605, 6,200,602, 6,197,763, 6,166,044, 6,166,024, 6,106,864, 6,074,689, 6,063,402, 6,039,975, 5,948,407, 5,914,132, 5,905,081, 5,889,028, 5,866,619, 5,849,327, 5,846,983, 5,843,479, 5,840,332, 5,814,336, 5,811,388, 5,744,166, 5,691,343, 5,686,106, 5,686,105, 5,681,584, 5,672,359, 5,670,158, 5,656,294, 5,656,290, 5,651,983, 5,631,022, 5,554,388, 5,525,634, 5,514,663, 5,482,718, 5,183,802, 5,122,376, 4,904,474, 4,705,515 and 4,627,851, and in US Patent Application No. 20070167416, 20070112075, 20070087939, 20070072828, 20070071820, 20070071806, 20070060580, 20070059366, 20070054945, 20070026067, 20070020254, 20070020197, 20070003626, 20060280795, 20060251720, 20060223787, 20060189635, 20060177507, 20060121091, 20060105045, 20060099243, 20060083718, 20060045865, 20060041109, 20060003995, 20050287276, 20050281781, 20050260262, 20050249716, 20050222040, 20050220861, 20050209271, 20050208132, 20050186267, 20050182134, 20050181053, 20050169996, 20050158408, 20050153908, 20050153907, 20050152978, 20050118326, 20050112201, 20050107334, 20050101611, 20050090473, 20050090451, 20050069550, 20050058701, 20050043298, 20050009848, 20050009768, 20050009767, 20050009766, 20050008702, 20050008688, 20040267240, 20040258754, 20040253304, 20040241173, 20040229831, 20040224898, 20040219216, 20040186045, 20040185107, 20040176319, 20040162263, 20040162259, 20040161459, 20040147445, 20040142880, 20040126422, 20040121967, 20040110837, 20040109894, 20040062778, 20040052846, 20040038866, 20040017387, 20040013687, 20030207851, 20030194439, 20030181380, 20030170181, 20030162717, 20030152617, 20030133978, 20030083232, 20030077326, 20030069170, 20030040497, 20030022843, 20030008914, 20020147156, 20020127198, 20020110593, 20020110590, 20020098235, 20020058061, 20020035139, 20020015729, 20010052137, 20010039262, 20010036473, 20010031748 and 20010026807, which are hereby fully incorporated by reference herein in their entirety.

Pharmaceutical compositions of the present invention can be prepared to provide targeted delivery of levorphanol, wherein the targeted delivery of the immediate or controlled release dosage forms can advantageously provide, among other things, (i) improved efficacy; (ii) improved safety; (iii) reduced appeal to drug addicts, drug abusers and recreational drug users; (iv) reduced nausea; (v) reduced drowsiness; (vi) reduced psychic effects desired by drug addicts, drug abusers and recreational drug users; (vii) reduced desirability for co-abuse with alcohol or other drugs of abuse; and (viii) reduced risk of diversion.

Pharmaceutical compositions of the present invention can be prepared using methods described in the art achieve delivery, release and absorption of drug from oral dosage forms, wherein said delivery, release and absorption is "targeted", e.g., by way of non-limiting examples, where said delivery, release and absorption is: (i) achieved at the desired anatomic location of the GI tract (e.g., upon arrival in the duodenum, or jejum, or ileum, or ileo-cecal junction, or cecum, or ascending colon, or transverse colon, or descending colon); (ii) substantially avoided at certain anatomic locations of the GI tract (e.g., stomach, or stomach and duodenum, or stomach, duodenum and jejunum, or stomach, duodenum, jejunum and ileum); (iii) achieved after a particular amount of time has elapsed post-ingestion (e.g., ≥ 1.5 hours or ≥ 2 hours, or ≥ 2.5 hours, or ≥ 3 hours, or ≥ 3.5 hours, or ≥ 4 hours, or ≥ 4.5 hours, or ≥ 5 hours, or ≥ 5.5 hours, or ≥ 6 hours, or ≥ 6.5 hours, or ≥ 7 hours, or ≥ 7.5 hours); (iv) achieved when the dosage form has come in contact or substantial contact or sustained contact with a desired gastrointestinal pH environment (e.g., pH > 3, or pH > 3.5, or pH > 4, or pH >4.5, or pH, > 5, or pH > 5.5, or pH > 6, or pH > 7, or pH > 7.5, or pH > 7.8); (v) achieved when the dosage form has come in contact with desired microbial flora (e.g., colonic microbial flora); (vi) achieved when the GI environment meets certain other conditions (e.g., electrolyte concentration, enzymes, hydration, and the like); (vii) a combination of two or more of the foregoing.

Pharmaceutical compositions of the present invention can be prepared using methods described, referenced or disclosed in Singh and Kim, Int J Pharm. 2007;341:143-51; Jain et al., Crit Rev Ther Drug Carrier Syst. 2006;23:349-400; Ugurlu et al., Eur J Pharm Biopharm. 2007;67:202-10; Sinha et al., J Pharm Pharmacol. 2007;59:359-65; Gazzaniga et al., Discov Med. 2006;6:223-8; Rhaman et al., AAPS PharmSciTech. 2006;7:E47; Akhgari et al., Int J Pharm. 2006;320:137-42; Bourgeois et al., J Drug Target. 2005;13:277-84; Curini et al., Bioorg Med Chem Lett. 2005;15:5049-52; Lamprecht et al., J Control Release. 2005;104:337-46; Verbeke et al., Aliment Pharmacol Ther. 2005;21:187-94; Bruce et al., Eur J Pharm Biopharm. 2005;59:85-97; Lamprecht et al., Eur J Pharm Biopharm. 2004;58:37-43; Mura et al., J Drug Target. 2003;11:365-71; Lamprecht et al., J Control Release. 2003;90:313-22; Wiwattanapatapee et al., J Control Release. 2003;88:1-9; Waterman et al., J Control Release. 2003;86:293-304; Park et al., Arch Pharm Res. 2002;25:964-8; Tuleu et al., Aliment Pharmacol Ther. 2002;16:1771-9; Gupta et al., Drug Dev Ind Pharm. 2002;28:207-15; Turkoglu et al., Eur J Pharm Biopharm. 2002;53:65-73; Tuleu et al., Methods Find Exp Clin Pharmacol. 2001;23:245-53; Stubbe et al., J Control Release. 2001;75:103-14; Shibata et al., J Pharm Pharmacol. 2001;53:441-7; Jeong et al., J Control Release. 2001;71:175-82; Hu et al., J Pharm Pharmacol. 2000;52:1187-93; Hu et al., Pharm Res. 2000;17:160-7; Ahrabi et al., Eur J Pharm Sci. 2000;10:43-52; Yoshikawa et al., J Pharm Pharmacol. 1999;51:979-89; Ahrabi et al., Drug Dev Ind Pharm. 1999;25:453-62; Hu et al., J Control Release. 1998;56:293-302; Kakoulides et al., J Control Release. 1998;52:291-300; Kakoulides et al., J Control Release. 1998;54:95-109; Muraoka et al., J Control Release. 1998;52:119-29; Takaya et al., J Control Release. 1998;50:111-22; Muraoka et al., Nippon Rinsho. 1998;56:788-94; Kenyon et al, Aliment Pharmacol Ther. 1997;11:205-13; Takaya et al., J Drug Target. 1997;4:271-6; Matsuda et al., J Drug Target. 1996;4:59-67; Jones SP, J Drug Target. 1996;3:477-8; Fedorak et al., Gastroenterology. 1995;108:1688-99; Takaya et al., J Pharm Pharmacol. 1995;47:474-8; Niwa et al., J Drug Target. 1995;3:83-9; Ashford and Fell, J Drug Target. 1994;2:241-57, Colonic Drug Delivery (page 287-294), Wilson CG, In: Modified- Release Drug Delivery Technology, Second Edition, Vol. 1, Rathbone MJ, Hadgraft J, Roberts MS, Lane ME (eds), Informa Healthcare USA Inc. 2008; Biopolymers and Colonic Delivery, Wilson CG, Mukherji G, Shah HK (pages 295-309), In: Modified- Release Drug Delivery Technology, Second Edition, Vol. 1, Rathbone MJ, Hadgraft J, Roberts MS, Lane ME (eds), Informa Healthcare USA Inc. 2008; Enteric Coating for Colonic Delivery, Shah HK, Mukherji G, Brogmann B, Wilson CG (pages 311-324), In: Modified- Release Drug Delivery Technology, Second Edition, Vol. 1, Rathbone MJ, Hadgraft J, Roberts MS, Lane ME (eds), Informa Healthcare USA Inc. 2008; Programmed Drug Delivery Systems and the Colon, Wilson CG, Shah HK, Lee WW, Brogmann B, Mukherji G (pages 325-335), In: Modified- Release Drug Delivery Technology, Second Edition, Vol. 1, Rathbone MJ, Hadgraft J, Roberts MS, Lane ME (eds), Informa Healthcare USA Inc. 2008; Targeting the Colon Using COLAL™ : A Novel Bacteria-Sensitive Drug Delivery System, McConnell EL, Basit AW (pages 343-348), In: Modified- Release Drug Delivery Technology, Second Edition, Vol. 1, Rathbone MJ, Hadgraft J, Roberts MS, Lane ME (eds), Informa Healthcare USA Inc. 2008, Niazi, S. Handbook of Pharmaceutical Manufacturing Formulations: Compressed Solid Products (Volume 1 of 6), CRC Press, 2004, which are hereby fully incorporated by reference herein in their entirety.

### DETAILED DESCRIPTION OF THE PREFERRED EMBODIMENTS

The following examples illustrate various aspects of the present invention. They are not to be construed to limit the claims in any manner whatsoever. A wide variety of methods known in the art for the preparation of oral extended release dosage forms may be incorporated into the invention. Similarly, a wide variety of methods known in the art for the in vitro and in vivo evaluation of analgesics and oral extended release dosage forms may be incorporated into the invention. Other suitable dosage forms may also be prepared by modification of the examples herein and by use of material other than those specifically disclosed herein, including those which may hereafter become known to the art to be capable of performing the necessary functions. Similarly, other suitable methods for the evaluation of dosage forms may also be used by modification of the examples herein and by use of methods other than those specifically disclosed herein, including those which may hereafter become known to the art to be capable of performing the necessary functions. Other suitable modifications and adaptations of the variety of conditions and parameters normally encountered and obvious to those skilled in the art are within the spirit and scope of the invention.

The dose and dosing frequency of extended release levorphanol will vary depending on of the nature of the patient population, the characteristics of the medical condition under treatment, and other factors described herein. Similarly, the in vitro and in vivo release characteristics of extended release levorphanol, including the dissolution rate and plasma concentration time profile may be modified based on the clinical need of the patient, and other factors described herein.

The percent loading of the levorphanol onto the dosage form may be varied depending on the physiochemical and pharmaceutical properties controlled release material, excipients, the selected salt of levorphanol and the desired release profile and duration of action.

The ingredients used for the preparation of the levorphanol dosage form may be modified depending on the selection, dose and desired duration of effect. In some embodiments, a change in the dose or amount levorphanol will not require a significant change in the amount of other ingredients. In other embodiments, a proportional change in the amount of other ingredients is required to maintain the desired properties. In yet other embodiments, a change in the dose or amount levorphanol necessitates a change in the nature and/or amount of ingredients to provide the required characteristics of the levorphanol (e.g., duration of effect, rate and extent of absorption, therapeutic concentrations and effect, etc.).

A wide variety of formulations of extended release levorphanol have been prepared and evaluated by the applicant.

### EXAMPLES

### Example 1

### Excipients and capsule size selection

The target capsule size for this project was size 2 gelatin capsule. A fill weight of 325 mg was selected to assist with patient compliance and to allow the possibility of increasing the active dosage quantity by scaling up the fill weight into a larger, but still within an acceptable, capsule size. This meant that levorphanol tartrate dihydrate would be present at about 3.08% w/w in this 325 mg overall, 10 mg levorphanol product. The dosage unit was to be designed with inbuilt abuse resistance.

A range of thermosoftening materials with melting points up to about 75°C were considered. Several potential release rate modifiers as described herein were considered but hydroxypropyl methylcellulose (HPMC) was chosen as the preferred release rate modifier. An HPMC (Methocel™ K 15M) was incorporated into the formulations to accelerate release and provide a level of abuse deterrence. Formulations containing only levorphanol, a water soluble material, with a water insoluble base excipient made separation of the active by extraction relatively easy in the setting of tampering for abuse. HPMC was chosen as we have found it to enhances abuse resistance, having the property of being water soluble, and thus would 'follow' levorphanol during attempted aqueous extraction, making separation of the levorphanol more difficult. HPMC is supplied in high viscosity grades which was found to impart a viscous nature to aqueous extracts of dosage units i.e. during scenarios when levorphanol was extracted with a small amount of water (e.g. in a small spoon) then, at best, an unpleasant mixture was produced that has a 'gummy' appearance and blocks attempts at filtration. Similarly this type of extract was much more difficult to draw into a syringe and provided a strong visual warning that it is not a pure solution of levorphanol. Additionally, we have found that HPMC behaves in an unusual manner in aqueous solution. Most water soluble materials increase in solubility as the water temperature rises. HPMC was most soluble in cold water, becoming less soluble with temperature increases until, at about 40°C, it was totally insoluble. Solutions of HPMC that heated to 40°C or above turned into solid gels. This means that although an HPMC may be added to increase release rates from a dosage unit, by providing channels through which the levorphanol can diffuse, it can also actively deter abuse by extraction from the dosage form and by other means of tampering. If an abuser tries to extract levorphanol with warm or hot water then the HPMC will become completely insoluble and actively resist the diffusion of levorphanol through the relatively impermeable base excipient. This resists the generation of injectable solutions, interferes with 'snorting' or 'dose dumping' (due to the viscous solutions produced) and resists extraction at elevated temperature.

A fumed silicon dioxide was chosen as both a thixotrope and contributor to abuse deterrence. Aerosil™ is the commercial name for fumed silicon dioxide manufactured by Degussa Hüls who produce a range of Aerosils™ with differing properties. These include different particle size, hydrophobic or hydrophilic characteristics or blended with additional materials for specific purposes. Aerosil™ 200 was chosen as the Aerosil™ of choice as it is pharmaceutically acceptable, 100% fumed silicon dioxide and effectively thickens aqueous systems. In this dosage form Aerosil™ 200 increased viscosity in the formulation. If an abuser attempts to add a small quantity of water to produce a solution (e.g. for injection), the Aerosil™ 200 (and other silicon dioxides and slica) contribute to maintain or increase the viscosity of any resulting solution. This complements the action of HPMC. Furthermore, silicon dioxide does not melt below 100°C (or even 1000°C) and is insoluble. The thickening effect of Aerosil™ is unaffected by heat; thus an abuser attempting to melt a dosage unit will find that the structure and shape of the dosage unit tends to remain unchanged when sufficient Aerosil™ is incorporated even though the melting point of all other excipients has been exceeded.

Miglyol™ 812N, a fractionated coconut oil, was used to impart softness and stickiness to formulations that contained waxes that were naturally hard and could potentially be ground into powders. Additionally, the solubility of oils in organic solvents added to the difficulty of attempted extraction and isolation of levorphanol using materials such as alcohols. This oil was selected as it is, pharmaceutically acceptable, of low viscosity and not susceptible to oxidation and rancidity.

### Example 2

**The material used included:**

| **Material** | **Material** |
|---|---|
| Aerosil™ 200 | Methocel™ K100M |
| Beeswax, yellow refined | Miglyol™ 812 |
| Cithrol™ GMS 0400 | Potassium dihydrogen orthophoshate |
| Compritol™ 888 ATO | Precirol™ AT05 |
| Ethanol 96% | Size 2 clear/clear gelatin capsules |
| Fractionated coconut oil | Size 2 white/white gelatin capsules |
| Hydrokote™ 112 | Sodium hydroxide |
| Levorphanol tartrate | Sodium metabisulphite (97%) |
| Methocel™ K15M | Sodium metabisulphite |
| Water | Sterotex™ NF |

### Example 3

### Dissolution Testing

Dissolution testing was carried out with the USP paddle method using standard round bottomed vessels, a temperature of 37°C, with a paddle speed of 75 rpm on a dissolution apparatus with thermostatically controlled water heater. Except where otherwise specified, the dissolution medium was 600 mL of Simulated Intestinal Fluid (SIF) USP, pH 6.8 without the inclusion of enzyme. Capsules were weighed down with 316 stainless steel sinking wire, wrapped round each capsule. The levorphanol dissolution release profiles were initially determined by UV measurement and later the process was changed to use HPLC.

Samples of suitable formulations were placed on stability stored at 25°C/60%RH and 40°C/75%RH in glass jars for one or more months (dependent on date of final formulation acceptance). Dissolution testing was carried out on these samples and the data compared with their T₀ data.

### Example 4

### Abuse resistance testing

Since levorphanol is an opioid analgesic, it is subject to tampering and non-medical use. Consequently, the dosage forms were also evaluated for abuse resistance. The series of tests chosen to evaluate abuse resistance and the source of the test included: Test 1a) Extraction with alcohol on whole dosage unit (Oral Drug Delivery patent application US 2004/0161382 A1 (P 11, [0122])). Method: An intact dosage unit was placed in 18 mL of 0.1N HCl in a 60 mL amber bottle and shaken at 240 rpm on an orbital shaker for 30 minutes. After 30 mins, 12 mL of ethanol (95-96%) was added to each bottle. A 1 ml sample was removed from each bottle (T0) after swirling by hand for one minute. The solution was placed back in the orbital shaker for further shaking at 240 rpm. Additional 1 mL samples were obtained after 10, 20, 30, 40, 60 and 180 min of further shaking for each bottle. Analyze and graph the results on a linear scale of cumulative release (%) vs. time (mins). Test 1b) Extraction with alcohol on a crushed or cut dosage unit (extension of test in above patent). Method: After crushing with a single crush using a spatula, the tampered tablet or capsule was placed in 18 ml of 0.1N HCl in a 60 ml amber bottle and shake at 240 rpm on an orbital shaker for 30 mins. The test was continued as in the preceding paragraph. Test 2) Extraction into water (Oral Drug Delivery patent application US 2004/0161382 A1 (P12, [0130])). Method: For capsules the outer shell was removed. The tablet or capsule plug was then crushed with a mortar and pestle and ground in 5 mL of water for 5 minutes. The resulting suspension was filtered through a 0.45 micron filter into a flask and diluted to 10 ml with water. The drug collected in the filtrate was then quantified. Test 3) Crush to small particles with mortar and pestle. Method: The dosage form was frozen in a domestic freezer for 24 hours. The capsule shell was then removed with a scalpel and the removed plug then ground with a mortar and pestle for one minute. The resulting powder was then sieved through a suitable sieve (ca 600 micron) and, by weighing the content of powder passing through the sieve was measured. Test 4 is intentionally omitted. Test 5) Extraction into acid. Method: The dosage form was crushed with a mortar and pestle and heated to boiling in 5 mL of vinegar (8% acetic acid). The resulting suspension was filtered through a 0.45 micron filter into a flask and diluted to 10 ml with water. The active drug content was then quantified. Test 6) Application of heat - melting temperature. Method: The squashed contents of the dosage were heated on a hot plate until melted. The temperature of melting was determined and the syringability of the melt was determined by aspirating and then expelling the aspirate from 1.2 mm needle attached to a syringe.

### Example 5

### Binary mix compatibility trials

Binary mixes were prepared of levorphanol in potential excipients (in some instances a third material, fractionated coconut oil was used to bring two non-melting materials into intimate contact). The excipients used were chosen according to their ability to influence the dissolution profile of the formulated product. The properties considered were aqueous solubility (from partially soluble to highly insoluble) and their ability to modify viscosity and/or release characteristics e.g. different viscosity grades of HPMC and thixotropic modifiers such as different grades of fumed silicon dioxide.

For the placebo samples, approximately 2g of excipient was weighed into two clear glass scintillation vials. One sample was placed in stability storage at 40C 75%RH and one at 5C for the initial analysis. For the active samples approximately 9.5g of excipient was weighed into a clear glass scintillation vials. Approximately 0.5g of levorphanol was weighed into the same vial. The mixture was stirred by spatula to thoroughly mix the components. The components were additionally mixed by high shear for 1 minute using a Silverson Mixer. Based on the results, it was decided to initially focus on Miglyol™ 812, Aerosil™ 200/Miglyol™ 812, Hydrokote™ 112, Sterotex™ NF, Compritol™ 888 ATO, Precirol™ ATO 5, Methocel™ K15M/Miglyol™ 812 and Methocel™ K100M/Miglyol™ 812 as the excipients for this project.

### Example 6

The release profile of three initial formulations were determined by UV monitoring. Formulations prepared and tested were:

| **Formula (Ref 052/149/1)** | **% (w/w)** | **mg/dose** |
|---|---|---|
| Sterotex™ | 48.92 | 159.00 |
| Coconut oil | 25.00 | 81.25 |
| Methocel™ K15M | 20.00 | 65.00 |
| Aerosil™ 200 | 3.00 | 9.75 |
| Levorphanol tartrate | 3.08 | 10.00 |

| **Formula (Ref 052/149/2)** | **% (w/w)** | **mg/dose** |
|---|---|---|
| Hydrokote™ 112 | 78.92 | 256.49 |
| Methocel™ K15M | 15.00 | 48.75 |
| Aerosil™ 200 | 3.00 | 9.75 |
| Levorphanol tartrate | 3.08 | 10.00 |

| **Formula (Ref 052/149/3)** | **% (w/w)** | **mg/dose** |
|---|---|---|
| Precirol™ ATO 5 | 78.92 | 256.49 |
| Methocel™ K15M | 15.00 | 48.75 |
| Aerosil™ 200 | 3.00 | 9.75 |
| Levorphanol tartrate | 3.08 | 10.00 |

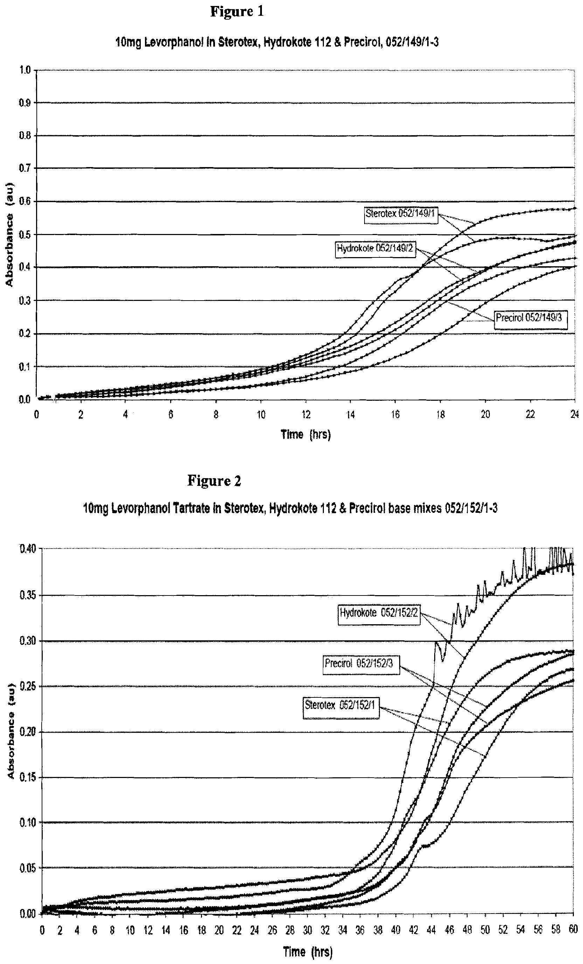

As shown in Figure 1, the absorbance values measured are surprisingly greatly in excess of the theoretical end absorbance. Without being bound by theory, this may be due to interference by excipients or microbial growth developing over the extended dissolution period.

### Example 7

The release profile of the three analogs to the above containing only the base wax and Methocel™ was determined to observe the effects of the loss of the other excipients (coconut oil and Aerosil™). Formulations prepared and tested were:

| **Formula (Ref 052/152/1)** | **% (w/w)** | **mg/dose** |
|---|---|---|
| Sterotex™ | 48.92 | 159.00 |
| Methocel™ K15M | 20.00 | 65.00 |
| Levorphanol tartrate | 3.08 | 10.00 |

| **Formula (Ref 052/152/2)** | **% (w/w)** | **mg/dose** |
|---|---|---|
| Hydrokote™ 112 | 78.92 | 256.49 |
| Methocel™ K15M | 15.00 | 48.75 |
| Levorphanol tartrate | 3.08 | 10.00 |

| **Formula (Ref 052/152/3)** | **% (w/w)** | **mg/dose** |
|---|---|---|
| Precirol™ ATO 5 | 78.92 | 256.49 |
| Methocel™ K15M | 15.00 | 48.75 |
| Levorphanol tartrate | 3.08 | 10.00 |

As show in Figure 2, the measured absorbance values increase slowly for the first 34 hours and then were surprisingly found to be greatly in excess of the theoretical end absorbance. Without being bound by theory, this may be due to interference by excipients or microbial growth developing over the extended dissolution period.

### Example 8

The dissolution profile of Methocel™ in combination with each of three base waxes was determined to investigate whether UV absorbance interference arose from the presence of excipients. Formulations prepared and tested were:

| **Formula (Ref 052/153/1)** | **% (w/w)** | **mg/dose** |
|---|---|---|
| Sterotex™ | 70.98 | 159.00 |
| Methocel™ K15M | 29.02 | 65.00 |

| **Formula (Ref 052/153/2)** | **% (w/w)** | **mg/dose** |
|---|---|---|
| Hydrokote™ 112 | 84.03 | 256.49 |
| Methocel™ K15M | 15.97 | 48.75 |

| **Formula (Ref 052/153/3)** | **% (w/w)** | **mg/dose** |
|---|---|---|
| Precirol™ ATO 5 | 84.03 | 256.49 |
| Methocel™ K15M | 15.97 | 48.75 |

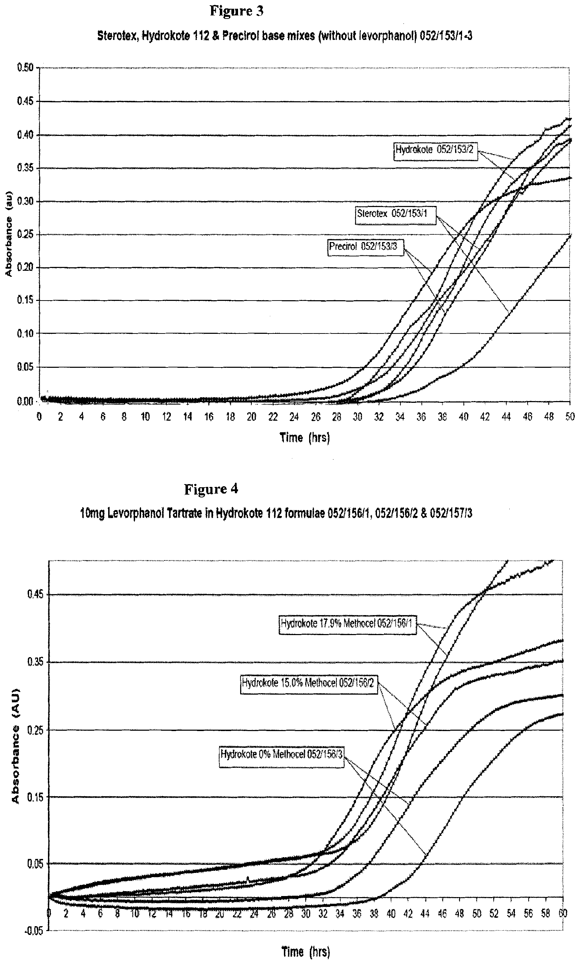

As show in Figure 3, the absorbance values showed no significant change for the first 20-30 hours then rose greatly in excess of the theoretical end absorbance for a levorphanol containing dosage unit. Without being bound by theory, the lack of any interference from the excipients for 20-30 hours followed by the rapid increase in absorbance is strongly indicative of the interference being due to microbial growth developing over the extended dissolution period.

### Example 9

Levorphanol was formulated incorporating different percentages of Methocel™. The dissolution profile was tracked by UV, as previously. Samples were also taken which were analyzed by HPLC. Plots were constructed to compare the results. Formulations prepared and tested were:

| **Formula (Ref 052/156/1)** | **% (w/w)** | **mg/dose** |
|---|---|---|
| Hydrokote™ 112 | 76.12 | 255.00 |
| Methocel™ K15M | 17.91 | 60.00 |
| Aerosil™ 200 | 2.99 | 10.00 |
| Levorphanol tartrate | 2.99 | 10.00 |

| **Formula (Ref 052/156/2)** | **% (w/w)** | **mg/dose** |
|---|---|---|
| Hydrokote™ 112 | 78.92 | 256.49 |
| Methocel™ K15M | 15.00 | 48.75 |
| Aerosil™ 200 | 3.00 | 9.75 |
| Levorphanol tartrate | 3.00 | 10.00 |

| **Formula (Ref 052/156/3)** | **% (w/w)** | **mg/dose** |
|---|---|---|
| Hydrokote™ 112 | 96.25 | 256.50 |
| Levorphanol tartrate | 3.75 | 10.00 |

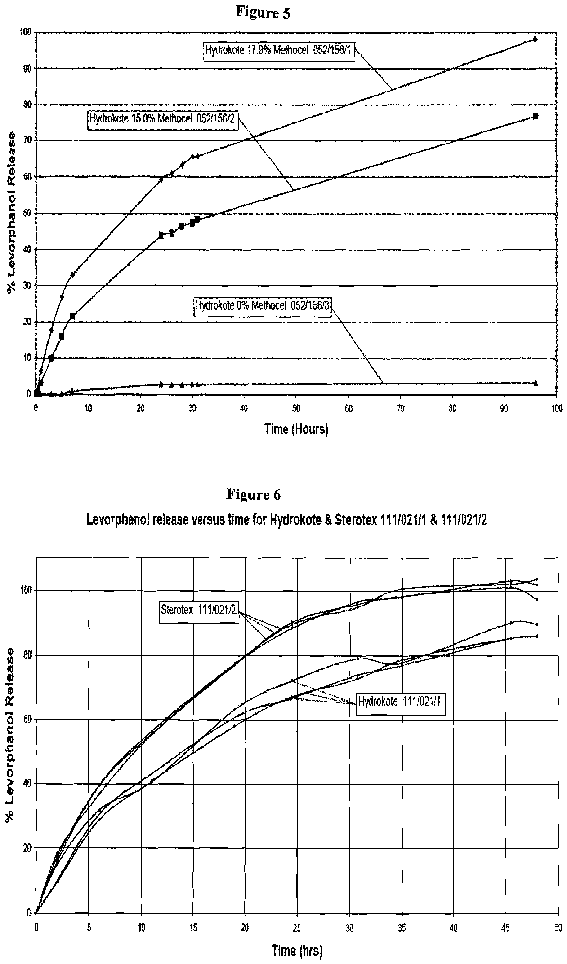

As shown in Figure 4, the UV the absorbance values measured increase slowly for the first 30 hours then rise to in excess of the theoretical end absorbance (0.076 au). This has been interpreted as due to interference by microbial growth developing over the extended dissolution period. During the first 30 hours the UV profiles did show a release pattern ordered in increasing content of Methocel™ but the small absorbance range exhibited made accurate quantification difficult. The HPLC analyzed data (Figure 5) show a similar release pattern, ordered in increasing content of Methocel™, but analysis shows a clear release pattern, unmodified by the interference that had occurred during UV monitoring. The release profile of levorphanol from a Hydrokote™ base formulation (no release rate modifiers incorporated; 052/156/3) shows that the release time was slow enough to meet or exceed (i.e. slower) the requirements for this project. The release rate of formulations can be accelerated by the addition of release rate modifiers but cannot be retarded beyond that of the base wax. Base formulations, without release rate, are likely to show similar release profiles for the other aqueous insoluble waxes selected.

### Example 10

Hydrokote™ 112, Sterotex™, Precirol™ ATO 5 and Compritol™ 888 ATO were selected as the base wax excipients for formulations. Formulations were prepared and their release profiles determined. The formulations were then adjusted to increase or decrease the release rates towards the intended target profiles. The dissolution profiles were determined by sampling and HPLC analysis. Formulations prepared and tested were:

| **Formula (Ref 111/021/1)** | **% (w/w)** | **mg/dose** |
|---|---|---|
| Hydrokote™ 112 | 75.4 | 245.00 |
| Methocel™ K15M | 18.5 | 18.5 |
| Aerosil™ 200 | 3.1 | 10.00 |
| Levorphanol tartrate | 3.1 | 10.00 |

| **Formula (Ref 111/021/2)** | **% (w/w)** | **mg/dose** |
|---|---|---|
| Sterotex™ | 47.1 | 153.00 |
| Miglyol™ 812 | 25.2 | 82.00 |
| Methocel™ K15M | 21.5 | 70.00 |
| Aerosil™ 200 | 3.1 | 10.00 |
| Levorphanol tartrate | 3.1 | 10.00 |

| **Formula (Ref 111/021/3)** | **% (w/w)** | **mg/dose** |
|---|---|---|
| Precirol™ ATO 5 | 75.4 | 245.00 |
| Methocel™ K15M | 18.5 | 60.00 |
| Aerosil™ 200 | 3.1 | 10.00 |
| Levorphanol tartrate | 3.1 | 10.00 |

| **Formula (Ref 111/021/4)** | **% (w/w)** | **mg/dose** |
|---|---|---|
| Compritol™ 888 ATO | 75.4 | 245.00 |
| Methocel™ K15M | 18.5 | 60.00 |
| Aerosil™ 200 | 3.1 | 10.00 |
| Levorphanol tartrate | 3.1 | 10.00 |

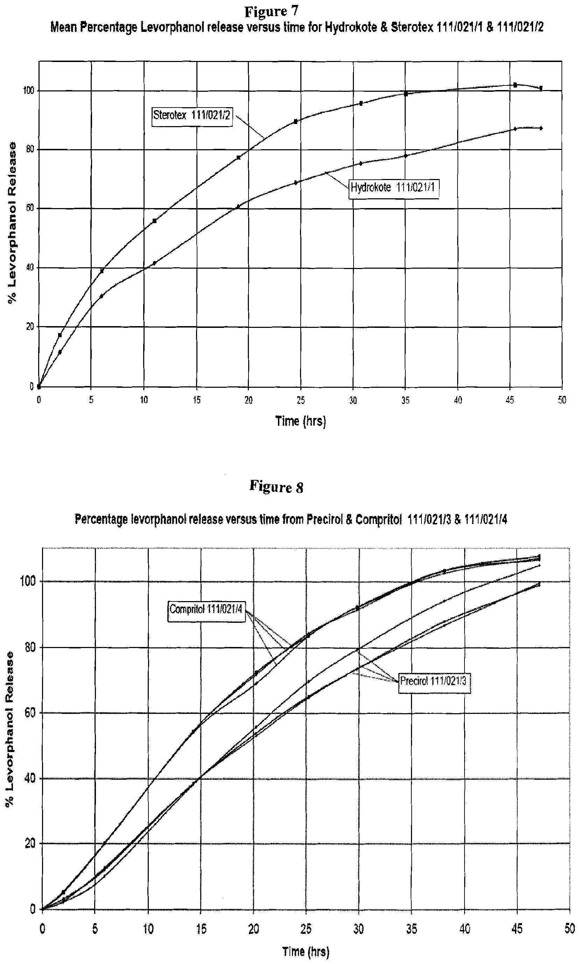

Figure 6, Figure 7, Figure 8 and Figure 9 show the dissolution profiles obtained from the above example. Three capsules of each formulation were used per trial. Each two plot set shows the release profile of two sets of three capsules and the average release profiles of each three capsule set.

### Example 11

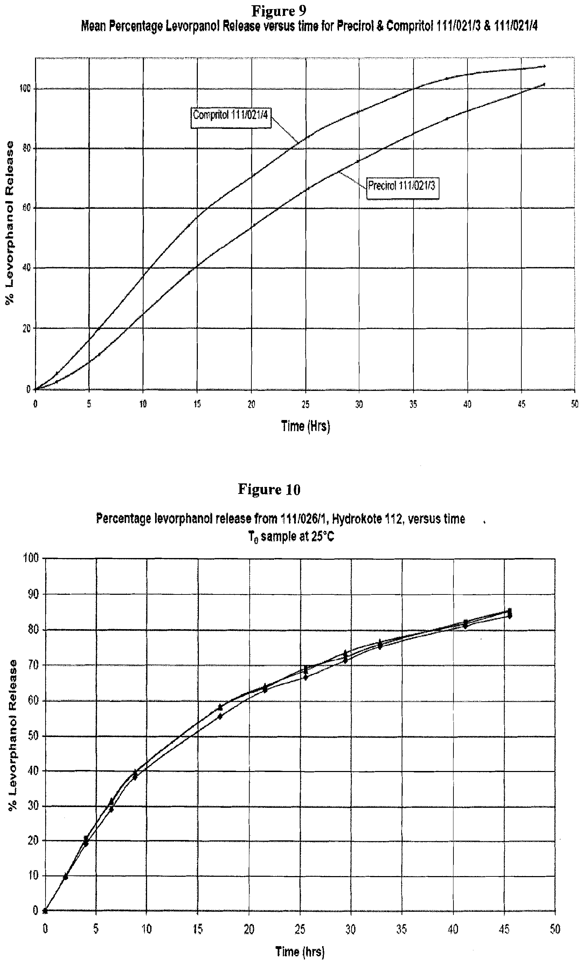

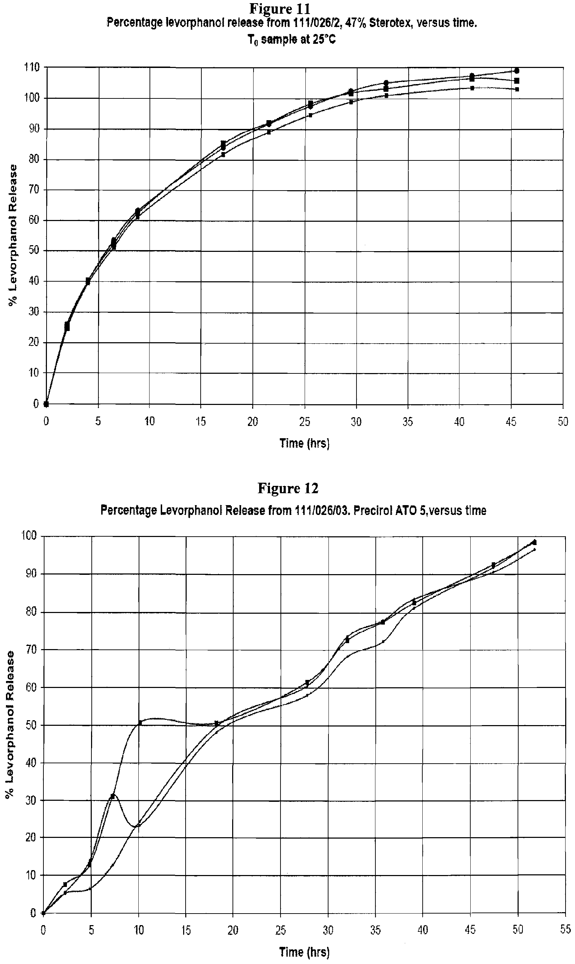

Four Levorphanol ER prototype batches were prepared having a spread of release rates. Formulations prepared and tested were 111/026/1 (Hydrokote™ based), 111/026/2 (Sterotex™ based), 111/026/3 (Precirol™ based) and 111/026/4 (Compritol™ based) as described herein. All mixes were filled on a HiBar bench filling machine. T₀ dissolution profiles were run on 3 capsule samples and the batches placed on stability at 25°C/60%RH and 40°C/75%RH in sealed glass vessels. The T₀ dissolution profiles are shown in Figure 10, Figure 11, Figure 12 & Figure 13.

### Example 12

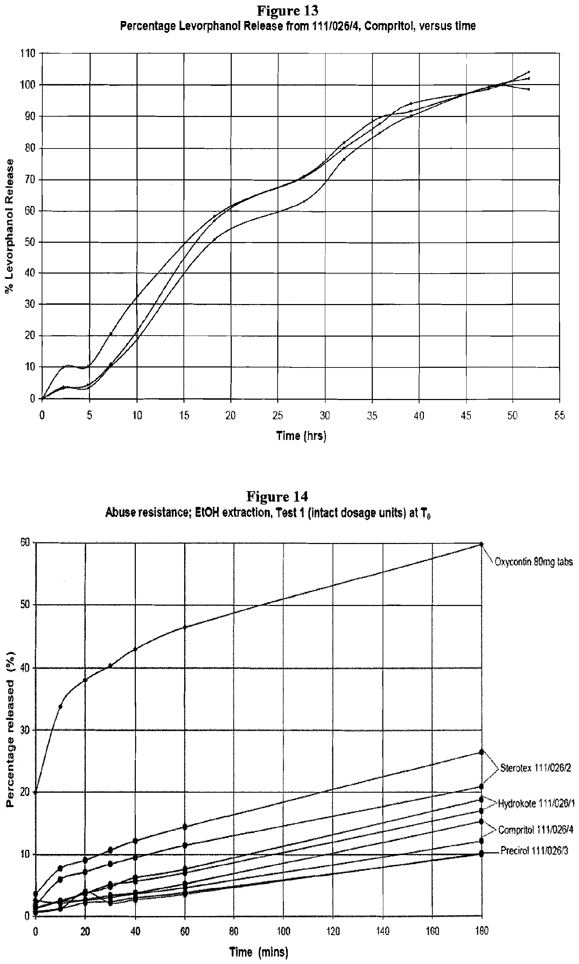

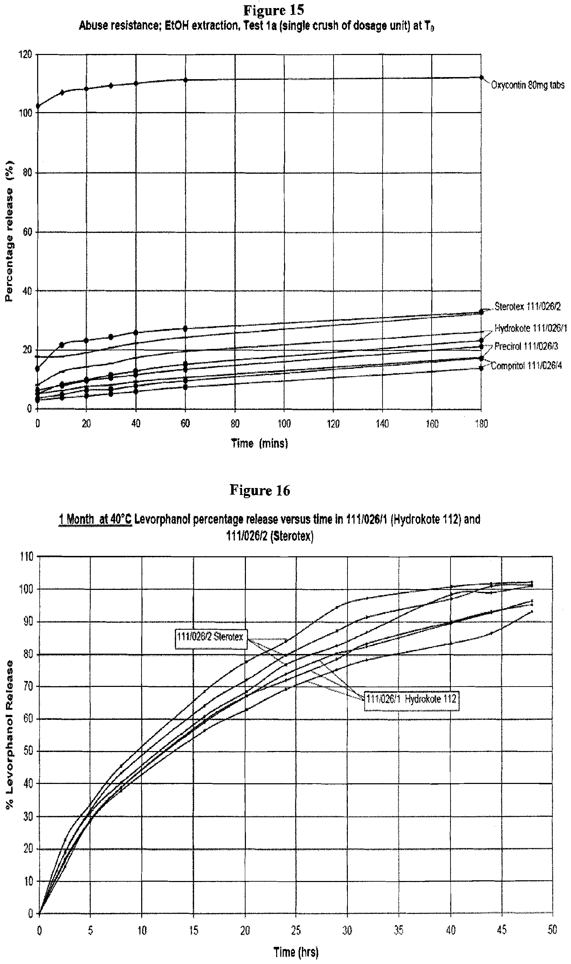

The above formulations underwent a battery of abuse deterrence tests. The results are shown in Figure 14 and Figure 15 and in the Tables below. Tests were carried out to determine the general resistance with each base excipient as it was not practical to carry out the (lengthy) testing while the formulations were being modified frequently. Abuse resistance testing was carried out on the stability samples following the methods previously. The extraction into water after grinding with a mortar and pestle in water [Test 2)] initially resulted in a viscous sticky mass that would not pass through a filter, pressure fed via a syringe so no liquid could be isolated in which active could be measured.

**Abuse Resistance Test 2 on Formulations 111/026/1-4 at T₀**

| **Extraction into water Test 2) T₀ (5 ml water; 5 mins using a mortar & pestle)** | | |
|---|---|---|
| Formulation | % released | Comment |
| Hydrokote™ | 0 | Forms sticky viscous mix which |
| 111/026/1 | | doesn't pass through filter |
| Sterotex™ NF | 0 | Forms sticky viscous mix which |
| 111/026/2 | | doesn't pass through filter |
| Precirol™ | 0 | Forms sticky viscous mix which |
| 111/038/3 | | doesn't pass through filter |
| Compritol™ | 0 | Forms sticky viscous mix which |
| 111/038/4 | | doesn't pass through filter |

The filter type (PTFE) was changed to that which had the highest permeability (PVDF or polyvinylidene difluoride) to permit testing that could discriminate between dosage units. The results on testing using these filters on samples at the three month storage time point are shown in below. Each sample was tested in duplicate and gave satisfactory results.

**Abuse Resistance Test 2 on Formulations 111/026/1-4 at T_{3 month}**

| **Extraction into water Test 2) T_{3 month} (5 ml water; 5 mins using a mortar & pestle)** | | |
|---|---|---|
| Formulation | % released | Comment |
| Hydrokote™ | 7.3; 6.4 | Viscous paste with waxy lumps |
| 111/026/1 | | |
| Sterotex™ NF | 6.7; 15.5 | Viscous paste with waxy lumps |
| 111/026/2 | | |

The Table below shows comparable data from test we undertook on an extended release tramadol (Zydol™ tablets) and on an extended release oxycodone (OxyContin™ tablets).

**Abuse Resistance Test 2 on Commercial Tablets Comparators**

| **Extraction into water Test 2 (5 ml water; 5 mins using a mortar & Pestle)** | | |
|---|---|---|
| Formulation | % released | Comment |
| Zydol™ (tablet) | 87 | Mobile easily filtered solution |
| OxyContin™ (tablet) | 82 | Mobile easily filtered solution |

### Example 13

This test was designed to assess the ease of powdering and percentage of resultant particles of 600 micron or less. Initial powdering tests were carried out using a laboratory stainless steel sieve of nominal 650 micron size. The sieve size used had been qualitatively determined as a size that could differentiate between the powders generated. Initially much finer sieves had been tested but were found to be too fine e.g. a 45 micron sieve was tested but this was too fine resulting in almost zero powder passing through the sieve from any samples. As a result of the initial tests, a certified sieve was obtained of 600 micron size for further trials. All of the above samples were subjected to the powdering test. All results for Hydrokote™ and Sterotex™ are acceptable.

**Abuse Resistance Test 3 on Formulations 111/026/1-4 at T₀**

| **Crush to small particles Test 3 (Freeze, grind in mortar, sieve through 600µ sieve)** | | |
|---|---|---|
| Formulation | % through sieve | Comment |
| Hydrokote™ | 0 | Forms sticky film on mortar |
| 111/026/1 | | |
| Sterotex™ NF | 0 | Forms sticky film on mortar |
| 111/026/2 | | |
| Precirol™ | 66.5 | Many particles pass through mesh |
| 111/026/3 | | |
| Compritol™ | 92.7 | Most particles pass through mesh |
| 111/026/4 | | |

Formulations 111/026/3 (Precirol™) and 111/026/4 (Compritol™) were temporarily discontinued at this stage as being too easily powdered, however, the samples present on stability were subjected to testing at the one month timepoint.

### Example 14

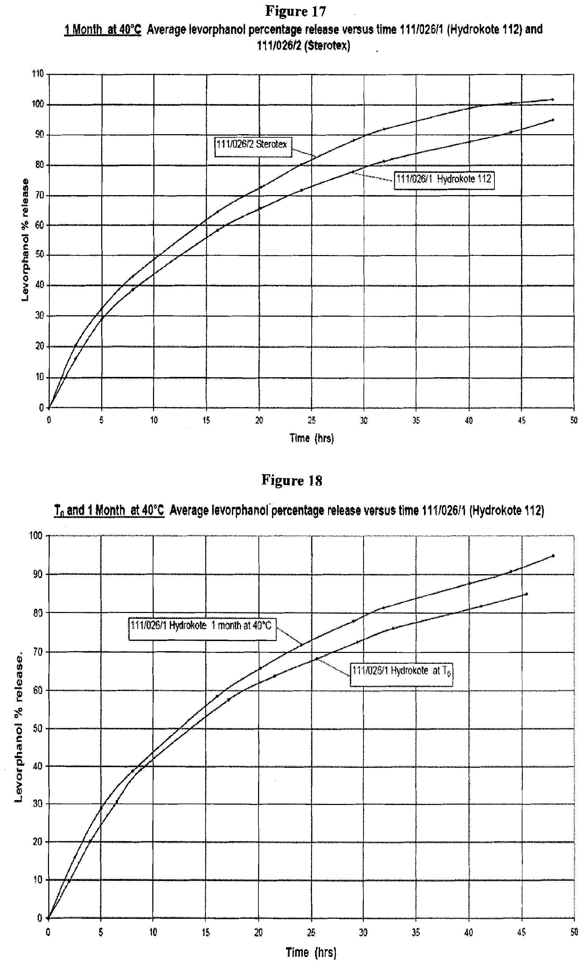

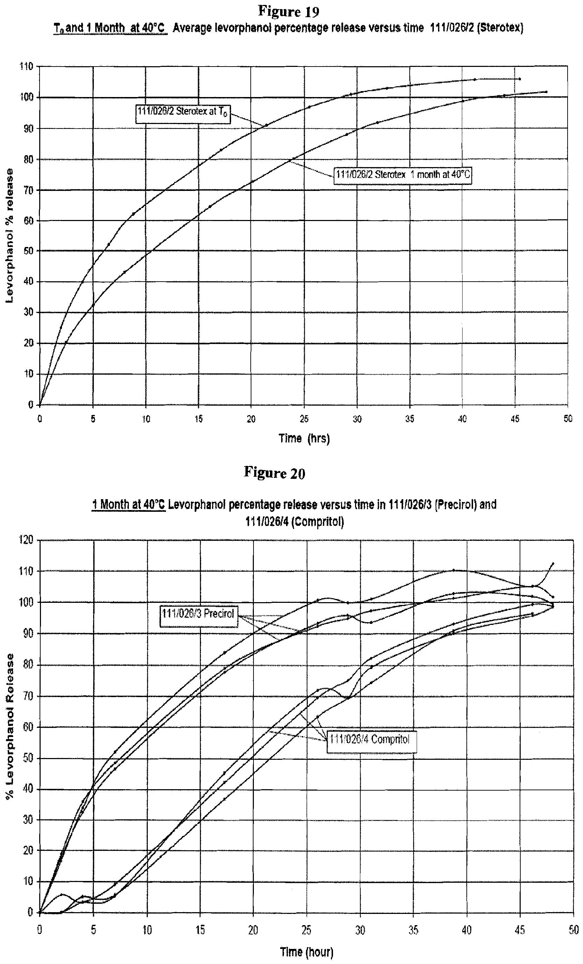

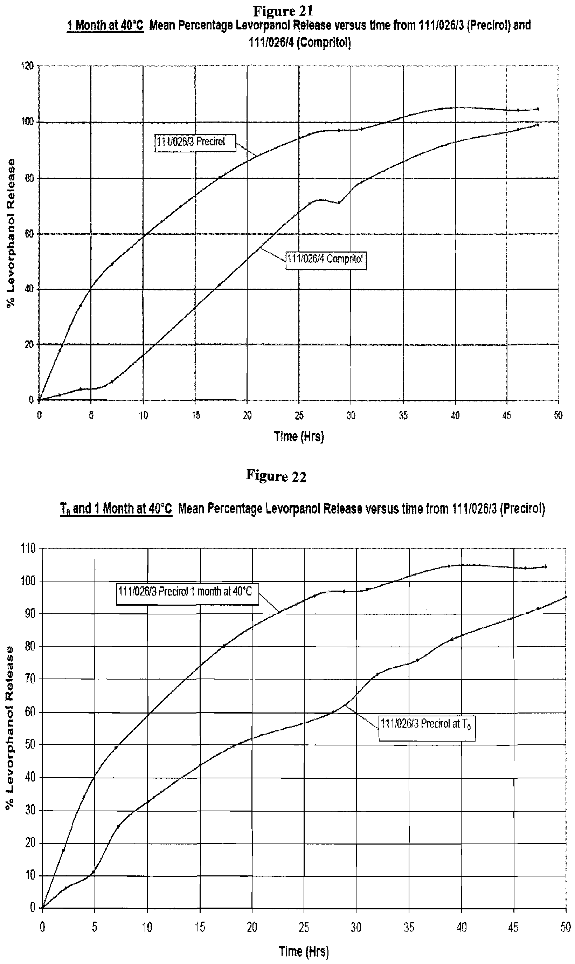

Figure 16, Figure 17, Figure 18, Figure 19, Figure 20, Figure 21, Figure 22 and Figure 23 show the dissolution data after storage at 40°C/75%RH for one month.

### Example 15

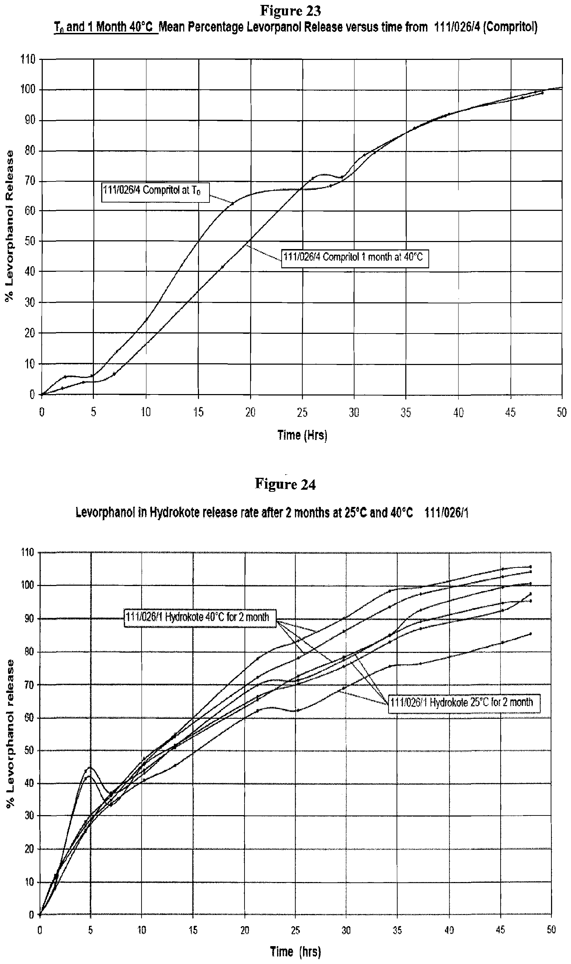

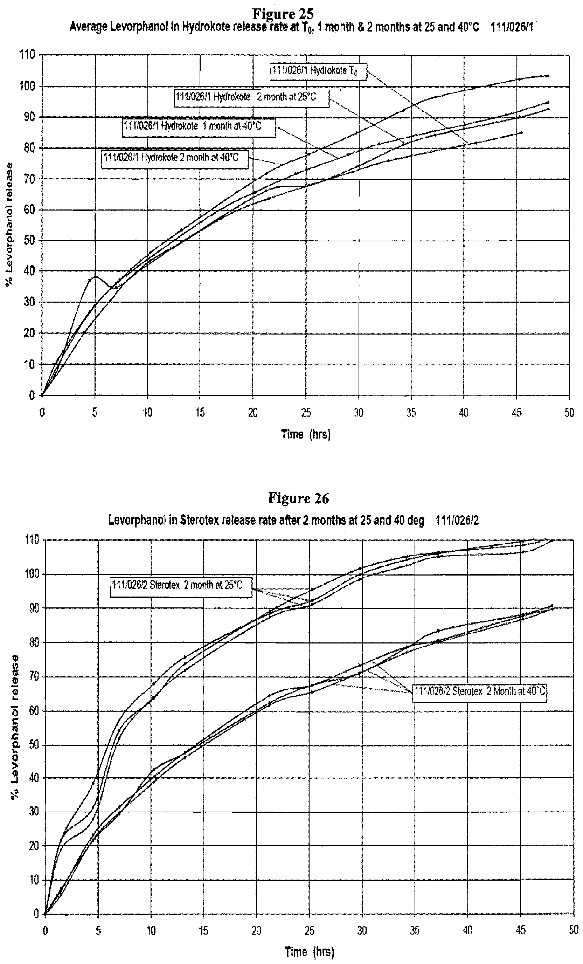

Figures 24, Figure 25, Figure 26 and Figure 27 show month dissolution data after storage at 25°C/60%RH and 40°C/75%RH for 111/026/1 (Hydrokote™) and 111/026/2 (Sterotex™).

### Example 16

Abuse resistance testing demonstrated of formulations 111/026/3 and 111/026/4 demonstrated that both formulations powdered too easily. Both formulations were modified by the inclusion of an oil to impart stickiness. Formulations prepared and tested were:

| **Formula (Ref 111/038/3)** | **% (w/w)** | **mg/dose** |
|---|---|---|
| Precirol™ ATO 5 | 53.2 | 173.00 |
| Miglyol™ 812 | 25.2 | 82.00 |
| Methocel™ K15M | 15.4 | 50.00 |
| Aerosil™ 200 | 3.1 | 10.00 |
| Levorphanol tartrate | 3.1 | 10.00 |

| **Formula (Ref 111/038/4)** | **% (w/w)** | **mg/dose** |
|---|---|---|
| Compritol™ 888 ATO | 53.2 | 173.00 |
| Miglyol™ 812 | 25.2 | 82.00 |
| Methocel™ K15M | 15.4 | 50.00 |
| Aerosil™ 200 | 3.1 | 10.00 |
| Levorphanol tartrate | 3.1 | 10.00 |

**Abuse Test 3 (Ease of Powdering) on Modified Precirol™ and Compritol™**

| **Crush to small particles Test 3 (Freeze, grind in mortar, sieve through 600µ sieve)** | | |
|---|---|---|
| Formulation | % through sieve | Comment |
| Precirol™ | 23.2 | Few particles pass through mesh |
| 111/038/3 | | |
| Compritol™ | 13.8 | Few particles pass through mesh |
| 111/038/4 | | |

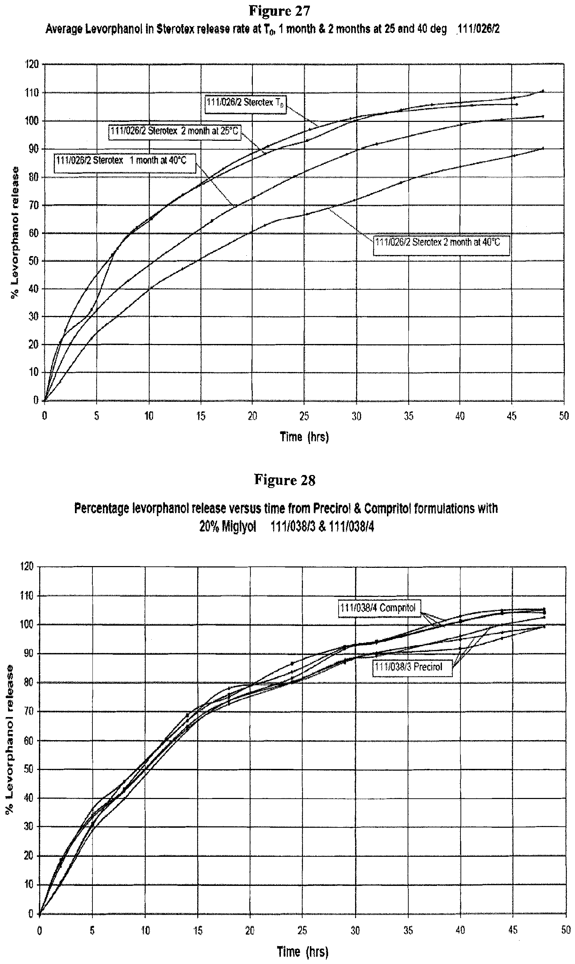

Dissolution testing of these formulations gave the release profiles shown in Figure 28. Both formulations had very similar release profiles.

### Example 17

A Hydrokote™ based formulation with a slower releasing profile than formulation 111/026/1 and a Sterotex™ based formulation with a faster releasing profile than formulation 111/026/2 were attempted. Formulations prepared and tested were:

| **Formula (Ref 111/043/1)** | **% (w/w)** | **mg/dose** |
|---|---|---|
| Hydrokote™ 112 | 48.9 | 159.00 |
| Miglyol™ 812 | 24.9 | 81.00 |
| Methocel™ K15M | 20.0 | 65.00 |
| Aerosil™ 200 | 3.1 | 10.00 |
| Levorphanol tartrate | 3.1 | 10.00 |

| **Formula (Ref 111/043/2)** | **% (w/w)** | **mg/dose** |
|---|---|---|
| Sterotex™ | 58.8 | 191.00 |
| Miglyol™ 812 | 24.9 | 81.00 |
| Methocel™ K15M | 10.1 | 33.00 |
| Aerosil™ 200 | 3.1 | 10.00 |
| Levorphanol tartrate | 3.1 | 10.00 |

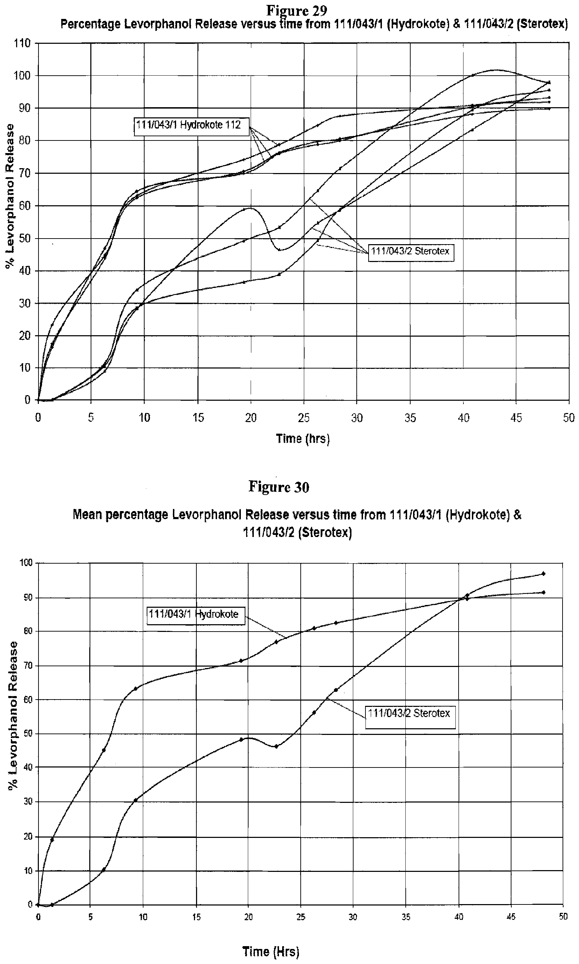

Dissolution testing of these formulations gave the release profiles shown in Figure 29 and Figure 30.

### Example 31

Precirol™ and Compritol™ based formulations with slower release profiles than formulation 111/038/3 and 111/038/4 were attempted. Formulations prepared and tested were:

| **Formula (Ref 111/045/3)** | **% (w/w)** | **mg/dose** |
|---|---|---|
| Precirol™ ATO 5 | 57.7 | 187.50 |
| Miglyol™ 812 | 25.2 | 81.90 |
| Methocel™ K15M | 10.0 | 32.50 |
| Aerosil™ 200 | 4.0 | 13.00 |
| Levorphanol tartrate | 3.1 | 10.00 |

| **Formula (Ref 111/045/4)** | **% (w/w)** | **mg/dose** |
|---|---|---|
| Compritol™ 888 ATO | 57.7 | 187.50 |
| Miglyol™ 812 | 25.2 | 81.90 |
| Methocel™ K15M | 10.0 | 32.50 |
| Aerosil™ 200 | 4.0 | 13.00 |
| Levorphanol tartrate | 3.1 | 10.00 |

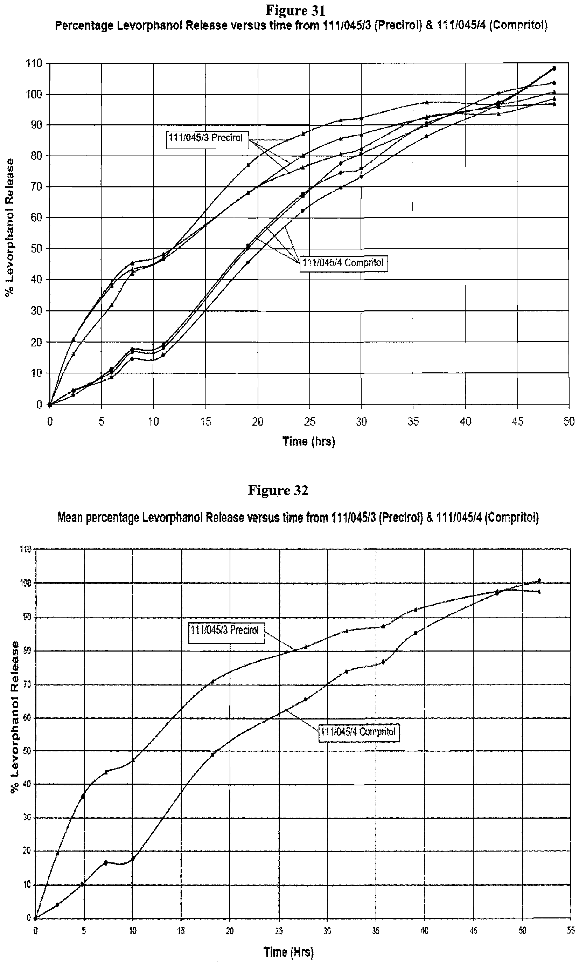

Dissolution testing of these formulations gave the release profiles shown in Figure 31 and Figure 32.

### Example 32

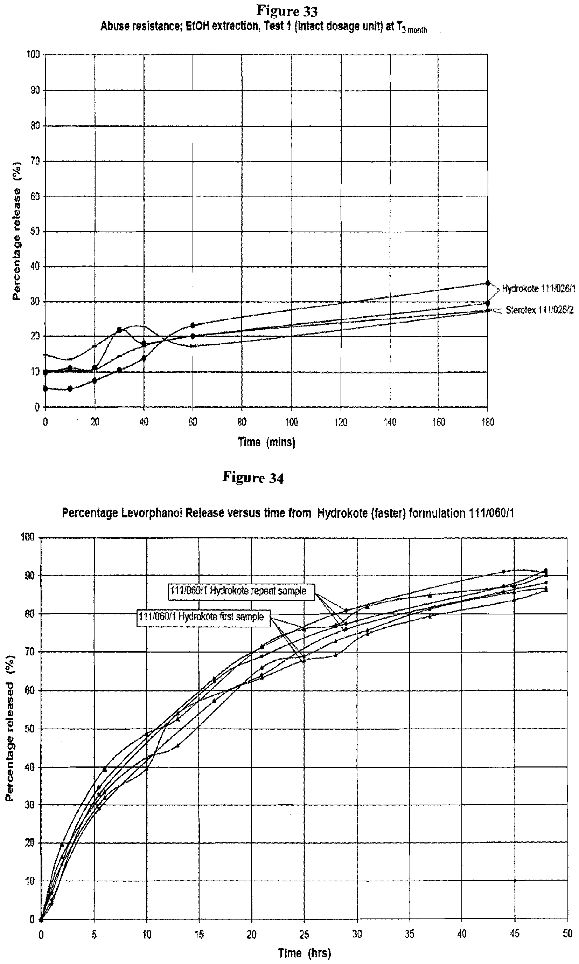

Abuse resistance testing on stability batches 111/026/1 and 111/026/2 was carried out at 3 months. See Figure 33 and the Tables below.

**Abuse Resistance Test 2 on Formulations 111/026/1&2 at T_{3 month}**

| **Extraction into water Test 2 T_{3 month} (5 ml water; 5 mins using a mortar & pestle)** | | |
|---|---|---|
| Formulation | % released | Comment |
| Hydrokote™ | 7.3; 6.4 | Viscous paste with waxy lumps |
| 111/026/1 | | |
| Sterotex™ NF | 6.7; 15.5 | Viscous paste with waxy lumps |
| 111/026/2 | | |

**Abuse Resistance Test 5 on Formulations 111/026/1&2 at T_{3 month}**

| **Extraction into acid Test 5 T_{3 month} (5 ml dil acetic; crush & bring to boil)** | | |
|---|---|---|
| Formulation | % released | Comment |
| Hydrokote™ | 0; 14.6 | Thick paste with large waxy lumps. Difficult to filter |
| 111/026/1 | | |
| Sterotex™ NF | 5.6; 3.0 | Lumpy paste difficult to draw into syringe |
| 111/026/2 | | |

### Example 33

A Hydrokote™ based formulation with a faster releasing profile than formulation 111/0436/1 and a Sterotex™ based formulation with a faster releasing profile than formulation 111/043/2 were attempted. Formulations prepared and tested were:

| **Formula (Ref 111/060/1)** | **% (w/w)** | **mg/dose** |
|---|---|---|
| Hydrokote™ 112 | 68.8 | 224.00 |
| Methocel™ K15M | 25.0 | 81.00 |
| Aerosil™ 200 | 3.1 | 10.00 |
| Levorphanol tartrate | 3.1 | 10.00 |

| **Formula (Ref 111/059/2)** | **% (w/w)** | **mg/dose** |
|---|---|---|
| Sterotex™ | 53.8 | 175.00 |
| Miglyol™ 812 | 24.9 | 49.00 |
| Methocel™ K15M | 15.0 | 33.00 |
| Aerosil™ 200 | 3.1 | 10.00 |
| Levorphanol tartrate | 3.1 | 10.00 |

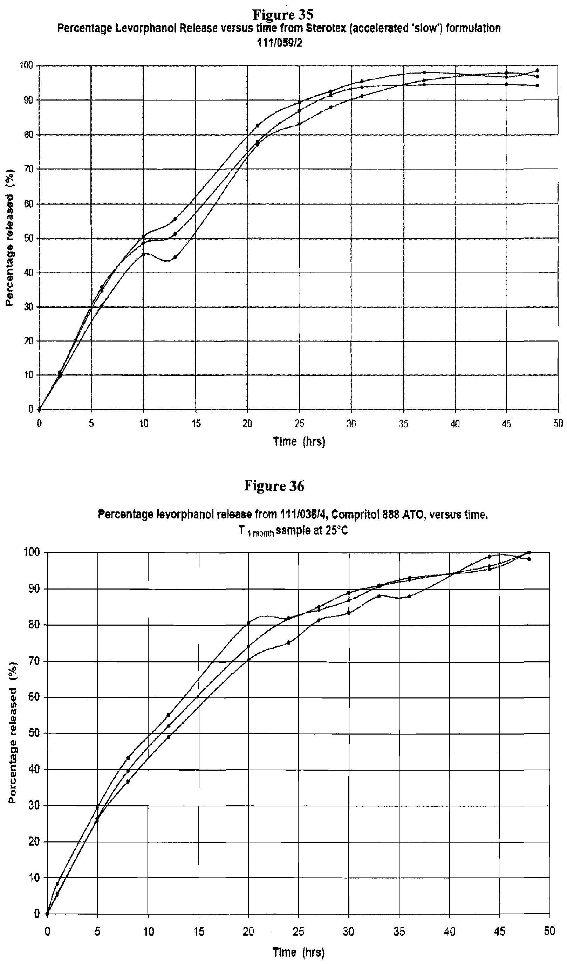

The dissolution profiles determined for these formulations are shown in Figure 34 and Figure 35

### Example 34

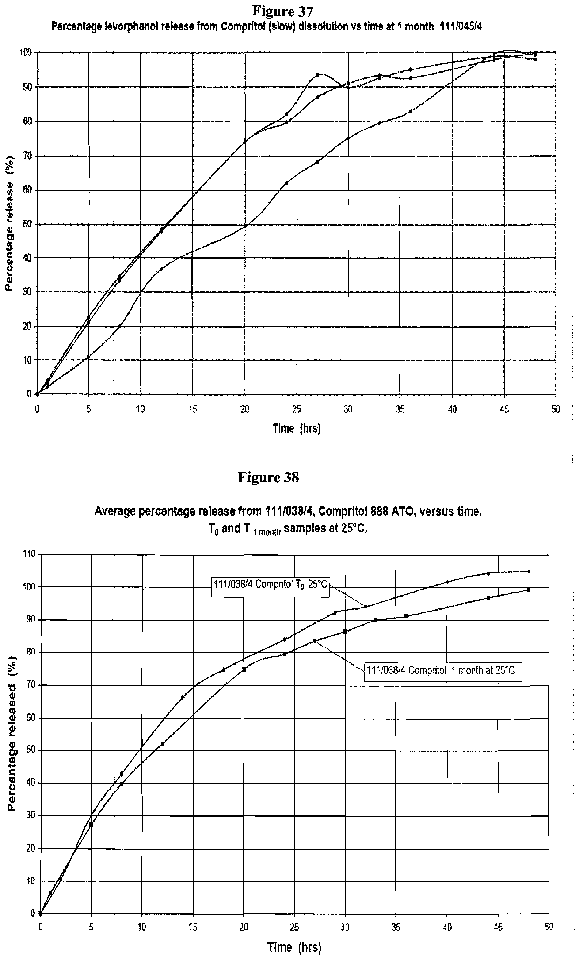

The dissolution profile of Compritol™ formulation 111/038/4 and 111/045/4 after 1 month at 25°C/60%RH and comparison with T₀ are shown in Figure 36, Figure 37, Figure 38 and Figure 39.

### Example 35

Two Sterotex™ based formulations were developed and their dissolution profiles determined to examine the magnitude in the change in release rate on Methocel™ variation. Formulations prepared and tested were:

| **Formula (Ref 111/068/2)** | **% (w/w)** | **mg/dose** |
|---|---|---|
| Sterotex™ | 57.1 | 185.60 |
| Miglyol™ 812 | 25.2 | 82.00 |
| Methocel™ K15M | 11.5 | 37.40 |
| Aerosil™ 200 | 3.1 | 10.00 |
| Levorphanol tartrate | 3.1 | 10.00 |

| **Formula (Ref 111/099/2)** | **% (w/w)** | **mg/dose** |
|---|---|---|
| Sterotex™ | 55.18 | 179.10 |
| Miglyol™ 812 | 25.2 | 82.00 |
| Methocel™ K15M | 13.5 | 43.90 |
| Aerosil™ 200 | 3.1 | 10.00 |
| Levorphanol tartrate | 3.1 | 10.00 |

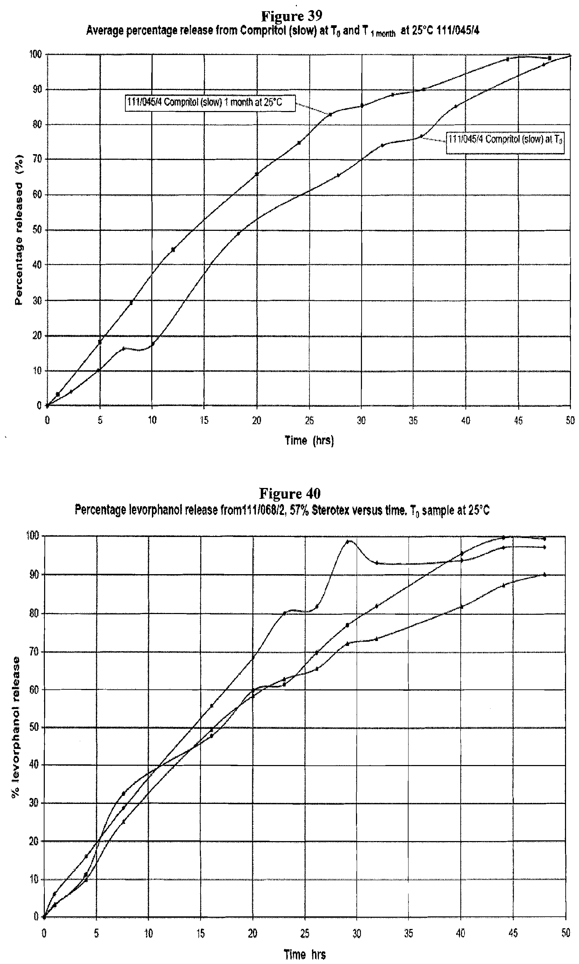

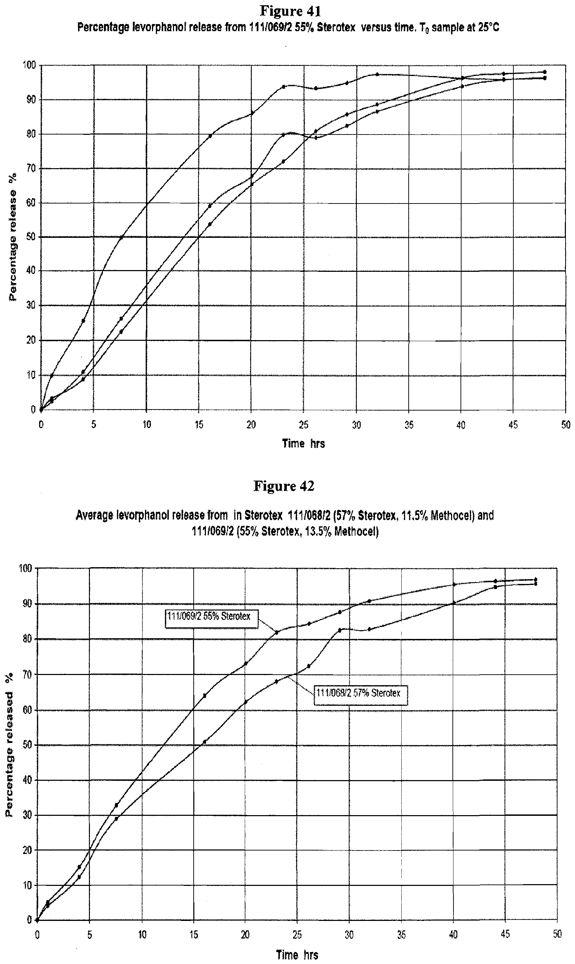

The dissolution profiles determined for these formulations are shown in Figure 40, Figure 41 and Figure 42.

### Example 36

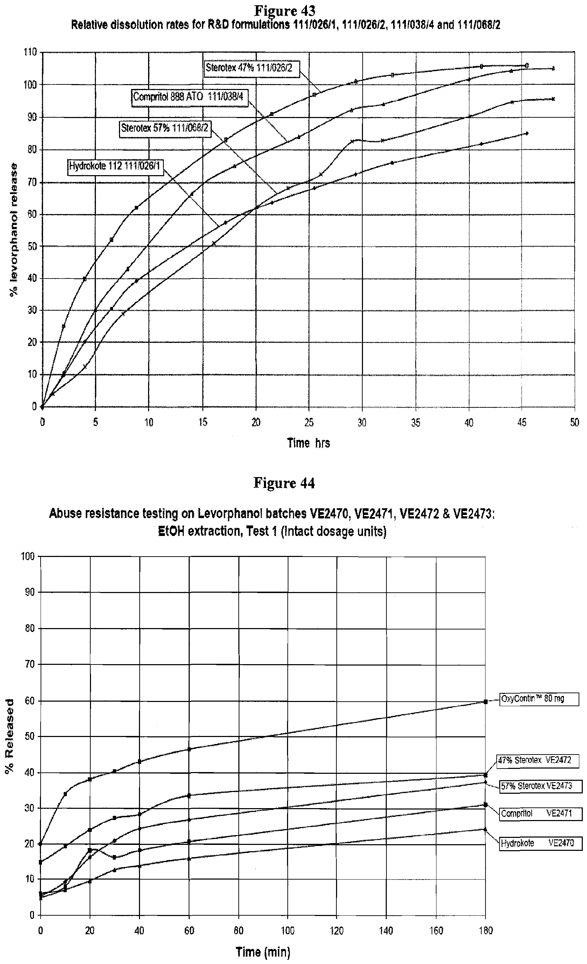

The relative dissolution profile of four R&D batches are shown in Figure 43

### Example 37

Four batches were manufactured. These formulations were Hydrokote™ formulation 111/026/1, Sterotex™ formulations 111/026/2 and 111/038/4, and Compritol™ formulation 111/038/4. See Table below.

| **Batch number** | **Description** | **Formulation Ref** |
|---|---|---|
| VE2470 | Levorphanol Tartrate ER 10 mg in Hydrokote™ 112 | 111/026/1 |
| VE2471 | Levorphanol Tartrate ER 10 mg in Compritol™ 888 ATO | 111/038/4 |
| VE2472 | Levorphanol Tartrate ER 10 mg in 47% Sterotex™ | 111/026/2 |
| VE2473 | Levorphanol Tartrate ER 10 mg in 57% Sterotex™ | 111/068/2 |

### Example 38

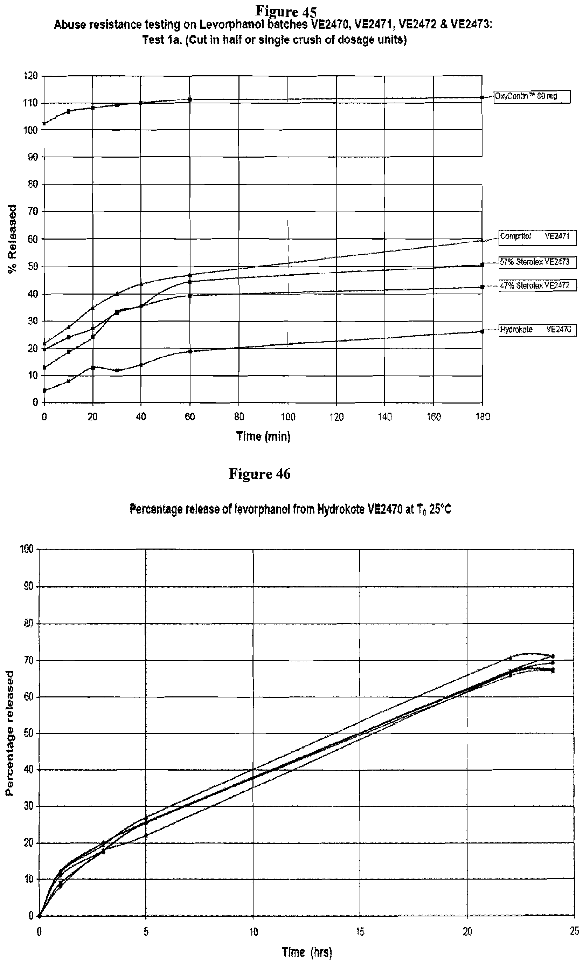

The above batches were tested for abuse resistance as shown in Figure 44 and Figure 45, and in the four Tables below.

### Example 39

**buse Resistance Test 2 on Batches VE2470, VE2471, VE2472 & VE2473**

| **Extraction into water Test 2 T₀ (5 ml water; 5 mins using a mortar & pestle)** | | |
|---|---|---|
| Formulation | % released | Comment |
| Hydrokote™ | 21.4 | Forms sticky viscous gel like paste with small lumps. Hard to filter |
| 111/026/1 | | |
| VE2470 | | |
| Compritol™ | 3.6 | Forms sticky gel which is very difficult to filter |
| 111/038/4 | | |
| VE2471 | | |
| 47% Sterotex™ | 14.1 | Forms sticky paste with small waxy lumps. Mix is difficult to filter. |
| 111/026/2 | | |
| VE2472 | | |
| 57% Sterotex™ | 27.1 | Forms sticky paste with small waxy lumps. Mix is difficult to filter. |
| 111/068/2 | | |
| VE2473 | | |
| OxyContin™ 80 mg | 82 | Mobile easily filtered solution |

**Abuse Resistance Test 3 0n Batches VE2470, VE2471, VE2472 & VE2473**

| **Crush to small particles Test 3 (Freeze, grind in mortar, sieve through 600µ sieve)** | | |
|---|---|---|
| Formulation | % through sieve | Comment |
| Hydrokote™ | 4.8 | Forms sticky film on mortar. Difficult to scrape off and pass sieve. |
| 111/026/1 | | |
| VE2470 | | |
| Compritol™ | 16.9 | Forms sticky film on mortar. Difficult to scrape off |
| 111/038/4 | | |
| VE2471 | | |
| 47% Sterotex™ | 12.5 | Forms sticky film on mortar. Difficult to scrape off a |
| 111/026/2 | | |
| VE2472 | | |
| 457% Sterotex™ | 7.0 | Forms sticky film on mortar. Difficult to scrape off a |
| 111/068/2 | | |
| VE2473 | | |
| OxyContin tablet 80 mg | 66.8 % | Powders as expected |

**Abuse Resistance Test 5 0n Batches VE2470, VE2471, VE2472 & VE2473**

| **Extraction into acid Test 5 T₀ (5 ml dilute acetic; crush & bring to boil)** | | |
|---|---|---|
| Formulation | % released | Comment |
| Hydrokote™ | 2.0 | Opaque suspension. Very difficult to filter |
| 111/026/1 | | |
| VE2470 | | |
| Compritol™ | 34.3 | Contents did not dissolve. Remainder filtered easily |
| 111/038/4 | | |
| VE2471 | | |
| 47% Sterotex™ | 4.8 | Opaque suspension with small waxy lumps. Very difficult to filter |
| 111/026/2 | | |
| VE2472 | | |
| 457% Sterotex™ | 8.5 | Opaque suspension with small waxy lumps. Very difficult to filter |
| 111/068/2 | | |
| VE2473 | | |
| OxyContin tablets 80 mg | 90 | Wax floats on mobile solution; filters easily |

**Abuse Resistance Test 6 0n Batches VE2470, VE2471, VE2472 & VE2473**

| **Base excipient** | **Excipientmp** | **Formulation** | **Formulation molten at** | **Comment** |
|---|---|---|---|---|
| Hydrokote™ 112 | 43-46°C | 111/026/1 | 45°C | VE2470 Sticky mass, can suck and eject negligible material from needle |
| Compritol™ 888 ATO | 69-74°C | 111/038/4 | 68° C | VE2471. Sticky mass, doesn't suck into syringe, sets instantly in needle tip |
| Sterotex™ | 60-63°C | 111/026/2 | 62°C | VE2472 Sticky mass, too viscous to be drawn into syringe needle. |
| Sterotex™ | 60-63°C | 111/0686/2 | 62°C | VE2473 Sticky mass, too viscous to be drawn into syringe needle. |

### Example 40

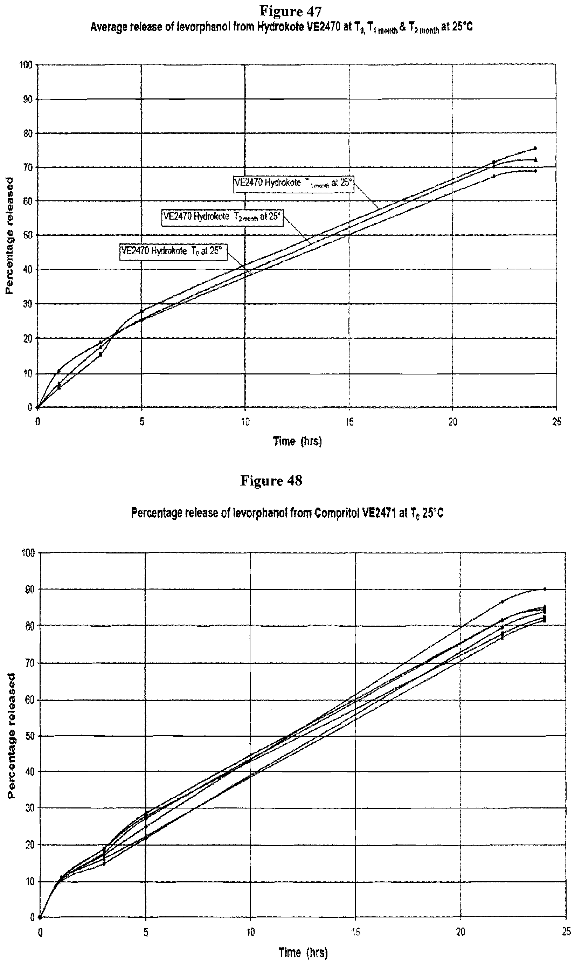

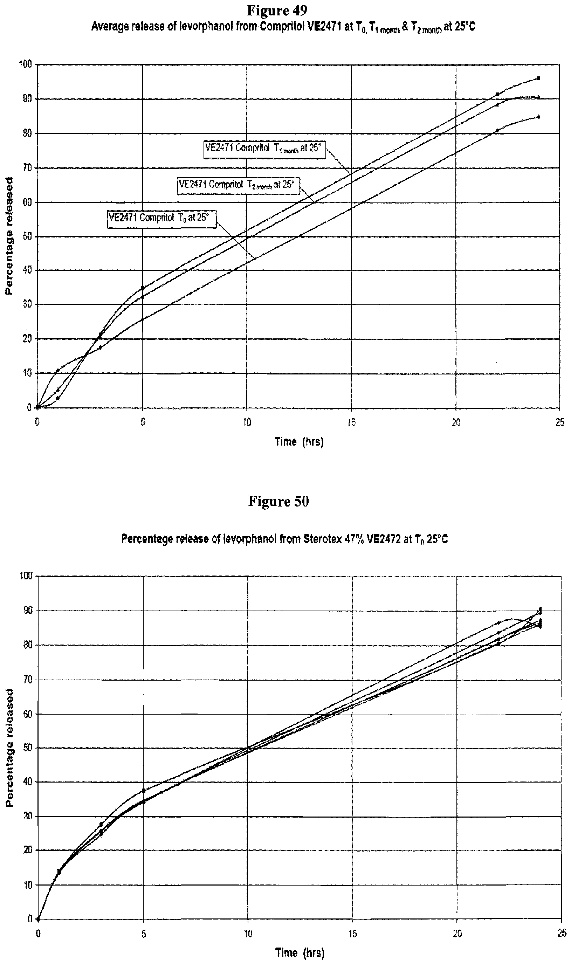

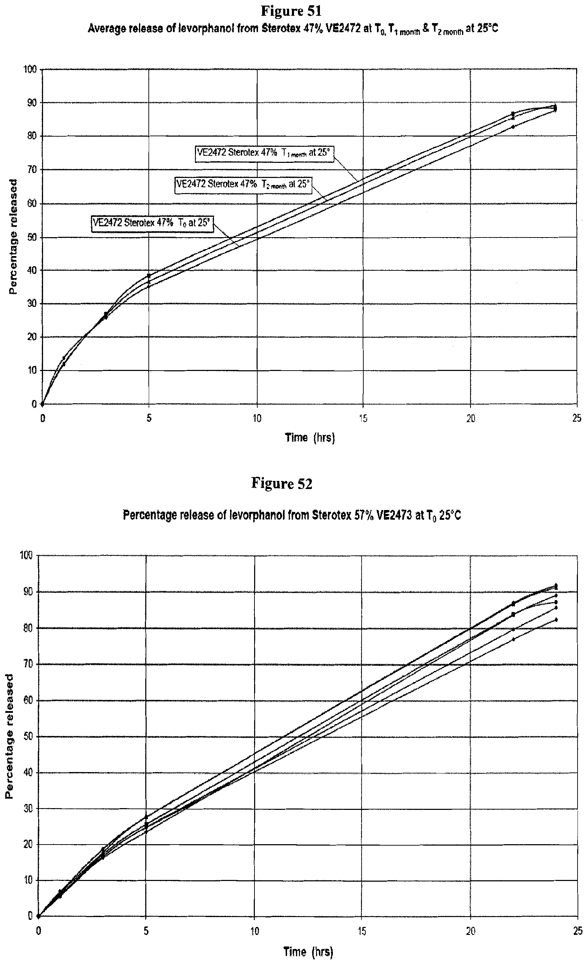

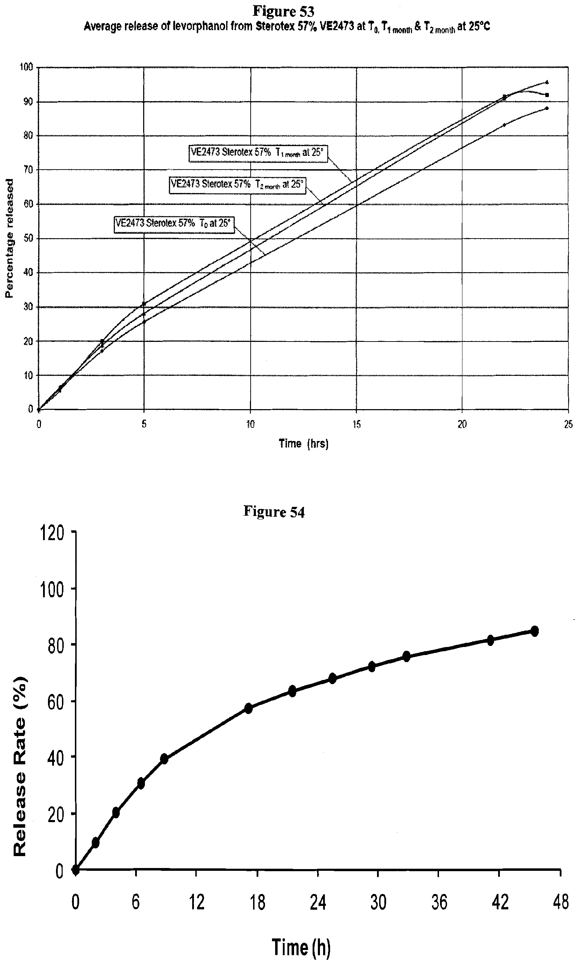

Dissolution profiles of Levorphanol ER batches after 0, 1 and two months storage at 25°C/60%RH are shown in Figure 46, Figure 47, Figure 48, Figure 49, Figure 50, Figure 51, Figure 52 and Figure 53. Formulations prepared and tested were:

| **Formula (Ref 111/026/1)** | **% (w/w)** | **mg/dose** |
|---|---|---|
| Hydrokote™ 112 | 75.4 | 245.00 |
| Methocel™ K15M | 18.5 | 60.00 |
| Aerosil™ 200 | 3.1 | 10.00 |
| Levorphanol tartrate | 3.1 | 10.00 |

| **Formula (Ref 111/026/2)** | **% (w/w)** | **mg/dose** |
|---|---|---|
| Sterotex™ (47%) | 47.1 | 153.00 |
| Miglyol™ 812 | 25.2 | 82.00 |
| Methocel™ K15M | 21.5 | 70.00 |
| Aerosil™ 200 | 3.1 | 10.00 |
| Levorphanol tartrate | 3.1 | 10.00 |

| **Formula (Ref 111/038/4)** | **% (w/w)** | **mg/dose** |
|---|---|---|
| Compritol™ 888 ATO | 53.2 | 173.00 |
| Miglyol™ 812 | 25.2 | 82.00 |
| Methocel™ K15M | 15.4 | 50.00 |
| Aerosil™ 200 | 3.1 | 10.00 |
| Levorphanol tartrate | 3.1 | 10.00 |

| **Formula (Ref 111/068/2)** | **% (w/w)** | **mg/dose** |
|---|---|---|
| Sterotex™ (57%) | 57.1 | 185.60 |
| Miglyol™ 812 | 25.2 | 82.00 |
| Methocel™ K15M | 11.5 | 37.40 |
| Aerosil™ 200 | 3.1 | 10.00 |
| Levorphanol tartrate | 3.1 | 10.00 |

### Example 41

Example 41 to 44 were prepared as follows: (i) ingredient (1) was dispensed into a mixer; (ii) ingredient (1) was heated until fully melted; (iii) ingredient (2) was dispensed into the same mixer; (iv) ingredient (1) and (2) were mixed until dispersed; (v) ingredient (3) was dispensed into the same mixer; (vi) ingredient (1), (2) and (3) were mixed until dispersed; (vii) ingredient (4) was dispensed into the same vessel; (viii) ingredient (1), (2), (3) and (4) were mixed thoroughly with a high shear mixer; (ix) the contents were transferred into a liquid filling machine and filled into hard gelatin capsules.

### Example 41

**Extended Release Levorphanol**

| | **Ingredients** | **% w/w** | **mg/capsule** |
|---|---|---|---|
| 1 | Hydrokote™ 112 | 75.38 | 245.0 |
| 2 | Methocel™ K15M | 18.46 | 60.0 |
| 3 | Aerosil™ 200 | 3.08 | 10.0 |
| 4 | Levorphanol Tartrate | 3.08 | 10.0 |

### Example 42

**Extended Release Levorphanol**

| | **Ingredients** | **% w/w** | **mg/capsule** |
|---|---|---|---|
| 1 | Beeswax | 70.74 | 237.00 |
| 2 | Methocel™ K15M | 23.88 | 80.00 |
| 3 | Aerosil™ COK 84 | 2.39 | 8.00 |
| 4 | Levorphanol Tartrate | 2.99 | 10.00 |

### Example 43

**Extended Release Levorphanol**

| | **Ingredients** | **% w/w** | **mg/capsule** |
|---|---|---|---|
| 1 | Cithrol™ GMS | 81.49 | 273.00 |
| 2 | Methocel™ K100 | 11.94 | 40.00 |
| 3 | Aerosil™ COK 84 | 3.58 | 12.00 |
| 4 | Levorphanol Tartrate | 2.99 | 10.00 |

### Example 44

**Extended Release Levorphanol**

| | **Ingredients** | **% w/w** | **mg/capsule** |
|---|---|---|---|
| 1 | Hydrokote™ 112 | 76.10 | 255.00 |
| 2 | Methocel™ K15M | 17.90 | 60.00 |
| 3 | Aerosil™ COK 84 | 3.0 | 10.00 |
| 4 | Levorphanol Tartrate | 3.0 | 10.00 |

Example 45 to 48 were prepared as follows: (i) ingredient (1) was dispensed into a mixer; (ii) ingredient (1) was heated until fully melted; (iii) ingredient (2) was dispensed into the same mixer; (iv) ingredient (1) and (2) were heated until fully melted; (v) ingredient (3) was dispensed into the same mixer; (vi) ingredient (1), (2) and (3) were mixed until dispersed; (vii) ingredient (4) was dispensed into the same mixer; (viii) ingredient (1), (2), (3) and (4) were mixed until dispersed; (ix) ingredient (5) was dispensed into the same vessel; (x) ingredient (1), (2), (3), (4) and (5) were mixed thoroughly with a high shear mixer; (ix) the contents were transferred into a liquid filling machine and filled into hard gelatin capsules.

### Example 45

**Extended Release Levorphanol**

| | **Ingredients** | **% w/w** | **mg/capsule** |
|---|---|---|---|
| 1 | Compritol™ 888 ATO | 53.23 | 173.00 |
| 2 | Miglyol™ 812 | 25.23 | 82.00 |
| 3 | Methocel™ K15M | 15.38 | 50.00 |
| 4 | Aerosil™ 200 | 3.08 | 10.0 |
| 5 | Levorphanol Tartrate | 3.08 | 10.0 |

### Example 46

**Extended Release Levorphanol**

| | **Ingredients** | **% w/w** | **mg/capsule** |
|---|---|---|---|
| 1 | Sterotex™ | 47.07 | 153.00 |
| 2 | Miglyol™ 812 | 25.23 | 82.00 |
| 3 | Methocel™ K15M | 21.54 | 70.00 |
| 4 | Aerosil™ 200 | 3.08 | 10.0 |
| 5 | Levorphanol Tartrate | 3.08 | 10.00 |

### Example 47

**Extended Release Levorphanol**

| | **Ingredients** | **% w/w** | **mg/capsule** |
|---|---|---|---|
| 1 | Sterotex™ | 57.11 | 185.60 |
| 2 | Miglyol™ 812 | 25.23 | 82.00 |
| 3 | Methocel™ K15M | 11.50 | 70.00 |
| 4 | Aerosil™ 200 | 3.08 | 10.00 |
| 5 | Levorphanol Tartrate | 3.08 | 10.00 |

### Example 48

**Extended Release Levorphanol**

| | **Ingredients** | **% w/w** | **mg/capsule** |
|---|---|---|---|
| 1 | Sterotex™ | 42.09 | 141.00 |
| 2 | Fractionated Coconut Oil | 29.85 | 100.00 |
| 3 | Methocel™ K15M | 23.89 | 80.00 |
| 4 | Aerosil™ 200 | 1.19 | 4.00 |
| 5 | Levorphanol Tartrate | 2.98 | 10.00 |

### Example 49

Example 49 is a capsule (or optionally tablet) formulation of oral extended release levorphanol for ileo-colonic and colonic delivery.

**Delayed Onset, Extended Release Levorphanol for Ileo-colonic or Colonic Delivery**

| **Ingredients** | **Qty./Unit** |
|---|---|
| 1. Levorphanol Tartrate | 20 mg |
| 2. HPMC 2208, USP | 150 mg |
| 3. Carnauba wax | 30 mg |
| 4. HPMC 2910, USP | 15 mg |
| 5. Magnesium Stearate | 2 mg |
| 6. Stearic acid | 8 mg |
| 7. Talc | 3 mg |

In Example 49, place the ingredients 1, 2 and 3 in the granulator and mix for 15 minutes. Dissolve ingredient 4 in water (mix in how water, then cool down) and spray into the fluidized mixture. Dry to approximately 5% moisture. Sequentially add ingredient 5, 6 and 7, with mixing steps between each addition. Encapsulate the dosage form in HPMC or other suitable capsules or compress into a tablet.

For the capsule formulation, prepare a composition of aqueous Eudragit™ FS30D dispersion to coat 1.3 kg HPMC capsules: Eudragit™ FS30D 1207 g (solids, 362 g), Triethyl citrate 18 g (solids 18 g), Glyceryl monostearate 11 g (solids 11 g), Tween 80 (33%) 13 g (solids 4 g) and Distilled Water 728 g. Spray the dispersion onto the HPMC capsules using an Accela-Cota 10. The temperature of the capsule bed during the coating process is maintained between 26 and 32 °C. The mean amounts of polymer applied ranges from 5 mg/cm² to 20 mg/ cm², preferably 6 mg/cm² to 10 mg/ cm².

For the tablet formulation, the composition of aqueous Eudragit™ FS30D dispersion described above may be applied with a typical coating thicknesses of 10 to 30 mg polymer per cm² of tablet surface, preferably, 10 to 18 mg polymer per cm² capsule surface.

The in vitro performance of the dosage form can be tested using methods know in the art and described herein. When the capsules are tested using the USP Paddle Method in 0.1N HCl (pH 1.2) for two hours, followed by a switch to phosphate buffer pH 6.8 for one to two hours and then again, a switch to phosphate buffer pH 7.4, they remain intact and substantially non-releasing at pH 1.2 and pH 6.8 and provide substantial release at pH 7.4.

In some embodiments, oral delayed onset, extended release capsule of levorphanol for ileo-colonic and colonic delivery in sustained release form can be made, as shown in Example 49 to 62.

Example 50 to 53 are prepared as follows: (i) dispense ingredient (1) into a mixer; (ii) heat ingredient (1) until fully melted; (iii) dispense ingredient (2) into the same mixer; (iv)mix ingredient (1) and (2) until dispersed; (v) dispense ingredient (3) into the same mixer; (vi) mix ingredient (1), (2) and (3) until dispersed; (vii) dispense ingredient (4) into the same vessel; (viii) mix ingredient (1), (2), (3) and (4) thoroughly with a high shear mixer; (ix) transfer the contents into a liquid filling machine and fill into hard gelatin capsules. Optionally, cure the capsules by setting them at room temperature for a period of 1 to 7 days.

For Example 50 to 53, prepare a composition of aqueous Eudragit™ FS30D dispersion to coat 1.3 kg HPMC capsules: Eudragit™ FS30D 1207 g (solids, 362 g), Triethyl citrate 18 g (solids 18 g), Glyceryl monostearate 11 g (solids 11 g), Tween 80 (33%) 13 g (solids 4 g) and Distilled Water 728 g. Spray the dispersion onto the HPMC capsules using an Accela-Cota 10. The temperature of the capsule bed during the coating process is maintained between 26 and 32 °C. The mean amounts of polymer applied ranges from 5 mg/cm² to 20 mg/ cm², preferably 6 mg/cm² to 15 mg/ cm².

The in vitro performance of the dosage form can be tested using methods know in the art and described herein. When the capsules are tested using the USP Paddle Method in 0.1N HCl (pH 1.2) for two hours, followed by a switch to phosphate buffer pH 6.8 for one to two hours and then again, a switch to phosphate buffer pH 7.4, the capsules will remain intact and substantially non-releasing at pH 1.2 and pH 6.8, and they will release the levorphanol at pH 7.4 over a prolonged period (e.g., over 12 to 48 hours).

### Example 50

**Delayed Onset, Extended Release Dosage for Ileo-colonic or Colonic Release**

| | **Ingredients** | **% w/w** | **mg/capsule** |
|---|---|---|---|
| 1 | Hydrokote™ 112 | 75.38 | 245.0 |
| 2 | Methocel™ K15M | 18.46 | 60.0 |
| 3 | Aerosil™ 200 | 3.08 | 10.0 |
| 4 | Levorphanol Tartrate | 3.08 | 10.0 |

### Example 51

**Delayed Onset, Extended Release Dosage for Ileo-colonic or Colonic Release**

| | **Ingredients** | **% w/w** | **mg/capsule** |
|---|---|---|---|
| 1 | Beeswax | 70.74 | 237.00 |
| 2 | Methocel™ K15M | 23.88 | 80.00 |
| 3 | Aerosil™ COK 84 | 2.39 | 8.00 |
| 4 | Levorphanol Tartrate | 2.99 | 10.00 |

### Example 52

**Delayed Onset, Extended Release Dosage for Ileo-colonic or Colonic Release**

| | **Ingredients** | **% w/w** | **mg/capsule** |
|---|---|---|---|
| 1 | Cithrol™ GMS | 81.49 | 273.00 |
| 2 | Methocel™ K100 | 11.94 | 40.00 |
| 3 | Aerosil™ COK 84 | 3.58 | 12.00 |
| 4 | Levorphanol Tartrate | 2.99 | 10.00 |

### Example 53

**Delayed Onset, Extended Release Dosage for Ileo-colonic or Colonic Release**

| | **Ingredients** | **% w/w** | **mg/capsule** |
|---|---|---|---|
| 1 | Hydrokote™ 112 | 75.38 | 253.00 |
| 2 | Methocel™ K15M | 18.46 | 60.00 |
| 3 | Aerosil™ COK 84 | 3.08 | 12.00 |
| 4 | Levorphanol Tartrate | 3.08 | 10.00 |

Example 54 to 57 are prepared as follows: (i) dispense ingredient (1) into a mixer; (ii) heat ingredient (1) until fully melted; (iii) dispense ingredient (2) into the same mixer; (iv) heat ingredient (1) and (2) until fully melted; (v) dispense ingredient (3) into the same mixer; (vi) mix ingredient (1), (2) and (3) until dispersed; (vii) dispense ingredient (4) into the same mixer; (viii) mix ingredient (1), (2), (3) and (4) until dispersed; (ix) dispense ingredient (5) into the same vessel; (x) mix ingredient (1), (2), (3), (4) and (5) thoroughly with a high shear mixer; (ix) transfer the contents into a liquid filling machine and fill into hard gelatin capsules.

For Example 54 to 57, prepare a composition of aqueous Eudragit™ FS30D dispersion to coat 1.3 kg HPMC capsules: Eudragit™ FS30D 1207 g (solids, 362 g), Triethyl citrate 18 g (solids 18 g), Glyceryl monostearate 11 g (solids 11 g), Tween 80 (33%) 13 g (solids 4 g) and Distilled Water 728 g. Spray the dispersion onto the HPMC capsules using an Accela-Cota 10. The temperature of the capsule bed during the coating process is maintained between 26 and 32 °C. The mean amounts of polymer applied ranges from 5 mg/cm² to 20 mg/ cm², preferably 6 mg/cm² to 15 mg/ cm².

The in vitro performance of the dosage form can be tested using methods know in the art and described herein. When the capsules are tested using the USP Paddle Method in 0.1N HCl (pH 1.2) for two hours, followed by a switch to phosphate buffer pH 6.8 for one to two hours and then again, a switch to phosphate buffer pH 7.4, the capsules will remain intact and substantially non-releasing at pH 1.2 and pH 6.8, and they will release the levorphanol at pH 7.4 over a prolonged period (e.g., over 12 to 48 hours).

### Example 54

**Delayed Onset, Extended Release Dosage for Ileo-colonic or Colonic Release**

| | **Ingredients** | **% w/w** | **mg/capsule** |
|---|---|---|---|
| 1 | Compritol™ 888 ATO | 52.23 | 173.00 |
| 2 | Miglyol™ 812 | 25.23 | 82.00 |
| 3 | Methocel™ K15M | 15.38 | 50.00 |
| 4 | Aerosil™ 200 | 3.08 | 10.0 |
| 5 | Levorphanol Tartrate | 3.08 | 10.0 |

### Example 55

**Delayed Onset, Extended Release Dosage for Ileo-colonic or Colonic Release**

| | **Ingredients** | **% w/w** | **mg/capsule** |
|---|---|---|---|
| 1 | Sterotex™ | 47.07 | 153.00 |
| 2 | Miglyol™ 812 | 25.23 | 82.00 |
| 3 | Methocel™ K15M | 21.54 | 70.00 |
| 4 | Aerosil™ 200 | 3.08 | 10.0 |
| 5 | Levorphanol Tartrate | 3.08 | 10.00 |

### Example 56

**Delayed Onset, Extended Release Dosage for Ileo-colonic or Colonic Release**

| | **Ingredients** | **% w/w** | **mg/capsule** |
|---|---|---|---|
| 1 | Sterotex™ | 57.11 | 185.60 |
| 2 | Miglyol™ 812 | 25.23 | 82.00 |
| 3 | Methocel™ K15M | 11.50 | 70.00 |
| 4 | Aerosil™ 200 | 3.08 | 10.00 |
| 5 | Levorphanol Tartrate | 3.08 | 10.00 |

### Example 57

**Delayed Onset, Extended Release Dosage for Ileo-colonic or Colonic Release**

| | **Ingredients** | **% w/w** | **mg/capsule** |
|---|---|---|---|
| 1 | Sterotex™ | 42.09 | 141.00 |
| 2 | Fractionated Coconut Oil | 23.88 | 80.00 |
| 3 | Methocel™ K15M | 29.85 | 100.00 |
| 4 | Aerosil™ COK 84 | 1.194 | 4 |
| 5 | Levorphanol Tartrate | 2.99 | 10.00 |

Examples 58 and 59 are oral delayed onset, extended release tablets of levorphanol for ileo-colonic and colonic delivery in sustained release.

The core tablet of levorphanol is prepared using the composition shown in the Tables for Examples 58 and 59 using the following procedure: (i) Mix 1, 2, (and 3), and 5 and pass through #20 mesh; (ii) Dissolve 4 in 7; (iii) Granulate the powder from (i) using a fluid bed granulator and then dry; (iv) Pass the dry granules through #20 mesh; (v) Mix the granules with 6; (vi) Compress to tablet using plain/plain tooling. Prepare a coating solution comprising: (a) Ethylcellulose (Ethocel PR100), 9.0 to 15 mg per tablet; (b) Polyvinylpyrrolidone (Kollidon™ 90F), 3.0 to 7.0 mg per tablet; (c) Dibutyl Sebacate, 2.0 to 4.0 mg per tablet; (d) Denatured Alcohol 150 to 300 mg per tablet (evaporates during process). Spray coating solution using coating pan until desired in vitro release is achieved as described herein. Adjust coating solution composition or coating weight/thickness as required. The coating weight is usually approximately 5 to 40% w/w. For the initial trial, prepare one coating comprising : (a) Ethylcellulose (Ethocel PR100), 9.2 mg per tablet; (b) Polyvinylpyrrolidone (Kollidon™ 90F), 4.15 mg per tablet; (c) Dibutyl Sebacate, 2.70 mg per tablet; (d) Denatured Alcohol 170 mg per tablet (evaporates during process). Prepare another coating comprising: (a) Ethylcellulose (Ethocel PR100), 14.15 mg per tablet; (b) Polyvinylpyrrolidone (Kollidon™ 90F), 5.1 mg per tablet; (c) Dibutyl Sebacate, 3.85 mg per tablet; (d) Denatured Alcohol 245 mg per tablet (evaporates during process).

### Example 58

**Delayed Onset, Extended Release Levorphanol Tartrate Tablets for Slow Ileo-colonic or Colonic Release**

| **Ingredients** | **Quantity** |
|---|---|
| | **(mg)** |
| 1. Levorphanol Tartrate | 40.00 |
| 2. Microcrystalline Cellulose | 90.00 |
| 3. HPMC (low viscosity grade) | 46.00 |
| 4. Polyvinyl Alcohol | 2.00 |
| 5. Colloidal Silicon Dioxide | 1.00 |
| 6. Sodium Stearyl Fumarate | 1.00 |
| 7. Purified Water * | As needed to form granules |

| | |
|---|---|
| * Evaporated during process | |

### Example 59

**Delayed Onset, Extended Release Levorphanol Tartrate Tablets for Slow Ileo-colonic or Colonic Release**

| **Ingredients** | **Quantity** |
|---|---|
| | **(mg)** |
| 1. Levorphanol Tartrate | 10.00 |
| 2. Microcrystalline Cellulose | 90.00 |
| 3. HPMC (low viscosity grade) | --- |
| 4. Polyvinyl Alcohol | 2.00 |
| 5. Colloidal Silicon Dioxide | 1.00 |
| 6. Sodium Stearyl Fumarate | 1.00 |
| 7. Purified Water * | 41.60 (to form granules) |

| | |
|---|---|
| * Evaporated during process | |

Alternatively, Examples 58 and 59 may be coated with a suitable amount of Eudragit™ S 100, or Eudragit™ S 12.5, or Eudragit™ FS 30D

### Example 60

Example 60 provides a method for manually sealing the overlapping region of the HPMC capsule body and cap using a process called banding. The banding solution is prepared as follows: (i) prepare the banding solution with 18 g of 4.5 cps HPMC along with 33 g of water and heated to 80 °C in a beaker; (ii) stir the mixture until the HPMC is well dispersed in the water; (iii) to completely solubilize the HPMC, add 50 mL of absolute alcohol and sonicate for 10 minutes; (iv) seal capsules using equipment such as the Lab band sealing machine (LBS 100) or the Qualicaps™ Lab-Top Capsule Band-Sealer (S-1); (v) apply banding solution uniformly to the external edge of the gap of the capsule to be sealed to form a liquid ring around the circumference of the capsule and remove excess sealing liquid from the exterior of the capsule; (vi) dry the capsules by applying thermal energy, such as hot air and a controlled temperature oven. For larger scale banding, automated equipment such as the Qualicaps S-40 or S-100 capsule band-sealing machine or the Hermetica™ is a capsule banding machine may be used to seal filled, two-piece capsules with a single or double band of substances such as gelatin or hypromellose at the joined portion of the cap and body.

### Example 61

Example 61 provides an alternative method of coating small batches of capsules to provide ileo-colonic or colonic deliver of the oral levorphanol dosage form for slow release. This method can be used in place of other described enteric coating methods. The method can be readily modified by those skilled in the art to coat tablet dosage forms. In this example, coating is carried out using 45% suspension of Eudragit™ FS30D. Coating solution is prepared by homogenizing talc and triethyl citrate in water for 10 minutes. This suspension is poured into Eudragit™ FS 30 D dispersion under stirring. This spray suspension is transferred through 0.5 mm sieve. Coating suspension is spread by spray gun on a moving capsule bed. The process is carried out in conventional coating pan (see Table below). The coating solution may be further modified to achieve targeted GI delivery or release in or distal to the duodenum, jejunum, ileum, ileo-cecal junction, ileo-colonic region or colon, using methods known in the art and those described herein.

**Composition of Coating Solution**

| **Ingredients** | **Enteric coating** |
|---|---|
| Eudragit™ FS 30 D | 150 g |
| Talc | 22.5 g |
| Triethyl citrate | 2.5 g |
| Water | 175 g |

The coating process parameters like the coating pan, baffles, inlet air temperature, product temperature, exhaust and spray air pressure are shown in Table below.

**Coating Process Parameters**

| | |
|---|---|
| Coating pan | 12 inch |
| Baffles | Present |
| Silicone tube od/id | 2 mm |
| Inlet air temperature | 40 °C |
| Product Temperature | 30 °C |
| Exhaust | ON |
| Blower | ON |
| Spray air pressure | 2 bar |

### Example 62

Example 62 provides an alternative method of coating small batches of capsules to provide ileo-colonic or colonic delivery of the oral levorphanol dosage form for slow release. This method can be used in place of other described enteric coating methods. The method can be readily modified by those skilled in the art to coat tablet dosage forms. The coating solution may be further modified to achieve targeted GI delivery or release in or distal to the duodenum, jejunum, ileum, ileo-cecal junction, ileo-colonic region or colon, using methods known in the art and those described herein. An initial coating solution is provided in the Table below. This can be modified further to achieve the desired delivery profile:

| **S. No.** | **Ingredients** | **% w/w** |
|---|---|---|
| 1 | Eudragit™ S 100 | 6.1 |
| 2 | Triethyl citrate | 0.9 |
| 3 | Talc | 3.0 |
| 4 | Isopropyl alcohol | 85.0 |
| 5 | Water | 5.0 |

The procedure for preparation of the above coating solution is: (a) dissolve Eudragit™ in 70% of total IPA and 100 % of water; (b) homogenize talc and triethyl citrate in 30% of remaining IPA; (c) add talc and triethyl citrate dispersion to Eudragit™ solution and stir gently. Apply the coat by dip coating using the ProCoater™ (Torpac, New Jersey), with the method recommended by the manufacturer or preferably described by described by Dodds and Podczeck (Tablets & Capsules, January 2008, CSC Publishing Inc). Once dry, check weight gain. Additional coating may be applied to provide further weight gain. Batches with polymer target weight gains of about 10 mg/cm2 to about 20 mg/cm2 are prepared (for example, 10 mg/cm2 or 15 mg/cm2 and 20 mg/cm2). The approximate surface area of Capsule Size 00, 0, 1 and 2 are 6.16 cm2, 5.07 cm2, 4.06 cm2, and 3.43 cm2, respectively (see Jones, Tablets & Capsules, January 2009 and April 2009, CSC Publishing Inc). This provides a target weight gain range shown below:

| **Capsule** | | **Total Capsule Weight Gain** | | |
|---|---|---|---|---|
| **Size** | **Surface Area** | **10 mg/cm²** | **15 mg/cm²** | **20 mg/cm²** |
| 00 | 6.16 cm² | 60 mg | 90 mg | 120 mg |
| 0 | 5.07 cm² | 50 mg | 75 mg | 100 mg |
| 1 | 4.06 cm² | 40 mg | 60 mg | 80 mg |
| 2 | 3.43 cm² | 35 mg | 50 mg | 70 mg |

To determine if the desired target delivery or release has been achieved, a disintegration tester is used (e.g. manually with the ERWEKA™ ZT 120, ZT 120, ZT 220, or ZT 320, or using the automated ZT 850). Alternatively, an orbital shaker or a beaker with an agitator (or magnetic stirrer at moderate or high speed) is used, first in 0.1N HCl for two hours, then with a change in media to water at pH >7 (or other suitable pH). The capsules shell remains intact in 0.1 N HCL (pH 1.2) for two hours and disintegrates or ruptures at the desired pH (e.g., pH 7) usually after 5 to 90 minutes (the higher the weight gain, the longer they stay intact at pH 7). For delayed onset, extended release dosage forms intended to provide targeted GI delivery (e.g., ileo-colonic or colonic delivery) for subsequent slow or sustained release, the capsule shell will rupture, disintegrate, or dissolve but the contents may remain intact (depending on the dosage form used).

A more specific and reliable method to determine coating integrity and attainment of target GI delivery (which does not rely on visual inspection of capsules) for delayed onset, extended release forms is the use of a dissolution methods where the drug release is quantified. When such the capsules are tested using the USP Paddle Method in 0.1N HCl (pH 1.2) for 2 hours at 37 °C, the capsules (or tablets) remain intact and substantially non-releasing. Following a switch to the USP Basket or Paddle Method at 100 rpm in 900 mL distilled water (time = 0 hour begins here) at 37 °C at the target pH, the dosage form begins to provide rapid or slow release of the drug usually after 5 to 90 minutes.

The optimal coating solution composition, weight gain and process parameters to achieve the desired target delivery or release is achieved by simultaneously testing the coated dosage form dosage form at various pH using the USP Paddle Method in 0.1N HCl (pH 1.2) for 2 hours at 37 °C, followed by a switch to the USP Basket or Paddle Method at 100 rpm in 900 mL distilled water (time = 0 hour begins here) at 37 °C at the target pH (e.g., pH 6.5, pH 6.8, pH 7.0, pH 7.5). For delayed onset, extended release dosage forms intended to provide targeted GI delivery distal to the stomach, preferably less than 10% of drug should be released at pH 1.2 at 1 or 2 hours, using the method shown above.

Various alternative small and large scale methods of organic and aqueous coating of tablets and capsules have been described or referenced herein, or are known those skilled in the art. Enteric coatings may also be applied over tablet and capsule osmotic delivery dosage forms of oral levorphanol, including push pull osmotic pumps, monolithic osmotic delivery systems and controlled porosity osmotic pumps to provide targeted delivery in the GI tract.

### Example 63

A matrix tablet formulation of extended release levorphanol is prepared using the method described here: (i) the Polyox and MCC are passed through a 44# sieve and then weighed; (ii) levorphanol tartrate is weighed and milled in mortar-pestle to make fine powder; (iii) the mixture of step (i) is mixed with step (ii) to provide a homogeneous mixture. Uniform mixture is achieved through sieving several times and mixing geometrically; (iv) the lubricants are added and passed through 25 # sieve in above mixture by sieving them with step (iii); (v) The mixture is well mixed and sieved through a 25# sieve twice and homogenized in a polybag; (vi) tablets are compressed using a standard concave 9 mm punch on single punch compression machine.

**Extended Release Levorphanol Tablets**

| | **Drug Layer** | **mg/Tablet** | **%w/w** |
|---|---|---|---|
| 1 | Levorphanol Tartrate | 10.0 | 5.0 |
| 2 | Polyox WSR 301 | 100.0 | 50.0 |
| 3 | MCC 101 | 80.0 | 40.0 |
| 4 | Talc | 3.0 | 1.5 |
| 5 | Mg. Stearate | 3.0 | 1.5 |
| 6 | SiO2 | 4.0 | 2 |

The tablet dissolution is evaluated in 900 mL of pH 1.2 for 2 hours, followed by a switch to pH 6.8 phosphate buffer using the USP paddle method at 50 to 100 rpm, and in 900 mL of pH 6.8 phosphate buffer using the USP paddle method at 100 rpm and.

### Example 64

A matrix tablet formulation of oral extended release levorphanol is prepared using the method described here: (i) the Polyox, HPMC K100M CR and MCC are passed through a 44 # sieve and then weighed; (ii) levorphanol tartrate is weighed and passed through a 25# sieve; (iii) the mixture of step (i) is mixed with step (ii) to provide a homogeneous mixture. Uniform mixture is achieved through sieving several times and mixing geometrically; (iv) the lubricants are added and passed through 100 # sieve in above mixture by sieving them with step (iii); (v) the mixture is well mixed and sieved through a 25# sieve twice and homogenized in a polybag; (vi) tablets are compressed using a standard concave 9 mm punch on single punch compression machine.

**Extended Release Levorphanol Tablets**

| | **Drug Layer** | **mg/Tablet** | **%w/w** |
|---|---|---|---|
| 1 | Levorphanol Tartrate | 10.0 | 5.0 |
| 2 | Polyox WSR 301 | 50.0 | 25.0 |
| 3 | HPMC K100M CR | 50.0 | 25.0 |
| 4 | MCC 101 | 80.0 | 40.0 |
| 5 | Talc | 3.0 | 1.5 |
| 6 | Mg. Stearate | 3.0 | 1.5 |
| 7 | SiO2 | 4.0 | 2 |

The tablet dissolution is evaluated in 900 mL of pH 1.2 for 2 hours at 50 to 100 rpm, followed by a switch to 900 mL pH 6.8 phosphate buffer using the USP paddle

A controlled porosity osmotic pump (CPOP) dosage form of oral levorphanol is prepared using the method described here.

Core Tablet: (i) levorphanol tartrate is mixed with lactose and sodium chloride after passing through 40# sieve; (ii) the mixture of step (1) is added to PVP K 30 solution in IPA; (iii) the mass is well mixed to achieve a homogeneous mixture; (iv) the mass is dried in a dryer at 50°C for 10 minutes; (v) the mass is passed through a #18 sieve once for granulation; (vi) the granules are further dried; (vii) magnesium stearate and talc are then added and mixed; (viii) the tablets are compressed using a standard concave 9 mm punch on single punch compression machine.

Coating Solution: (i) cellulose acetate is dissolved in acetone; (ii) D-sorbitol is dissolved in water half quantity; (iii) PEG is dissolved in rest of the one fourth of water; (iv) step (ii) is mixed with step (1) with high stirring; (v) step (iii) is mixed with the mixture of step (i) and step ii); (vi) the solution is stirred to homogenize; (vii) dip the tube of the coating solution dispenser into the product of step (vi).

**Extended Release Osmotic Dosage Form Core Tablet**

| | **Drug Layer** | **mg/Tablet** | **%w/w** |
|---|---|---|---|
| 1 | Levorphanol Tartrate | 10.0 | 4.3 |
| 2 | Sodium Chloride | 100.0 | 43.5 |
| 3 | Lactose | 100.0 | 43.5 |
| 4 | PVP K 30 in IPA 3 mL | 12.0 | 5.2 |
| 5 | Mg. Stearate | 3.0 | 1.3 |
| 6 | Talc | 3.0 | 1.3 |
| 7 | Aerosil™ 200 | 2.0 | 0.87 |

**Extended Release Osmotic Dosage Form Coating Solution**

| | **Coating Formula** | **mg/Tablet** | **%w/w** |
|---|---|---|---|
| 1 | Cellulose Acetate | 22.95 | 2.39 |
| 2 | D -Sorbitol | 4.05 | 0.42 |
| 3 | PEG - 400 | 4.05 | 0.42 |
| 4 | Acetone | 855.00 | 88.97 |
| 5 | Water | 75.00 | 7.80 |

**Extended Release Osmotic Dosage Form Coating Process Parameters**

| **Coating Parameters** | |
|---|---|
| Solvent | Acetone : Water (90 : 10) |
| Solids content (%w/w) | 4 % (3.23 %) |
| Weight gain (%) | 15 % Target / 13.71 Achieved |
| Inlet air temperature (°C) | 50 - 55 °C (Set) 49 °Actual |
| Out let air temperature (°C) | 45-50 °C (Set) 40° Actual |
| Inlet air CFM | 32 |
| Outlet air CFM | 43 |
| Tablet surface bed temp (°C) | 40-42 °C |
| Pre-warm tablet bed (°C) | 41-43 °C |
| Load | 175 gm |
| Atomizing air pressure (kg/cm²) | 3.0 kg/cm² |
| Gun-to-bed distance (inches) | Three to Four inch |
| Spray rate (g/min) | 4.5 to 5.5 g\min |
| Baffles | 6 |
| Pan speed (rpm) | 3.0 RPM |
| Peristaltic Pump RPM | 10 RPM |
| Fluid nozzle (mm) | 1.5 |
| Spray equipment | Solace |
| Spray pan size | 12" |

The tablet dissolution is evaluated in 900 mL of pH 6.8 phosphate buffer using the USP paddle method at 50 to 100 rpm after application of coating in the following w/w% amounts: (i) 5.7%; (ii) 9.6%; and (13.7%).

### Example 65

A push-pull osmotic pump (PPOP) dosage form of extended release levorphanol is prepared using the method described below. Ingredients 1, 2 and 3 of drug layer are sifted (through BSS 22), dry mixed, and granulated with IPA. The granules, after drying and sifting (through BSS 22) are lubricated with ingredients 4 to 6 (passed through 30 BSS) of drug layer blend. Similarly, ingredients 1, 2, 3 and 4 of push layer are granulated with IPA. The granules, after drying and sifting are lubricated with ingredients 5 to 7. Bilayer tablets are compressed on a single punch compression machine using 8 mm punch. First the push layer is filled into die cavity and pressed lightly followed by addition of drug layer and mass is compressed to obtain tablets having minimum hardness of 4.5 kg/cm². A 4% w/v solution containing cellulose acetate and PEG 4000 in ratio of 90:10 is used for the coating of the bilayer tablets. Cellulose acetate and PEG 4000 are dissolved in acetone, stirred and sonicated to obtain clear transparent solution. Coating is done until the weight gain of 20-22 % w/w which takes around 2.5 hrs. Coated tablets are dried in the oven for 16 to 20 hours; 55 °C. Coated tablets are drilled using 8.0 mm drill at the centre of tablet on the drug layer side to create an appropriate orifice.

**Extended Release Osmotic Dosage Form Drug Layer**

| | **Drug Layer** | **mg/Tablet** | **%w/w** |
|---|---|---|---|
| 1 | Levorphanol Tartrate | 10 | 6.45 |
| 2 | Polyox WSR N 80 | 136 | 87.74 |
| 3 | HPMC E5 | 7 | 4.51 |
| 4 | Magnesium Stearate | 1 | 0.65 |
| 5 | Colloidal Silicon Dioxide | 0.5 | 0.32 |
| 6 | Talc | 0.5 | 0.32 |

**Extended Release Osmotic Dosage Form Push Layer**

| | **Push Layer** | **mg/Tablet** | **%w/w** |
|---|---|---|---|
| 1 | Polyethylene oxide (Polyox WSR Coagulant) | 63 | 63 |
| 2 | Sodium Chloride | 29 | 29 |
| 3 | HPMC E5 | 5 | 5 |
| 4 | Red Ferric Oxide | 1 | 1 |
| 5 | Magnesium Stearate | 0.8 | 0.8 |
| 6 | Colloidal Silicon Dioxide | 0.7 | 0.7 |
| 7 | Talc | 0.5 | 0.5 |

The tablet dissolution is evaluated in 900 mL of pH 6.8 phosphate buffer using the USP paddle method at 50 to 100 rpm.

### Example 66

A push-pull osmotic pump (PPOP) dosage form of extended release levorphanol is prepared using the method described below. Ingredients 1, 2 and 3 of drug layer are sifted (through BSS 22), dry mixed, and granulated with IPA. The granules, after drying and sifting (through BSS 22) are lubricated with ingredients 4 to 6 (passed through 30 BSS) of drug layer blend. Similarly, ingredients 1, 2, 3 and 4 of push layer are granulated with IPA. The granules, after drying and sifting are lubricated with ingredients 5 to 7. Bilayer tablets are compressed on a single punch compression machine using 8 mm punch. First the push layer is filled into die cavity and pressed lightly followed by addition of drug layer and mass is compressed to obtain tablets having minimum hardness of 7.5 kg/cm². A 4% w/v solution containing cellulose acetate and PEG 4000 in ratio of 95:5 is used for the coating of the bilayer tablets. Cellulose acetate and PEG 4000 are dissolved in acetone, stirred and sonicated to obtain clear transparent solution. Coating is done until the weight gain of 20-22 % w/w which takes around 2.5 hrs. Coated tablets are drilled using 8.0 mm drill at the centre of tablet on the drug layer side to create an appropriate orifice.

**Extended Release Osmotic Dosage Form Drug Layer**

| | **Drug Layer** | **mg/Tablet** | **%w/w** |
|---|---|---|---|
| 1 | Levorphanol Tartrate | 10 | 6.45 |
| 2 | (Polyox WSR N 80) | 136 | 87.74 |
| 3 | HPMC E5 | 7 | 4.51 |
| 4 | Magnesium stearate | 1 | 0.65 |
| 5 | Colloidal silicon dioxide | 0.5 | 0.32 |
| 6 | Talc | 0.5 | 0.32 |

**Extended Release Osmotic Dosage Form Push Layer**

| | **Push Layer** | **mg/Tablet** | **%w/w** |
|---|---|---|---|
| 1 | Polyethylene oxide (Polyox WSR Coagulant) | 63 | 63 |
| 2 | Sodium Chloride | 29 | 29 |
| 3 | HPMC E5 | 5 | 5 |
| 4 | Red Ferric Oxide | 1 | 1 |
| 5 | Magnesium Stearate | 0.8 | 0.8 |
| 6 | Colloidal Silicon Dioxide | 0.7 | 0.7 |
| 7 | Talc | 0.5 | 0.5 |

The tablet dissolution is evaluated in 900 mL of pH 6.8 phosphate buffer using the USP paddle method at 50 to 100 rpm.

### Example 67

A push-pull osmotic pump (PPOP) dosage form of extended release levorphanol is prepared using the method described below. Ingredients 1, 2 and 3 of drug layer are sifted (through BSS 22), dry mixed, and granulated with IPA. The granules, after drying and sifting (through BSS 22) are lubricated with ingredients 4 to 6 (passed through 30 BSS) of drug layer blend. Similarly, ingredients 1, 2, 3 and 4 of push layer are granulated with IPA. The granules, after drying and sifting are lubricated with ingredients 5 to 7. Bilayer tablets are compressed on a single punch compression machine using 8 mm punch. First the push layer is filled into die cavity and pressed lightly followed by addition of drug layer and mass is compressed to obtain tablets having minimum hardness of 6.5 kg/cm². A 4% w/v solution containing cellulose acetate and PEG 4000 in ratio of 90:10 is used for the coating of the bilayer tablets. Cellulose acetate and PEG 4000 is dissolved in acetone, stirred and sonicated to obtain clear transparent solution. Coating is done until the weight gain of 20-22 % w/w which takes around 2.5 hrs. Coated tablets are drilled using 8.0 mm drill at the centre of tablet on the drug layer side to create an appropriate orifice.

**Extended Release Osmotic Dosage Form Drug Layer**

| | **Drug Layer** | **mg/Tablet** | **%w/w** |
|---|---|---|---|
| 1 | Levorphanol Tartrate | 10 | 6.45 |
| 2 | (Polyox WSR N 80) | 136 | 87.74 |
| 3 | HPMC E5 | 7 | 4.51 |
| 4 | Magnesium stearate | 1 | 0.65 |
| 5 | Colloidal silicon dioxide | 0.5 | 0.32 |
| 6 | Talc | 0.5 | 0.32 |

**Extended Release Osmotic Dosage Form Push Layer**

| | **Push Layer** | **mg/Tablet** | **%w/w** |
|---|---|---|---|
| 1 | Polyethylene oxide (Polyox WSR Coagulant) | 63 | 63 |
| 2 | Sodium Chloride | 29 | 29 |
| 3 | HPMC E5 | 5 | 5 |
| 4 | Red Ferric Oxide | 1 | 1 |
| 5 | Magnesium Stearate | 0.8 | 0.8 |
| 6 | Colloidal Silicon Dioxide | 0.7 | 0.7 |
| 7 | Talc | 0.5 | 0.5 |

The tablet dissolution is evaluated in (i) 900 mL of pH 6.8 phosphate buffer and in and (ii) at pH 1.2 for 2 hrs, followed by pH 6.8 phosphate buffer for the remaining time using the USP paddle method at 50 to 100 rpm.

### Example 68

A matrix dosage form of extended release levorphanol is prepared using the method described below. 1. Weigh the levorphanol tartrate, HPMC E15 LV, HPMC K15 M CR, MCC, ascorbic acid, citric acid, sodium citrate, EDTA and colloidal silicon dioxide. 2. Sift ingredients of step 1 using mesh ASTM # 40. 3. Blended step 2 ingredients for 10 minutes. 4. Weigh PVP K30 and alpha-tocopherol acetate and put in IPA and prepare binder solution. 5. Granulate step 3 blend using step 4 binder solution. 6. Dry step 5 granules in oven at 50 °C. 7. Sift dry granules through mesh ASTM # 25. 8. Weigh and sift magnesium stearate through mesh # 60. 9. Lubricate step 7 granules using pre-sifted magnesium stearate for 5 minutes in a polybag. 10. Compress the granules using 7.5 mm round standard concave punches.

| **Components** | **mg/Tablet** |
|---|---|
| Levorphanol Tartrate | 10 |
| HPMC E 15 LV | 13.3 |
| HPMC K 15 M CR | 26.7 |
| MCC (Avicel™ PH 102) | 56.4 |
| Ascorbic Acid | 2.5 |
| Alpha-tocopherol Acetate | 0.5 |
| Citric Acid Anhydrous | 4 |
| Sodium Citrate Trihydrate | 4 |
| PVP K30 | 3 |
| EDTA | 0.1 |
| Aerosil™ | 3 |
| Magnesium stearate | 1.5 |
| Isopropyl Alcohol | qs |

The tablet dissolution is evaluated in (i) 600 mL of pH 6.8 phosphate buffer and in and (ii) in pH 1.2 (0.1 N HCl) for 24 hours using the USP paddle method at 50 to 100 rpm.

### Example 69

**Tablets Composition of Extended Release Levorphanol**

| **Ingredients** | **Qty./Unit** |
|---|---|
| 1. Levorphanol Tartrate | 20 mg |
| 2. HPMC 2208, USP | 150 mg |
| 3. Carnauba wax | 30 mg |
| 4. HPMC 2910, USP | 15 mg |
| 5. Magnesium Stearate | 2 mg |
| 6. Stearic acid | 8 mg |
| 7. Talc | 3 mg |

In Example 69, place the ingredients 1, 2 and 3 in the granulator and mix for 15 minutes. Dissolve ingredient 4 in water (mix in how water, then cool down) and spay into the fluidized mixture. Dry to approximately 5% moisture. Sequentially add ingredient 5, 6 and 7, with mixing steps between each addition. Compress using capsule shaped tooling.

### Example 70

**Tablets Composition of Extended Release Levorphanol**

| Ingredients | Amt/Unit (mg) |
|---|---|
| Levorphanol Tartrate | 30 |
| Spray Dried Lactose | 60 |
| Povidone | 5 |
| Eudragit RS30D (solids) | 10 |
| Triacetin | 2 |
| Stearyl Alcohol | 25 |
| Talc | 2.5 |
| Magnesium Stearate | 1.25 |
| Opadry Pink Y-S-14518A | 4.0 |

In Example 70: 1. Granulation: Spray the Eudragit/Triacetin dispersion onto the Levorphanol, Spray Dried Lactose and Povidone using a fluid bed granulator. 2. Milling: Discharge the granulation and pass through a mill. 3. Waxing: Melt the stearyl alcohol and add to the milled granulation using a mixer. Allow to cool. 4. Milling: Pass the cooled granulation through a mill. 5. Lubrication: Lubricate the granulation with talc and magnesium stearate using a mixer. 6. Compression: Compress the granulation into tablets using a tablet press. 7. Film coating: Apply an aqueous film coat to the tablets.

### Example 71

**Capsule Composition of Extended Release Levorphanol**

| Ingredients | Amt/Unit (mg) |
|---|---|
| Levorphanol Tartrate | 20 |
| Eudragit RSPO | 76 |
| Eudragit RLPO | 4 |
| Stearyl Alcohol | 25 |

In Example 71: 1. Blend milled Stearyl Alcohol, Eudragit RLPO, Levorphanol Tartrate, and Eudragit RSPO using a Hobart Mixer. 2. Extrude the granulation using a Powder Feeder, Melt Extruder (equipped with the 6 x 1 mm die head), Conveyor, Lasermike, and Pelletizer. Powder feed rate-40 g/min; vacuum-about 980 mBar; Conveyor, such that diameter of extrudate is 1 mm, Pelletizer, such that pellets are cut to 1 mm in length. Screen pellets using #16 mesh and #20 mesh screens. Collect material that passes through the #16 mesh screen and is retained on the #20 mesh screen. 4. Fill capsules with the pellets.

### Example 72

**Capsule Composition of Extended Release Levorphanol**

| Ingredients | Amt/Unit (mg) |
|---|---|
| Levorphanol Tartrate | 12 |
| Eudragit RSPO | 77 |
| Ethocel | 4.5 |
| Stearic acid | 27 |

In Example 72: 1. Blend milled Stearic acid, ethocel, Levorphanol Tartrate, and Eudragit RSPO using a V-blender. 2. Extrude the mixture using a Powder Feeder, Melt Extruder (equipped with the 6 x 1 mm die head), Conveyor, Lasermike, and Pelletizer. Powder feed rate, 1.2 kg/hr; vacuum, about 980 mBar; Conveyor, such that diameter of extrudate is 1 mm; Pelletizer, such that pellets are cut to 1 mm in length. 3. Screen pellets using #16 mesh and #20 mesh screens. Collect material that passes through the #16 mesh screen and is retained on the #20 mesh screen. Fill pellets in capsules.

### Example 73

**Capsule Composition of Extended Release Levorphanol**

| | Ingredients | Amt/unit (mg) |
|---|---|---|
| Steps | | |
| 1 | Levorphanol tartrate | 12 |
| | Non-pareil beads (30/35 mesh) | 45 |
| | Opadry Clear | 2.5 |
| 2 | Eudragit RS3-D (dry) | 7.2 |
| | Eudragit RL30D (dry) | 0.4 |
| | Triethyl citrate | 1.5 |
| | Cabosil | 0.4 |
| 3 | Opadry Clear (HPMC) | 1.9 |
| | Cabosil | 0.28 |

In Example 73: 1. Dissolve Levorphanol tartrate and Opadry (HPMC) in water. Spray the drug solution onto nonpareil beads in a fluid bed coater with Wurster insert. 2. Disperse Eudragit RS, Eudragit RL, triethyl citrate, and Cabosil in water. Spray the dispersion onto the beads in the fluid bed coater. 3. Dissolve Opadry in water. Spray the solution onto the beads in the fluid bed coater. 4. Cure the beads at 60.degree. C. for 24 hours.

### Example 74

**Tablet Composition of Extended Release Levorphanol**

| Ingredient | Amt/unit (mg) |
|---|---|
| Levorphanol Tartrate | 15 |
| Anhydrous Dicalcium | 44 |
| Phosphate (Powdered) | 62 |
| Microcrystalline Cellulose | 62 |
| Glyceryl Behenate | 20 |
| Magnesium Stearate | 2 |
| Opadry Red | 10 |
| Purified Water | 57* |

| | |
|---|---|
| *Remains in product as residual moisture only. | |

In Example 74: 1. Pass the Stearyl Alcohol flakes through an oscillating mill. 2. Mix the Levorphanol Tartrate, milled Stearyl Alcohol, Anhydrous Dicalcium Phosphate, Microcrystalline Cellulose, and Glyceryl Behenate in a twin shell blender. 3. Continuously feed the blended material into a twin screw extruder and collect the resultant heated material on a conveyor. 4. Allow the extrudate to cool on the conveyor. 5. Mill the cooled extrudate using an oscillating mill. 6. Blend the milled extrudate and Magnesium Stearate. 7. Compress the resultant granulation using a tablet press, preferably into a caplet. 8. Prepare a film coating solution by dispersing the Opadry in Purified Water and applying it to the tablet.

### Example 75

**Tablet Composition of Extended Release Levorphanol**

| Ingredient | Amt/unit (mg) |
|---|---|
| Levorphanol Base | 10 |
| Spray Dried Lactose | 60 |
| Povidone | 5 |
| Eudragit RS 30D (dry wt.) | 10 |
| Triacetin | 2 |
| Stearyl Alcohol | 25 |
| Talc | 2.5 |
| Magnesium Stearate | 1.25 |
| Opadry Pink | 6 |
| Purified Water | 34* |

| | |
|---|---|
| *Remains in product as residual moisture only. | |

In Example 75: Plasticize the Eudragit with Triacetin by mixing. 2. Place the Levorphanol Base, Spray Dried Lactose, and Povidone into a fluid bed granulator and apply the above solution. 3. Pass the granulation through a rotating impeller mill. 4. Dry granulation if moisture content is too high. 5. Melt Stearyl Alcohol and wax the above granulation by adding melted Stearyl Alcohol onto granulation while mixing. 6. Cool the waxed granulation in a fluid bed dryer. 7. Pass the cooled waxed granulation through a rotating impeller mill. 8. Blend the milled waxed granulation, Talc and Magnesium Stearate. 9. Compress the resultant granulation using a tablet press. 10. Prepare a film coating solution by dispersing the Opadry in Purified Water and applying it to the tablet.

### Example 76

**Capsule Composition of Extended Release Levorphanol**

| Ingredient | Amt/unit (mg) |
|---|---|
| Levorphanol Tartrate | 8 |
| Eudragit RSPO | 76.5 |
| Ethylcellulose | 4.5 |
| Stearyl Alcohol | 27 |

In Example 76: 1. Pass Stearyl Alcohol flakes through an impact mill. 2. Mix the Levorphanol Tartrate, Eudragit, Ethylcellulose and milled Stearyl Alcohol in a twin shell blender. 3. Continuously feed the blended material into a twin screw extruder and collect the resultant strands on a conveyor. 4. Allow the strands to cool on the conveyor. 5. Cut the cooled strands into pellets using a Pelletizer. 6. Screen the pellets and collect desired sieve portion. 7. Fill the extruded pellets into capsules.

Example 77 to 88 may be prepared as follows: (i) Dispense the specified hydrophobic controlled release material (e.g., hydrogenated Type I vegetable oil, hydrogenated Type II vegetable oil, polyoxyethylene stearates, polyoxyethylene distearates, glycerol monostearate, poorly water soluble, or high melting point waxes) into a mixer; (ii) Heat until fully melted; (iii) dispense the hydroxypropyl methyl cellulose (HPMC) into the mixer; (iv) Mix until dispersed; (v) Dispense the Aerosil into the same vessel; (vi) Mix until dispersed; (vii) Dispense levorphanol into the same vessel; (viii) Stir thoroughly with a high shear mixer; (ix) Transfer the mix into a liquid filling machine; (x) Fill into hard gelatin (or HPMC) capsule; (xi) Optionally, transfer the capsules to a banding machine and band the capsules. (xii) Optionally, dispense (i), (iii) and (v) into a mixer, then continue heat the mixture of (i), (iii) and (v) simultaneously until fully melted, then continue from (vii). Optionally, dispense (i), (iii), (v) and (vii) into a mixer, then continue heat the mixture until (i), (iii) and (v) have until fully melted, then continue from (viii).

### Example 77

**Capsule Composition of Extended Release Levorphanol**

| **Ingredients** | **Quantity (mg)/Dose** |
|---|---|
| Sterotex® NF | 200 |
| Fractionated coconut oil | 70 |
| Methocel® K 15M | 81 |
| Aerosil® COK 84 | 4 |
| Levorphanol Tartrate | 5 |

### Example 78

**Capsule Composition of Extended Release Levorphanol**

| **Ingredients** | **Quantity (mg)/Dose** |
|---|---|
| Beeswax | 200 |
| HPMC, K15M | 80 |
| Aerosil COK 84 | 8 |
| Levorphanol Tartrate | 5 |

### Example 79

**Capsule Composition of Extended Release Levorphanol**

| **Ingredients** | **Quantity (mg)/Dose** |
|---|---|
| Sterotex NF | 150 |
| HPMC, K15M | 75 |
| Coconut oil | 75 |
| Aerosil COK 84 | 5 |
| Levorphanol Tartrate | 10 |

### Example 80

**Capsule Composition of Extended Release Levorphanol**

| **Ingredients** | **Quantity (mg)/Dose** |
|---|---|
| Cithrol GMS | 275 |
| HPMC, K100M | 40 |
| Aerosil COK 84 | 10 |
| Levorphanol Tartrate | 5 |

### Example 81

**Capsule Composition of Extended Release Levorphanol**

| **Ingredients** | **Quantity (mg)/Dose** |
|---|---|
| Hydrokote 112 | 250 |
| HPMC, K15M | 60 |
| Aerosil COK 84 | 10 |
| Levorphanol Tartrate | 10 |

### Example 82

**Capsule Composition of Extended Release Levorphanol**

| **Ingredients** | **Quantity (mg)/Dose** |
|---|---|
| Beeswax | 200 |
| HPMC, Pharmacoat 606 | 62.5 |
| Aerosil COK 84 | 7.5 |
| Levorphanol Tartrate | 5 |

### Example 83

**Capsule Composition of Extended Release Levorphanol**

| **Ingredients** | **Quantity (mg)/Dose** |
|---|---|
| Gelucire 50/02 | 190 |
| Methocel K 100M | 35 |
| Aerosil COK 84 | 10 |
| Levorphanol Tartrate | 5 |

### Example 84

**Capsule Composition of Extended Release Levorphanol**

| **Ingredients** | **Quantity (mg)/Dose** |
|---|---|
| Cetyl alcohol | 280 |
| Methocel K 100M | 50 |
| Aerosil COK 84 | 10 |
| Levorphanol Tartrate | 10 |

### Example 85

**Capsule Composition of Extended Release Levorphanol**

| **Ingredients** | **Quantity (mg)/Dose** |
|---|---|
| Sterotex NF | 320 |
| Methocel K 15M | 60 |
| Aerosil COK 84 | 10 |
| Levorphanol Tartrate | 10 |

### Example 86

**Capsule Composition of Extended Release Levorphanol**

| **Ingredients** | **Quantity (mg)/Dose** |
|---|---|
| Cithrol GMS | 320 |
| Methocel K 100M | 55 |
| Aerosil COK 84 | 15 |
| Levorphanol Tartrate | 10 |

### Example 87

**Capsule Composition of Extended Release Levorphanol**

| **Ingredients** | **Quantity (mg)/Dose** |
|---|---|
| Sterotex® NF | 100 |
| Fractionated coconut oil | 70 |
| Beeswax | 100 |
| Methocel® K 15M | 81 |
| Aerosil® COK 84 | 4 |
| Levorphanol Tartrate | 10 |

### Example 88

**Capsule Composition of Extended Release Levorphanol**

| **Ingredients** | **Quantity (mg)/Dose** |
|---|---|
| Glyceryl behenate | 135 |
| Fractionated coconut oil | 50 |
| Methocel® K 15M | 60 |
| Aerosil® COK 84 | 3 |
| Levorphanol Tartrate | 5 |

### Example 89

**Tablet Composition of Extended Release Levorphanol**

| **Ingredients** | **Quantity (mg)/Dose** |
|---|---|
| Lactose (spray dried) | 230 |
| Eudragit™ RS PM | 60 |
| Purified water | q.s.* |
| Stearyl Alcohol | 90 |
| Talc | 8 |
| Magnesium Stearate | 4 |
| Levorphanol Tartrate | 5 |

| | |
|---|---|
| *Remains in product as residual moisture only. | |

In Example 89: The required quantities of levorphanol tartrate, spray-dried lactose, and Eudragit ™ RS PM are transferred into an appropriate-size mixer, and mixed for approximately 5 minutes. While the powders are mixing, the mixture is granulated with enough water to produce a moist granular mass. The granules are then dried in a fluid bed dryer at 60 °C, and then passed through an 8-mesh screen. Thereafter, the granules are re-dried and pushed through a 12-mesh screen. The required quantity of stearyl alcohol is melted at approximately 60 to 70 °C, and while the granules are mixing, the melted stearyl alcohol is added. The warm granules are returned to the mixer. The coated granules are removed from the mixer and allowed to cool. The granules are then passed through a 12-mesh screen. Then granulate is then lubricated by mixing the required quantity of talc and magnesium stearate in a suitable blender. Tablets are compressed on a suitable tableting machine.

### PHARMACOLOGIC EVALUATION

The in vitro pharmacologic profile of levorphanol was evaluated using standard methods. The results were analyzed and expressed as shown below.

### Binding Assays

The specific ligand binding to the receptors was defined as the difference between the total binding and the nonspecific binding determined in the presence of an excess of unlabelled ligand. The results were expressed as a percent of control specific binding ((measured specific binding/control specific binding) x 100) and as a percent inhibition of control specific binding (100-((measured specific binding/control specific binding) x 100)) obtained in the presence of the test compounds. The IC₅₀ values (concentration causing a half-maximal inhibition of control specific binding) and Hill coefficients (nH) were determined by non-linear regression analysis of the competition curves generated with mean replicate values using Hill equation curve fitting (Y = D + [(A - D)/(1 + (C/C₅₀)^{nH})], where Y = specific binding, D = minimum specific binding, A = maximum specific binding, C = compound concentration, C₅₀ = IC₅₀, and nH = slope factor). This analysis was performed using validated proprietary software. The inhibition constants (Kᵢ) were calculated using the Cheng Prusoff equation Kᵢ = IC₅₀/(1+(L/K_{D})), where L = concentration of radioligand in the assay, and K_{D} = affinity of the radioligand for the receptor). A scatchard plot was used to determine the Kd.

### In Vitro Pharmacology: Cellular Functional Assays

The results were expressed as a percent of control specific agonist response ((measured specific response/control specific agonist response) x 100) and as a percent inhibition of control specific agonist response (100 - ((measured specific response/control specific agonist response) x 100)) obtained in the presence of the test compounds. The EC₅₀ value (conc. producing a half-maximal specific response) and IC₅₀ value (conc. causing a half-maximal inhibition of the control specific agonist response) were determined by non-linear regression analysis of the conc. response curves generated with mean replicate values using Hill equation curve fitting (Y = D + [(A - D)/ (1 + (C/C₅₀)^{nH})], where Y = specific response, D = minimum specific response, A = maximum specific response, C = compound concentration, and C₅₀ = EC₅₀ or IC₅₀, and nH = slope factor). This analysis was performed using validated proprietary software. For the antagonist, the apparent dissociation constant (K_{B}) was calculated using the modified Cheng Prusoff equation (K_{B} = IC₅₀/(1+(A/EC₅₀A)), where A = concentration of reference agonist in the assay, and EC₅₀A = EC₅₀ value of the reference agonist).

### Enzyme Assays

The results were expressed as a percent of control specific activity ((measured specific activity/control specific activity) x 100) and as a percent inhibition of control specific activity (100 - ((measured specific activity/control specific activity) x 100)) obtained in the presence of the test compounds. The IC₅₀ values (concentration causing a half-maximal inhibition of control specific activity) and Hill coefficients (nH) were determined by non-linear regression analysis of the inhibition curves generated with mean replicate values using Hill equation curve fitting (Y = D + [(A - D)/(1 + (C/C₅₀)^{nH})], where Y = specific activity, D = minimum specific activity, A = maximum specific activity, C = compound concentration, C₅₀ = IC₅₀ and nH = slope factor). This analysis was performed using validated proprietary software.

### Example 90

Example 90 provides a brief summary of the experimental conditions for assessment of NMDA antagonism. Testing was done using an adaptation of the method described by Vignon et al, Brain Research, 1986;378:133-41. Levorphanol Tartrate provided 91% inhibition of control specific binding at concentration of 1 x 10⁻⁶ M levorphanol tartrate, equal to 5.8 x 10⁻⁶ M levorphanol base.

| **Assay** | **Origin** | **Ligand** | **Conc.** |
|---|---|---|---|
| NMDA (antagonist radio ligand) | rat cerebral cortex | [³H]TCP | 10 nM |

| **Kd** | **Non Specific** | **Incubation** | **Detection Method** |
|---|---|---|---|
| 13 nM | MK 801 | 120 min | Scintillation counting |
| | (10 µM) | 37°C | |

### Example 91

Example 91 provides a brief summary of the experimental conditions for assessment of 5-HT uptake assay. Testing was done using an adaptation of the method described by Perovic and Muller, Arzneim-Forsch Drug Res 1995;45:1145-48. Levorphanol tartrate provided an IC₅₀ of 5.2 x 10⁻⁸ M levorphanol tartrate, equal to 3.02 x 10⁻⁸ M levorphanol base. See Figure 83.

| **Origin** | **Assay** | **Substrate/Tracer** |
|---|---|---|
| rat brain synaptosomes | 5-HT uptake | [³H]5-HT |
| | | (0.2 µCi/ml) |

| **Reaction Product** | **Incubation** | **Method of Detection** |
|---|---|---|
| [³H]5-HT incorporation into synaptosomes | 15 min 37°C | Scintillation counting |

### Example 92

Example 92 provides a brief summary of the experimental conditions for assessment of norepinephrine uptake assay. Testing was done using an adaptation of the method described by Perovic and Muller, Arzneim-Forsch Drug Res 1995;45:1145-48. Levorphanol tartrate provided an IC₅₀ of 2.1 x 10⁻⁶ M levorphanol tartrate, equal to 1.22 x 10⁻⁶ M levorphanol base. See Figure 84.

| **Assay** | **Origin** | **Substrate/Tracer** |
|---|---|---|
| rat hypothalamus synaptosomes | norepinephrine uptake | [³H]NE |
| | | (0.2 µCi/ml) |

| **Incubation** | **Reaction Product** | **Method of Detection** |
|---|---|---|
| [³H]NE incorporation into synaptosomes | 20 min | Scintillation counting |
| | 37°C | |

The in vivo pharmacologic profile of levorphanol was evaluated using standard methods. The results were analyzed and expressed as shown below.

### Example 93

Objectives: To evaluate the efficacy and potency of levorphanol in a model of inflammatory pain and to correlate its potency relative to that of morphine.

Methods: Male Sprague-Dawley rats (200-300 grams) were used. Peripheral inflammation was induced by subdermal injection of 0.05 ml of a 0.6% carrageenan suspension into the plantar aspect of lightly ether-anesthetized rats. This procedure reliably produces a marked inflammatory response within 3 hours of carrageenan injection which is indicated by swelling of the hindpaw, edema, rubor and hyperalgesia and tactile allodynia. Drugs were administered 150 minutes after carrageenan-induced inflammation. Behavioral tests were performed 2, 3 and 4 hours after induction of inflammation. Tactile hyperesthesia was measured by determining the withdrawal threshold of the hindpaw to probing the plantar aspect with a series of von Frey filaments. A significant (p < 0.05) reduction in paw withdrawal threshold indicated tactile hyperesthesia. Responses to thermal nociception were determined by placing rats within plexiglass enclosures on a clear glass plate. After an acclimatization period, an infrared source was focused onto the plantar surface of the hindpaw. Paw withdrawal was detected by a motion sensor that halted the IR source and the timer, indicating paw withdrawal latency. Thermal hyperalgesia was indicated by a significant (p < 0.05) reduction in paw withdrawal latency and antinociception was indicated by a significant increase in latency. The levorphanol data for mechanical allodynia and thermal hyperalgesia were compared to historical morphine data. In order to analyze dose-effect relationships, the data were converted to % Maximal Possible Effect (%MPE) by the formula: % MPE = 100 x (test value - basal value)/(cut-off - basal value). Dose-response curves along with A50 values and confidence intervals were determined from linear regression analysis of the log dose-effect curves. Significant shifts in the dose-effect curves were indicated by Student's t-statistic applied to the A50 values obtained for levorphanol and morphine.

Conclusions: Carrageenan injected into the hindpaw produced behavioral sensitivity to tactile and thermal stimuli and increased paw volume within 3 hours, indicating that inflammation was successfully induced. Levorphanol was more potent than morphine at all times for reversal of tactile hyperalgesia occurring in the inflamed hind paw and for paw withdrawal after noxious radiant heat. Surprisingly levorphanol base (which is approximately 58% of the active amount of tested material, levorphanol tartrate dihydrate) was only approximately 2 to 3 times more potent than morphine base for reversal of tactile hyperalgesia and 4 to 5 times more potent than morphine base for paw withdrawal after noxious radiant heat. When compared in the way the dose is expressed in pharmaceutical compositions (levorphanol is dosed by as mg of levorphanol tartrate dihydrate, while morphine is dose morphine as mg of morphine base), levorphanol was surprisingly only approximately 1.2 to 1.7 times more potent than morphine for reversal of tactile hyperalgesia and 2.3 to 2.9 times more potent than morphine base for paw withdrawal after noxious radiant heat.

### Example 94

Objective: To evaluate the efficacy and potency of levorphanol and to compare its potency relative to that of morphine in a model of thermal nociception (hot-plate test).

Methods: Male Sprague-Dawley rats (200-300 grams) were used. Antinociceptive activity was determined by the 53°C hot-plate test. The rat is placed on a metal plate heated to 53°C, and the latency to withdrawing or shaking or licking of a paw, or escape attempts by jumping were documented. A cut-off latency of 15 sec was employed. Drugs were prepared administered after baseline measurements were performed. Behavioral testing was performed 30, 45 and 60 minutes after dosing. The levorphanol data for were compared to historical morphine data. In order to analyze dose-effect relationships, the data were converted to % Maximal Possible Effect (%MPE) by the formula: % MPE = 100 x (test latency - basal latency)/(15 sec - basal latency). Dose-response curves along with A50 values (i.e., dose calculated to produce a 50% MPE) and confidence intervals were determined from linear regression analysis of the log dose-effect curves. Significant differences in potency between 2 dose-response curves were determined by applying the Student's t-statistic to variances of the A50 values obtained for each dose-response curve. The antinociceptive effect of levorphanol was determined in the 53°C rat hot-plate test 30, 45 and 60 minutes after dosing. Hot-plate latencies for each time-point were compared to the mean baseline response by ANOVA followed by the post-hoc Fisher's Least Significant Difference test.

Conclusions: Levorphanol produced dose-dependent antinociception in rats in the classic 53°C rat hot-plate test of antinociception. Levorphanol was more potent than morphine at all times. Surprisingly, levorphanol base (which is approximately 58% of the active amount of tested material, levorphanol tartrate dihydrate) was only approximately 1.9 to 4.7 times more potent than morphine base in the hot-plate test of antinociception. When compared in the way the dose is expressed in pharmaceutical compositions (levorphanol is dosed by as mg of levorphanol tartrate dihydrate, while morphine is dose morphine as mg of morphine base), levorphanol was surprisingly only approximately 1.1 to 2.7 times more potent than morphine as an analgesic in this pain model. Perhaps more surprising, the duration of analgesic action of levorphanol was shorter than that of morphine, which supports the development of extended release dosage forms of levorphanol.

### Example 95

Objective: To evaluate the efficacy and potency of levorphanol and to compare its potency relative to that of morphine in a model of thermal nociception (tail flick test).

Methods: Male Sprague-Dawley rats (200-300 grams) were used. Antinociceptive activity was determined by a significant increase in the latency to a reflexive flicking of the tail away from a radiant heat (infrared) source projected onto a small area on the ventral aspect of the tail. Baseline tail flick latencies were determined prior to any drug administration. The antinociceptive effect of levorphanol was determined 30, 45 and 60 minutes after dosing. The levorphanol data for were compared to historical morphine data. Tail flick latencies for each time-point were compared to the mean baseline response by ANOVA followed by the post-hoc Fisher's Least Significant Difference test. A significant increase from the baseline was considered antinociceptive. In order to analyze dose-effect relationships, the data were converted to % Maximal Possible Effect (%MPE) by the formula: % MPE = 100 x (test latency - basal latency)/(15 sec - basal latency). Dose-response curves along with A50 values (i.e., dose calculated to produce a 50% MPE) and confidence intervals were determined from linear regression analysis of the log dose-effect curves. Significant differences in potency between 2 dose-response curves were determined by applying the Student's t-statistic to variances of the A50 values obtained for each dose-response curve.

Conclusions: Levorphanol produced dose-dependent antinociception in rats in the classic tail flick model of antinociception. Levorphanol was more potent than morphine at all times. Surprisingly, levorphanol base (which is approximately 58% of the active amount of tested material, levorphanol tartrate dihydrate) was only approximately 3.6 to 5.8 times more potent than morphine base in the tail flick test of antinociception. When compared in the way the dose is expressed in pharmaceutical compositions (levorphanol is dosed by as mg of levorphanol tartrate dihydrate, while morphine is dose morphine as mg of morphine base), levorphanol was surprisingly only approximately 2.1 to 3.4 times more potent than morphine as an analgesic in this pain model. Perhaps more surprising, the duration of analgesic action of levorphanol was shorter than that of morphine, which supports the development of extended release dosage forms of levorphanol.

### HUMAN STUDIES

### Example 96

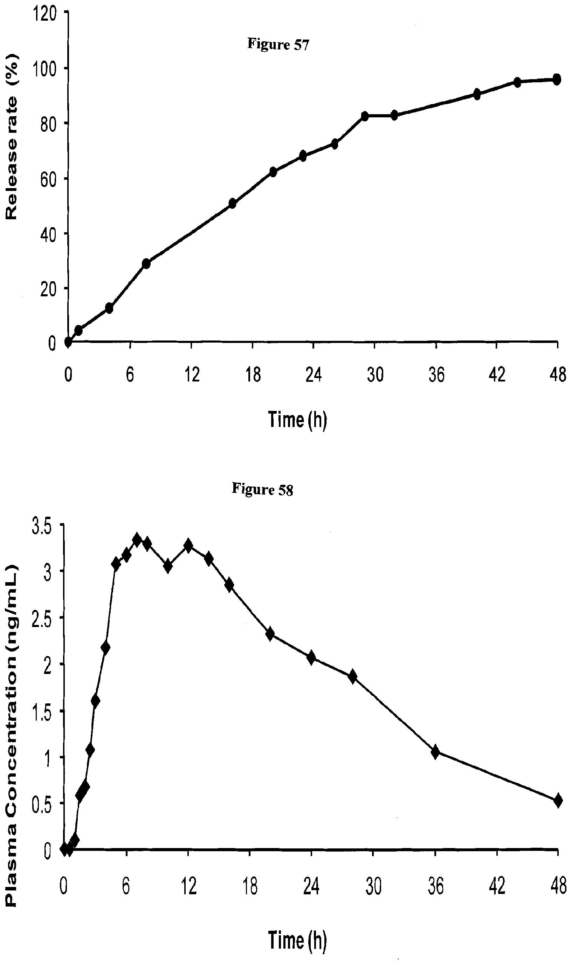

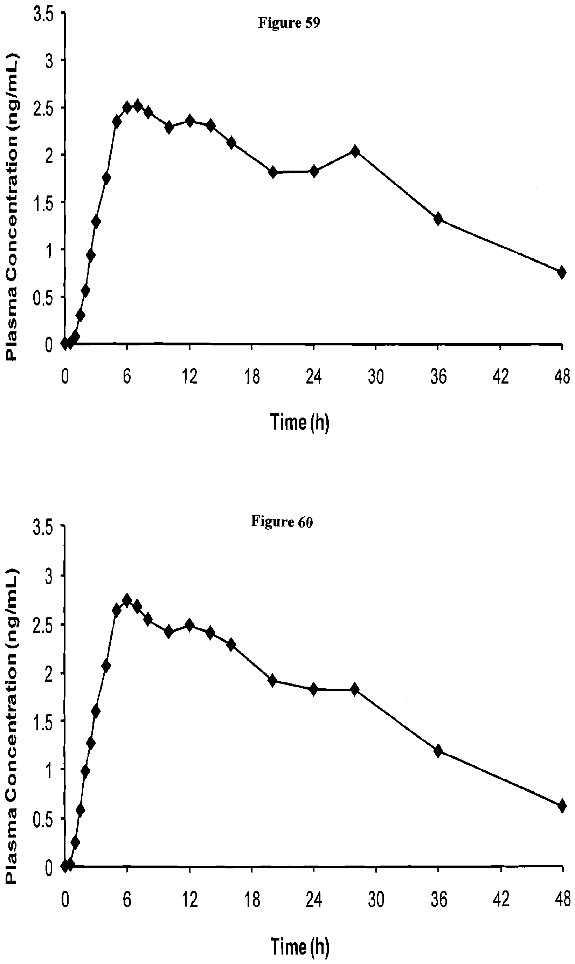

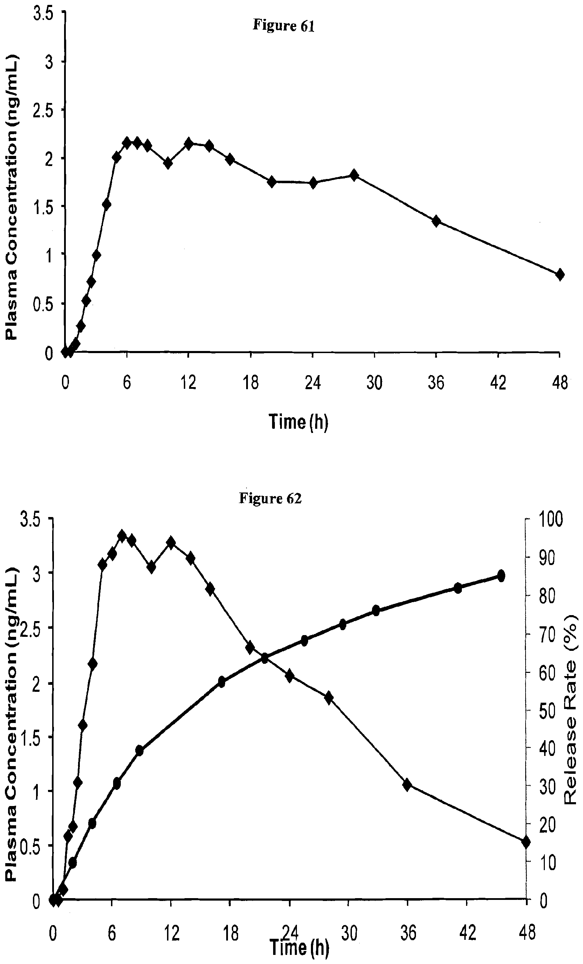

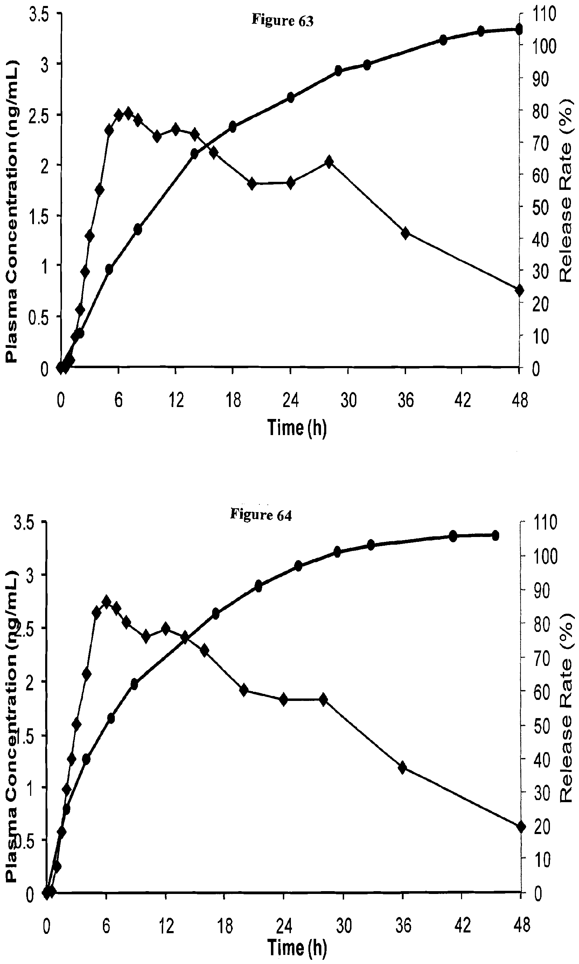

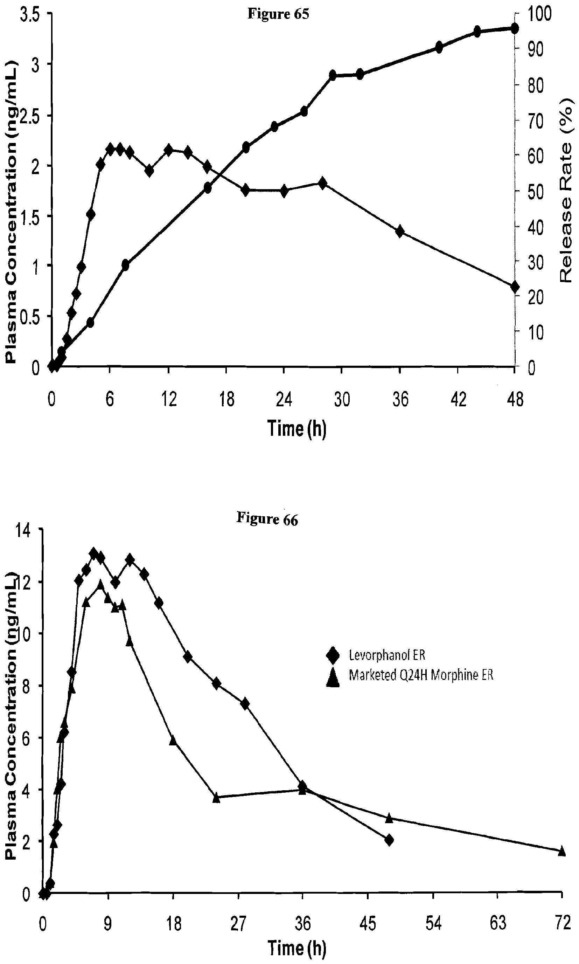

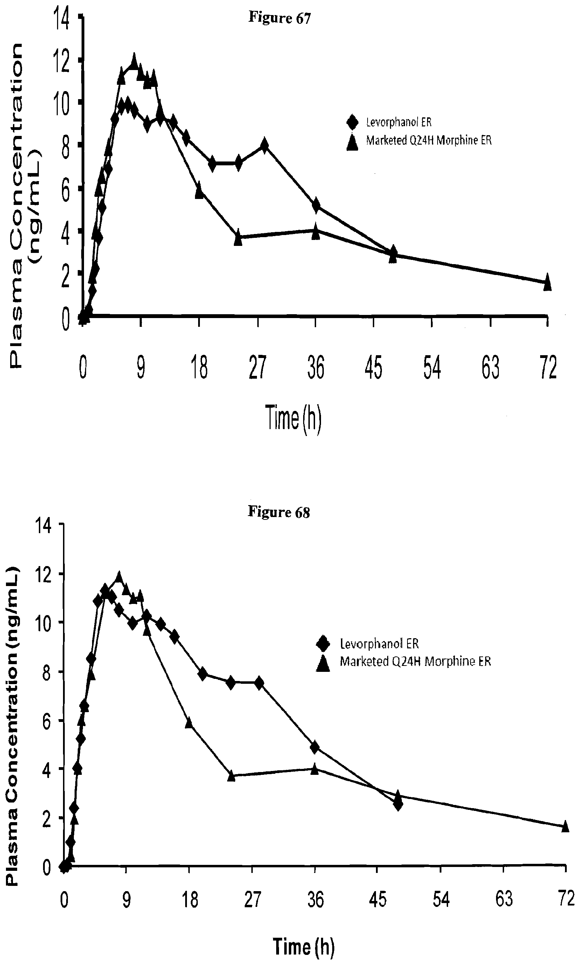

This study was a 5-way, analytically masked, single-dose crossover bioavailability study of four formulations of extended release (ER) levorphanol tartrate relative to immediate release (IR) levorphanol tartrate tablets in 15 subjects. Eligible subjects reported to the clinical unit in the evening, at least 13 hours prior to dosing in each treatment period. All subjects fasted for ≥ 12 hours prior to dosing. Water was restricted for 2 hours pre-levorphanol dosing to 2 hours post-levorphanol dosing. A single dose of levorphanol was administered. Subjects took their study medication in each treatment period with 240 mL of water and received a standardized meal four hours after dosing. All subjects remained in the unit until the completion of the last scheduled study procedure in each treatment period (approximately 48 hours post-dose). There was a minimum of 7 and up to 14 day washout period between each of the 5 dosing periods. Since the doses of levorphanol ER in the treatment groups were different, as was the dose of levorphanol IR, the plasma concentration data were normalized to the same dose to allow comparisons between treatments. Physical examination, vital signs, 12-lead ECG, clinical laboratory tests were performed at the screening visit and at the beginning and again upon exit from the study. Vital signs including respiratory rate, blood pressure and pulse will be obtained at screening, at pre-dose, 4-hours post-dose (prior to ambulation) and prior to the last blood collection in each study period. Vital signs at Screening, at pre-dose and at 4-hours post dose were taken in a sitting and standing position. Other scheduled vital signs were obtained after the subject had been in a sitting position for five minutes. Spontaneous reporting of adverse events was monitored during the course of the study. In the Levorphanol ER treatment periods, blood samples were collected for levorphanol at 0.0 (pre-dose) and at 0.5, 1.0, 1.5, 2.0, 2.5, 3.0, 4.0, 5.0, 6.0, 7.0, 8.0, 10.0, 12.0, 14.0, 16.0, 20.0, 24.0, 28.0, 36.0, and 48.0 hours post-dose (21 samples). In the Levorphanol IR treatment, blood samples were collected for levorphanol at 0.0 (pre-dose) and at 0.25, 0.5, 1.0, 1.5, 2.0, 2.5, 3.0, 4.0, 5.0, 6.0, 7.0, 8.0, 10.0, 12.0, 14.0, 16.0, 20.0, 24.0, 36.0, and 48.0 hours post-dose (21 samples). Harvested plasma samples was stored at -70°C and subsequently processed for bioanalysis for levorphanol. The assay for levorphanol in plasma was done with a sensitive, specific, robust and validated analytical method. Data from 15 subjects were included in the pharmacokinetic and statistical analyses. Full precision concentration data (not rounded to three significant figures) and actual sample times were used for all pharmacokinetic and statistical analyses. Data from 15 subjects were included in the pharmacokinetic and statistical analyses. Concentration-time data were transferred from Watson LIMS directly to WinNonlin™ Enterprise Edition (Version 4.0, Pharsight Corporation) using the Custom Query Builder option for analysis. Data were analyzed by noncompartmental methods in WinNonlin. Data were also analyzed using compartmental models. The results of this study confirm that various tested prototypes of levorphanol ER provide an extended release profile, when compared with Levorphanol IR, as evidenced by the time of peak concentration, mean residence times, mean absorption time, W₅₀ and HVD. Figure 58, Figure 59, Figure 60 and Figure 61 show the mean levorphanol plasma concentration time profile obtained with the prototypes of Levorphanol ER in this example. Figure 62, Figure 63, Figure 64 and Figure 65 show the surprising relationship between the release rate of levorphanol and the plasma concentration time profile. Levorphanol ER provides a prolonged in vitro dissolution rate that is substantially longer than the dosing interval in vivo. Levorphanol ER continues to robustly release drug in vitro well past the dosing interval, even when plasma concentrations are declining. Figure 66, Figure 67, Figure 68 and Figure 69 show a superior extended release human pharmacokinetic profile for various dosages of Levorphanol ER described in this example, when compared with a brand name multiparticulate extended release once-daily morphine (Morphine ER), making the Levorphanol ER dosage form suitable for up to once a day dosing (e.g., twice-a-day [Q12H or Q12H PRN] or once-a-day [QD, Q24H or Q24H PRN]. To allow meaningful comparison (and preserve the overall shape of the plasma-concentration time profile), the plasma concentration of each Levorphanol ER dosage form has been put on the same scale as the Morphine ER ("normalized") by applying a fixed ratio of mean AUC_{0-inf} of Morphine ER to Levorphanol ER for each plasma concentration.

The results of the study are summarized in part below.

| **Parameter** | **Levo ER** | **Levo ER** | **Levo ER** | **Levo ER** | **Levo IR** |
|---|---|---|---|---|---|
| | **(1)** | **(2)** | **(3)** | **(4)** | |
| Tₘₐₓ (hr) | 10.36 | 12.29 | 9.15 | 11.53 | 2.40 |
| λ_{z} (hr⁻¹) | 0.0658 | 0.0575 | 0.0557 | 0.0541 | 0.0649 |
| T_{1/2}(hr) | 10.93 | 14.11 | 13.65 | 14.35 | 10.85 |
| K₀₁ | 0.264 | 0.5458 | 0.4621 | 0.4977 | 1.3543 |
| K₁₀ | 0.0662 | 0.0292 | 0.0379 | 0.0269 | 0.1304 |
| T_{lag} | 1.35 | 1.56 | 1.08 | 1.46 | 0.51 |
| MRTₗₐₛₜ (hr) | 19.06 | 21.39 | 20.30 | 22.27 | 12.44 |
| MRTINF_obs (hr) | 22.93 | 28.96 | 27.19 | 29.50 | 14.76 |
| MAT (hr) | 5.14 | 3.39 | 3.24 | 3.47 | 1.25 |
| W₅₀ (hr) (6 hour dosing) | - | - | - | - | 4.5 |
| W₅₀ (hr) (8 hour dosing) | - | - | - | - | 6.0 |
| W₅₀ (hr) (12 hour dosing) | 7.8 | 8.6 | 8.8 | 8.4 | 6.6 |
| W₅₀ (hr) (24 hour dosing) | 16.5 | 19.9 | 20.00 | 20.4 | 6.6 |
| HVD (hr) | 20.0 | 29.6 | 24.3 | 32.1 | 6.6 |

### Example 97

The pharmacokinetics of a prototype dosage form of extended release levorphanol (Levorphanol ER) was compared with immediate release levorphanol (Levorphanol IR) in 15 fasted medically supervised subjects. Eligible subjects reported to the clinical unit the evening prior to dosing in each treatment period and all subjects fasted for ≥ 12 hours prior to dosing. Water was restricted for 2 hours pre-levorphanol dosing to 2 hours post-levorphanol dosing. Levorphanol ER and levorphanol IR were given as a single administration using a randomized crossover design with an intervening washout period between the treatments. Subjects took the total study dose with 240 mL of water and receive a standardized meal four hours after dosing. All subjects remained in the unit until the completion of the last scheduled study procedure (approximately 48 hours post-dose). Physical examination, vital signs, 12-lead ECG, clinical laboratory tests were performed at the screening visit and at the beginning and again upon exit. A screen for drugs of abuse was performed at screening and prior to dosing. An ethanol breath test was performed prior to dosing. Vital signs including respiratory rate, blood pressure and pulse were obtained at screening, at pre-dose, 4-hours post-dose and prior to the last blood collection. Adverse events were monitored during the course of the study. Blood samples were collected for levorphanol at 0.0 (pre-dose) and at 0.5, 1.0, 1.5, 2.0, 2.5, 3.0, 4.0, 5.0, 6.0, 7.0, 8.0, 10.0, 12.0, 14.0, 16.0, 20.0, 24.0, 28.0, 36.0, and 48.0 hours post-dose (21 samples). Harvested plasma samples were stored at -70°C and subsequently processed for bioanalysis for levorphanol. The assay for levorphanol in plasma was done with a sensitive, specific, robust and validated analytical method. A total of 14 subjects completed the Levorphanol ER treatment (one subject was excluded due to non-compliance with study procedures) and all 15 subjects completed the Levorphanol IR treatment. All completed subjects were included in the pharmacokinetic and statistical analyses. Bioavailability parameters based on actual dose (assayed dose) are presented below. The time of peak plasma concentration, terminal elimination apparent half life, HVD and W₅₀ are reported as median values. To allow meaningful comparison, Cₘₐₓ and AUC data are reported per mg of levorphanol dose. The results of the study (summarized in the Table below) show that levorphanol ER provided robust extended release properties, full bioavailability compared to Levorphanol IR. The dosage form is suitable for Q12H or Q24H dosing.

| **Parameter** | **Levorphanol ER** | **Levorphanol IR** |
|---|---|---|
| Cₘₐₓ (pg/mL/mg) | 407 | 838 |
| AUC_{inf} (hr*pg/mL/mg) | 10440 | 10710 |
| Tₘₐₓ (hr) | 10.00 | 2.00 |
| T_{1/2} (hr) | 10.63 | 10.68 |
| W₅₀ (hr) (6 hour dosing) | - | 4.5 |
| W₅₀ (hr) (8 hour dosing) | - | 6.0 |
| W₅₀ (hr) (12 hour dosing) | 8.0 | 6.0 |
| W₅₀ (hr) (24 hour dosing) | 19.0 | 6.0 |
| HVD (hr) | 23.0 | 6.0 |
| Relative Bioavailability | ER (Test) | IR (Reference) |
| Cₘₐₓ Ratio (ER/IR) | 50.15 | - |
| AUC_{inf} Ratio (ER/IR) | 99.27 | - |

A wide variety of materials can be used for preparing the dosage form according to this invention. This invention therefore contemplates the use of materials other than those specifically disclosed herein, including those which may hereafter become known to the art to be capable of performing the necessary functions. In all instances wherein prophetic examples are provided, these compositions are intended to be exemplary and it should be understood that the specific procedures, constituents, amounts thereof and the like can be varied in order to obtain a composition possessing desired properties. The percent loading of the levorphanol onto the dosage form will depend on the physiochemical and pharmaceutical properties of levorphanol API (base or salt, type of salt, particle size), choice and amount of excipient, desired route (e.g., orally ingested, sublingual), desired tablet or capsule size, and the desired release profile. Levorphanol, its pharmaceutically salts or mixture thereof, and any incorporated excipients in the above examples may require milling to assure that they meet the particle size requirements of some embodiments of the invention. The choice of particle size will vary depending on the desired properties of the dosage form. The dosage form may comprise any pharmaceutically acceptable excipients, in any desired amounts and with any desired physicochemical or mechanical properties (e.g., particle size, melting point, viscosity). Preferably, the pharmaceutical excipient is about 0.0001% to about 99.5%, more preferably, about 0.001 % to about 95%, and even more preferably, about 0.01% to about 90%.

Levorphanol and the any excipients comprising the dosage form may be incorporated in any desired order to prepare the dosage form. Levorphanol, and any incorporated excipients may be processed (e.g., cured, milled, co-mingled, heated, made into a liquid, adjusted for moisture content, purified) prior to incorporation into the dosage form and the dosage form may also be further processed after manufacture (e.g., cured at ambient temperature, cured at a specified temperature, film coated, enteric coated) prior to packaging and sale. The ingredients used for the preparation of the levorphanol dosage form agent may be modified. In some embodiments, a change in the dose or amount levorphanol will not require a significant change in amount of other ingredients. In other embodiments, a proportional change in the amount of other ingredients is required to maintain the desired properties. In yet other embodiments, a change in the dose or amount levorphanol necessitates a change in the nature and/or amount of ingredients to provide the required characteristics of the levorphanol (e.g., onset of effect, duration of effect, rate and extent of absorption, therapeutic concentrations and effect, etc.).

These and other embodiments of the present invention will readily occur to those of ordinary skill in the art in view of the disclosure herein. The included examples are illustrative but not limiting of the methods and composition of the present invention. Other suitable modifications and adaptations of the variety of conditions and parameters normally encountered and obvious to those skilled in the art are within the spirit and scope of the invention. Additionally, it is understood that each of the various embodiments of the pharmaceutical compositions described herein may be used with each of the various embodiments of the described method of the present invention as described herein. Furthermore, it is understood that each of the embodiments disclosed herein may alternatively be described as "consisting of' or "consisting essentially of' the listed components.

## Claims

1. A dosage form for orally administering levorphanol to a human patient, the dosage form comprising
an amount of levorphanol or a pharmaceutically acceptable salt thereof equivalent to 4 to 80 milligrams of levorphanol tartrate, functionally combined with
a controlled release material selected from the group consisting of waxes, vegetable oils, esters of vegetable oils, and hydrogenated vegetable oils
when the controlled release material is selected from the group consisting of waxes, vegetable oils and esters of vegetable oils, the controlled release material has been treated by application of heat at least up to its melting point and admixed with a thixotrope and a release rate modifier; and,
when the controlled release material is hydrogenated vegetable oils, the controlled release material has been treated by application of heat at least up to its melting point and admixed with a thixotrope, a release rate modifier and medium chain triglycerides
formulated as an orally-administrable dosage form, said dosage form resistant or substantially resistant to dissolution and/or absorption in the stomach, and/or the duodenum, and/or the jejunum, and/or the ileum.

2. The dosage form of claim 1, formulated such that substantially no release of levorphanol therefrom occurs less than about 2 hours, optionally 4 hours, after oral administration of the dosage form to the patient.

3. The dosage form of any preceding claim, formulated such that, following oral administration of the dosage form to the patient, substantially no release of levorphanol therefrom occurs proximal to the stomach or in any portion of the gastrointestinal tract distal to the stomach that has a pH less than 5.

4. The dosage form of any preceding claim, further comprising at least one of
i) a release rate modifier selected from the group consisting of hydroxylpropyl celluloses, cellulose acetates, powdered celluloses, cellulose acetate phthalates, hydroxyethylmethyl celluloses, carboxymethylcelluloses,hypromellose acetates, hypromellose phthalates, ethylcelluloses, and mixtures of these; and
ii) a thixotrope selected from the group consisting of metal oxides, microcrystalline methylcelluloses, clays, silicates, carbon black, and combinations of these.

5. The dosage form of any preceding claim, said dosage form resistant or substantially resistant to dissolution and/or absorption in the stomach.

6. The dosage form of any preceding claim, said dosage form resistant or substantially resistant to dissolution and/or absorption in the duodenum.

7. The dosage form of any preceding claim, said dosage form resistant or substantially resistant to dissolution and/or absorption in the jejunum.

8. The dosage form of any preceding claim, said dosage form resistant or substantially resistant to dissolution and/or absorption in the ileum.

9. The dosage form of any preceding claim for use as a medicament.

## Patentansprüche

1. Arzneiform für orale Verabreichung von Levorphanol an einen menschlichen Patienten, wobei die Arzneiform umfasst:
Eine Menge von Levorphanol oder ein pharmazeutisch akzeptables Salz davon gleichwertig 4 bis 80 Milligramm von Levorphanol Tartrat, funktional kombiniert mit einem Material mit kontrollierter Freisetzung, das aus der Gruppe selektiert ist, die aus Wachsen, Pflanzenölen, Estern von Pflanzenölen und hydrierten Pflanzenölen besteht;
wenn das Material mit kontrollierter Freisetzung aus der Gruppe selektiert wird, die aus Wachsen, Pflanzenölen und Estern von Pflanzenölen besteht, ist das Material mit kontrollierter Freisetzung durch Anwendung von Wärme zumindest bis zu seinem Schmelzpunkt behandelt und einem Thixotrop und einem Modifikator für die Freisetzungsrate beigemischt worden; und, wenn das Material mit kontrollierter Freisetzung hydrierte Pflanzenöle ist, ist das Material mit kontrollierter Freisetzung durch Anwendung von Wärme zumindest bis zu seinem Schmelzpunkt behandelt und einem Thixotrop, einem Modifikator für die Freisetzungsrate und mittelkettigen Triglyceriden beigemischt worden
formuliert als eine oral verabreichbare Arzneiform ist die Arzneiform resistent oder im Wesentlichen resistent gegen Auflösung und/oder Absorption im Magen, und/oder im Duodenum, und/oder im Jejunum und/oder im Ileum.

2. Arzneiform nach Anspruch 1, derartig formuliert, dass im Wesentlichen keine Freisetzung von Levorphanol daraus weniger als ca. 2 Stunden, optional 4 Stunden nach oraler Verabreichung der Arzneiform an den Patienten vorkommt.

3. Arzneiform nach einem vorhergehenden Anspruch, derartig formuliert, dass, nach oraler Verabreichung der Arzneiform an den Patienten, im Wesentlichen keine Freisetzung von Levorphanol daraus nahe dem Magen oder in irgendeinem Abschnitt des Gastrointestinaltrakts fern des Magens vorkommt, der einen pH-Wert von weniger als 5 aufweist.

4. Arzneiform nach einem vorhergehenden Anspruch, die ferner zumindest eins umfasst von:
i) Einem Modifikator für Freisetzungsrate, der aus der Gruppe selektiert ist, die besteht aus:
Hydroxylpropylcellulosen, Celluloseacetaten, pulverisierten Cellulosen, Celluloseacetatphthalaten, Hydroxyethylmethylcellulosen, Carboxymethylcellulosen, Hypromelloseacetaten, Hypromellosephthalaten, Ethylcellulosen und Mischungen von diesen; und
ii) Ein Thixotrop, das aus der Gruppe selektiert ist, die besteht aus:
Metalloxiden, mikrokristallinen Methylcellulosen, Lehmen, Silikaten, Kohleschwarz und Kombinationen von diesen.

5. Arzneiform nach einem vorhergehenden Anspruch, wobei die Arzneiform gegen Auflösung und/oder Absorption im Magen resistent oder im Wesentlichen resistent ist.

6. Arzneiform nach einem vorhergehenden Anspruch, wobei die Arzneiform gegen Auflösung und/oder Absorption im Duodenum resistent oder im Wesentlichen resistent ist.

7. Arzneiform nach einem vorhergehenden Anspruch, wobei die Arzneiform gegen Auflösung und/oder Absorption im Jejunum resistent oder im Wesentlichen resistent ist.

8. Arzneiform nach einem vorhergehenden Anspruch, wobei die Arzneiform gegen Auflösung und/oder Absorption im Ileum resistent oder im Wesentlichen resistent ist.

9. Die Arzneiform nach einem vorhergehenden Anspruch zur Verwendung als ein Medikament.

## Revendications

1. Forme posologique pour l'administration orale de lévorphanol à un patient humain, la forme posologique comprenant
une quantité de lévorphanol ou d'un sel pharmaceutiquement acceptable de celui-ci équivalant à de 4 à 80 milligrammes de tartrate de lévorphanol, combinée fonctionnellement avec un matériau à libération contrôlée choisi dans le groupe constitué des cires, des huiles végétales, des esters d'huiles végétales et des huiles végétales hydrogénées
lorsque le matériau à libération contrôlée est choisi dans le groupe constitué des cires, des huiles végétales et des esters d'huiles végétales et d'huiles végétales hydrogénées, le matériau à libération contrôlée a été traité par application de chaleur jusqu'à au moins son point de fusion et mélangé avec un thixotrope et un modificateur de vitesse de libération ; et,
lorsque le matériau à libération contrôlée est une huile végétale hydrogénée, le matériau à libération contrôlée a été traité par application de chaleur jusqu'à au moins son point de fusion et mélangé avec un thixotrope, un modificateur et des triglycérides à chaîne moyenne
formulé sous une forme posologique pour administration orale, ladite forme posologique étant résistante ou essentiellement résistante à la dissolution et/ou à l'absorption dans l'estomac, et/ou dans le duodénum, et/ou dans le jéjunum, et/ou dans l'iléon.

2. Forme posologique selon la revendication 1, formulée de sorte que pratiquement aucune libération de lévorphanol à partir de celle-ci ne se produit moins d'environ 2 heures, facultativement 4 heures, après administration orale de la forme posologique au patient.

3. Forme posologique selon l'une quelconque des revendications précédentes, formulée de sorte que, après administration orale de la forme posologique au patient, pratiquement aucune libération de lévorphanol ne se produit à proximité de l'estomac ou dans une quelconque partie du tractus gastro-intestinal distale à l'estomac dont le pH est inférieur à 5.

4. Forme posologique selon l'une quelconque des revendications précédentes, comprenant en outre au moins l'un parmi
i) un modificateur de vitesse de libération choisi dans le groupe constitué par les hydroxylpropyl celluloses, les acétates de cellulose, les celluloses en poudre, les phtalates d'acétate de cellulose, les hydroxyéthylméthyl celluloses, les carboxyméthylcelluloses, les acétates d'hypromellose, les phtalates d'hypromellose, les éthylcelluloses et leurs mélanges ; et
ii) un thixotrope choisi dans le groupe constitué par les oxydes métalliques,
les méthylcelluloses microcristallines, les argiles, les silicates, le noir de carbone et leurs combinaisons.

5. Forme posologique selon l'une quelconque des revendications précédentes, ladite forme posologique étant résistante ou essentiellement résistante à la dissolution et/ou à l'absorption dans l'estomac.

6. Forme posologique selon l'une quelconque des revendications précédentes, ladite forme posologique étant résistante ou essentiellement résistante à la dissolution et/ou à l'absorption dans le duodénum.

7. Forme posologique selon l'une quelconque des revendications précédentes, ladite forme posologique étant résistante ou essentiellement résistante à la dissolution et/ou à l'absorption dans le jéjunum

8. Forme posologique selon l'une quelconque des revendications précédentes, ladite forme posologique étant résistante ou essentiellement résistante à la dissolution et/ou à l'absorption dans l'iléon.

9. Forme posologique selon l'une quelconque des revendications précédentes, à utiliser comme médicament.
